# EUROPEAN PATENT SPECIFICATION

(11) **EP 2 277 124 B1**
(45) Date of publication and mention of the grant of the patent: **09.11.2016**
(21) Application number: 08852916.9
(22) Date of filing: 21.11.2008
(51) Int. Cl.: G06F 17/50, G06F 19/16, A61K 31/33

(54) **MEANS FOR TREATING MYOSIN-RELATED DISEASES**
MITTEL ZUM BEHANDELN VON MIT MYOSIN ZUSAMMENHÄNGENDEN ERKRANKUNGEN
MOYENS POUR TRAITER DES MALADIES LIÉES À LA MYOSINE

(30) Priority: 21.11.2007 US 989773 P
(43) Date of publication of application: 26.01.2011
(73) Proprietor: Medizinische Hochschule Hannover, 30625 Hannover (DE)
(72) Inventor: MANSTEIN, Dietmar, 30559 Hannover (DE); FEDOROV, Roman, 30629 Hannover (DE); TSIAVALIARIS, Georgios, 30159 Hannover (DE); KNÖLKER, Hans-Joachim, 01187 Dresden (DE); MARTIN, René, 01159 Dresden (DE); KIRST, Juliane, 01099 Dresden (DE); GUTZEIT, Herwig, 01309 Dresden (DE); BÖHL, Markus, 01187 Dresden (DE)
(74) Representative: Vossius & Partner Patentanwälte Rechtsanwälte mbB
(86) International application number: PCT/EP2008/009891
(87) International publication number: WO 2009/065600

(56) References cited:
- ALLINGHAM JOHN S ET AL: "The structural basis of blebbistatin inhibition and specificity for myosin II" NATURE STRUCTURAL & MOLECULAR BIOLOGY, vol. 12, no. 4, April 2005 (2005-04), pages 378-379, XP002529525 ISSN: 1545-9985
- CHEUNG A ET AL: "A small-molecule inhibitor of skeletal muscle myosin II" NATURE CELL BIOLOGY, vol. 4, no. 1, January 2002 (2002-01), pages 83-88, XP002529526 ISSN: 1465-7392 cited in the application
- YANG ET AL: "Rigor-like Structures from Muscle Myosins Reveal Key Mechanical Elements in the Transduction Pathways of This Allosteric Motor" STRUCTURE, CURRENT BIOLOGY LTD., PHILADELPHIA, PA, US, vol. 15, no. 5, 10 May 2007 (2007-05-10), pages 553-564, XP022069543 ISSN: 0969-2126
- GEEVES M A ET AL: "Molecular mechanism of actomyosin-based motility" CMLS CELLULAR AND MOLECULAR LIFE SCIENCES, BIRKHÄUSER-VERLAG, BA, vol. 62, no. 13, 1 July 2005 (2005-07-01), pages 1462-1477, XP019200773 ISSN: 1420-9071
- FEDOROV R. ET AL: "Structural studies of myosin in complex with isoform-specific inhibitors" [Online] 13 February 2008 (2008-02-13), HASYLAB ANNUAL REPORT 2007 , XP002529612 Retrieved from the Internet: URL:http://hasyweb.desy.de/science/annual_ reports/2007_report/part2/contrib/72/20545 .pdf> [retrieved on 2009-05-27] page 211 - page 212
- FEDOROV ROMAN ET AL: "The mechanism of pentabromopseudilin inhibition of myosin motor activity" NATURE STRUCTURAL & MOLECULAR BIOLOGY, vol. 16, no. 1, January 2009 (2009-01), pages 80-88, XP002529527 ISSN: 1545-9985

## Description

This invention relates to a method of designing an inhibitor of a myosin, the method comprising in silico molecular modeling of a compound such that the modeled compound interacts by means of one or more of charge-charge interactions, charge-dipole interactions, dipole-dipole interactions, hydrogen bonds and hydrophobic interactions with at least three amino acid residues of said myosin, said residues being selected from (a) positions K265, A420, K423, A424, R428, L431, D590, I617, and A618 of SEQ ID NO: 2, wherein said residues comprise K265 and said myosin comprises or consists of (i) the sequence of SEQ ID NO: 2; (ii) the sequence encoded by the sequence of SEQ ID NO: 1; or (iii) a sequence being at least 40% identical to the sequence of SEQ ID NO: 2 or to the sequence encoded by the sequence of SEQ ID NO: 1; wherein said sequence of (iii) comprises said three amino acid residues; or (b) positions of any one of SEQ ID NOs: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 or 108, respectively, said positions aligning with the positions of SEQ ID NO: 2 as defined in (a), wherein the alignment of said positions occurs over the entire length of the respective ranges of (a) and (b), wherein said residues comprise the residue aligning with K265 of SEQ ID NO: 2 and said myosin comprises or consists of (i) the sequence of any one of SEQ ID NO: 4, 6, 8, 10, 12, 14. 16. 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 or 108, respectively; (ii) the sequence encoded by the sequence of any one of SEQ ID NO: 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 or 109, respectively; or (iii) a sequence being at least 40% identical to the sequence of any one of SEQ ID NO: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 or 108, respectively, or to the sequence encoded by the sequence of any one of SEQ ID NO: 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 or 109, respectively; wherein said sequence of (iii) comprises said three amino acid residues; thereby obtaining said inhibitor of a myosin.

Myosins are motor proteins which are involved in a large number of motility processes. By decomposing, more specifically hydrolysing ATP, myosins generate a directed force which permits movement along actin filaments. Known myosin-dependent processes include muscle contraction, cell division, movements of entire cells, intracellular transport of organelles and vesicles, endocytosis as well as maintenance and modification of actin-rich structures such as the cytoskeleton.

Cellular motility as well as aberrant forms thereof are involved in numerous diseases and conditions including cardiovascular diseases, cancer, malaria, and diseases of the central nervous system. Known inhibitors of myosin such as Blebbistatin (Kovacs M, Toth J, Hetenyi C, Malnasi-Csizmadia A, Sellers JR. J. Biol. Chem. 2004, 279: 35557-35563), BDM (2,3-butanedione monoxime) and BTS (N-benzyl-p-toluol-sulfonamide) (Cheung A, Dantzig JA, Hollingworth S, Baylor SM, Goldman YE, Mitchison TJ, Straight AF. Nat. Cell. Biol. 2002, 4: 83:8) lend themselves, in view of their lack of specifity, high toxicity, and associated side effects, not or only to a very restricted extent to therapeutic applications.

Laatsch et al. (Chem. Pharm. Bull. 43(4) 537-546 (1995)) and Fenical (Chem. Rev. 1993, 93, 1673-1683) describe pentabromopseudilin (2,3,4-tribromo-5-(3,5-dibromo-2-hydroxyphenyl)-1*H*-pyrrole), further pseudilins as well as their anti-tumor and anti-microbial properties.

Allingham et al. (Nature Structural & Molecular Biology 12, 378-379, 2005) describe a structural basis of blebbistatin inhibition and specificity for myosin II. Cheung et al. (Nature Cell Biology 4, 83-88, 2002) describe a small-molecule inhibitor of skeletal muscle myosin II.

In view of the limitations of the means and methods of the prior art, the technical problem underlying the present invention was therefore the provision of improved or alternative means and methods for the treatment of myosin-related diseases.

Accordingly, this invention relates to a method of designing an inhibitor of a myosin, the method comprising in silico molecular modeling of a compound such that the modeled compound interacts by means of one or more of charge-charge interactions, charge-dipole interactions, dipole-dipole interactions, hydrogen bonds and hydrophobic interactions with at least three amino acid residues of said myosin, said residues being selected from (a) positions K265, A420, K423, A424, R428, L431, D590, 1617, and A618 of SEQ ID NO: 2, wherein said residues comprise K265 and said myosin comprises or consists of (i) the sequence of SEQ ID NO: 2; (ii) the sequence encoded by the sequence of SEQ ID NO: 1; or (iii) a sequence being at least 40% identical to the sequence of SEQ ID NO: 2 or to the sequence encoded by the sequence of SEQ ID NO: 1; wherein said sequence of (iii) comprises said three amino acid residues; or (b) positions of any one of SEQ ID NOs: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 or 108, respectively, said positions aligning with the positions of SEQ ID NO: 2 as defined in (a), wherein the alignment of said positions occurs over the entire length of the respective ranges of (a) and (b), wherein said residues comprise the residue aligning with K265 of SEQ ID NO: 2 and said myosin comprises or consists of (i) the sequence of any one of SEQ ID NO: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 or 108, respectively; (ii) the sequence encoded by the sequence of any one of SEQ ID NO: 3, 5, 7, 9, 11, 13, 15, 17, 19, 21, 23, 25, 27, 29, 31, 33, 35, 37, 39, 41, 43, 46, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 or 109, respectively; or (iii) a sequence being at least 40% identical to the sequence of any one of SEQ ID NO: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 or 108, respectively, or to the sequence encoded by the sequence of any one of SEQ ID NO: 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 or 109, respectively; wherein said sequence of (iii) comprises said three amino acid residues; thereby obtaining said inhibitor of a myosin.

Described is also a method of designing a modulator of a myosin, the method comprising molecular modeling of a compound such that the modeled compound interacts with at least three amino acid residues of said myosin, said residues being selected from (a) ranges K265-V268, V411-L441, N588-Q593, D614-T629, and V630-E646 of SEQ ID NO: 2, said myosin comprising or consisting of (i) the sequence of SEQ ID NO: 2; (ii) the sequence encoded by the sequence of SEQ ID NO: 1; (iii) a sequence being at least 40% identical to the sequence of SEQ ID NO: 2 or to the sequence encoded by the sequence of SEQ ID NO: 1; or (iv) a sequence encoded by a sequence being at least 40% identical to the sequence of SEQ ID NO: 1; wherein said sequence of (iii) or (iv) comprises said three amino acid residues, and wherein said residues comprise K265; or (b) ranges of any one of SEQ ID NOs: 4, 6, 8, 10,.12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 or 108, respectively, said ranges aligning with the ranges of SEQ ID NO: 2 as defined in (a), said myosin comprising or consisting of (i) the sequence of any one of SEQ ID NO: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72; 74, 76, 78, 80, 82, 84, 86, 88, 90, 92, 94, 96, 98, 100, 102, 104, 106 or 108, respectively; (ii) the sequence encoded by the sequence of any one of SEQ ID NO: 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 or 109, respectively; (iii) a sequence being at least 40% identical to the sequence of any one of SEQ ID NO: 4, 6, 8, 10, 12, 14, 16, 18, 20, 22, 24, 26, 28, 30, 32, 34, 36, 38, 40, 42, 44, 46, 48, 50, 52; 54, 56, 58, 60, 62, 64, 66, 68, 70, 72, 74, 76, 78, 80, 82, 84, 86, 88, 90, 92, 94, 96, 98, 100, 102, 104, 106 or 108, respectively, or to the sequence encoded by the sequence of any one of SEQ ID NO: 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 or 109, respectively; or (iv) a sequence encoded by a sequence being at least 40% identical to the sequence of any one of SEQ ID NO: 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 or 109, respectively; wherein said sequence of (iii) or (iv) comprises said three amino acid residues, and wherein said residues comprise the residue aligning with K265 of SEQ ID NO: 2; thereby obtaining said modulator of a myosin.

The term "designing" refers to the creation of a molecule by *in silico* methods. In addition to *in silico* methods, further steps may be performed in the method of designing according to the invention, which may include synthesizing of a compound and/or optimization of said compound. Such optional further steps are detailed below.

As regard the *in silico* methods for creating molecules, these are commonly referred to as molecular modelling. Particularly envisaged for the present invention are molecular modeling tools which are also referred to as ligand construction tools. Such methods for rational drug design typically take into account properties including shape, charge distribution, the distribution of hydrophobic groups, ionic groups and groups capable of forming hydrogen bonds at a site of interests of the protein molecule under consideration. Using this information, that can be derived from the high resolution structure of proteins and protein-ligand complexes (see, e.g. Example 2), these methods either suggest improvements to existing molecules, construct new molecules on their own that are expected to have good binding affinity, screen through virtual compound libraries for such molecules or fragments thereof, or otherwise support interactive design of new drug compounds *in silico.* Typically, ligand construction makes use of dedicated software and involves interactive sessions in front of a computer display of the three-dimensional structure of the target molecule, i.e., myosin, and of candidate molecules or fragments thereof. Suitable software packages are known in the art and include Chemoffice (CambridgeSoft Corporation), CNS *(*Acta Cryst. D54, 905-921), CCP4 (Acta Cryst. D50, 760-763), ADF (Computational Chemistry, David Young, Wiley-Interscience, 2001. Appendix A. A.2.1 p. 332) and Gold (G. Jones, P. Willett and R. C. Glen, J. Mol. Biol., 245, 43-53, 1995; G. Jones, P. Willett, R. C. Glen, A. R. Leach and R. Taylor, J. Mol. Biol., 267, 727-748, 1997; M. L. Verdonk, J. C. Cole, M. J. Hartshorn, C. W. Murray and R. D. Taylor, Proteins, 52, 609-623, 2003). As a result of modifications of candidate or starting molecules, the modeled compound is obtained.

The coordinates of the target molecule, i.e. myosin, may be experimentally determined e.g. by NMR spectroscopy and/or X-ray crystallography, or may be obtained by molecular modeling, preferably homology modeling using the high resolution structure of a myosin, said high resolution structure being determined by experimental means, to estimate and calculate the structure of a different myosin for which an experimentally determined high resolution structure is not yet available. Suitable software is known in the art and includes the software packages described in Example 2 enclosed herewith. Structures of myosin-2 from *Dictyostelium* with and without exemplary or preferred compounds are provided in Examples 2 and 7.

The term "interact" or "interaction" as used herein refers to a relation between at least two molecular entities. This relation may *inter alia* be described in terms of intermolecular distances and/or free energies of interaction. In the first case, an interaction may be defined by at least one intermolecular distance, preferably by more than one such as two, three, four, five or more intermolecular distances. If an interaction according to the invention is to be described in terms of intermolecular distances only, it is envisaged to use at least three such distances. Typically, intermolecular distances are determined as distances between the centers of atoms of the respective interacting molecular entities. In this case, intermolecular distances according to the invention are referred to as interatomic distances. Preferably, a such determined interatomic distance is less than 4 Angstroms, more preferably in the range from 3.6 and 2.8 Angstroms. Preferred values include 3.4, 3.2 and 3.0 Angstroms. Alternatively or in addition, an interaction may be defined in terms of free energy. The free energy may be a total free energy determining the strength of an intermolecular interaction in its entirety or a partial free energy, said partial free energy resulting from, for example, one atom-atom interaction within a plurality of atom-atom interactions within the intermolecular interaction under consideration. Preferably, the total free energy of an interaction according to the invention is at least 60 kJ/mol. This value corresponds to the binding energy of about three hydrogen bonds. More preferred are total free energies of an interaction of at least 100, at least 150 or at least 200 kJ/mol.

As an alternative or additional parameter, and in case of inhibitors, the IC₅₀ concentration may be used to characterize the strength of an intermolecular interaction. The IC₅₀ concentration is the concentration of an inhibitor that is required to inhibit 50% of the target, in the present case myosin. Preferably, the modelled compound, in case it is an inhibitor, interacts with said at least three residues such that the IC₅₀ concentration is in the two-digit micomolar range, i.e. below 100 µM. More preferred are IC₅₀ concentrations below 50 µM, below 10µM or below 1µM. Yet more preferred are nanomolar or even picomolar inhibitors, e.g. inhibitors with an IC₅₀ concentration below 100 nM, below 10 nM, below 1 nM or below 100 pM. More generally speaking, and applicable to any binding molecule, the concentration that is required to achieve binding to 50% of the target or modulating of 50% of the target may be used. Preferred values of IC₅₀ concentrations as recited above apply also to these concentrations.

An intermolecular interaction comprises one or more types of interactions, interactions being selected from charge-charge, charge-dipole, and dipole-dipole interactions and furthermore hydrogen bonds and hydrophobic interactions. Dipoles may be permanent, induced or fluctuating. Interactions involving permanent dipoles and hydrogen bonds may be of particular relevance, since they are capable of specifically positioning and orienting a ligand or modulator in a binding pocket.

The positions according to part (a) of the main embodiment provide a generic description of the binding pocket according to the present invention. Since at least three residues within these ranges interact with the compound to be modelled, the present invention also provides an implicit definition of pharmacophores capable of binding to said binding pocket. The term "pharmacophore" is known in the art and refers to the molecular framework responsible for the biological or pharmacological activity of a compound (Osman F. Güner (2000) Pharmacophore Perception, Development, and use in Drug Disgn ISBN 0-9636817-6-1; Thierry Langer and Rémy D. Hoffmann (2006) Pharmacophores and Pharmacophore Searches ISBN 3-527-31250-1). Preferred activities of the compound include the modifying of myosin activities as described herein below. Typical pharmacophore features include hydrogen bond donors, hydrogen bond acceptors, dipoles, charges, ions and hydrophobic moieties. The pharmacophore furthermore includes information on the spatial arrangement of one or more of such moieties.

The term "modulator" as used herein refers to any molecule capable of modifying the activity of the target, i.e. myosin. "Modifying" includes increasing and decreasing said activity. The activity includes any molecular, biochemical, biomechanical or biological activity. A preferred activity is the capability of myosin to produce force and/or movement in an actin- and/or Mg-ATP dependent way; see below. A further activity is ATPase activity, i.e., the capability to hydrolyze adenosine trisphosphate (ATP), preferably to yield ADP and Pᵢ. In structural terms, the modulator is not limited. Preferably, the modulator is a small organic molecule, the term "small" preferably referring to molecules with a molecular weight below 1200, 1000, 800, 600, 500, 400 or 300 Da.

The term "myosin" refers to a well-known class of proteins with ATPase activity and having the capability of generating force and/or directional movement; see also the background section herein above. Table 1 below provides further information on myosins to be used in the present invention.

### 1: Summary of Myosins

| **Class** | **Subclass** | **Myosin (name)** | **NCBI Accession number** | **Function** | **Literature** |
|---|---|---|---|---|---|
| 1 | BBM | *Hs* Myosin-1A (MYO1A) | NM_005379;AF127026;AF009961 | Cell motility | Bement et al., 1994; Hofmann et al., |
| | Iα | *Hs* Myosin-1B (MYO1B) | NM_012223;XM_290989 | Vesicular transport | 2006 |
| | Iβ | *Hs* Myosin-1C (MYO1C) | NM_001080779 | Endocytosis | Ruppert et al., 1993; Salas-Cortes et al., |
| | Iγ | Hs Myosin-1D (MYOID) | NM_015194;XM_050041 | Exocytosis | 2005 |
| | IC | *Hs* Myosin-1E (MYO1E) | NM_004998 | Signal transduction | Bement et al., 1994; - |
| | - | *Hs* Myosin-1F (MYO1F) | NM_012335 | transcription | Hasson et al., 1996; - |
| | - | *Hs* Myosin-1G (MYO1G) | NM_033054;XM_291223;XM_37 | Trafficking | Bement et al., 1994; Krendel et al., 2007 |
| | - | *Hs* Myosin-1H (MYO1H) | 4431 | Neurosensory functions | Crozet et al., 1997; - |
| | | | NM_001101421;XM_001125815 | Cell morphology | Scherer et al., 2003; - |
| | | | | Left-right asymmetry | Venter et al., 2001; - |
| | | | | | |
| | | | | | |
| 2 | Skeletal muscle | *Hs* heavy chain 1 (MYH1), skeletal muscle | NM_005963 | Muscle contraction | Leinwand et al., 1983 |
| | | (adult) | NM_017534 | | |
| | | *Hs* heavy chain 2 (MYH2), skeletal muscle | NM_001100112 | | |
| | | (adult, var1) | NM_002470 | | |
| | | *Hs* heavy chain 2 (MYH2), skeletal muscle | NM_017533 | | Eller et al., 1989 |
| | | (adult, var2) | NM_003802 | | Winters et al., 1998; Schachat and |
| | | *Hs* heavy chain 3 (MYH3), skeletal muscle | | | Briggs, 2002 |
| | Cardiac muscle | (embryonic) | | Muscle contraction | |
| | | *Hs* heavy chain 4 (MYH4), skeletal muscle | NM_002471 | | |
| | | *Hs* heavy chain 13 (MYH13), skeletal muscle | NM_000257 | | Solomon et al., 1990 |
| | | | XM_371398 | | Solomon et al., 1990; Pegoraro et al., |
| | | *Hs* heavy chain 8 (MYH8), skeletal perinatal | | | 2007 |
| | non-muscle | *Hs* heavy chain 6 (MYH6), cardiac muscle | NM_002473 | Cytokinesis, cortical | Desjardins et al., 2002 |
| | | alpha | XM_290747 | bundling, morphogenesis, | |
| | | *Hs* heavy chain 7 (MYH7), cardiac muscle beta | | cell motility/migration, | Simons et al., 1991 |
| | | *Hs* heavy chain 7 (MYH7B), cardiac muscle | | carcinogenesis | Andzelm et al., 2007 |
| | | beta | | | |
| | smooth muscle | | NM_002474;NM_022870 | Muscle contraction | |
| | | *Hs* heavy chain 9 (MYH9), non-muscle | NM_001040114 | | |
| | | *Hs* heavy chain 10 (MYH 10), non-muscle | NM_022844 | | Matsuoka et al., 1993; Zhang et al., 2006 |
| | | | NM_001040113 | | |
| | Extra ocular | | | | |
| | | *Hs* heavy chain 11 (MYH11), smooth muscle | | - | |
| | Not defined | varSM1A | NM_001077186 | | |
| | Not defined | *Hs* heavy chain 11 (MYH11), smooth muscle | NM_024729 | - | |
| | Not defined | varSM1B | NM_014981;XM_036988;XM_93 | - | Desjardins et al., 2002 |
| | | *Hs* heavy chain 11 (MYTH11), smooth muscle | 8225 | - | Desjardins et al., 2002; Donaudy et al., |
| | | varSM2A | | | 2004 |
| | | *Hs* heavy chain 11 (MYH 11), smooth muscle | | | Desjardins et al., 2002 |
| | | varSM2B | | | |
| | | *Hs* heavy chain 13 (MYH13) | | | |
| | | *Hs* heavy chain 14 (MYH 14), var1 | | | |
| | | *Hs* heavy chain 14 (MYH14), var2 | | | |
| | | *Hs* heavy chain 15 (MYH15) | | | |
| | | | | | |
| 3 | Myosin-3a | *Hs* myosin-3A (Myo3A) | NM_017433 | Phototransduction and | Dose et al., 2000; Walsh et al., 2002 |
| | Myosin-3b | *Hs* myosin-3B (Myo3B), var1 | NM_001083615 | hearing processes | Dose and Burnside, 2002 |
| | | | | | |
| 5 | Myosin-5a | *Hs* Myosin-5a (MYO5A) | NMR_000259 | Intracellular trafficking | Engle and Kennett, 1994; Desnos et al., |
| | Myosin-5b | *Hs* Myosin-5b (MYO5B) | NM_001080467;XM_371116 | and transport processes | 2007 |
| | Myosin-5c | *Hs* Myosin-5c (MYO5C) | NM_018728 | (mitochondrial transport, melanosomal transport, mRNA, organelles, cargo) in neuronal cells and other cell types | Bement et al., 1994; Lapierre et al., 2001 Bement et al., 1994; Rodriguez and Cheney, 2002 |
| | | | | | |
| 6 | Myosin-6 | *Hs* myosin-6 (MY06) | NM_004999;XM376516 | Reverese directional transport processes, clathrin mediated endocytosis, neurosensory functions | Bement et al., 1994; Dunn et al., 2006 |
| 7 | Myosin-7a | *Hs* myosin-7a (MY07A) | NM_000260 | Cell adhesion, filopodia | Bement et al., 1994; Jaijo et al., 2007 |
| | | | | extensions, stereocilia | |
| | | | | organization, | |
| | | | | neurosensory functions | |
| 9 | Myosin-9a | *Hs* myosin-9a (MY09A) | NM_006901 | Signaltransduction | Bement et al., 1994 |
| | Myosin-9b | *Hs* myosin-9b (MYO9B) | NM_004145 | Lamellipodia, fillopodia extensions | Bement et al., 1994 ; Sanchez et al., 2007 |
| 10 | Myosin-10 | *Hs* myosin 10 (MYO10) | NM_012334 | Filopodia | Hasson et al., 1996; Bohil et al., 2006 |
| | | | | Function in cell motility | |
| | | | | Neuronal growth cone | |
| 11 | Myosin-11 | Plant myosins (tobacco, Arabidopsis | | Cytoplasmic streaming | Shimmen, 2007; Lee and Liu, 2004; |
| | | thaliana,...) | | Transport | Volkmann et al., 2003 |
| 14 | *Toxoplasma* | *Tg* myosin-A (*Tg*MyoA) | AF006626 | Gliding motility, host cell | Heintzelman and Schwartzmm, 1997 |
| | *gondii* | *Tg* myosin-B (*Tg*MyoB) | AF438184 | invasion processes | Delbac et al., 2001 |
| | | Tg myosin-E (*Tg*MyoE) | AF221131 | | Delbac and Soldati, unpublished |
| | *Plasmodium falciparum* | *Pf* myosin-A | AF105117 | | Foth et al., 2006 |
| 15 | Myosin-15a | *Hs* unconventional myosin-15a(Myo 15A) | AF144094 | Neurosensory functions | Liang et al., 1999 |
| | Myosin-15b | *Hs* unconventional myosin-15b (Myo15B) | NR_003587 | | Berg et al., 2001; Boger et al., 2001 |
| 16 | Myosin-16 | *Hs* myosin-16 (Myo16) | BC146791 | Neuronal functions | Strausberg et al., 2002 |
| 18 | Myosin-18a | *Hs* myosin-18a (MYO18A), var1 | NM_078471 | "Carcinogenesis" | Furusawa et al., 2000; Mori et al., 2003 |
| | Myosin-18b | *Hs* myosin-18a (MYO18A), var2 | NM 203318 | | Mori et al., 2003; Yang et al., 2005 |
| | | *Hs* myosin-18b (MYO18B) | NM_032608 | | Dunham et al., 1999; Edakuni et al., 2006 |
| 19 | Myosin-19 | *Hs* myosin-19 (Myo19) | BC008900 | ? | Strausberg et al., 2002 |
| 22 | *Toxoplasma gondii* | Tg myosin-F (*Tg*MyoF) | DQ131541 | Invasion processes,... | Foth et al., 2006 |

Sequence alignments of myosins motor domains are shown in the Figures enclosed herewith. These sequence alignments have been created with methods well known and established in the art. The multiple sequence alignments have been generated with the software ClustalW (see below) by using the following parameters: Gap Penalty: 15.00; Gap Length Penalty: 0.5; Delay Divergent Seqs (%): 15; Protein Weight Matrix: Gonnet Series.

The invention is applicable to all myosin isoforms, in particular to the myosins having sequences of the sequence listing. Several sequences of the sequence listing correspond to sequences with the below accession numbers for the NCBI databases (www.pubmed.com) in the versions of November 11, 2008. Preferred myosin isoforms are human, Apicomplexa and plant isoforms.

### HUMAN MYOSINS

1. *H.s*. myosin IA (MYO1A), NM_005379
2. *H.s*. myosin IB (MYO1B), NM_012223
3. *H.s*. myosin IC (MYO1C), NM_001080779
4. H.s. myosin ID (MYO1D), NM_015194
5. H.s. myosin IE (MYO1E), NM_004998
6. H.s. myosin IF (MYO1F), NMR_012335
7. *H.s*. myosin IG (MYO1G), NM_033054
8. *H.s*. myosin IH (MYO1H). NM_001101421
9. H.s. myosin heavy chain 1, skeletal muscle, adult (MYH1), NM_005963
10. H.s. myosin heavy chain 2, skeletal muscle, adult (MYH2), NM_017534
11. *H.s*. myosin heavy chain 3, skeletal muscle, embryonic, (MYH3), NM_002470
12. *H.s*. myosin heavy chain 4, skeletal muscle (MYH4), NMR_017533
13. *H.s*. myosin heavy chain 6, cardiac muscle alpha (MYH6), NM_002471
14. *H.s.* myosin heavy chain 7, cardiac muscle, beta (MYH7), NM_000257
15. H.s. myosin heavy chain 7B, cardiac muscle, beta (MYH7B), NM_020884
16. *H.s.* myosin heavy chain 8, skeletal muscle perinatal (MYH8), NM_002472
17. H.s. myosin heavy chain 9, non-muscle (MYH9), NM_002473
18. *H.s.* myosin heavy chain 10, non-muscle (MYH10), NM_005964
19. H.s. myosin heavy chain 11, smooth muscle A (MYH11), NM_002474; NM_022844
20. *H.s.* myosin heavy chain 11, smooth muscle B (MYH11), NMR_001040114; NM_001040113
21. *H.s*. myosin heavy chain 13, skeletal muscle (MYH13), NM_003802
22. H.s. myosin heavy chain 14 (MYH14), AY165122
23. H.s. myosin heavy chain 14 (MYH14), NM_001077186, NM_024729
24. H.s. myosin heavy chain 15 (MYH15), NM_014981
25. *H.s*. myosin IIIA (MYO3A), NM_017433
26. *H.s*. myosin IIIB (MYO3B), NM_001083615
27. H.s. myosin VA (MYO5A), NM_000259
28. *H.s*. myosin VB (MYO5B), NM_001080467
29. *H.s*. myosin VC (MYO5C), NP_061198
30. *H.s*. myosin VI (MYO6), NM_004999
31. *H.s*. myosin VIIA (MY07A). NM_000260
32. H.s. myosin IXA (MYO9A), NM_006901
33. H.s. myosin IXB (MYO9B), NM_004145
34. *H.s.* myosin X (MYO10), NM_012334
35. H.s. myosin XVA (MYO15A), AF144094
36. H.s. myosin XVI (MYO16, Myr8), NP_055826
37. H.s. myosin XVIIIA (MYO18A), NM_078471; NM_203318
38. *H.s.* myosin XVIIIB (MYO18B), NM_032608
39. *H.s.* myosin XIX (MYO19), BC008900

### PLASMODIUM AND TOXOPLAMSA MYOSINS

1. *P.f.* MyoA, AF105117
2. *P.f.* MyoF, Q286V9
3. T.g. MyoA, AF006626
4. *T.g.* MyoB, AF438184
5. T.g. MyoC, AF438183
6. T.g. MyoD, AF105118
7. T.g. MyoF, DQ131541

The positions as defined in part (b) of the main embodiment are readily determined based on (i) the information regarding the positions in the sequence of SEQ ID NO: 2 (myosin-2) as defined in part (a) of the main embodiment and (ii) said sequence alignments.

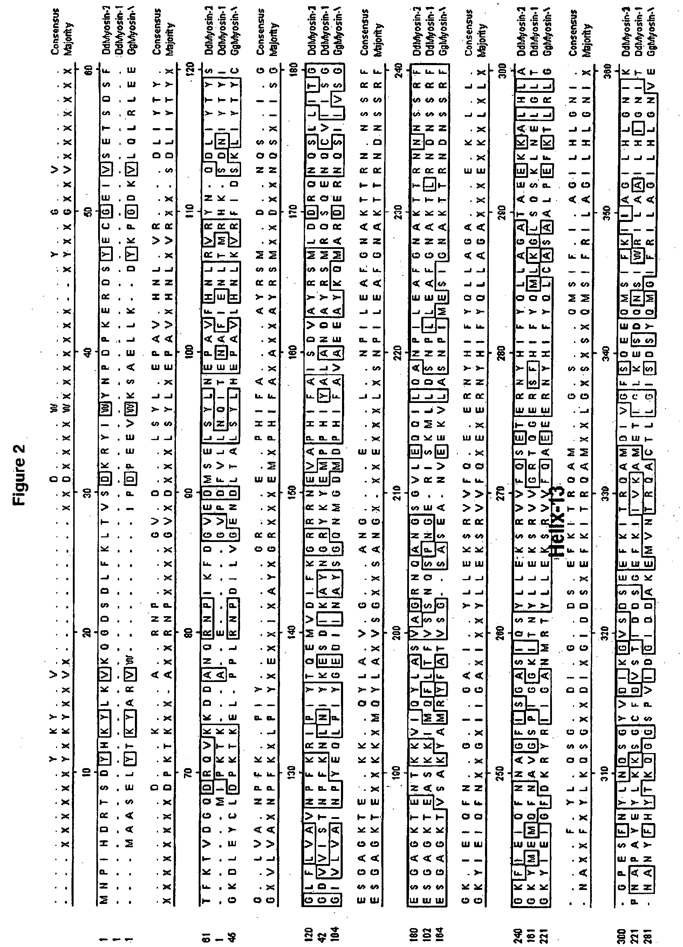

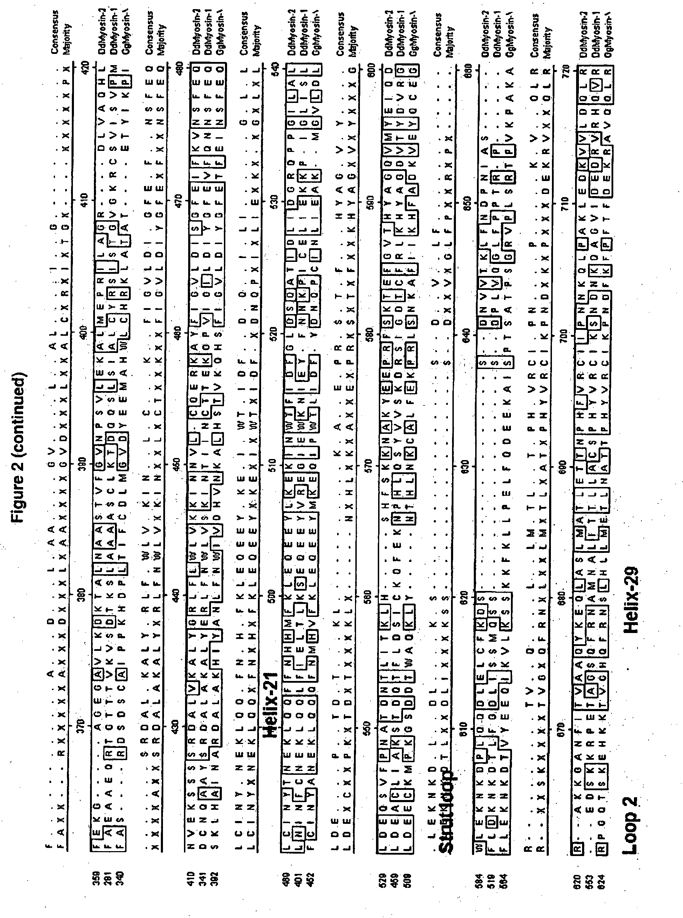

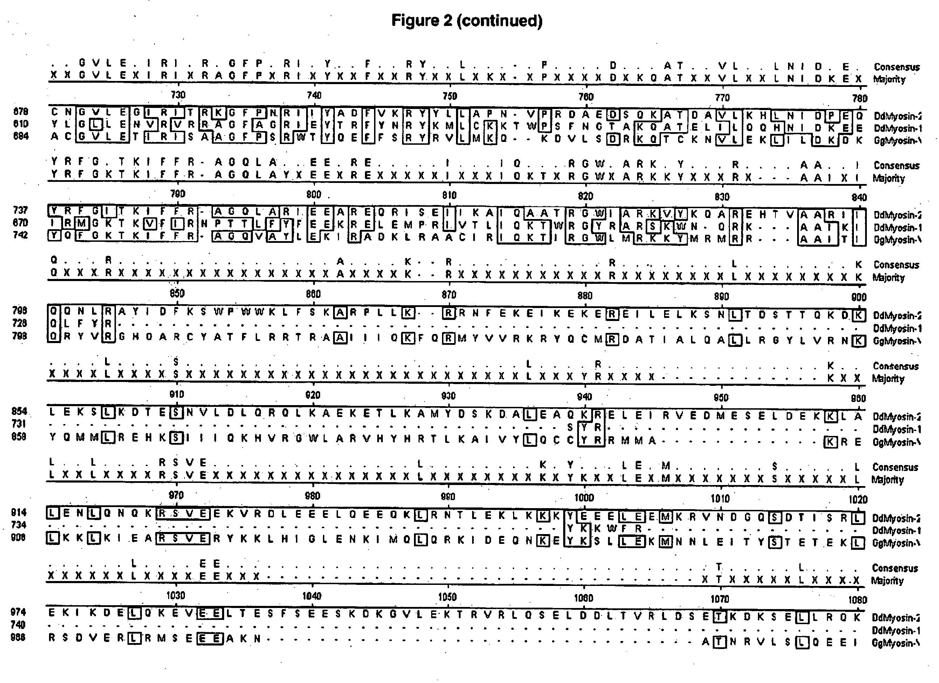

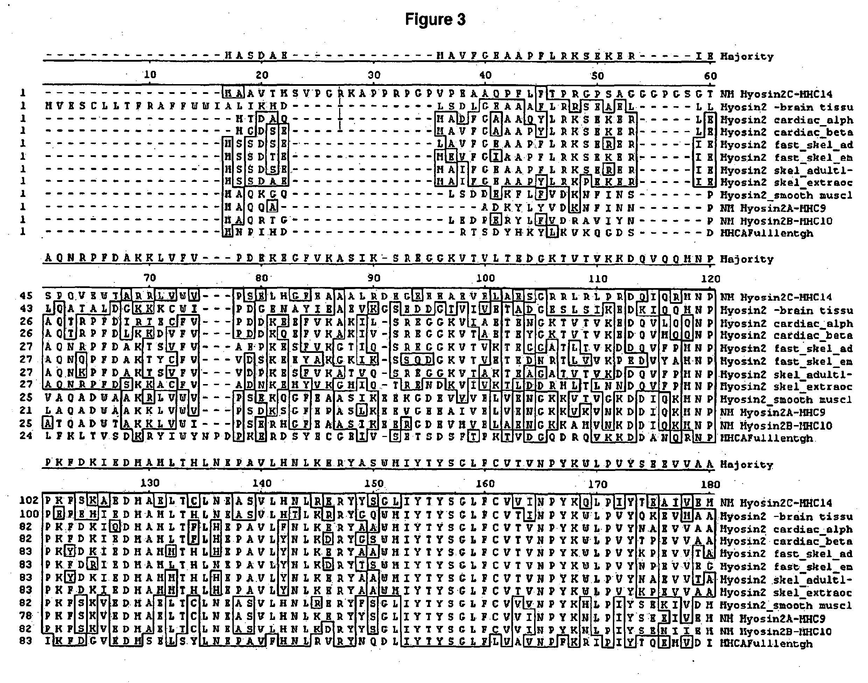

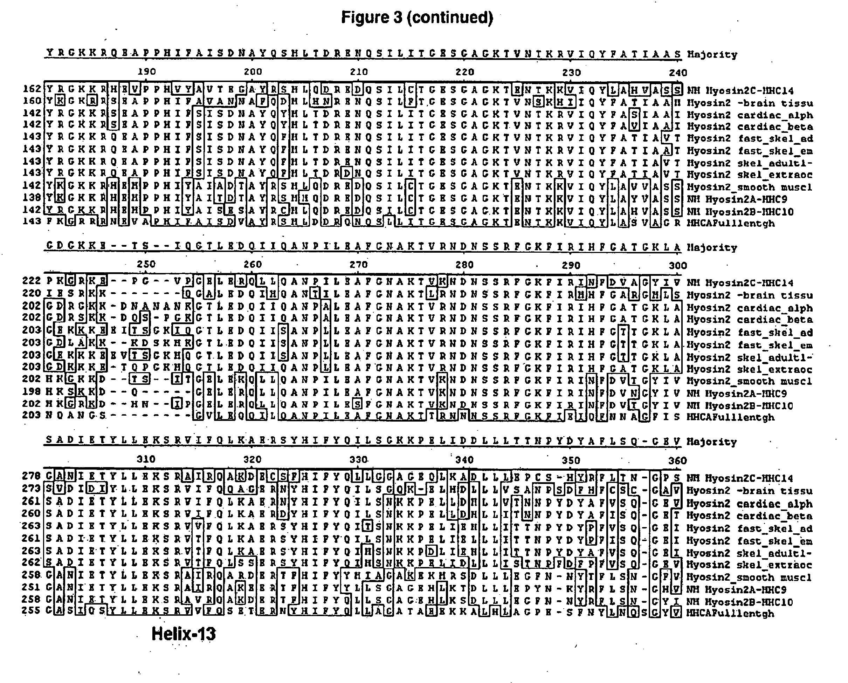

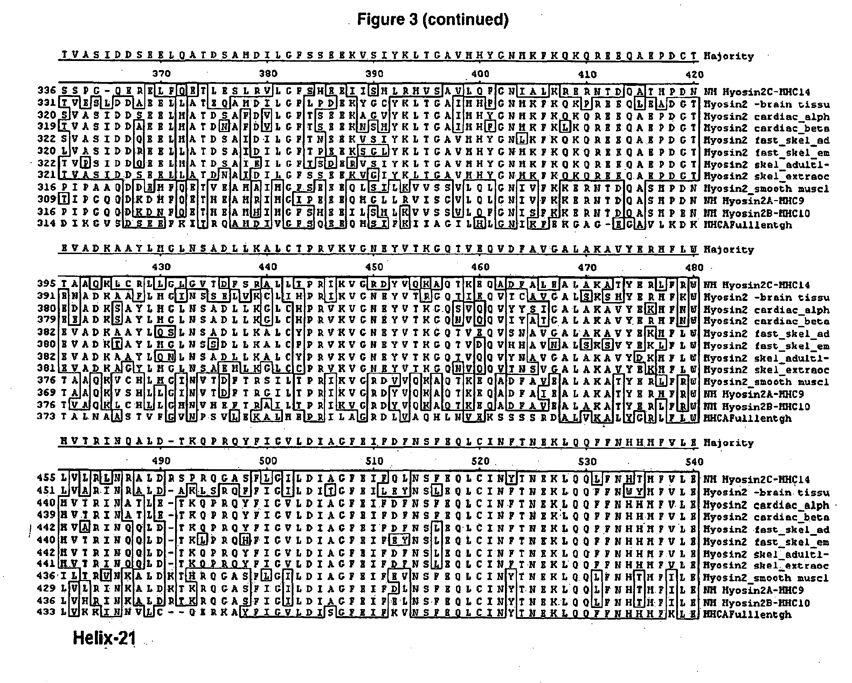

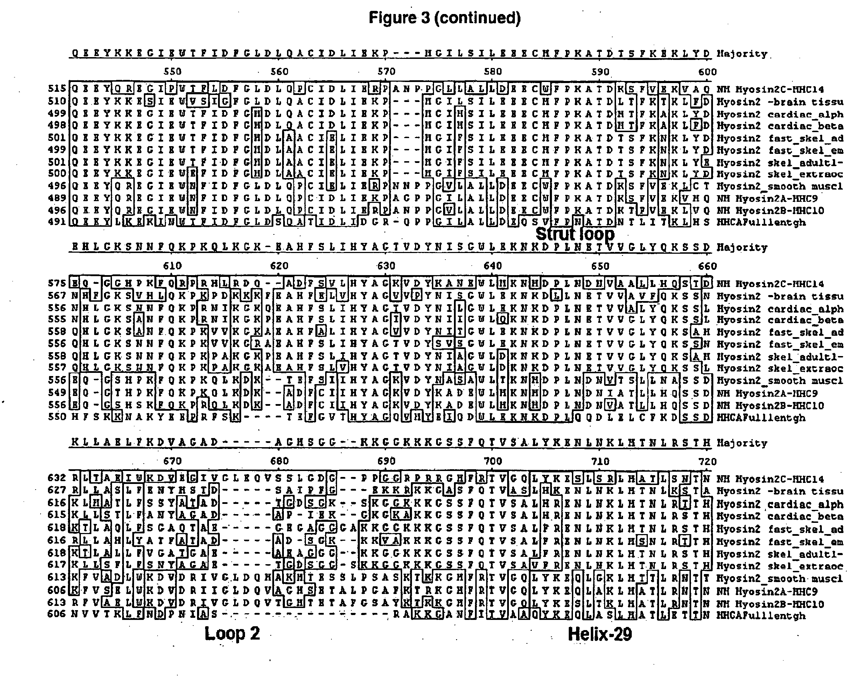

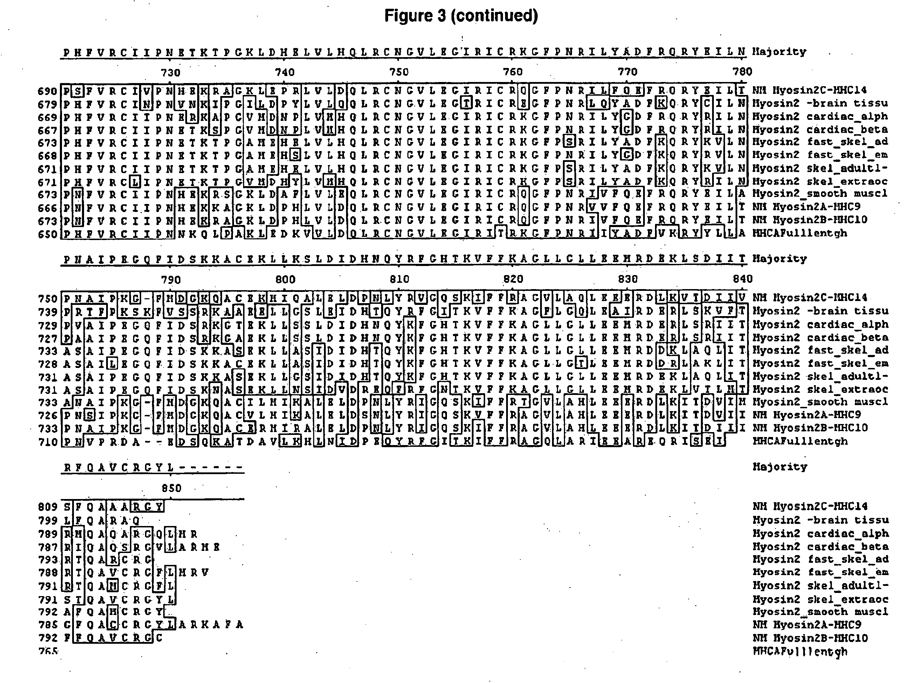

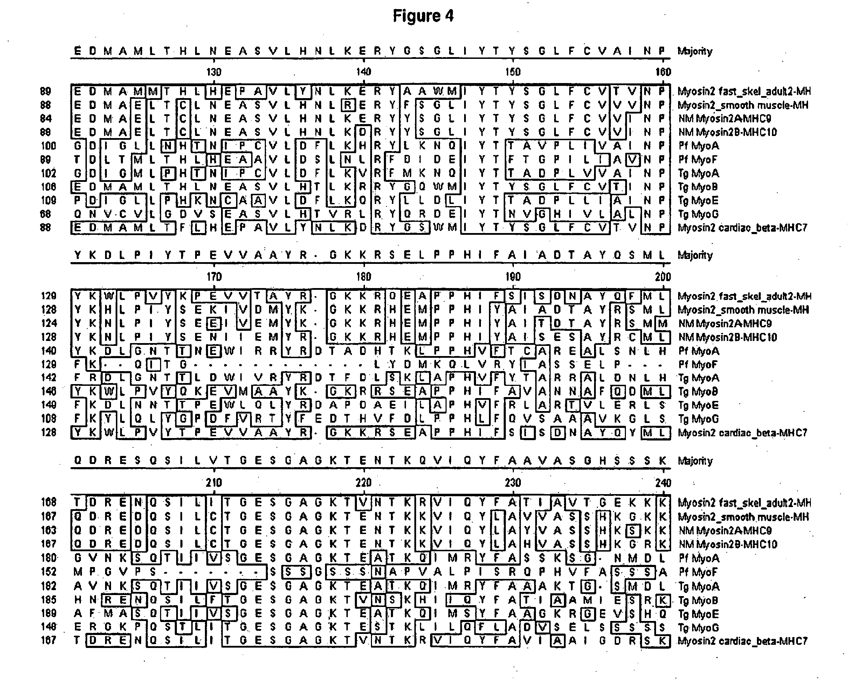

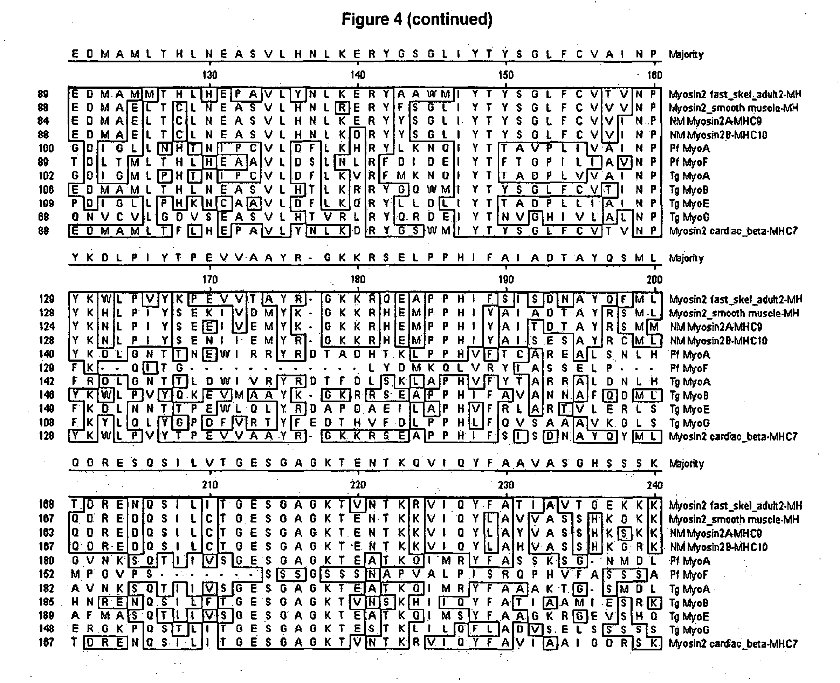

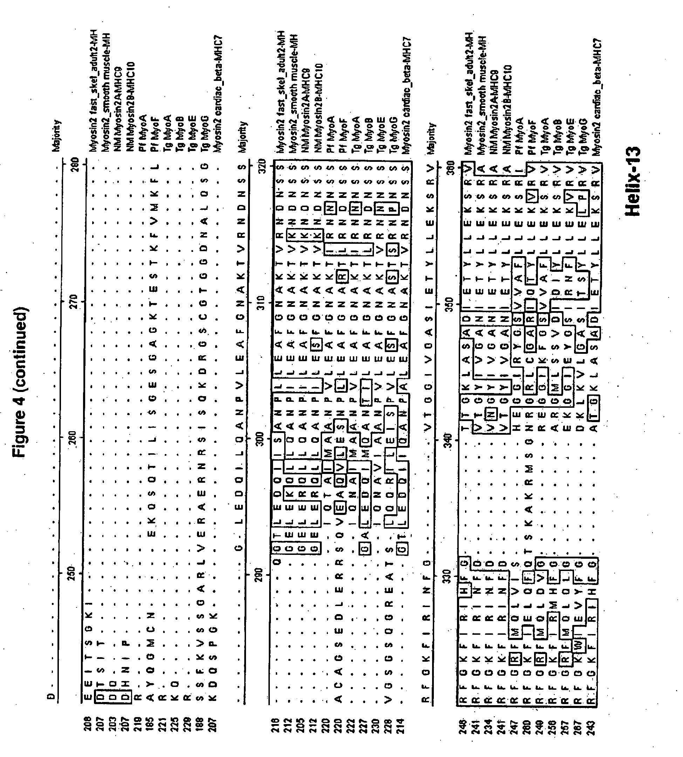

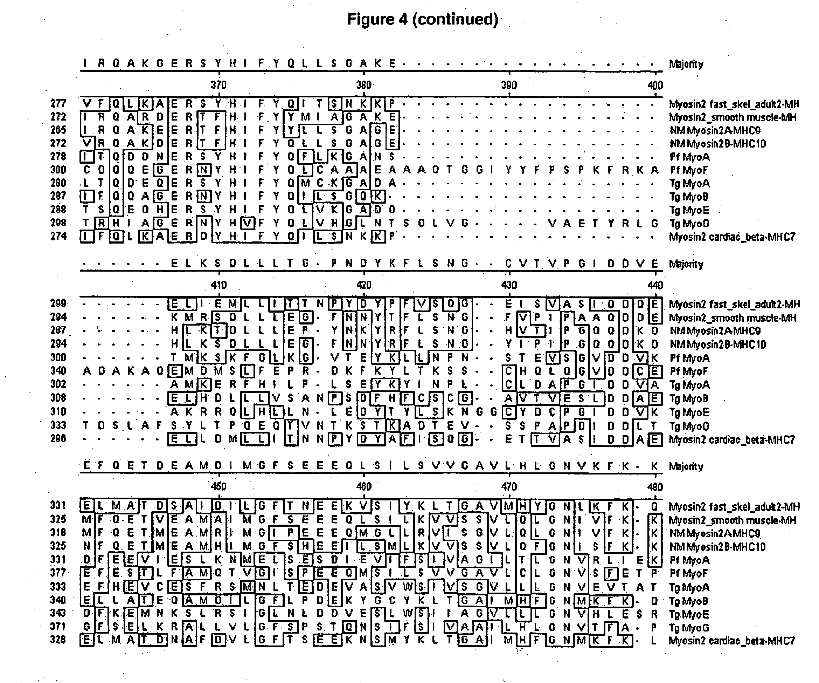

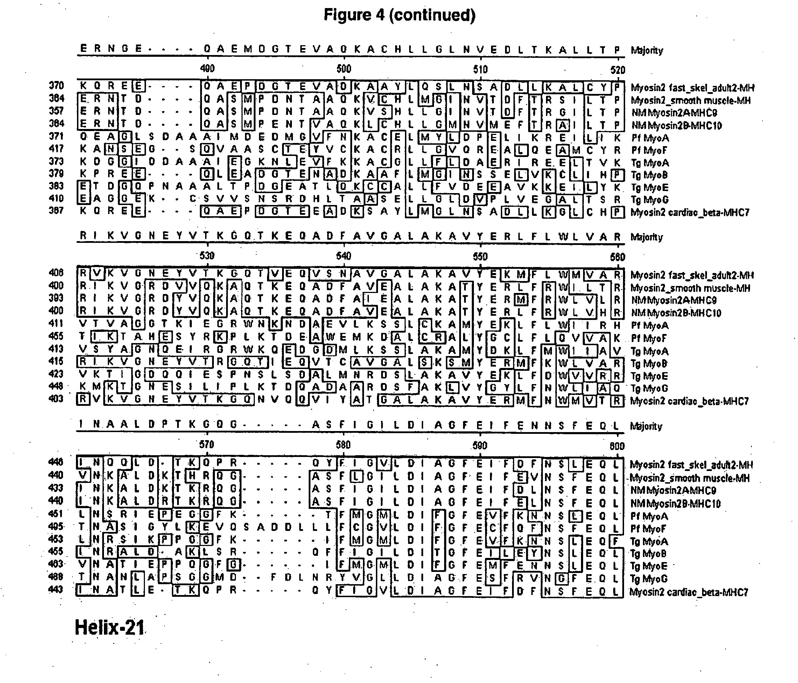

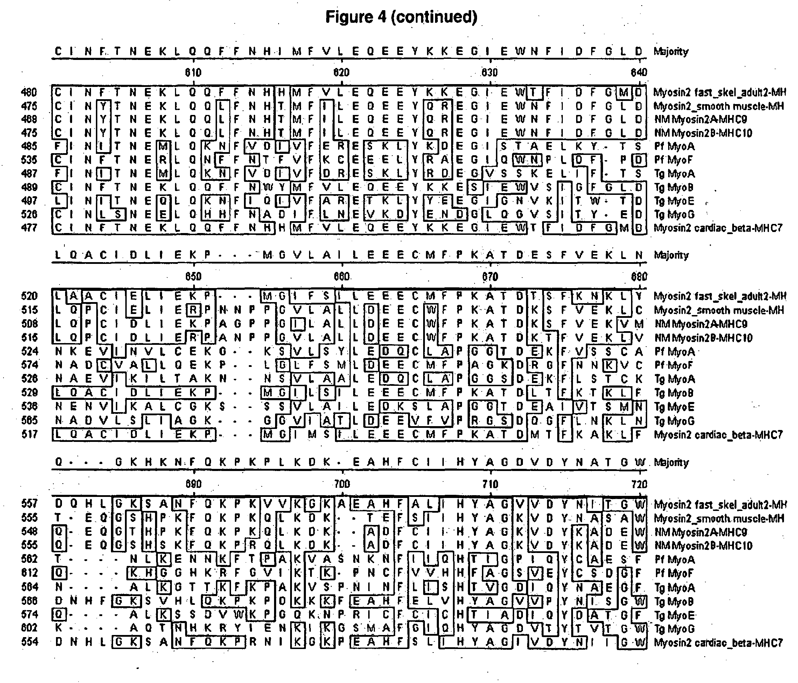

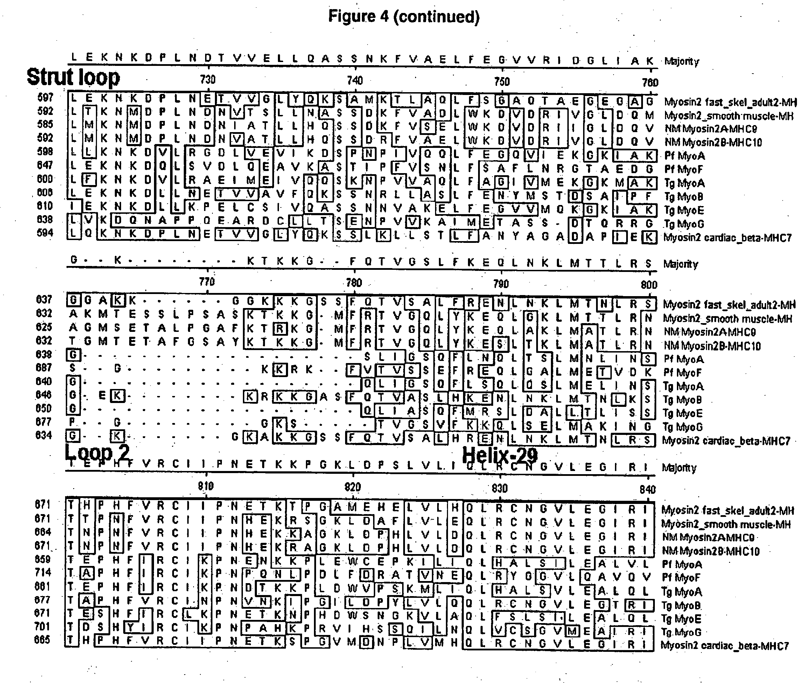

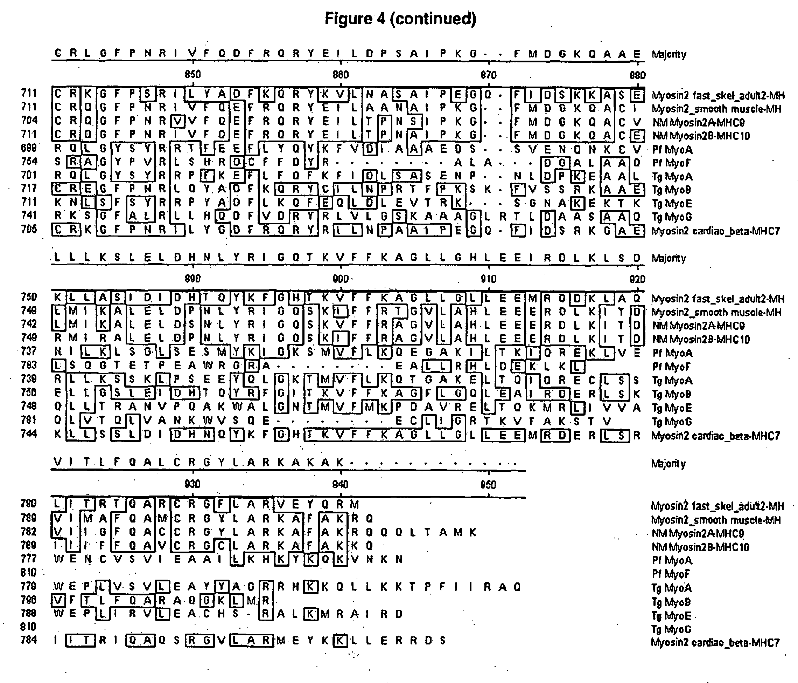

Similarly, the ranges as defined in part (b) of the described method are readily determined based on (i) the information regarding the ranges in the sequence of SEQ ID NO: 2 (myosin-2) as defined in part (a) of the described method and (ii) said sequence alignments. Preferably, the alignment of said ranges occurs over the entire length of the respective ranges as recited in parts (a) and (b); see also the enclosed alignments (in particular Figures 2 to 4).

For example, the corresponding, i.e., aligning ranges in the sequence of SEQ ID NO: 4 (myosin-1) are as follows: K186-V190, C342-N362, N523-F528, P549-T561, and A562-L577, and the corresponding ranges in the sequence of SEQ ID NO: 6 (myosin-5) are as follows: K246-V250, K392-N413, N568-Y573, L613-T635, and V636-L651.

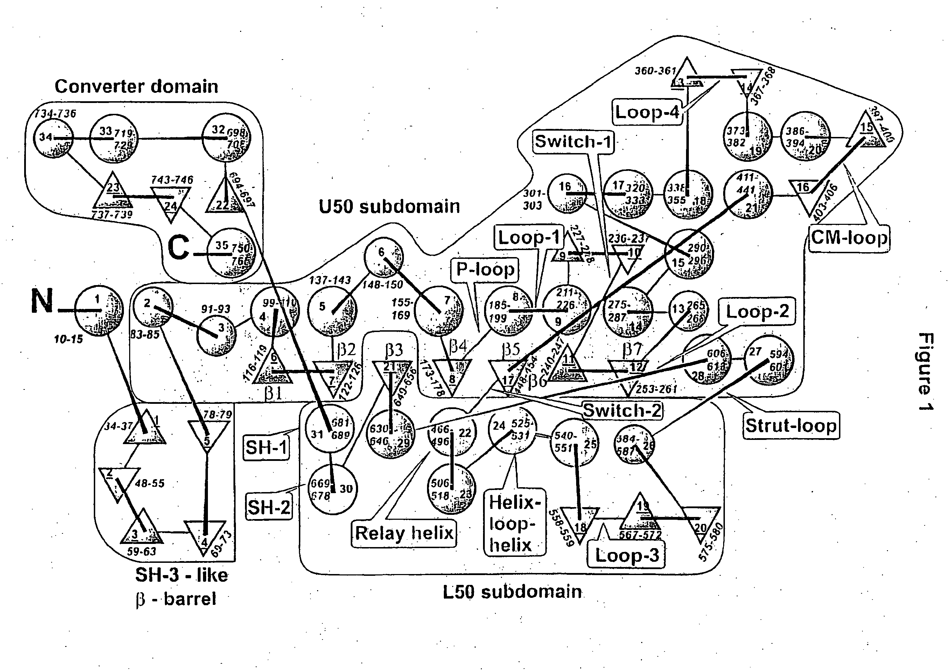

These ranges as well as the ranges recited in part (a) of the described method define structural elements of the three-dimensional structure of the respective myosin or secondary structure elements thereof. In particular, the five ranges in their respective order correspond to the following structural elements, the designations of which are established in the art: helix 13, helix 21, strut loop, loop2, and helix-29; see also the enclosed multiple sequence alignments (in particular Figures 2 to 4) as well as the schematic drawing of the topology of myosin-2 (Figure 1). As shown in the enclosed Figures, these structural elements form a previously unknown binding pocket of myosins. This binding pocket was found by the present inventors to be an allosteric binding pocket. The term "allosteric" is known in the art and refers to an alteration of conformation in response to ligand binding. Ligand binding occurs at a site which is not the active site or one of the active sites of the target (here myosin), but exerts a modulating effect on the active site(s). This modulation involves structural changes which in turn frequently entail functional changes. In other words, binding of ligands to this pocket induces or locks, respectively, a conformation of myosin. The induced or locked conformation may be an active or inactive conformation. The term "activity" is defined herein above. Preferably, said induced or locked conformation is an inactive conformation, e.g. a conformation of myosin with reduced or absent ATPase activity and/or reduced or absent actin binding capability and/or force generating activity. Alternatively, said induced or locked conformation may be an activated conformation. It is preferred that the activated conformation has a higher force generating activity.

The amino acid residues K265 as recited in part (a) is an anchor residue for the interaction of a modulator with the binding pocket defined by the ranges recited in part (a). A corresponding anchor residue in the sequence of SEQ ID NO: 4 (myosin-1) is K186. A corresponding anchor residue in the sequence of SEQ ID NO: 6 (myosin-5) is K246. Corresponding anchor residues of further myosins can be determined from the enclosed multiple sequence alignments without further ado. As used herein, the term "anchor residue" refers to residues of particular relevance for the interaction between a myosin and a modulator thereof. The residue of any myosin which aligns with K265 of SEQ ID NO: 2 (myosin-2) is almost invariably a Lys residue. It may also be an Arg residue.

Sequence identity levels as recited in the main embodiment may be determined by methods well known in the art. Two nucleotide or protein sequences can be aligned electronically using suitable computer programs known in the art. Such programs comprise BLAST (Altschul et al. (1990), J. Mol. Biol. 215, 403-410), variants thereof such as WU-BLAST (Altschul & Gish (1996), Methods Enzymol. 266, 460-480), FASTA (Pearson & Lipman (1988), Proc. Natl. Acad. Sci. USA 85, 2444-2448), CLUSTALW (Higgins et al. (1994), Nucleic Acids Res. 22, 4673-4680) or implementations of the Smith-Waterman algorithm (SSEARCH, Smith & Waterman (1981), J. Mol. Biol. 147, 195-197). These programs, in addition to providing a pairwise sequence alignment (multiple sequence alignment in case of CLUSTALW), also report the sequence identity level (usually in percent identity) and the probability for the occurrence of the alignment by chance (P-value).

Preferably the sequence identity at the amino acid sequence level between myosins is at least 45%, 50%, 55%, 60%, 65%, 70%, 75%, 80%, 85%, 90%, 95%, 96%, 97%, 98% or 99%. Preferred sequence identities at the nucleic acid sequence level are at least 50%, 60%, 70%, 75%, 80%, 85%, 90%, 92%, 94%, 95%, 96%, 97%, 98% or 99%. In any case, it is deliberately envisaged to use myosins from mammalian and avian species including *Rattus norvegicus, Mus musculus, Gallus gallus, Lepus europaeus* and *Canis lupus.*

In a further embodiment, the method of designing of the invention further comprises synthesizing said modulator, thereby producing said modulator. The subject-matter of this embodiment is a method of producing said modulator. This embodiment is particularly envisaged for those cases where the modulator is a small organic molecule, a peptide or protein. Means and methods for synthesizing peptides and proteins are well known in the art and may involve organic synthesis and/or the recombinant production using the methods of molecular biology and protein biochemistry. As regards small organic molecules, reference is made to the Beilstein database available from MDL Information Systems as an example. Means and methods for preparing the preferred compound classes (for details see below) to be used in screens according to the invention are detailed in the Examples enclosed herewith. In a further preferred embodiment, said molecular modeling comprises (i) measuring at least one intermolecular distance; and/or (ii) calculating at least one free energy of interaction. Described is also (iii) determining the accessibility to the allosteric binding pocket. Accessibility of the binding pocket limits the size of the allosteric effector and thereby the maximal intermolecular interaction energy.

Tools for molecular modeling as described above typically provide the option of measuring one or more intermolecular distances. Preferably, the intermolecular distances are determined as distances between the centers of atoms. Tools for molecular modeling also generally provide the option of calculating free energies of interaction. The term "free energy" in relation to an interaction is well known in the art and is related to the equilibrium binding constant by the equation ΔG = -RT *In* K, wherein ΔG is the change in free energy upon binding, K is the binding constant, T is the temperature and R is the universal gas constant. Free energies to be calculated may be, as described above, total free energies and/or partial free energies.

The positions as defined in part (a) of the main embodiment are as follows: K265, A420, K423, A424, R428, L431, D590, I617, and A618. These positions or at least three of these positions provide a preferred definition of the binding pocket according to the invention. By defining the binding pocket, they implicitly also define the pharmacophore which is capable of binding to said binding pocket.

In case of the sequence of SEQ ID NO: 4 (myosin-1), the positions are K186, A355, E358, R359 N362, D521 and L527. In case of the sequence of SEQ ID NO: 6 (myosin-5), the positions are K246, A402, H406, A409, N410, N413, D570, V572 and H632. Obviously, in further embodiments of part (b) of the main embodiment (said embodiments relating to SEQ ID NOs: 8 to 108 (even numbers)), the positions are those positions which align with K265, A420, K423, A424, R428, L431, D590, 1617, and A618 of SEQ ID NO: 2. Analogous considerations apply to all preferred positions or ranges.

Further preferred anchor residues for modulator binding (in addition to the Lys residue described above) include D590 in the strut loop in *Dictyostelium* myosin-2 (SEQ ID NO: 2), which corresponds to D521 in *Dictyostelium* myosin-1 (SEQ ID NO: 4) and to D570 in *Gallus gallus* myosin-5 (SEQ ID NO: 6), respectively.

On the other hand, there residues in the allosteric binding pocket according to the invention which are specific for a given subclass of myosins such as class 2 myosins. Preferably, the method of designing according to the invention makes use of such subclass-specific residues to the effect that said modeled compound interacts with at least one of said subclass-specific residues. Modeled compounds interacting with at least one of said subclass-specific residues are candidates for subclass-specific modulators. More specifically, specificity is mostly mediated by residues in helix-21 that connects the actin binding cardiomyopathy loop (formed by residues 403 to 406 in myosin 2 (SEQ ID NO: 2)) with strand β5 which is part of the central β-sheet and adjacent to switch-2, forming part of the nucleotide binding pocket. The multiple contacts in helix-21 are structurally conserved but diverse as regards the nature of side chains involved in interactions. Conserved residues are A424, R428, and L431 in myosin-2 (SEQ ID NO: 2), A355, R359, and N362 in myosin-1 (SEQ ID NO: 4), and H406, N410, N413 in myosin-5 (SEQ ID NO: 6), respectively. Additional, preferably subclass-specific contacts with a compound binding to the allosteric pocket are provided by residues belonging to loop-2 and helix-29. The latter contacts are neither structurally conserved nor at the level of the side chains and thereby provide further possibilities in fine-tuning inhibitor binding and subclass-specificity thereof. Residues belonging to loop-2 and helix-29 are of particular importance in forming the opening of the access channel to the allosteric binding site of the invention. Specific residues in this region can be selected from the annotated sequence alignments and the topology diagram enclosed herewith (Figures 1 to 4).

The present invention furthermore provides a method of identifying an inhibitor of a myosin, the method comprising (a) bringing into contact a myosin and a test compound; (b) determining whether said test compound interacts by means of one or more of charge-charge interactions, charge-dipole interactions, dipole-dipole interactions, hydrogen bonds and hydrophobic interactions with at least three amino acid residues selected from (ba) positions K265, A420, K423, A424, R428, L431, D590, I617, and A618 of SEQ ID NO: 2, wherein said residues comprise K265 and said myosin comprises or consists of (i) the sequence of SEQ ID NO: 2; (ii) the sequence encoded by the sequence of SEQ ID NO: 1; or (iii) a sequence being at least 40% identical to the sequence of SEQ ID NO: 2 or to the sequence encoded by the sequence of SEQ ID NO: 1; wherein said sequence of (iii) comprises said three amino acid residues; or (bb) positions of any one of SEQ ID NOs: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 or 108, respectively, said positions aligning with the positions of SEQ ID NO: 2 as defined in (a), wherein the alignment of said ranges occurs over the entire length of the respective positions of (a) and (b), wherein said residues comprise the residue aligning with K265 of SEQ ID NO: 2 and said myosin comprises or consists of (i) the sequence of any one of SEQ ID NO: 4, 6, 8, 10, 12, 14, 16, 18, 20, 22, 24, 26, 26, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 or 108, respectively; (ii) the sequence encoded by the sequence of any one of SEQ ID NO: 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 or 109, respectively; or (iii) a sequence being at least 40% identical to the sequence of any one of SEQ ID NO: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 or 108, respectively, or to the sequence encoded by the sequence of any one of SEQ, ID NO: 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 or 109, respectively; wherein said sequence of (iii) comprises said three amino acid residues; wherein optionally said determining is effected by X-ray crystallography and/or NMR spectroscopy; and (c) identifying those compounds which interact with said at least three amino acid residues, thereby identifying said inhibitor of a myosin.

Described is also a method of identifying a modulator of a myosin, the method comprising (a) bringing into contact a myosin and a test compound; (b) determining whether said test compound interacts with at least three amino acid residues selected from (ba) ranges K265-V268, V411-L441, N588-Q593, D614-T629, and V630-E646 of SEQ ID NO: 2, said myosin comprising or consisting of (i) the sequence of SEQ ID NO: 2; (ii) the sequence encoded by the sequence of SEQ ID NO: 1; (iii) a sequence being at least 40% identical to the sequence of SEQ ID NO: 2 or to the sequence encoded by the sequence of SEQ ID NO: 1; or (iv) a sequence encoded by a sequence being at least 40% identical to the sequence of SEQ ID NO: 1; wherein said sequence of (iii) or (iv) comprises said three amino acid residues, and wherein said residues comprise K265; or (bb) ranges of any one of SEQ ID NOs: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 or 108, respectively, said ranges aligning with the ranges of SEQ ID NO: 2 as defined in (a), said myosin comprising or consisting of (i) the sequence of any one of SEQ ID NO: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 or 108, respectively; (ii) the sequence encoded by the sequence of any one of SEQ ID NO: 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 or 109, respectively; (iii) a sequence being at least 40% identical to the sequence of any one of SEQ ID NO: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 or 108, respectively, or to the sequence encoded by the sequence of any one of SEQ ID NO: 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 or 109, respectively; or (iv) a sequence encoded by a sequence being at least 40% identical to the sequence of any one of SEQ ID NO: 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 or 109, respectively; wherein said sequence of (iii) or (iv) comprises said three amino acid residues, and wherein said residues comprise the residue aligning with K265 of SEQ ID NO: 2; and (c) identifying those compounds which interact with said at least three amino acid residues, thereby indentifying said modulator of a myosin.

This embodiment relates to a screen for the identification of myosin modulators which in turn are suitable as medicaments or lead compounds for the development of a medicament. The screen may be implemented in various ways such as a biochemical screen or a cellular screen. In case of a cellular screen said "bringing into contact a myosin and a test compound" may be effected by bringing into contact a cell producing a myosin with a test compound. The bringing into contact is performed under conditions which allow binding of the test compound to myosin, in case the test compound is in principle capable of binding. Suitable conditions include conditions in liquid phase such as aqueous solutions, preferably buffered solutions. Furthermore, ionic strength may be adjusted, e.g., by the addition of sodium chloride. The concentration of sodium chloride may be between 0 and 2 M, preferably between 100 and 200 mM. Alternatively, sodium chloride is absent from the assay. For biological assays in many cases the presence of one or more further substances, including other salts than sodium chloride, trace elements, anti-oxidants, amino acids, vitamins, growth factors, ubiquitous co-factors such as ATP or GTP, is required. Said further substances may either be added individually or provided in complex mixtures such as serum. These and further accessory substances are well known in the art as are concentrations suitable for biological assays. The skilled person is aware of suitable conditions in dependency of the particular assay format to be used in the method of screening according to the invention.

Described is also that the screen may be implemented as a virtual screen, i.e., the screen may be performed *in silico.* Virtual screens may be implemented by computer-based docking of one test compound at a time into the allosteric binding site defined above, wherein both the test compound and the binding site are represented *in silico.* Thereby, the binding position and conformation is calculated. The binding site may, for example, be comprised in a representation of the entire myosin molecule or, alternatively, of those parts only which line the binding pocket. Upon completion of docking, the binding affinity (or equivalently the free energy of interaction as defined herein above) is determined based on the parameters of the computer representation of the involved molecules. A threshold may be chosen such as to select those test compounds which are candidate high affinity blinders. Suitable software packages are known in the art and include Chemoffice, CNS, CCP4, ADF and Gold (see above).

Preferably, said determining in step (b) is effected by X-ray crystallography and/or NMR spectroscopy.

In other words, the question of whether an interaction involving at least three residues of myosin occurs, is answered by determining structural parameters by using NMR spectroscopy or X-ray crystallography. These structural parameters may comprise the coordinates of the complex between said test compound and myosin. Alternatively, structural parameters may be determined only to the extent necessary to determine whether binding according to the invention, in particular interaction with at least three residues of myosin occurs. Examples of the latter, more selective methods include NMR spectroscopic methods exploiting the nuclear Overhauser effect or saturation transfer difference (STD). Suitable methods include the recording of NOESY and/or ROESY spectra. The required NMR spectra can be obtained in medium to high throughput manner, wherein throughput may be further increased by assessing a mixture of ligands, for example 10, 20 or 100 ligands at a time and further analyzing only those mixtures which are found to comprise one or more binding molecules. Also, means and methods for high throughput crystallization are available; see, for example, Stevens (Current Opinion in Structural Biology 2000, 10: 558-564) and Kuhn et al. (Current Opinion in Chemical Biology 2002, 6: 704-710).

X-ray crystallography may comprise (a) generating a crystal of a complex formed by said test compound bound to myosin; (b) generating and recording x-ray diffraction data; (c) digitising the data; (d) calculating an electron density map; (e) determining the three-dimensional structure of the crystal components; and (f) storing the crystal coordinates generated on a data carrier.

X-ray diffraction may be performed on a beamline such as the ID29 beamline of ESRF, Grenoble or using in-house devices such as a Bruker X8PROTEUM. Data may be further processed with XDS (W. Kabsch, J. Appl. Cryst. 21, 67 (1988)) and refined with CNS (A. T. Brunger et al. Acta Cryst. D 54, 905 (1998)). Alternatively, the PROTEUM2 software (Bruker) may be used. Structure can finally be solved with, for example, AmoRe (J. Navaza, Acta Crystallogr. A 50, 157 (1994)) and analysed with Xfit (D. E. McRee, J. Struct. Biol. 125, 156 (1999)) while structure validatation may be performed with PROCHECK (R. A. Laskowski, M. W. MacArthur, J. Appl. Crystallogr. 26, 283 (1993)) and WHATCHECK (R.W.W. Hooft, G. Vriend, C. Sander, E. E. Abola, Nature 381, 272 (1996)). The final map containing the atomic coordinates of the constituents of the crystal may be stored on a data carrier, typically the data is stored in PDB format or in X_PLOR format, both of which are known to the person skilled in the art. However, crystal coordinates may as well be stored in simple tables or text files.

The myosin used in the screen involving X-ray crystallography according to the invention may be myosin-2. A co-crystal of myosin-2 with an exemplary inhibitor has been solved; see Example 2. Methods well know in the art such as soaking permit the generation of co-crystals of myosin-2 with other modulators. Performing X-ray crystallography according to the invention with such co-crystals is a means of providing the necessary information for identifying compounds which interact with said at least three amino acid residues.

In a preferred embodiment of the method of identifying according to the invention, said method further comprises the step of (a') (i) determining whether said test compound binds to said myosin; and/or (ii) determining whether said test compound modulates the activity and/or conformation of said myosin; and/or (iii) determining the cytotoxicity of said test compound; wherein step (a) is to be effected after step (a) and prior to step (b), and wherein said determining in step (b) is performed with test compounds determined to bind, to modulate, and/or to be cytotoxic in step (a').

This embodiment provides for filtering a subset of test compounds testing positive in one or more of the assays of (a') (i) to (iii). The advantage of such filtering is that the subsequent determining whether the test compound interacts with at least three residues has to be done only with said test compounds testing positive.

Means and methods for determining binding are well known in the art and include assays based on fluorescence such as fluorescence resonance energy transfer (FRET) assays and fluorescence polarization (FP) assays, immunological assays such as ELISA, surface plasmon resonance, isothermal titration calorimetry and Fourier Transformed Infrared Spectroscopy (FTIR).

Assays for myosin activity are discussed further below.

Since the method of identifying a modulator according to the invention is designed to identify compounds binding to the aliosteric pocket of myosins, an additional or alternative filtering step involves the determining whether the test compound modulates or changes the conformation of said myosin. A change in conformation may be determined by using methods known in the art including the determination of electrophoretic or chromatographic mobility and fluorescence-based methods. In the latter case, changes in intramolecular distances between fluorophors arising from a change of conformation may be determined.

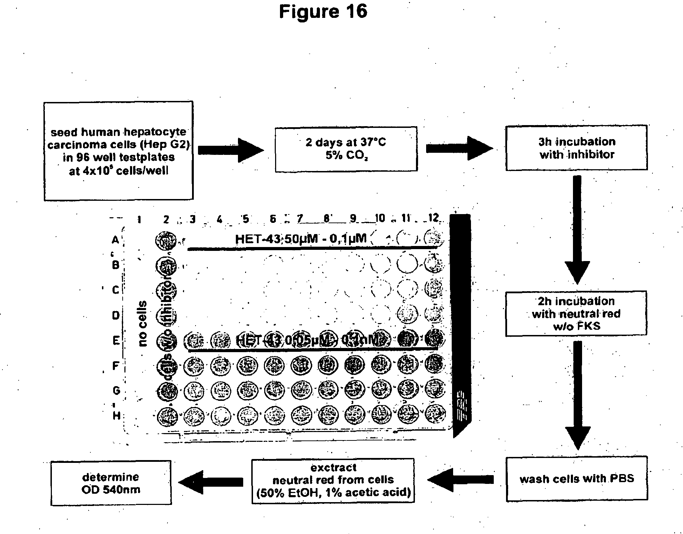

Said cytotoxicity correlates with the inhibition of the ATPase activity of myosin. Cytotoxicity may be assayed by determining the cellular uptake of neutral red. Only living cells are capable of neutral red uptake via an active transport mechanism. For an exemplary workflow of a cytotoxicity assay, see Figure 16.

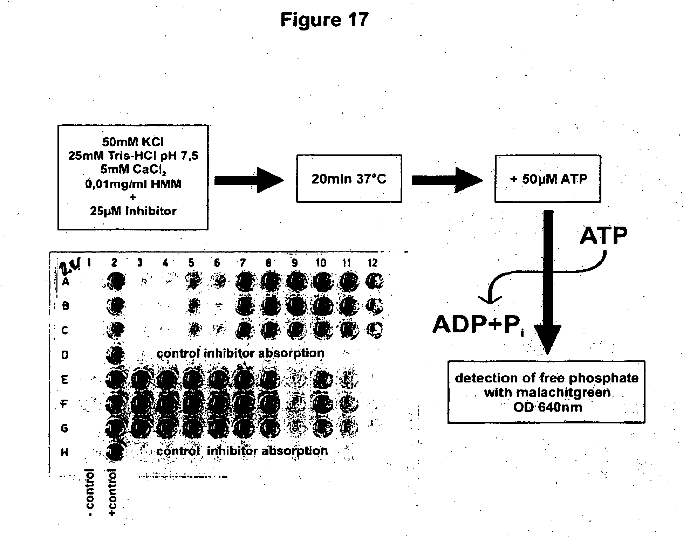

Preferably, said activity is the capability of said myosin (i) to bind actin; (ii) to convert ATP into ADP and Pi; and/or (iii) to generate force and/or movement. Exemplary or preferred binding assays are described herein above and can be applied for determining whether myosin binds actin, preferably F-actin, and/or nucleotides. Exemplary or preferred activity assays are described herein below and can be applied for determining whether myosin is capable of converting ATP into ADP and Pᵢ or of force production and generation of movement. An exemplary assay for ATPase activity is based on the detection of free phosphate formed upon cleavage of ATP, wherein the detection is effected using malachite green. For an exemplary workflow, see Figure 17.

In preferred embodiments, molecular modeling according to the invention starts from a compound selected from compounds of the general formulae (1) to (4) or a salt or solvate thereof, or the test compound to be used in methods of identifying of the invention is selected from compounds of the general formulae (1) to (4) or a salt or solvate thereof 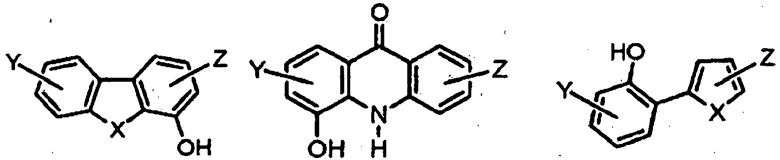 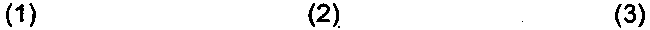 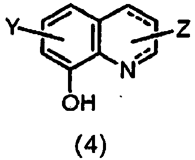 wherein X is selected from NH, O and S; and Y and Z designate, as valence permits, one or more substituents, wherein each occurrence of Y and Z is independently selected from F, Cl, Br, I, R and OR; R being selected from (i) H, (ii) (CO)CH₃, and (iii) linear or branched alkyl, alkenyl or alkinyl with one to four carbon atoms, the moieties (ii) and (iii) being optionally substituted with one or more F, Cl, Br and/or I.
Formula (1) represents carbazoles, dibenzofurans and dibenzothiophenes according to the invention. Formula (2) represents acridones according to the invention. Formula (3) represents a group of alkaloids known as pseudilines as well as their structural analogues having furan or thiophene in place of the pyrrole ring. Formula (4) represents quinolines according to the invention. The nitrogen-containing ring of quinolines according to the invention may be partially or fully hydrogenated.

Generally, R - as used in conjunction with any of the general formulae disclosed herein above and below - may have one or two carbon atoms. Particularly preferred is R = CH₃ , CCl₃ or CF₃. Also preferred are partially halogenated or mixed halogenated methyl groups such as CH₂F, CHF₂, CH₂Cl, CHCl₂, CF₂Cl or CHFCI.

Further compounds are disclosed herein below in conjunction with medical uses. All compounds described in conjunction with medical uses, in particular classes of compounds represented by generic formulae, are described for use in the methods according to the present invention.

Also described is the use of a compound selected from compounds of the general formulae (1) to (4) or a salt or solvate thereof as a lead compound in the development of a modulator of a myosin.

The term "lead compound" is known in the art and refers to a compound providing a starting point for developing a pharmaceutically active agent. Generally, said pharmaceutically active agent is different from, preferably optimized as compared to the lead compound. In other words, the development of a lead compound preferably involves the optimization of the pharmacological properties of said lead compound.

Methods for the optimization of the pharmacological properties of compounds identified in screens, generally referred to as lead compounds, are known in the art and comprise a method of modifying a compound identified as a lead compound to achieve: (i) modified site of action, spectrum of activity, organ specificity, and/or (ii) improved potency, and/or (iii) decreased toxicity (improved therapeutic index), and/or (iv) decreased side effects, and/or (v) modified onset of therapeutic action, duration of effect, and/or (vi) modified pharmacokinetic parameters (resorption, distribution, metabolism and excretion), and/or (vii) modified physico-chemical parameters (solubility, hygroscopicity, color, taste, odor, stability, state), and/or (viii) improved general specificity, organ/tissue specificity, and/or (ix) optimized application form and route by (i) esterification of carboxyl groups, or (ii) esterification of hydroxyl groups with carboxylic acids, or (iii) esterification of hydroxyl groups to, e.g. phosphates, pyrophosphates or sulfates or hemi-succinates, or (iv) formation of pharmaceutically acceptable salts, or (v) formation of pharmaceutically acceptable complexes, or (vi) synthesis of pharmacologically active polymers, or (vii) introduction of hydrophilic moieties, or (viii) introduction/exchange of substituents on aromates or side chains, change of substituent pattern, or (ix) modification by introduction of isosteric or bioisosteric moieties, or (x) synthesis of homologous compounds, or (xi) introduction of branched side chains, or (xii) conversion of alkyl substituents to cyclic analogues, or (xiii) derivatisation of hydroxyl group to ketales, acetales, or (xiv) N-acetylation to amides, phenylcarbamates, or (xv) synthesis of Mannich bases, imines, or (xvi) transformation of ketones or aldehydes to Schiff's bases, oximes, acetales, ketales, enolesters, oxazolidines, thiazolidines or combinations thereof.

The various steps recited above are generally known in the art. They include or rely on quantitative structure-action relationship (QSAR) analyses.(Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000).

Preferably, said general formulae are the general formulae (1) or (2).

In accordance with the invention, said modulator is an inhibitor.

The term "inhibitor" refers to compounds lowering or abolishing the activity of myosin, whereas the term "activator" refers to compounds increasing the activity of myosin, said activity being defined herein above. In preferred embodiments, inhibition refers to a reduction in activity of at least 10, 20, 30, 40, 50, 60, 70, 80, 90, 95, 98 or 99%. More preferred, activity drops to less than 10⁻², less than 10⁻³, less than 10⁻⁴ or less than 10⁻⁵ times the activity in absence of the inhibitor. "Activation" preferably refers to an increase in activity by 10, 20, 50 or 100%. More preferred activation involves a rise in activity to 3-fold, 5-fold, 10-fold or 15-fold or more of the activity in absence of the activator.

Described is furthermore a pharmaceutical composition comprising one or more compounds selected from compounds of the general formulae (1), (2) and (4) as defined herein above; 2,3,4-tribromo-5-(1'-methoxy-2',4'-difluorophenyl)-pyrrole; and the compounds shown below, wherein CF₃ may replace one, more or all occurrences of F, Cl, Br and/or MeO in said compounds shown below: - 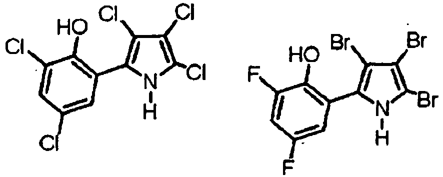 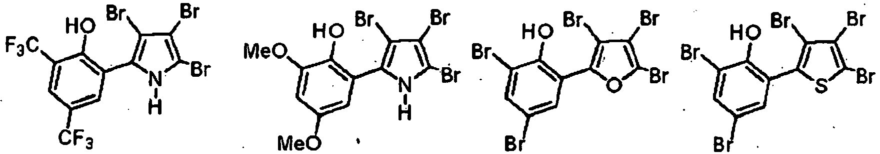 or a salt or solvate thereof.

The pharmaceutical composition may further comprise pharmaceutically acceptable carriers, excipients and/or diluents. Examples of suitable pharmaceutical carriers, excipients and/or diluents are well known in the art and include phosphate buffered saline solutions, water, emulsions, such a oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. It is particularly preferred that said administration is carried out by injection and/or delivery, e.g., to a site in the pancreas or into a brain artery or directly into brain tissue. The compositions may also be administered directly to the target site, e.g., by biolistic delivery to an external or internal target site, like the pancreas or brain. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Pharmaceutically active matter may be present in amounts between 1 ng and 10 mg/kg body weight per dose; however, doses below or above this exemplary range are envisioned, especially considering the aforementioned factors. If the regimen is a continuous infusion, it is preferably in the range of 1 µg to 10 mg units per kilogram of body weight per minute.

Also described is the use of one or more compounds as defined above for the manufacture a pharmaceutical composition for the treatment and/or prevention of cardiovascular diseases; cancer, diseases of the central nervous system, viral infections, and infections by parasites of the *Apicomplexa* family.

Example 2 provides proof of in vitro and in vivo activity of several preferred or exemplary compounds of the invention.

Myosins play an essential role in force generating processes and are essential in several common diseases like cancer, cardiovascular diseases, and parasite and viral infections. Also the immune response during inflammation, which is triggered by the synapse between T cells and antigen presenting cells, depends on the activation of myosin-2 and myosin-5 and is up-regulated during T-cell activation (Rey et al., 2007, in press).

With respect to cancer, in particular the formation of metastasis severely complicates the therapy and negatively affects the prognosis. At the beginning of tumor metastasis cells may enter into an amoeboid migratory state. For the migration, non-muscle myosins-2A and 2B play an essential role (Betapudi et al., 2006). The inhibition of myosin-2 (in particular isoform A) was shown to strongly inhibit tumor cell migration. Furthermore, in a clinical study on lung cancer patients a positive correlation was found between the expression of a myosin acitivating enzyme (myosin light chain kinase) and the likelihood of disease recurrence and metastasis (Minamiya et al., 2005). These and additional observations give strong evidence that the myosin-2 isoforms are excellent target proteins for cancer treatment, in particular against tumors that may form metastasis. This holds true in particular for the prevalent cancer types with high incidence (U.S. cancer statistics from 2003) like cancer of the oral cavity and pharynx, the digestive system including pancreas and respiratory system, melanomas of the skin, the genital and urinary system, and cancer of the brain and the nervous system. Further cancer forms which are deliberately envisaged for treatment by the present invention are listed below.

The myosin isoform 2B was found to be important for lamellar protrusions of migrating cells. Cellular protrusions such as filopodia are known to provide a gateway for the entry of various viruses into a host cell. Myosin-2 (Lehmann et al., 2005) or myosin-6 (Sun and Whittaker, 2007) are indispensable for virus uptake. After multiplication the exit of virus particles from the infected cell again depend on Myosin-2A or B; examples are vaccinia virus (M. Way, London; personal communication to HOG) and herpes simplex virus (van Leeuwen, 2002)). Myosin-2A is furthermore required for the internalization of the CXCR4 receptor, which plays a role in HIV uptake into the cell. The uptake of the receptor can be inhibited by silencing nonmuscle myosin -2A with siRNA or using the myosin inhibitor blebbistatin (Rey et al, 2007). Furthermore, the release of HIV-1 from the host cell depends on myosin activity, since this process can be blocked with Wortmannin, an effective inhibitor of myosin light chain kinase (Sasaki et al., 1995). Based on these findings the inhibition of myosin activity is envisaged as an efficient treatment of viral infections. The fact that myosin plays a key role in viral spreading is reflected in the observation that even plant viruses (Tobacco mosaic virus, Kawakami et al., 2004) require myosin activity for spreading.

Members of the myosin superfamily are known to be involved in the host cell invasion of apicomplexan parasites. This group of protozoa.includes species which are the cause of various human diseases of which malaria, toxoplasmosis or Eimeria infections are prominent examples. The gliding motility and invasion during infection depends on the activity of the unusual class-14, 23, and 24 myosins which is found in all *Apicomplexa* (Soldati et al., 2004).

Class-5 myosins are very abundant in the CNS (central nervous system) and mutations in the encoding gene give rise to severe neurological defects in mice and humans. In neurons, myosin-5a controls the targeting of IP3 (inositol 1,4,5-trisphosphate)-sensitive Ca²⁺ stores to dendritic spines and the transport of mRNA (Desnos et. al., 2007). Whereas deletion of non-muscle myosins-2 A and B are accompanied by abnormalities in the brain and a defect in the migration of neuronal cells (facial neurons, cerebellar granule cell and pontine neurons (Kim et. al., 2005).

Said infection by parasites of *Apicomplexa* family may be selected from malaria, toxoplasmosis and coccidiosis (e.g. by *Eimeria).*

Said cancer may be selected from the group consisting of:
Anal Cancer (Squamous Cell Carcinoma of the Anus), Bladder Cancer (Squamous Cell Carcinoma of the Bladder), Bone Cancer (Chondrosarcoma of Cartilage), Osteosarcoma, Cancer of the bone marrow including Myelodysplastic syndrome (MDS), Acute Lymphoblastic Leukaemia (ALL), Promyelocytic Leukaemia (PML), Acute Myeloid Leukaemia (AML), Chronic Myeloid Leukaemia (CML), Chronic Lymphocytic Leukaemia (CLL), Multiple Myeloma, Brain Tumour including as Astrocytoma, Glioblastoma, Lymphoma of the Brain, Neuroblastoma, Breast Cancer including including Ductal Carcinoma of the Breast (DCIS). and Lobular Carcinoma of the Breast (LCIS), Bowel Cancer, Cervical Cancer, Colorectal Cancer including Adenocarcinoma of the Rectum and of the Colon, Head and Neck Cancer including Lymphoma of the Tonsil, Squamous Cell Carcinoma of the Floor of the Mouth (Oral Cancer). Squamous Cell Carcinoma of the Larynx, Pharynx, Tongue, and Squamous Cell Carcinoma of the Tonsil (Throat cancer), Kidney Cancer (Renal Cell Carcinoma), Liver Cancer (Hepatocellular Carcinoma), Lung Cancer including Malignant Mesothelioma of the Pleura (Malignant Mesothelioma), Adenocarcinoma of the Lung, Large Cell Carcinoma of the Lung, Non₋small cell lung cancer (NSCLC), Small Cell Carcinoma of the Lung, Pleural effusion, Cancer of the Lymphatic System
Hodgkin's lymphoma and non-Hodgkin's lymphoma including Anaplastic Large Cell Lymphoma (ALCL), Burkitt's lymphoma, Cerebral Lymphoma, Cutaneous T cell Lymphoma, Diffuse large B cell lymphoma (DLBCL), Muscle Cancer including Biliary Cancer (Cholangiocarcinoma), Leiomyosarcoma of Muscle, Rhabdomyosarcoma of Muscle, Soft tissue Sarcomas, Oesophagus Cancer, Ovarian Cancer, Pancreatic Cancer (Adenocarcinoma of the Pancreas), Pituitary gland Gland Cancer, Prostate Cancer including Neuroendocrine Carcinoma, Adenocarcinoma of the Prostate, Skin Cancer including Basal Cell Carcinoma of the Skin, Malignant Skin Melanoma,
Small Intestine Cancer including Small Bowel cancer, Spinal Cord Tumours including Lymphoma, Astrocytoma, Glioma, Meningioma, and Metastases of the Spinal Cord, Stomach cancer, Testicular Cancer including Seminoma and Teratoma of the Testicle, Thyroid Cancer, Uterus Cancer (Adenocarcinoma of the Endometrium), Squamous Cell Carcinoma of the Vulva.

Viral infections according to the invention include infection by double-stranded DNA viruses such as viruses of *Herpesviridae* family include Herpes Simplex Virus (HSV). Furthermore deliberately envisaged are lentiviral and retroviral infections, including HIV infection (AIDS), Hepatitis-A, HBV (hepatitis-B) and HCV (hepatitis-C) infections.

Diseases of the central nervous system (CNS) include:
Alzheimer Disease, Brain lschemia, Cerebellar Ataxia, Cerebrdvascular Accident, Corticotiasal Ganglionic Degeneration (CBGD), Creutzfeldt-Jakob Syndrome, Dandy-Walker Syndrome, Dementia, Vascular, Encephalitis, Encephalomyelitis, Epilepsy, Hallervorden-Spatz Syndrome, Huntington Disease, Hydrocephalus, Ischemic Attack, Lacunar Syndromes, Landau-Kleffner Syndrome, Lewy Body Disease, Machado-Joseph Disease, Meige Syndrome, Meningitis, Multiple System Atrophy, Neuroaxonal Dystrophies, Parkinsonian Disorders, Shy-Drager Syndrome, Spinocerebellar Ataxias, Spinocerebellar Degenerations and Tourette Syndrome.

Furthermore described is a compound of the following formula A - L - B, wherein A is selected from compounds of the general formulae (1) to (4) as defined above, L is a linker, B is biebbistatin or an analogue, and "-" is a covalent bond.

This relates to divalent or multivalent compounds binding simultaneously to two or more binding sites of myosin. Such simultaneous binding to two or more binding sites may significantly enhance affinity and/or specificity. Blebbistatin as such has been described in the prior art; see e.g. Kovacs et al. (loc. cit.). Analogues of blebbistatin according to the invention are compounds binding to the blebbistatin binding pocket of myosins and/or exerting substantially the same modulating effect as blebbistatin. Biebbistatin interferes with myosin-2 function by inhibiting ATPase activity by blocking entry into the strong binding state, additionally it reduces the rate of ADP release. Preferred compounds A are carbazoles according to the invention (formulae (2) and (2')). A preferred attachment site for the linker L on carbazoles according to the invention is the position Y₆. The linker L may be the substituent designated Y₆. Preferably the linker is a poly-methylene linker with four to eight, more preferred five to seven, most preferred six carbon atoms, i.e., - (CH₂)₆-. One or more CH₂ groups of said linker may be replaced with oxygen or sulphur. Accordingly, envisaged linkers include -O-(CH₂)₂-O-(CH₂)₂-. Further linkers are known in the art and can be used for the present invention, wherein it is preferred that the length of said linkers is the same or substantially the same as the length of a poly-methylene linker with four to eight, more preferred five to seven, most preferred six carbon atoms.

For the pharmaceutical composition, the use, or the compound of the formula A - L - Blebbistatin, said one or more compounds or said compound A, respectively, are selected from compounds of the formulae (1'), (2') and (3'): 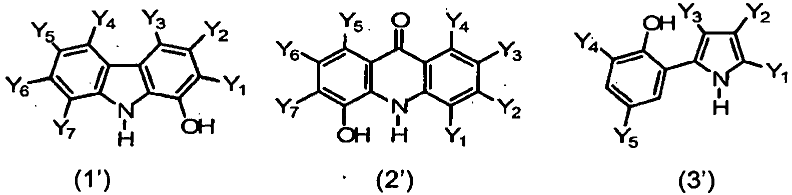 wherein Y₁, Y₂, Y₃, Y₄, Y₅, Y₆, and Y₇ are independently selected from H, F, Cl, Br, I, R and OR; R being selected from (i) H, (ii) (CO)CH₃, and (iii) linear or branched alkyl, alkenyl or alkinyl with one to four carbon atoms, optionally substituted with one or more F, Cl, Br and/or I; or a salt or solvate thereof.

Y₁ to Y₇, to the extent they are not H, may be selected from Cl, Br, OCH₃ and CF3. Particularly preferred are Br and CF₃.
(i) Y₁, Y₄ and Y₆ of formula (1') may be H; and/or (ii) Y₁, Y₄, Y₅ and Y₇ of formula (2') may be H.
(i) Y₇ of formula (1') may be H; and/or (ii) Y₂ of formula (2') may be H.

Accordingly, carbazoles include

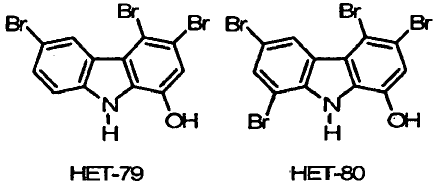

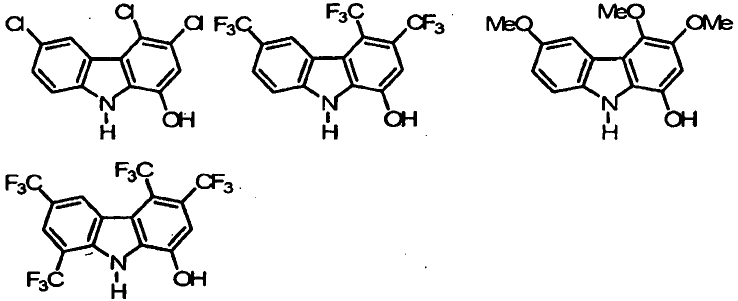

Acridones include

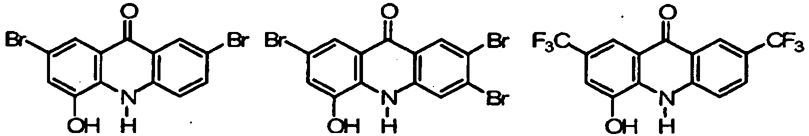

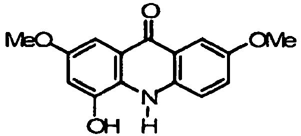

The substitution patterns as defined in the preceding embodiments apply *mutatis mutandis* O*-* and S-analoga of the compounds of formulae (1'), and (3'), i.e., to the corresponding dibenzo-furans and dibenzo-thiophenes (O- and S-analoga of the compounds of formulae (1')), as well as to the corresponding phenyl-furans and phenyl-thiophenes (O- and S-analoga of the compounds of formulae (3')). Also these O- and S-analoga are embraced by the present invention. Accordingly, a preferred dibenzo-furan is

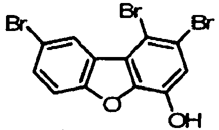

The substituents Y₁ to Y₇ on carbazoles, dibenzo-furans, dibenzo-thiophenes and acridones according to the invention, to the extent said substituents are present, i.e., different from H, may be equal. Also, substituents Y₁, Y₂ and Y₃ of pseudilines, phenyl-furans and phenyl-thiophenes of the invention may be equal. Furthermore, and independently, substituents Y₄ and Y₅ of said pseudilines, phenyl-furans and phenyl-thiophenes may be equal. Said substituents which are equal may be all Cl, Br, OCH₃ (herein also abbreviated "MeO") or CF₃, respectively, particularly Br and CF₃.

Further compounds are phenols which are substituted in positions 2, 4 and 6. The substituents may be selected from Br and CF₃. A particular phenol is 2,4,6-tribromo-phenol.

A further described class of compounds are fluorenes. Fluorenes include 1-amino-9-hydroxy-fluorenes and 2-amino-9-hydroxy-fluorenes, which are optionally substituted, as valence permits, with one or more substituents selected from F, Cl, Br, I, R and OR; R being selected from (i) H, (ii) (CO)CH₃, and (iii) linear or branched alkyl, alkenyl or alkinyl with one to four carbon atoms, optionally substituted with one or more F, Cl, Br and/or I. A particular fluorene is 2-amino-3-bromo-9-hydroxy-fluorene.

Further compounds are shown below. These compounds - as are all compounds described herein - are *inter alia* envisaged as starting compounds for the method of designing according to the invention and as lead compounds which may be further optimized by using the lead optimization methods as described herein above. The "HET" series of compounds is herein also referred to as the "KIN" series of compounds. Accordingly, and as an example, "HET-43" and "KIN-43" designate the same compound.

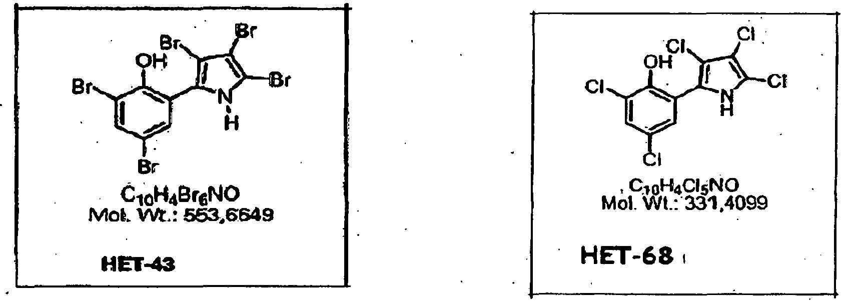

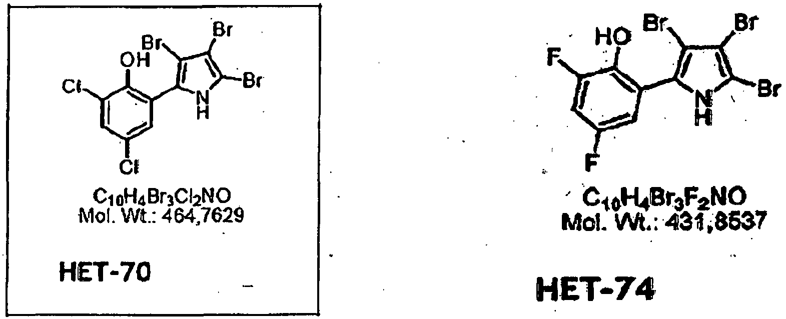

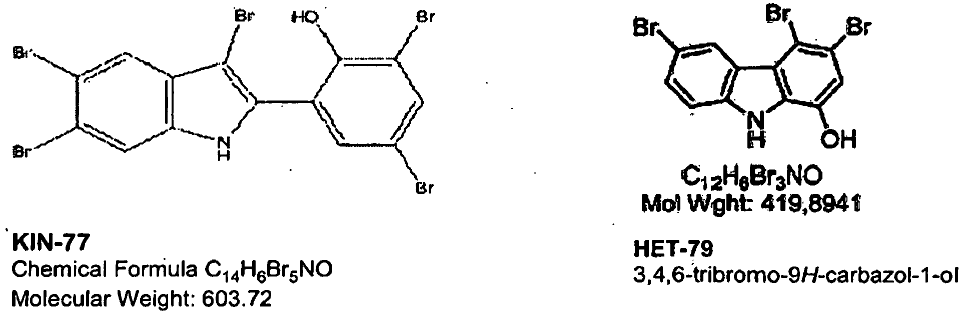

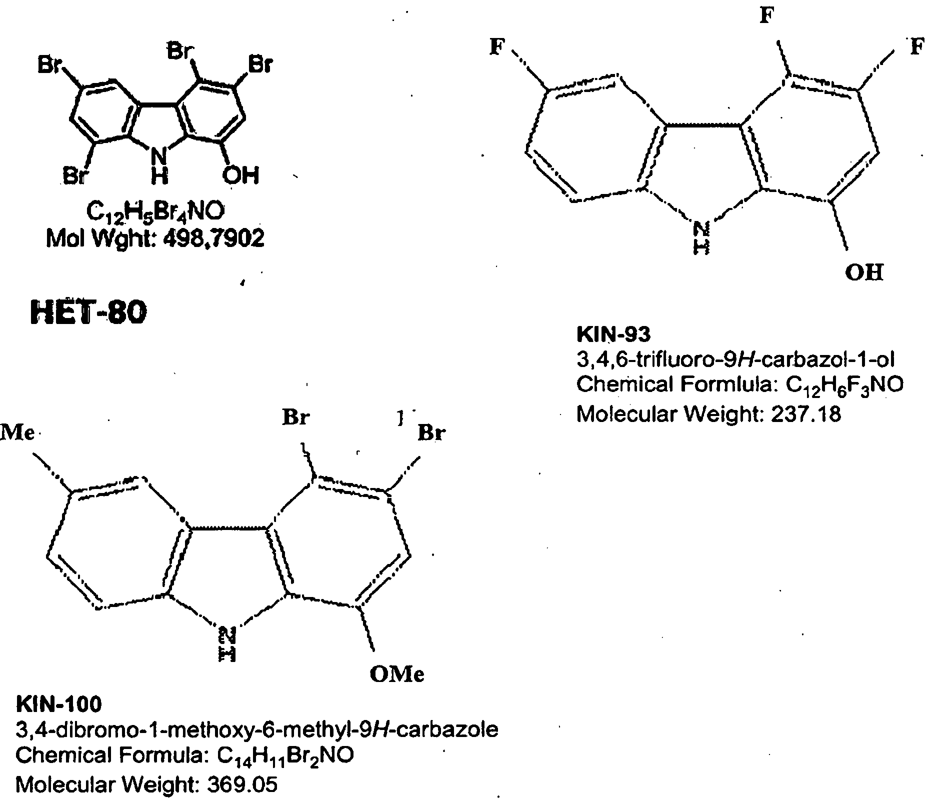

HET-43 is also known as pentabromopseudilin (PBP).

Described is furthermore a method of treating a disease selected from the group consisting of cardiovascular diseases, cancer, diseases of the central nervous system, viral infections, and infections by parasites of the *Apicomplexa* family, the method comprising administering to the subject in need thereof a therapeutically effective amount of one or more compounds as defined herein above.

Also described is a method of treating a patient suffering from or at risk of developing a disease or condition selected from the group consisting of cardiovascular diseases, cancer, diseases of the central nervous system, viral infections, and infections by parasites of the *Apicomplexa* family, the method comprising administering to the patient a composition comprising one or more of the compounds defined above in a therapeutically effective dose, thereby treating the patient.

Particular compounds and indications to be treated or prevented by the above methods are described herein above in conjunction with medical uses and pharmaceutical compositions.

The Figures show:
**Figure 1****:** Topology of myosin-2. Helices are shown as circles and beta-sheets as triangles.
**Figure 2****:** Sequence alignment of three representative myosins (*Dictyostelium* myosin-1 E, *Dictyostelium* myosin-2 and *Gallus gallus* myosin-5). The ligand-binding sites are shaded. Dd *= Dictyostelium discoideum,* Gg = *Gallus gallus.*
**Figure 3**: Alignment of human myosin-2 isoforms. The isoforms are human myosins-2 from cardiac muscle, smooth muscle, non-muscle (NM) and brain. tissue. The ligand-binding sites of the respective human myosins NM Myosin-MHC14, brain tissue-MHC15, cardiac alpha-MHC6, cardiac beta-MHC7, fast skeletal adult2-MHC2, fast skeletal embryonal-MHC3, skeletal adult1-MHC1, skeletal extraocular=MHC13, smooth muscle-MHC11, NM 2A-MHC9, NM 2B-MHC10 as compared to *Dictyostelium* myosin-2 ("MHCA Fulllength") and MHG myosin heavy chain are shaded.
**Figure 4****:** Alignment of human and apicomplexan myosins. The ligand-binding sites of the respective human myosins cardiac beta-MHC7, fast skeletal adult2-MHC2, smooth muscte-MHC11, NM 2A-MHC9, NM 2B-MHC10 as compared to myosins-1 from *Plasmodium falciparum* (Pf MyoA und Pf MyoF), and from *Toxoplasma gondii (Tg MYo A, Tg Myo B, Tg Myo E and Tg MyoG)* are shaded. MHC=myosin heavy chain.

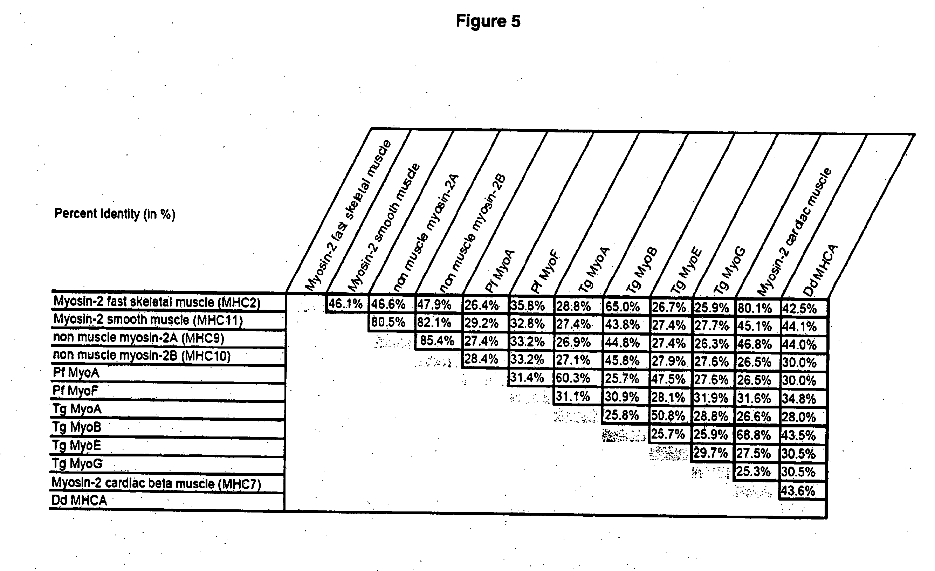

**Figure 5****:** Amino acid sequence pair distance of myosins from human, *Apicomplexa* and *Dictyostelium. The* underlying sequences are the sequence from amino acid 265 to 635 of Dd myosin and the corresponding subsequences of the other myosins aligning with this sequence.

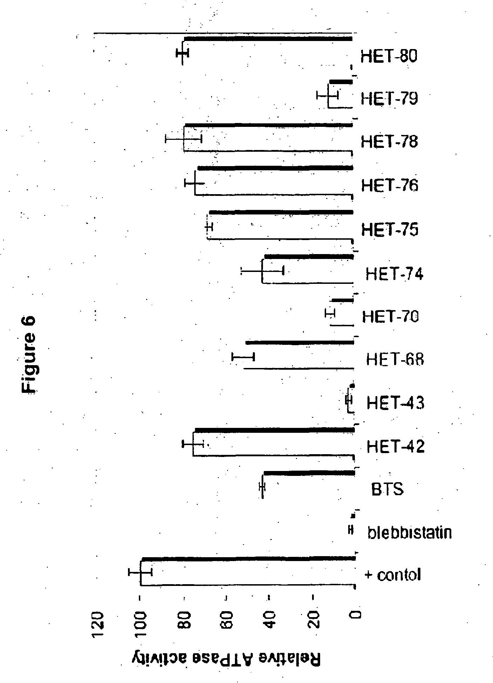

**Figure 6****:** Histogram showing the relative myosin-2 ATPase activity measured in the absence (+ control) and presence of halogenated alkaloids. Blebbistatin und BTS were used as controls. Compounds of the HET-series, BTS and blebbistatin were used at 25µM final concentration. HET-43 is PBP (pentabromopseudilin). HET-68 = pentachloropseudilin; Het-70 = 2,3,4-tribromo-5 (1' hydroxy, 2',4'-dichlorophenyl) pyrrole; Het-74 = 2,3,4-tribromo-5 (1' hydroxy, 2',4'-difluorophenyl) pyrrole; HET-76 = 2,3,4-triiodo-5 (1' methoxy, 2',4'-dichlorophenyl) pyrrole; HET-78 = 2,3,4-tribromo-5 (1' methoxy, 2',4'-diiodophenyl) pyrrole. The bars represent the mean value of three measurements. The error bars indicate the standard deviation.
**Figure 7****:** 2 Fo-Fc electron density omit map showing Het-43 (left) and the nucleotide (right) binding sites. The map was calculated using the final model of myosin-2(Het-43) complex with the inhibitor and the substrate omitted. The map was contoured at 1,0 σ.
   Overall view of myosin(Het-43) complex (left). The details of Het-43 positioning in the binding site are shown in the close up (right). The transparent surface represents van-der-waals radii of the atoms forming Het-43 binding site.
**Figure 8****:** Comparison of the PBP-binding sites of *Dictyostelium* myosin-2, *Dictyostelium* myosin-1E, chicken myosin-5a, *Saccharomyces cerevisiae* myosin-2p, and *Dictyostelium discoideum* myosin-5b (from top to bottom).
**Figure 9****:** Inhibition of myosin functions by PBP. (a) Concentration-dependence of the inhibition of the actin-activated ATPase activity displayed by different myosin constructs. The semi-logarithmic plot shows the concentration dependence for myosin-1E (o), myosin-2 (■), and myosin-5b (Δ) from *D. discoideum* and for chicken myosin-5a (▼). (b) ATP turn-over rates of Dictyostelium myosin-2 (o) and Dictyostelium myosin-5b (■) at increasing F-actin concentrations in the absence (filled symbols) and presence (open symbols) of 25 µM pentabromopseudilin.
**Figure 10****:** ATP binding kinetics. For details see Example 2.
**Figure 11****:** (a) The histograms shows the average sliding velocity of Rh/Ph-labeled actin filaments in the absence and presence of 10 µM PBP. (b) Inhibition of chicken myosin-5a by PBP in the *in vitro* motility assay. The histograms show the average sliding velocity of Rh/Ph-labeled actin filaments in the absence and presence of 10 µM PBP. Myosin-5a moves actin filaments with an average velocity of 0.6 µm/s ± 0.05 (dark bars); addition of 10 µM PBP into the same flow cell decreases the average sliding velocity to 0.013 µm/s ± 0.002 (light grey bars).
**Figure 12****:** Isometric force measurements.
**Figure 13****:** Myo2p-dependent changes in mitochondrial morphology. In yeast cells, the class-5 myosins Myo2p is responsible for the distribution of mitochondria between mother and daughter cells during cytokinesis and has be shown to affect mitochondrial morphology during interface. (a) Wild-type yeast cells; (b) Myo2p-depleted cells; (c) the phenotype of Myo2p-depleted cells can be phenocopied by exposure of witd-type cells to 500nM PBP. Insets show the same cells viewed under transmitted light. Bar: 5µm
**Figure 14(A)****:** Overview of HET-79 binding site. Het-79 binds bind in the same region and makes similar contacts as HET-43. However, there exist significant differences in the detailed interactions, the size of the contact area with the protein and the accessibility of the binding pocket.

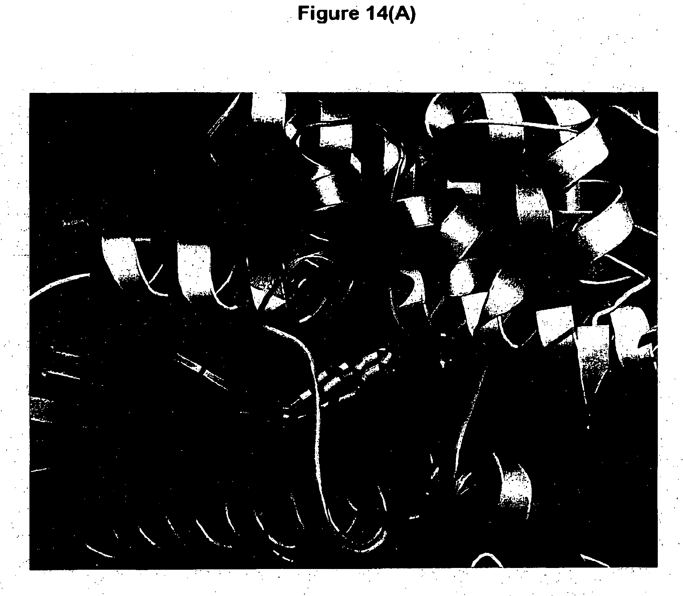

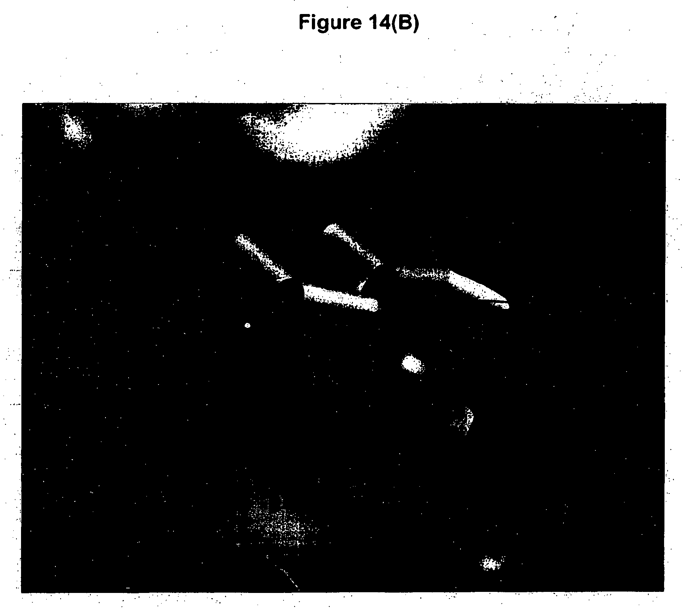

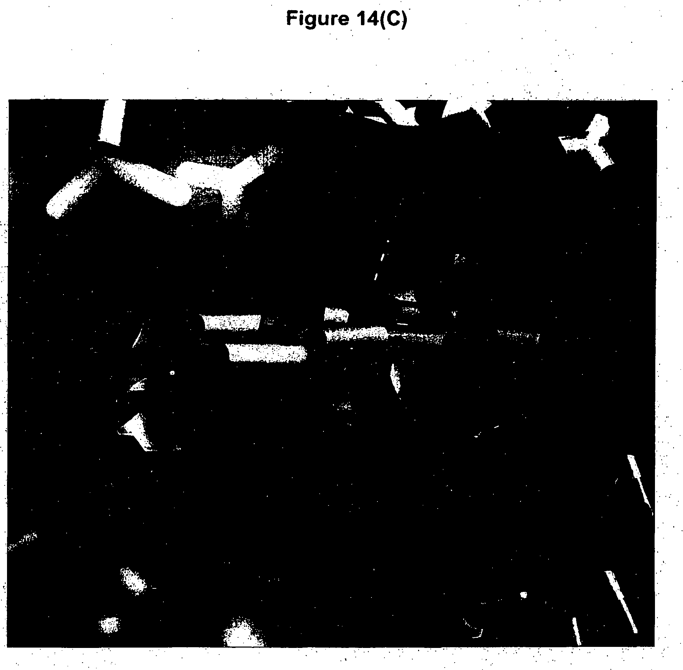

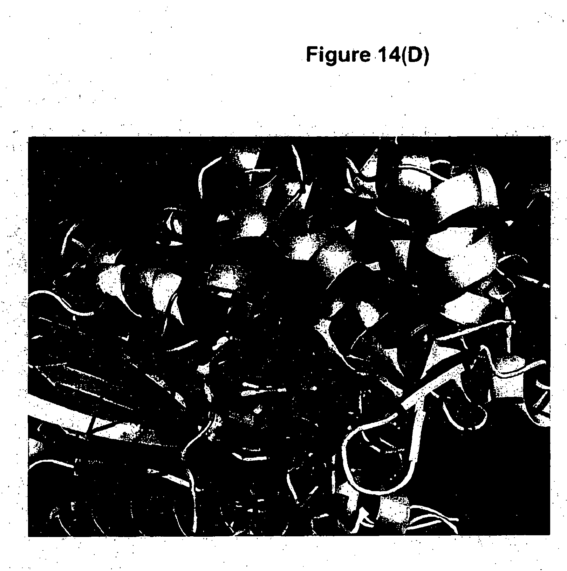

**Figure 14(B)****:** HET-79 binding pocket has three access channels. HET-79 binds in a pocket that is formed by residues at the interface between the 50kDa upper domain (U50) and the 50kDa lower domain (L50). The secondary structure elements that form the pocket include helix-13 (K265-V268), helix-21 (V411-L441), the strut loop (N588-Q593), loop 2 (D614-T629) and helix-29 (V630-E646). Both polar and apolar residues contribute to the binding of PBP. The HET-79 pocket has three major access channels. The first is located between loop-2 and the strut loop, the second is formed by residues from the loop connecting β7, the edge β-strand of the central β-sheet, with helix-13, helix-21, loop-2 and helix-29. The third is formed by residues from β7, helix-13, the loop connecting both, helix-21, and the strut loop.
**Figure 14(C)****:** Detailed contacts with the OH-group of the carbazol derivative HET-79. K265 forms a hydrogen bond with the hydroxyl group. Additionally, K265 is in hydrogen bonding distance from a water molecule that is also in contact with D590. K423 and the carbonyl group of A424 form additional contacts with the OH-group of HET-79.
**Figure 14(D)****:** Comparison with HET-43 Binding pocket.

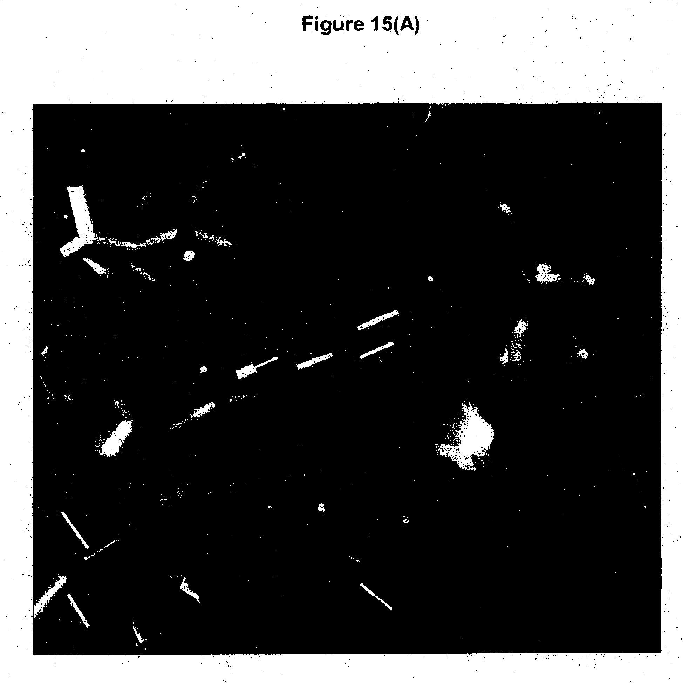

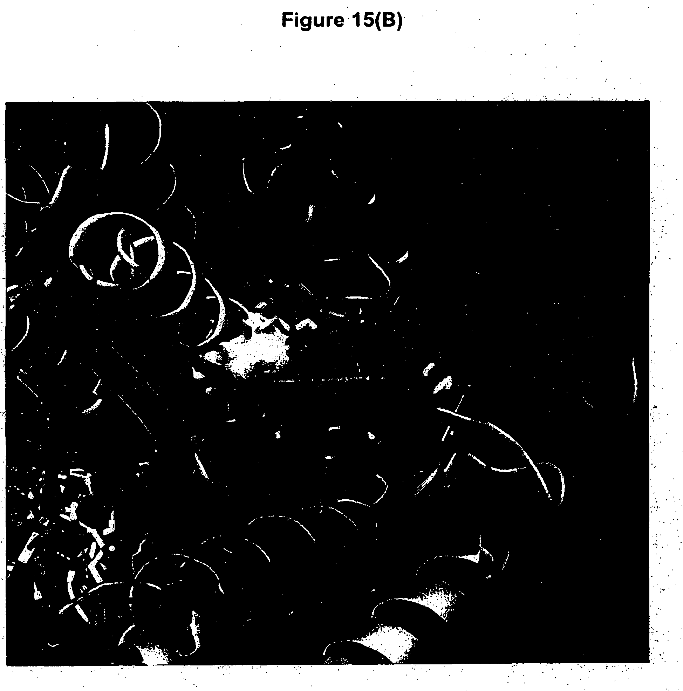

**Figure 15(A)****:** Het-68 (Pentachloropseudilin) binding shows major differences to that of the closely related pentabromopseudilin. Differences include major rearrangements of the loop-2 region, a reversal of the orientation of the inhibitor in the binding pocket, the complete planarity of HET-68, and its trans orientation of phenyl and pyrrol rings. Accordingly, there are also major changes in the contacts made between inhibitor and protein. K265 makes electrostatic interactions with the chlorine groups at positions 2 and 3 of the pyrrol ring; the chlorine in position 4 makes electrostatic interactions with D590. R428 forms a hydrogen bond with the OH group of the phenyl ring and the aliphatic part makes staggering interactions with pyrrol and phenyl ring. The aliphatic part of R620 makes a staggering interaction with the phenyl ring and its guanidinium group contributes an electrostatic interaction with the OH group. A618 and R620 form a tight indentation of the binding pocket that accommodates the 4' chlorine, where the 4' chlorine makes strong electrostatic interactions with the main chain amide groups of S619 and R620. Finally, E597 forms a hydrogen bond with a water molecule that is positioned close to the 2' chlorine group of the phenyl ring.
**Figure 15(B)****:** Overview of the Het-68 binding pocket. The second helix of the helix-loop-helix motif preceding loop-2 appears unstructured here and has moved closer towards the inhibitor. The inhibitor is completely planar and the binding pocket is more open towards the central β-sheet. The nucleotide binding pocket is seen in the lower-left corner.
**Figure 16****:** Cytotoxicity assay.
**Figure 17****:** ATPase activity assay.

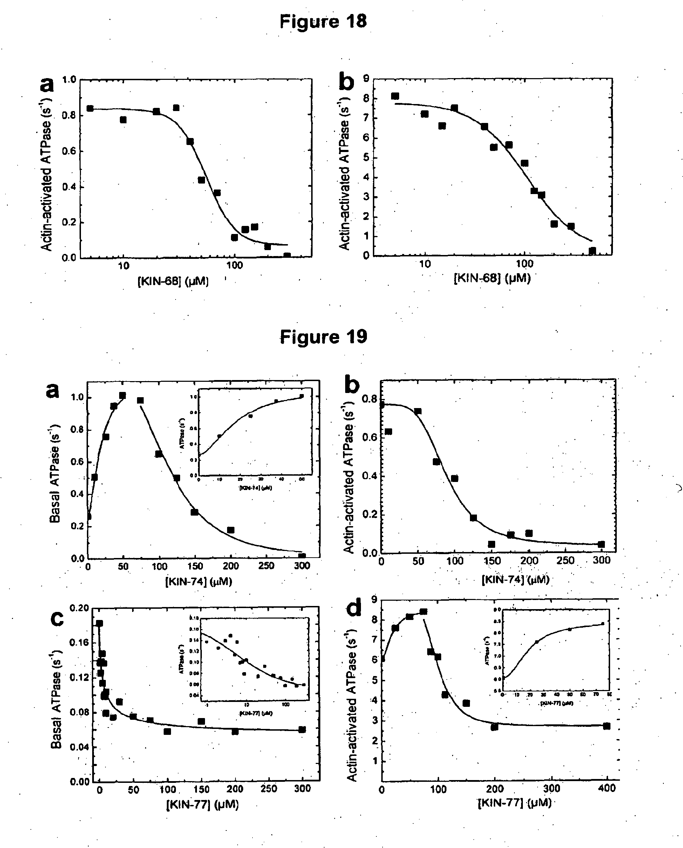

**Figure 18****:** KIN-68-inhibition of the basal ATPase activity of *Dictyostelium* myostn-1E and myosin-5b. The concentrations of KIN-68 required for half-maximal inhibition of myosin-1E ATPase activity is 55.8 µM **(a)** and 104.6 µM for myosin-5b **(b).**
**Figure 19****:** Effects of KIN-74 and KIN-77 on the basal and actin-activated ATPase activity of *Dictyostelium* myosin-2 and rabbit myosin-2.
   (a) Concentrations of KiN-7 in the range from 1 to 50 µM activate the basal ATPase activity of *Dictyostelium* myosin-2 with an apparent half maximum activation constant AC50 of 18 µM. At concentrations of KIN-74 >50 µM the basal ATPase activity of *Dictyostelium* myosin-2 is inhibited with IC50 = 90 µM.
   (b) In the presence of 30 µM actin KIN-74 inhibits the ATPase activity of Dictyostelium myosin-2 with IC50 = 88.8 µM
   (c) KIN-77 inhibits the basal ATPase activity of myosin-2 approximately 3-fold with IC50 = 5.3 µM.
   (d) Concentrations of KIN-77 in the range from 1 to 100 µM activate the basal ATPase activity of rabbit myosin-2 from 6.1 s-1 to 8.5 s-1 with a half maximum activation constant AC50 = 19.2 µM. At concentrations of KIN-77 >100 µM the actin-activated ATPase activity of rabbit myosin-2 is inhibited 3.5-fold with an IC50 = 95.6 µM.

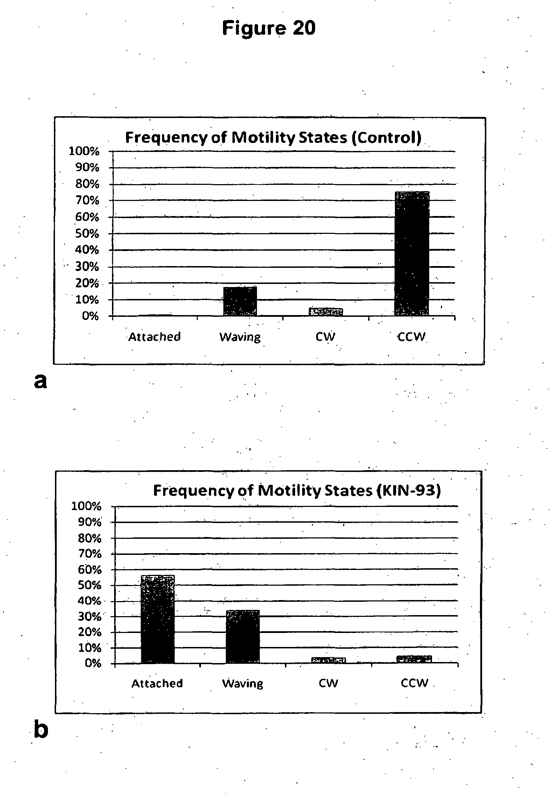

**Figure 20****:** (a) Plasmodium sporozoites adopt motility states, shown in the photos from the left to the right, attached, waving, clockwise (CW), and counterclockwise (CCW). In the absence of inhibitors sporozoites preferably move CW and CCW at 0.97 µm/s and 1.13 µm/s. (b) The addition of KIN-93 switched the population to attached and waving states and reduced the velocity for CW and CCW motility to 0.58 µm/s and 0.59 µm/s.

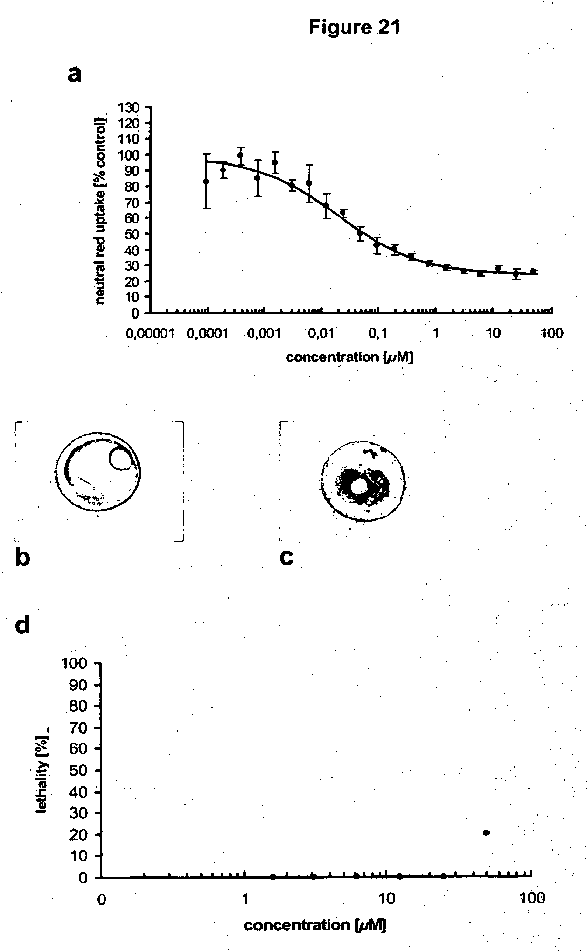

**Figure 21****:** Development toxicity testing of KIN-compounds on medaka embryos. (a) KIN-43 in concentrations between 0.5 µM and 100 µM were incubated for 48 h with 20 medaka embryos per concentration. The half lethal concentration of KIN-43 was at LC50 = 3.0 µM. (b) Light microscopy image of embryo after 48 h in the absence of KIN-43. (c) At higher concentration of KIN-43 the embryos agglomerate and die. (d) KIN-93 shows no lethal effect on medaka embryos in the lower micromolar range below 50.0 µM. LC50 is therefore well above a concentration of 50.0 µM.

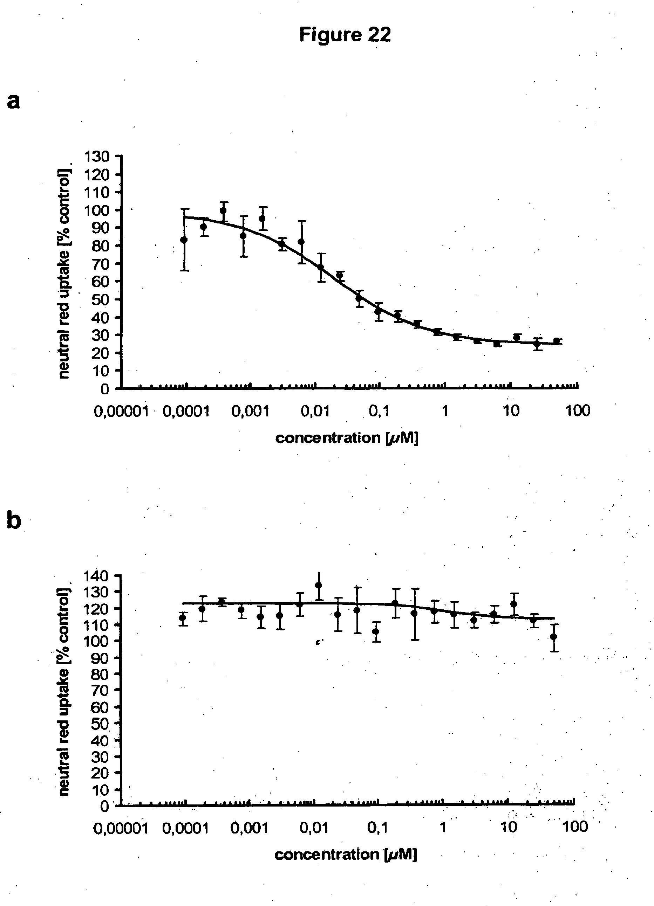

**Figure 22****:** Cytotoxic testing of KIN-compounds by neutral red uptake assays. (a) human hepatocyte carcinoma cells (Hep G2) were exposed to KIN-43 for 4h in the in presence of 1% FBS. KIN-43 shows a half effective concentration EC50 of 0.03 µM. (b) exposure of the same amount of KIN-93 did not show a measurable effect in the concentration range between 0.1 nM and 100 µM resulting in a LC50 well above a concentrations of 50.0 µM. The following examples illustrate the invention but should not be construed as being limiting.

### Example 1

### Metal-Catalyzed Syntheses of Polyheterocyclic Compounds

### Introduction

Over the past two decades, applications of transition metals for selective C-H bond activation have emerged to a powerful tool in organic synthesis.

Methodologies have been developed for the cyclization of appropriately substituted primary or secondary alkyl- and arylamines, which open up simple and direct approaches to nitrogen-containing heterocyclic ring systems. These transformations are efficiently induced by using either stoichiometric or even catalytic amounts of transition metals (*e.g*., iron, molybdenum, palladium, or silver). The advantages of this synthetic approach are mild reaction conditions and toleration of a broad range of functional groups. Therefore, the construction of nitrogen-containing heterocyclic frameworks by fusion of fully functionalized building blocks is achieved in just a few synthetic steps. Application of this chemistry in heterocyclic synthesis provides convergent and highly efficient short-step approaches to a variety of biologically active compounds.¹

### 1. Silver(I)-Catalyzed Cyclization to Pyrroles

The pyrrole ring system represents a pivotal substructure in naturally occurring alkaloids and pharmaceutical products. Following the classical Hantzsch, Knorr, and Paal-Knorr syntheses numerous alternative assemblies of pyrroles have been reported. Homopropargylamines represent easily available building blocks for pyrrole synthesis. We recently described a novel pyrrole synthesis by silver(I)-mediated oxidative cyclization of homopropargylamines to pyrroles.² The required precursors are readily accessible by condensation of simple arylaldehydes to Schiff bases and subsequent Lewis acid-promoted addition of 3-trimethylsilylpropargylmagnesium bromide (Scheme 1). Under optimized reaction conditions, treatment with 1.1 equivalents of silver acetate at room temperature affords the corresponding pyrroles almost quantitatively. This procedure represents a versatile and simple synthetic route to 1,2-diarylpyrroles, which are of interest due to their biological activities.

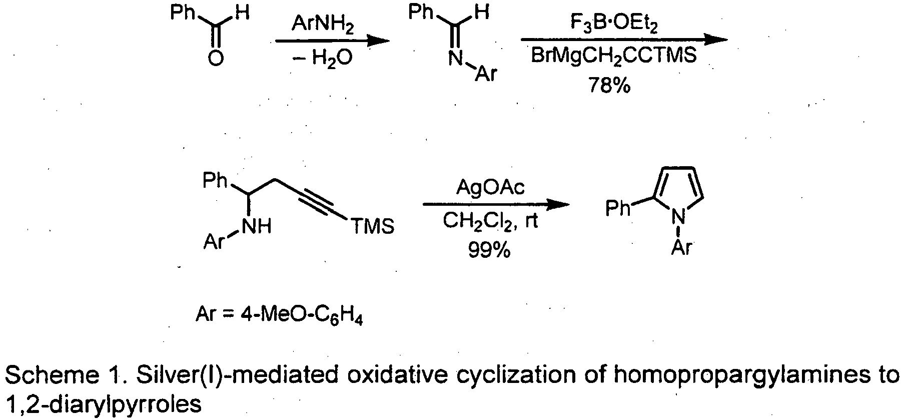

It is known that silver(I) salts form stable π-complexes with terminal acetylenes. On the other hand, silylacetylenes on treatment with silver nitrate were reported to afford silver acetylides. Based on these considerations and additional experimental evidence, the following mechanistic rationale has been provided for the silver(I)-mediated oxidative cyclization of homopropargylamines to pyrroles (Scheme 2). Activation of the acetylene by coordination of the triple bond to the silver cation enables a 5-endo-dig cyclization *via* nucleophilic attack of the amine. Protonation of the resulting vinyl silver complex leads to an iminium ion. Subsequent β-hydride elimination affords metallic silver and a pyrrylium ion which aromatizes by proton loss to the pyrrole. For trimethylsilyl-substituted homopropargylamines (R3 = SiMe₃), the resulting pyrrole (R³ = SiMe₃) undergoes protodesilylation to the 1,2-disubstituted pyrrole.

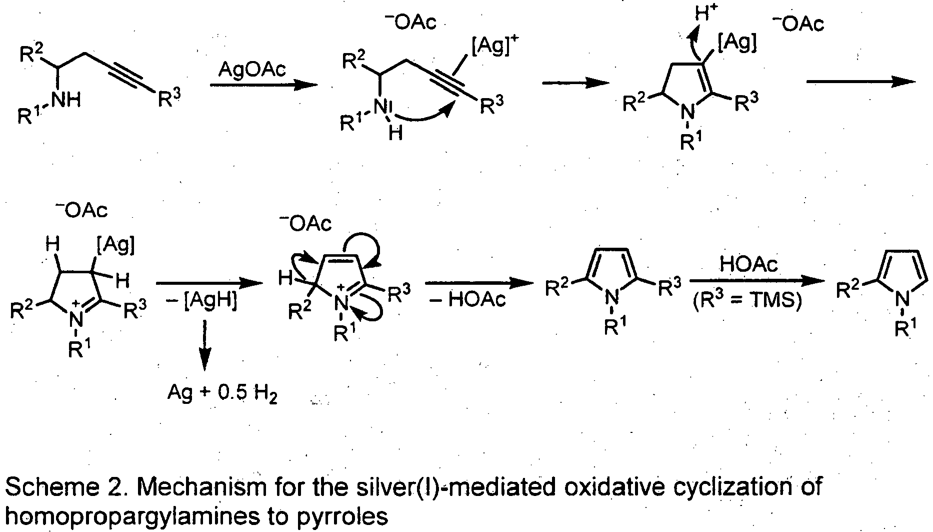

### Application: Synthesis of Pentabromopseudilin and Structural Analogues

Highly halogenated bioactive natural products as pentabromo- and pentachloropseudilin are often obtained from marine sources.³ For the construction of their heterocyclic framework a catalytic variant of our silver(I)-mediated pyrrole synthesis was applied: a silver(I)-catalyzed cyclization of the corresponding *N*-tosylhomopropargylamines (Scheme 3).

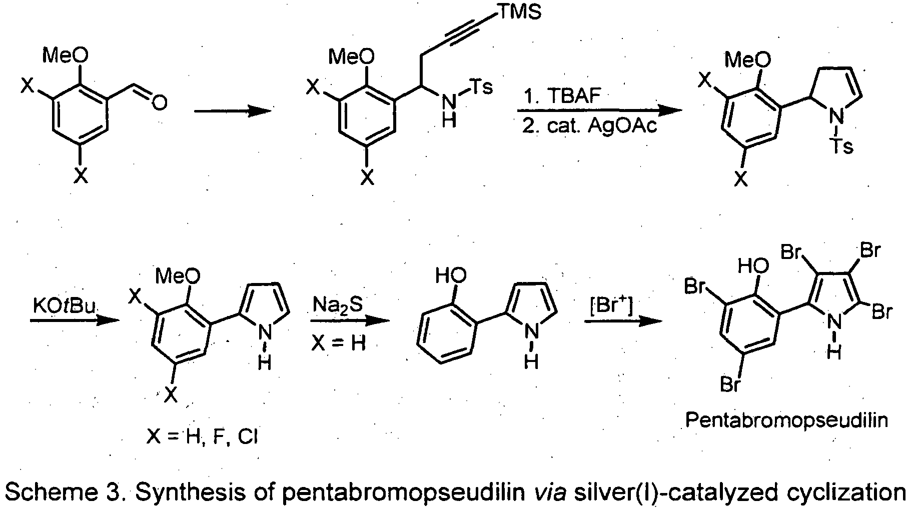

Starting from the appropriately substituted precursor, the dichloro- and the difluoro-*O*-methylpseudilin are available (Scheme 4). Further chlorination and subsequent cleavage of the ether provides pentachloropseudilin, while further bromination followed by ether cleavage leads to the non-natural dichlorotribromopseudilin and difluorotribromopseudilin. Our direct approach can be easily exploited for the generation of a whole series of structural analogues.

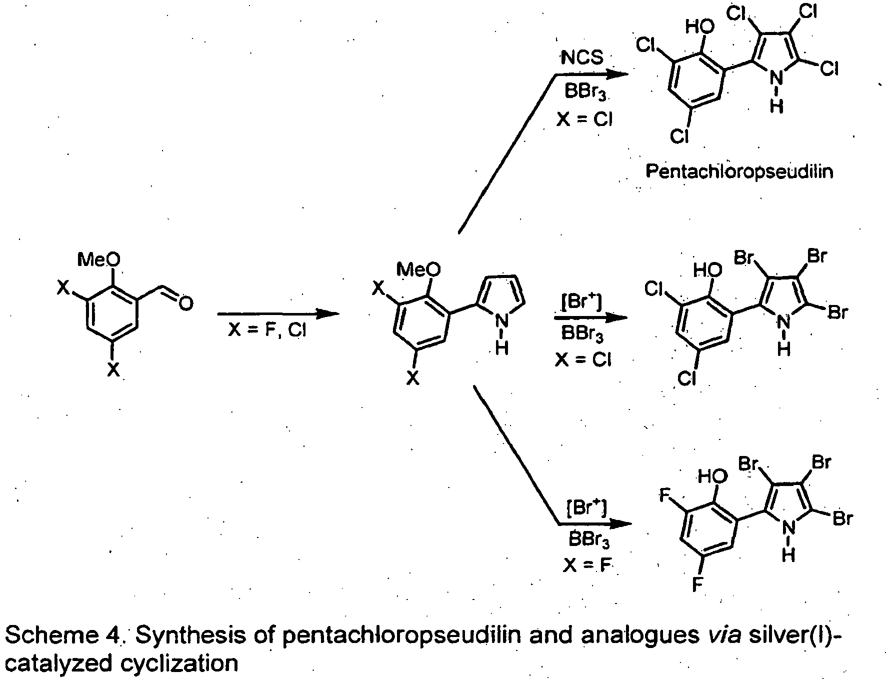

### 2. Transition Metal-Catalyzed Oxidative Cyclization to Carbazoles

A broad structural variety of carbazole alkaloids with useful biological activities has been isolated from different natural sources (Chinese and Indian medicinal plants, marine algae, streptomyces, etc.). The pharmacological potential of this class of natural products led to the development of diverse methodologies for the synthesis of carbazoles.⁴ We elaborated an efficient iron-mediated construction of the carbazole framework by consecutive C-C and C-N bond formation (Scheme 5). Electrophilic aromatic substitution by reaction of the iron-coordinated cyclohexadienylium salt 1 and the arylamine 2 generates the aryl-substituted iron-cyclohexadiene complex 3. Application of an appropriate oxidizing agent results in oxidative cyclization with concomitant aromatization and demetalation to afford directly the carbazole 4.

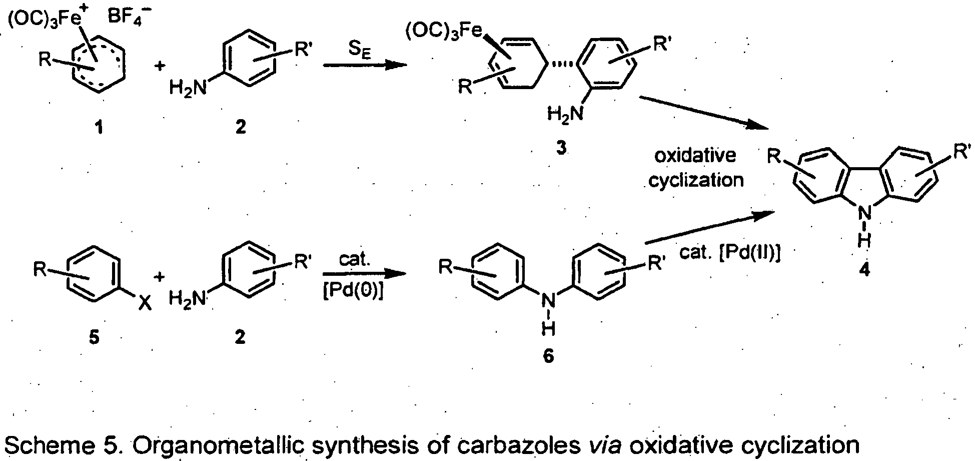

An alternative route to the carbazole framework represents the palladium(II)-catalyzed oxidative cyclization of *N*,*N*-diarylamines 6, which are readily available by palladium(0)-catalyzed amination of the aryl bromides 5 with the arylamines 2. Heating of the *N*,*N*-diarylamines 6 with catalytic amounts of palladium(II) acetate in the presence of copper(II) acetate in acetic acid at reflux results in smooth oxidative cyclization to the corresponding carbazoles 4 (Scheme 5).⁴

### Application: Synthesis of Polybrominated 1-Hydroxycarbazoles

Using the palladium-catalyzed construction of the carbazole framework, an easy access to 1-methoxycarbazote has been achieved (Scheme 6). Dependent on the reagent and the reaction conditions, the subsequent bromination provides either 3,4,6-tribromo-1-methoxycarbazole or 3,4,6,8-tetrabromo-1-methoxycarbazole, which by cleavage of the ether are converted to the corresponding polybrominated 1-hydroxycarbazoles.

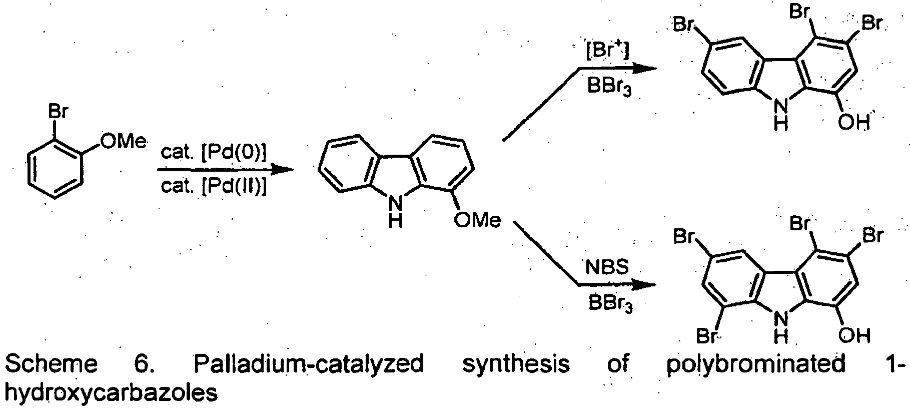

### Example 2

### Allosteric inhibition of myosin motor activity by pentabromopseudilin

The inventors surprisingly found several promising lead compounds; amongst them flavinoids and halogenated alkaloids; see Figure 6. Furthermore, it was surprisingly found that pentabromopseudilin (PBP) was a very potent inhibitor of rabbit skeletal muscle heavy meromyosin (HMM) ATPase activity. The systematic name of PBP is 2,3,4-tribromo-5-(1' hydroxy, 2',4'-dibromophenyl)-pyrrole.

To identify the PBP binding site and to elucidate the inhibitory mechanism, we solved the cocrystal structure of PBP bound to the *Dictyostelium* myosin-2 motor domain. The structure shows the inhibitor to bind in a pocket close to the actin binding region at the tip of the 50-kDa domain. Based on the structure, we modeled the binding of PBP to *Dictyostelium* myosin-1E and chicken myosin-5a. Functional assays confirmed the predictions from the modeling that PBP is most potent as an inhibitor of class-5 myosins, less active for class-2 myosins, and displays weaker activity with class-1 myosins.

### Identification of the PBP-binding site

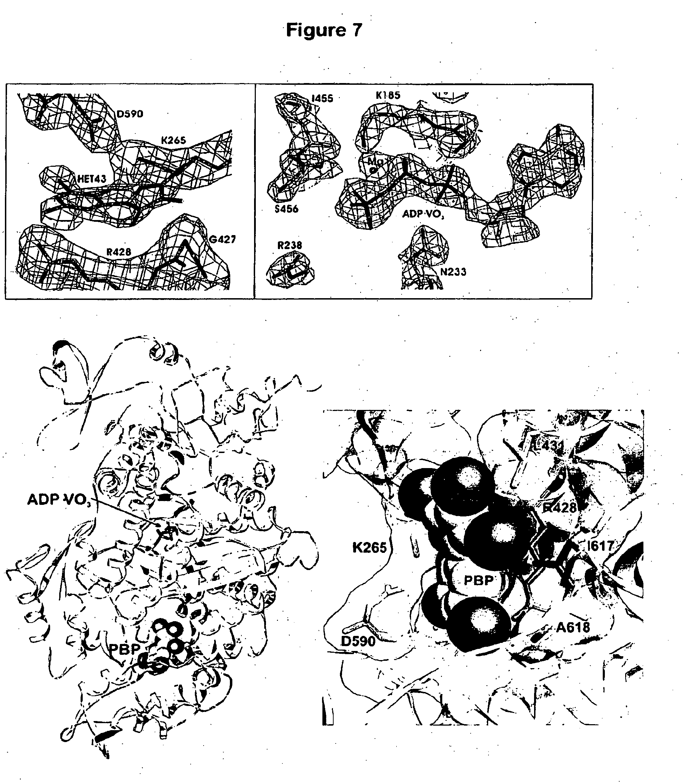

To identify the binding mode of PBP, we crystallized the *Dictyostelium* myosin-2 motor domain MgATP-metavanadate complex in the presence and absence of PBP. We solved the complex structures by molecular replacement and refined them to 2.1Å and 2.8Å resolution, respectively. The overall fold of the motor domain in both structures is similar to that reported for the prepowerstroke conformation. The α-carbon atoms in both structures superimpose with an r.m.s. deviation of 0.695. The electron density for PBP is unambiguous and shows the inhibitor in a conformation where the phenyl and pyrrole ring systems are bent by 12° out of plane and twisted by 20° against each other (Fig. 7). The inhibitor binds in a pocket that is formed by residues at the interphase between the 50kDa upper domain (U50) and the 50kDa lower domain (L50). The secondary structure elements that form the pocket include helix-13 (K265-V268), helix-21 (V411-L441), the strut loop (N588-Q593), loop.2 (D614-T629) and helix-29 (V630-E646). Both polar and polar residues contribute to the binding of PBP. The pocket has a large opening that is surrounded by residues from loop-2, helix-29, the loop connecting β7, the edge β-strand of the central β-sheet, with helix-13, and helix-21. Additionally, a smaller access channel is located between loop-2 and the strut loop. The total protein surface area in contact with PBP comprises 255Å².

Coordinates for the myosin-2 motor domain - ADP • VO₃ and myosin-2 motor domain - ADP • VO₃ - PBP complexes have been deposited in the Protein Data Bank (PDB) with the accession codes 2JJ9 and 2JHR, respectively.

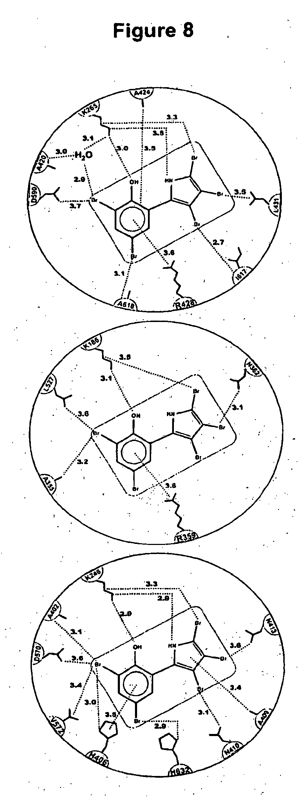

Binding of PBP requires several rearrangements of residues inside and near the binding pocket. The most extensive of these changes involve residue K265. To allow PBP to bind, the ammonium group of K265 needs to move 3.4A towards K423. The movement of K265 involves a change in rotamer and the stabilization of the side chain in its new position by an intricate network of interactions (Fig. 8). These include the formation of a key hydrogen bond with the hydroxyl group of the PBP phenol ring, a second hydrogen bond that is mediated via a water molecule from the ammonium group of K265 to the 2'-bromo group of phenyl ring, and contacts with the NH-group and the 2-bromo group of the pyrrol ring. Further stabilization is derived from a salt-bridge that forms between the ammonium group of K265 and the carboxyl group of D590. Formation of this salt-bridge requires a shift in the Cα-backbone at position D590. The side chains of A424, R428, and D590 and the main chain carbonyl of A420and A618 are involved in the binding of the phenyl-ring, whereas the pyrrol ring has additional interactions with the side chain of L431 and the main chain carbonyl of 1617. The carbonyl groups of 1617 and A618 are in hydrogen-bonding distance to the bromo substituents in positions 3 and 4', while the hydrogen bond between the carbonyl group of A420 and the 2'-bromo group is mediated by the same water molecule as the hydrogen bond between K265 and the hydroxyl group. The extent of side chain rearrangements associated with these interactions varies from 0.8 A for L431 to 3.5 A for R428. Residues R620 and Q633 are not in contact with PBP but their side chains undergo coordinated conformational changes, bringing them in positions were they form part of the large opening of the pocket. The guanidinium group moves 3.5 A due to a 90° rotation around the Cδ-Cε bond, while a rotation around the Cα-Cβ bond leads to a 1.5 A shift in the position of the side chain of Q633.

### Specificity for myosin isoforms

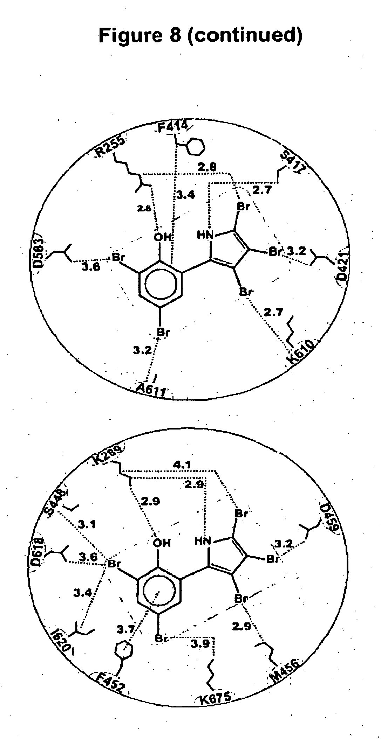

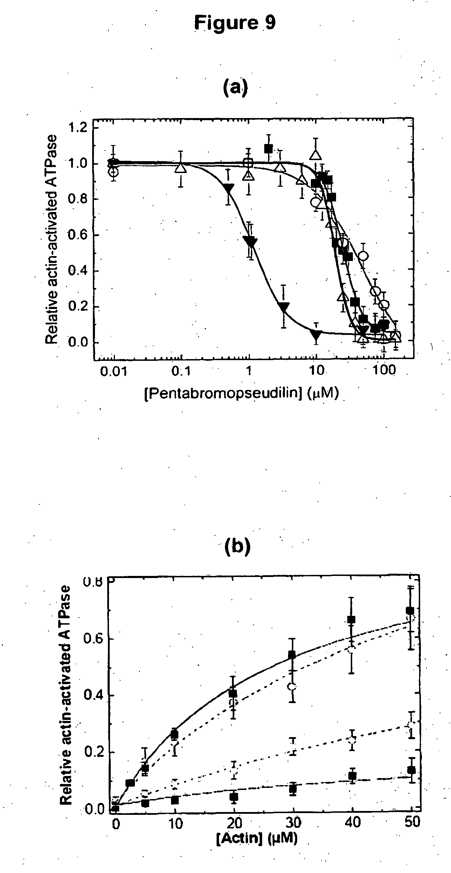

Based on the structure of the myosin-2 motor domain with bound PBP, we performed molecular modeling studies to elucidate the specificity of PBP-binding to myosins from different classes. Here, we show the results for then modeling of complexes formed with class-1 and class-5 myosins, representing isoforms that are predicted to interact considerably stronger and weaker with the inhibitor **(****Fig. 8****).** The interaction between the lysine residue corresponding to K265 in myosin-2 is conserved in the other myosins. However, the number of interactions and their strength varies considerably between individual myosins. Myosin-1E is predicted to form only three hydrogen bonds and three weaker interactions, whereas myosin-5a is predicted to form twice as many interactions. To test the predictions from the modeling studies, we determined actin-activated ATPase activities in the presence of 0 to 150 µM PBP with rabbit skeletal myosin-2 heavy meromyosin (HMM), myosin-5a isolated from chicken brain, and recombinant *Dictyostelium* myosin class-1, 2 and 5 motor-domain constructs (Fig. 9a). The semi-logarithmic plot shows the concentration dependence for myosin-1E (o), myosin-2 (■), myosin-5b (Δ) from *D*. *discoideum,* and for chicken myosin-5a (▼). The concentrations of PBP required for half-maximal inhibition of the different myosin motors was calculated from fitting the data to hyperbolae. The strongest inhibition was observe for chicken myosin-5a *Kᵢ* = 1.19 µM ± 0.20), followed by *Dictyostelium* myosin-5b (*Kᵢ* = 19.3 µM ± 0.85), *Dictyostelium* myosin-2 (*Kᵢ* = 24 µM ± 1.5), HMM (*Kᵢ* = 23 µM ± 5), and *Dictyostelium* myosin-1E (*Kᵢ* = 49.5 µM ± 1.5) **(****Fig. 9a****).** The ATPase activity at 30 µM F-actin decreased in the presence of > 100 µM PBP for chicken myosin-5a from 8.5 s⁻¹ to 0.34 s⁻¹, for *Dictyostelium* myosin-5b from 5.5 s⁻¹ to 0.05 s⁻¹, for *Dictyostelium* myosin-2 from 0.7 s⁻¹ to 0.02 s⁻¹, and for *Dictyostelium* myosin-1E from 3.5 s⁻¹ to 0.10 s⁻¹. To determine the extend to which PBP impairs the coupling between the actin- and nucleotide-binding sites of myosin, we measured the ATP turn-over rates of *Dictyostelium* myosin-2 and myosin-5b motor domain constructs over the range from 0 to 50 µM F-actin in the absence and presence of 25 µM PBP.

Values for the rate of maximum ATP turnover (kcat), the concentration of F-actin at which half-maximal activation is achieved (Kapp), and the apparent second order rate constant for actin binding (kcat/Kapp) were estimated from a fit of the data to a hyperbolic function. kcat/Kapp is a direct measure of the coupling efficiency between actin and nucleotide binding and can be determined from the initial slope at F-actin concentrations much smaller than Kapp . Coupling was 26-fold weaker for myosin-5b (0.32 µM-1s-1/0.012.µM-1s-1), whereas a 3.7-fold reduction in coupling was observed for myosin-2 (0.037 µM-1s-1/ 0.010 µM-1s-1). ATP turn-over rates of Dictyostelium myosin-2 (o) and Dictyostelium myosin-5b (■) at increasing F-actin concentrations in the absence (filled symbol) and presence (open symbols) of 25 µM pentabromopseudilin **(****Fig. 9b****).**

### ATP binding kinetics

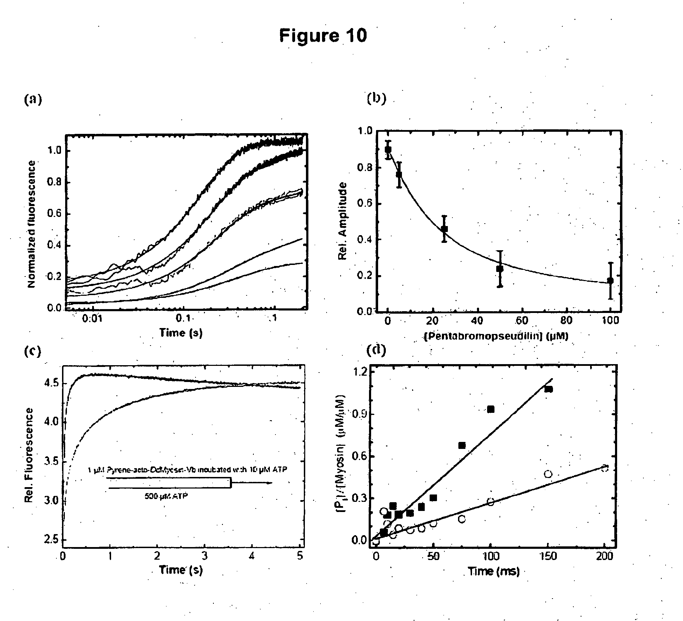

Effect of Pentabromopseudilin on ATP binding kinetics to *Dd*Myosin-5b and acto-*Dd*Myosin-5b. **(****Fig. 10a****)** Fluorescence transients obtained upon mixing 1 µM *Dd*Myosin-5b with 10 µM mantATP in the absence and presence of 5 µM, 25 µM, 50µM and 100 µM PBP. Mant-fluorescence was excited at 365 nm and detected after passing through a KV 389 nm cut-off filter. The fluorescent signal of mantATP binding to *Dd*Myosin-5b in the absence of PBP follows a single exponential function (upper curve). The presence of increasing concentrations of PBP lead to reduced amplitudes of the fluorescent signals that are best fit by double exponentials. **(****Fig. 10b****)** Plot of the relative amplitudes of mantATP binding to Dd Myosin-5b at different PBP concentrations. A hyperbolic fit to the data yields an apparent constant K_{d} of 21 ± 2 µM that is well consistent with the Kᵢ value (19.14 µM) determined from the steady state ATPase measurements in figure 9b .**(****Fig. 10c****)** Fluorescence transients of the ATP induced dissociation of pyrene-acto-*Dd*Myosin-5b in the absence (black curve) and presence (grey curve) of 50 µM PBP. **(****Fig. 10d****)** Quench-flow experiments of the ATP hydrolysis reaction upon mixing 5 µM M761 with 25 µM ATP in the absence (solid aquares) and presence (open circles) of 50 µM PBP. The Pᵢ-burst (k_{burst} = 7.8 ± 0.2 s⁻¹ in the absence and 2.5 ± 0.1 s⁻¹ in the presence of PBP) was followed by a linear steady-state phase. The amplitude of the burst was 0.95 in the absence and 0.5 in the presence of PBP.

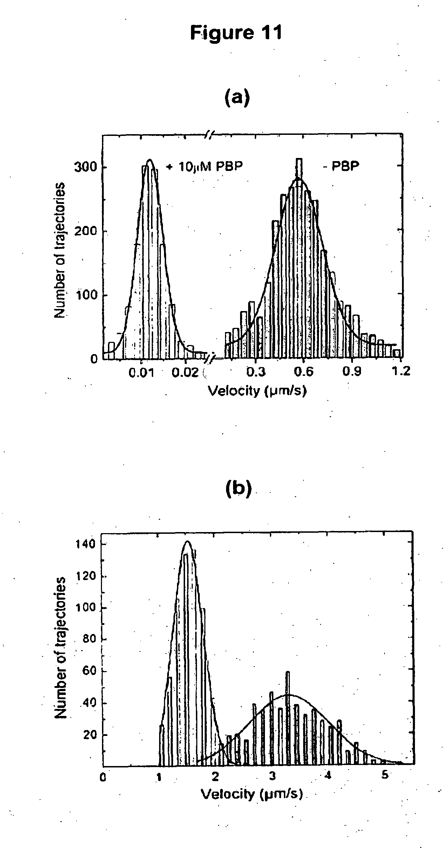

The extent and order of isoform-specific differences in the potency of the inhibitor were additionally confirmed by the results of *in vitro* motility assays.). The histograms shows the average sliding velocity of Rh/Ph-labeled actin filaments in the absence and presence of 10 µM PBP. **(****Fig. 11a****)** Myosin-5a moves actin filaments with an average velocity of 0.6 µm/s ± 0.05 (dark bars); addition of 10 µM PBP into the same flow cell lead to a 46-fold reduction of the average sliding velocity to 0.013 µm/s ± 0.002 (light grey bars). A 2-to-3-fold reduction in the average gliding velocity was observed for myosin-2 at 100 µM PBP. concentrations **(****Fig 11b****).** No significant inhibition was seen with myosin-1E (data not shown).

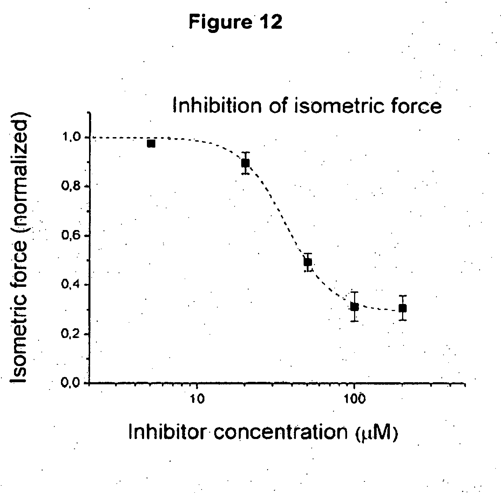

To test the effect of PBP on fully assembled myofilament arrays, we examined the contractile properties of skinned muscle fibre preparations from rabbit psoas muscle. Pentabromopseudilin leads to a more than 4-fold reduction in isometric force development of skinned muscle fibre preparations from rabbit psoas muscle. The Ki is in the same range as that determined for actomyosin ATPase activity. **(****Fig. 12****)**

### Inhibition of myosin-5-dependent cellular processes

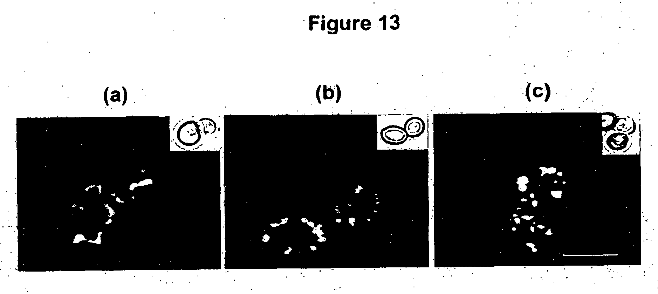

To test the specific inhibition of myosin-5 function in a cellular context, we exposed *Saccharomyces cerevisiae* cells to 100 to 500 nM PBP. S. *cerevisiae* has five myosin heavy chain genes: a myosin-2, two class-1 myosins, and the class-5 myosins Myo2p and Myo4p. Myo2p plays a crucial role in polarized distribution of mitochondria and is required for retention of newly inherited mitochondria in yeast cells during cell division⁴⁻⁶. Myo4p is required for mRNA transport and facilitates movement of ER tubules into the growing bud. Myo4p null mutants are viable and display no detectable phenotype. Deletion of the *myo3* and *myo5* genes, encoding class-1 myosins, leads to severe defects in growth and actin cytoskeletal organization. Severe growth defects are also observed upon depletion of the class-2 myosin Myo1p. Therefore, we expected that selective inhibition of class-5 myosins will produce readily detectable changes in the morphology of mitochondria without affecting cell growth and cytokinesis. To facilitate the visual inspection of mitochondrial morphology, we transfected the cells used for these experiments with an expression vector for the production of mitochondria-targeted GFP⁷. A yeast mutant strain with *myo2* expression under the control of the TetO₇ promoter was used as control⁸. Repression of the TetO₇ promoter by the addition of 10µg/ml deoxycycline to the culture medium of this strain led to an apparent fragmentation of mitochondria. Similar phenotypic changes were observed when wild-type cells were exposed to 500 nM PBP for 12 hours (Fig.13c). At lower concentrations the phenotype was less severe and at 100 nM PBP the mitochondrial morphology closely resembled that of untreated wild-type-cells. Yeast cells exposed to 500 nM PBP showed similar growth kinetics as the wild type control cultures. These observations confirm the more potent inhibition of class-5 myosins observed the *in vitro* experiments and predicted by the *in silico* studies.

The results from the actin-activated ATPase measurements clearly demonstrate that PBP impairs the ability of myosins to effectively interact with nucleotides and actin; however the extent of inhibition by PBP is quite different for the individual myosins. The strongest inhibition was observed for class-5 myosins. In this case, the inhibitor caused a more than 25-fold increase in Kₐₚₚ and an approximately 8-fold decrease in k_{cat}, while the same Michaelis-Menten parameters were less affected for a corresponding myosin-2 motor-domain construct.

### Methods

*Protein preparation.* Rabbit fast skeletal muscle heavy meromyosin (HMM) was prepared as described by Kron and Spudich⁹. Motor domain constructs of myosin-1E, myosin-2, and myosin-5b from *Dictyostelium discoideum* comprising amino acids 1-698, 1-765, and 1-839 respectively, were prepared as described previously¹⁰⁻¹². Myosin-5a heavy meromyosin from chicken brain was provided by Dr. T. Scholz (Hannover Medical School, Germany) and F-actin was prepared as described by Lehrer and Kerwar¹³.

Crystallization experiments were performed with myosin motor domain construct M761-c14 consisting of an N-terminal His-tag, amino acids 3 to 761 of myosin-2 from *D. discoideum* followed by a leucine and a glutamate residue and the 14 C-terminal residues of EcoSSB¹⁴. The protein was expressed in D. *discoideum* AX3-ORF+ cells and purified by Ni2⁺-chelate affinity chromatography as described previously¹⁵. Subsequently the protein was applied onto a Resource Q column (GE Healthcare) equilibrated with storage buffer (1 mM magnesium acetate, 0.5 mM EDTA, 0.2 mM EGTA, 1 mM benzamidine, 1 mM DTT, 50 mM Tris/HCl pH 7.5), followed by an elution with a gradient from 0 to 0.5 M KCl. The peak fractions were concentrated to 10 mg/ml using ultrafiltration (Vivaspin 20, Vivascience) and dialyzed against storage buffer containing 3% (w/v) sucrose. Aliquots of 100 µl were flash frozen in liquid nitrogen and stored at -80°C.

*Synthesis of halogenated pseudilins.* PBP and the other halogenated pseudilin derivates used in this study were synthesized using silver(I)-catalyzed pyrrole synthesis for the cyclization reaction to the heterocyclic system as described previously¹⁶.

*Inhibitor screening.* To screen compound libraries for myosin effectors, colorimetric high-throughput assays for myosin ATPase activity were performed with heavy meromyosin (HMM) prepared from rabbit skeletal muscle myosin-2. *N*_{σ}-Tosyl-L-lysine chloromethyl ketone hydrochloride treated σ-chymotrypsin was used for the generation of HMM. Each reaction mixture including the controls contained 2.5% DMSO that was used as solvent for the small organic compounds. Reactions containing HMM (0,01mg/ml), assay buffer (50mM KCI, 5mM CaCl₂, 25mM Tris-HCl pH 7.5) and 25µM of the respective inhibitor were started by the addition of 50µM ATP and incubated in for 20 min at 37°C. Reactions were terminated by the addition of Biomol Green (Biomol, Hamburg, Germany). The amount of dye formed after 20 min color development at room temperature was determined with a Tecan Infinite™ microplate reader (Crailsheim, Germany).

*Crystallizafion and data collection.* The complex of M761-c14 with HET43 and ADP-VO₃ was obtained by adding 2mM of PBP, MgCl₂, ADP and sodium meta-vanadate to the protein solution and incubation on ice for 1 hour. Crystals of the complex were grown in the dark at 4°C using the hanging drop geometry. 2 µl of complex solution and 2 µl of reservoir solution were mixed, the latter contained 50mM HEPES pH 7.4, 140mM NaCl, 11% w/v PEG8000, 2% (v/v) MPD, 5mM MgCl₂, 5mM DTT, and 1mM EGTA. Rectangular shaped crystals appeared within 3 weeks. Prior to diffraction data collection the crystals were soaked for 5 minutes at 4°C in a cryo-protection solution containing reservoir solution supplemented with 25% ethylene glycol, 2mM sodium meta-vanadate, 2mM ADP and 2mM HET43. Subsequently, crystals were flash-cooled in liquid nitrogen. Diffraction data were collected in house using a Bruker X8PROTEUM equipped with cryo-system, κ-goniometer and CCD-detector. We processed the data with PROTEUM2 software (Bruker AXS).

*Molecular modeling.* The motor domain structures of *Dictyostelium* myosin-1 E (PDB entry 1 LKX) and chicken myosin-5A (PDB entry 1OE9) were used to model the complexes of class-1 and class-5 myosins with Het43. Initial models were created by inserting HET43 coordinate from superimposed myo2-HET43 complex. Next molecular mechanics energy minimization procedures were performed for HET43 in the new protein environment using the CNS software suit¹⁷. The resulting structures were subjected to a more precise quantum chemical energy minimization procedure, using *ab initio* DFT QM/MM calculations with the 6-31G* basis set of atomic orbitals and B3LYP hybrid functional. The quantum chemical calculations were performed using the PC GAMESS version developed by Alex A. Granovsky (http://www.classic.chem.msu.su/gran/gamess/index.html) of the GAMESS (US) Quantum Chemistry package¹⁸.

*Steady-state kinetics.* Basal and actin-activated Mg²⁺-ATPase activities were measured with the NADH-coupled assay¹⁹ in a buffer containing 25 mM HEPES, 25 mM KCl, and 4 mM MgCl₂, 0.5 mM DTT at pH 7.0 in the presence of 1 mM ATP at 25 °C. PBP was added to the reaction mixture in the absence of nucleotide and incubated for 20 minutes before the reaction was started by the addition of ATP. NADH oxidation was followed using the change in absorption at 340 nm in a Beckman DU-800 spectrophotometer.

*Transient kinetic experiments*: Stopped-flow measurements were performed with an Applied Photophysics PiStar Instrument in a buffer containing. 25 mM HEPES, 25 mM KCI, 1 mM DTT, 4 mM MgCl2, pH 7.0 at 20°C using procedures and kinetic models described previously (1-3). The binding and hydrolysis of ATP and mantATP, respectively by myosin head fragments were analyzed in terms of the seven-step model described by Bagshaw and coworkers (4). Transients in the presence of actin were analyzed according to the mechanisms described in references 5 and 6.

*Direct functional Assays.* Actin-sliding motility was performed at 25 °C using an Olympus IX81 inverted fluorescence microscope. Experimental flow cells were constructed using bovine serum albumin (0.5 mg/ml in assay buffer)-coated glass slides and nitrocellulose-coated coverslips. Myosins were actin affinity-purified immediately before use, to remove, rigor heads²⁰. Sliding movement was started by adding assay buffer containing 4 mM ATP, 10 mM DTT, 0.5 mg/ml bovine serum albumin, anti-fade solution, and 0.5% methylcellulose to the flow cell. Average sliding velocity was determined from the Gaussian distribution of automatically tracked actin filaments using DiaTrack 3.0 and Origin 7.0.

*Quench-flow Experiments.* Experiments were performed in a BioLogic QFM-400 apparatus. 20 µl of a 50 µM γ-³³P-ATP solution (0.3 µCi/µl) were mixed with an equal amount of 10 µM myosin solution in the absence and presence of 50 µM PBP in the assay buffer (25 mM HEPES, pH 73, 4 mM MgC12, 1 mM DTT, and 25 mM KCI at 25 °C). The reaction was quenched after a well defined period of time by the addition of 176 µl 1 M perchloric acid and rapidly neutralized by 60 µl of an 8 M KOAc. After centrifugation, 2 µl aliquots of the supernatant were spotted on a thin-layer chromatography (TLC) plate (Polygram CEL 300 PEI, cellose-molyethylenimin, Machery-Nagel). The hydrolysis products were separated in a 1.2 M LiCl solution containing 1 M HCOOH. The relative amounts of Pᵢ and ATP were quantified using a phosphorimager (Fujix BAS 1000, Fuji), the software MacBAS V 2.4, TINA V 2.09f, and Origin 7.0.

*Myo2p-dependent changes in mitochondrial morphology.* The predicted strong inhibitory effect of PDP on myosin activity was tested *in vivo,* making use of the phenotypic changes induced by the depletion of the class-5 myosin Myo2p in S. *cerevisiae.* Yeast strains producing mitochondria-targeted GFP were supplied by Dr. B. Westermann⁷, allowing us to study the morphology of yeast mitochondria in a fluorescence microscope. Repression of the TetO₇ promoter that controls *myo2* in one of the mutant strains was achieved by the addition of 10µg/ml deoxycycline to the culture medium⁸. Cells were analyzed at the end of the logarithmic growth phase. Yeast cells exposed to 500 nM PDP showed almost the same growth kinetics as wild type control cultures. Addition of 0.05% DMSO used as solvent vehicle for PBP had no effect on growth. Images of the mitochondrial phenotype were acquired using a Zeiss confocal microscope (LSM 510) equipped with a 63x oil immersion objective. Additional images of the same cells in transmitted light were acquired using a Hamamatsu ORCA camera.

**Table 2. Data collection, structure solution and refinement statistics for the ternary myosin-2 motor domain - ADP•VO₃- Pentabromopseudilin complex**

| **Complex** | **Myosin**-**2** - **ADP•VO₃**-**Pentabromopseudilin** |
|---|---|
| *Crystal parameters* | |
| Group | *C222,* |
| Cell parameters: a, b, c; α, β, γ | 89.8, 150.5, 154.6; 90, 90, 90 |
| *Data collection* | |
| X-ray source | X8PROTEUM, Bruker |
| Wave length (Å) | 1.54178 |
| Resolution of data (Å) | 2.8 |
| No. of observations / unique reflections | 134833 / 25708 |
| Completeness (total/high) % | 98.2/95.5 |
| <I/σ(I)> (total/high) | 10.9/2.4 |
| R_{sym} (total/high) % | 8.8/40.4 |

| ***Refinement*** | |
|---|---|
| Resolution range (Å) | 8.0-2.8 |
| Included amino acids | 776 |
| No. of protein atoms | 6240 |
| No. of waters | 475 |
| R_{work} / R_{free} % | 21.7/26.5 |
| r.m.s. deviation for bonds (Å) / angles (deg) | 0.009/1.4 |

### Example 3

### Selectivity for myosin isoforms

KIN-43 shows preferred selectivity for mammalian myosin-5a, KIN-68 displays preferred selectivity for myosin-1 isoforms. In this context, preferred selectivity means a more than 20-fold smaller IC50 value compared to fast skeletal muscle myosin-2 and other references myosins.

### Example 4

### Activators and uncouplers of myosin function

Compounds KIN-74, KIN-77 are activators of myosin-2 function. KIN-74 activates the ATPase activity of *Dictyostelium* myosin-2 in the absence of actin. ATPase activity is uncoupled from motor activity in this situation. The apparent half-maximum activation constant AC₅₀ corresponds to 18 µM. Uncouplers have potential applications in inhibiting the overcontraction and overextension of cardiac muscles without being accompanied with a cardioinhibitory action. Therefore, compounds derived from KIN-74 have potential applications in the treatment of acute myocardial infarction helping to prevent the necrosis of cardiac muscles.

KIN-77 activates the actin-activated ATPase and motor activity of rabbit myosin-2 with a half maximum activation concentration Also = 19.2 µM. KIN-77 is an ideal lead compounds for the development of optimized activators of myosin motor activity. Optimized activators have potential applications in diseases where myosin motility is impaired and contractility needs to be restored, as in the case of cardiomyopathies and congestive heart failures.

### Example 5

### KIN-93 is an inhibitor of myosin-14-dependet motility in Plasmodium

*Plasmodium* sporozoites are moving at very low speed in the salivary glands of infected mosquitoes but are moving at high speed (2µm/s) upon transmission into the vertebrate host. In vivo microscopy showed that sporozoites can move extensively within the dermis and when associated with blood and lymph vessels, which they can both invade. We followed single parasites using in vitro imaging approaches in combination with our bioactive compounds and discovered some intriguing features of sporozoite motility. Under normal conditions sporozoites move in circles, either counterclockwise or clockwise. Two other states of attachment and waving are less populated. In the presence of 10 µM KIN-93, the number of sporozoite that followed counterclockwise or clockwise movement was strongly reduced. In addition, their gliding velocity was only half as fast as compared with control parasites. In addition to sporozoite impairment, *in vitro* growth assays indicate that the compound applied at concentration of 25 µM has an effect on the blood stages of malaria, too, by reducing the number of *Plasmodium* merozoites. No general cytotoxicity for erythrocytes or for *Plasmodium* sporozoites was recorded in any of the assays.

### Example 6

### Optimization of pharmacological properties by in silico assisted methods

Based on the X-ray structures of myosin-2 motor domains in complex with KIN-43 and related inhibitors of myosin function, we have gained a detailed understanding of the pharmacophore requirements of the allosteric binding site. By applying *in silico* assisted methods, we optimized the pharmacological properties of the initial bioactive compounds. We are using a library of common organic molecules and functional groups and are applying an empirical force field description of the nonbonding interactions between a ligand and the binding site in order to build up *de novo* compounds with spatial and electrostatic properties complementary to the allosteric binding site. KIN-93 is the result of a target directed drug design that shows improved pharmacological properties in relation to KIN-43. Cell viability is vastly improved in the presence of KIN-93. Neutral red uptake experiments do not reveal any defects. In addition, Medaka fish-based assays show that embryonic development is not impaired in the presence of KIN-93.

### Examples 7

### Structure of the myosin-2-KIN79 co-crystal

The pdb coordinate file below shows the structure of the complex of the carbazole KIN-79 with myosin-2.

| | |
|---|---|
| REMARK | coordinates from minimization refinement |
| REMARK | refinement resolution: 500.0 - 2.2 A |
| REMARK | starting r= 0.3111 free_r= 0.3624 |
| REMARK | final r= 0.3067 free_r= 0.3617 |
| REMARK | rmsd bonds= 0.008031 rmsd angles= 1.30849 |
| REMARK | wa= 4.4505 |
| REMARK | target= mlf cycles= 1 steps= 200 |
| REMARK | sg= C222(1) a= 88.18 b= 149.90 c= 154.27 alpha= 90.0 beta= 90.0 gamma= 90 |
| REMARK | parameter file 1 : CNS_TOPPAR:protein_rep.param |
| REMARK | parameter file 2 : CNS_TOPPAR:ion.param |
| REMARK | parameter file 5 : |
| REMARK | CNS_TOPPAR:water_rep.param molecular structure file: gen.mtf |
| REMARK | input coordinates: anneal_1.pdb |
| REMARK | reflection file= myo2het79.cv |
| REMARK | ncs= none |
| REMARK | B-correction resolution: 6.0 - 2.2 |
| REMARK | initial B-factor correction applied to fobs : |
| REMARK | B11= 0.541 B22=-12.873 B33= 12.332 |
| REMARK | B12= 0.000 B13= 0.000 B23= 0.000 |
| REMARK | B-factor correction applied to coordinate array B: -2.640 |
| REMARK | bulk solvent: density level=-0.319696 e/A^3, B-factor= 33.6052 A^2 |
| REMARK | reflections with \|Fobs\|/sigma_F < 0.0 rejected |
| REMARK | reflections with \|Fobs\| > 10000 * rms(Fobs) rejected |
| REMARK | theoretical total number of refl. in resol. range: 52178 (100.0 %) |
| REMARK | number of unobserved reflections (no entry or \|F\|=0): 81 (0.2%) |
| REMARK | number of reflections rejected: 0(0.0%) |
| REMARK | total number of reflections used: 52097 (99.8 %) |
| REMARK | number of reflections in working set: 49492 (94.9%) |
| REMARK | number of reflections in test set: 2605 (5.0%) |

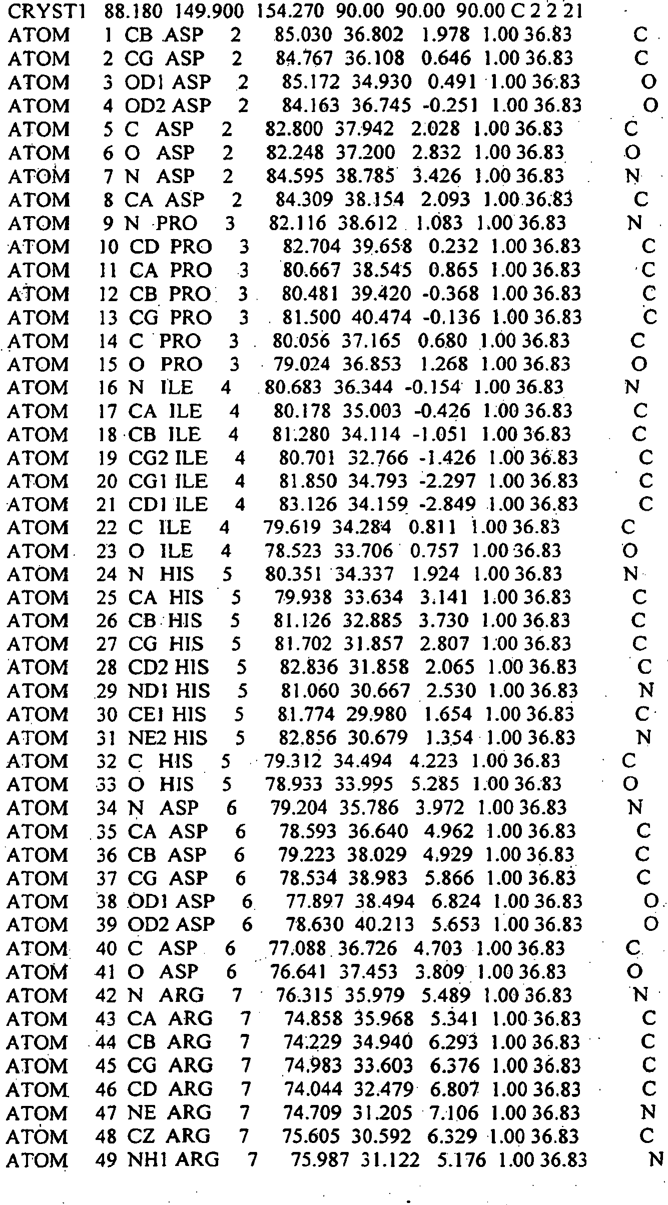

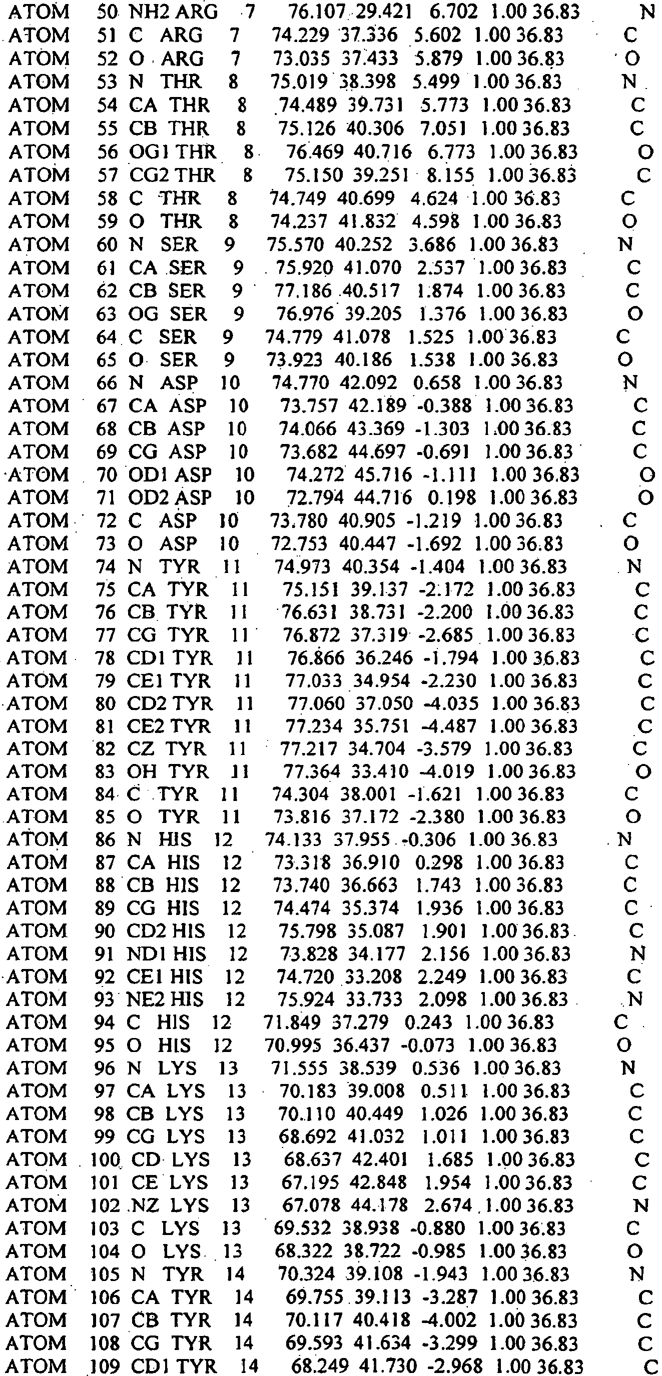

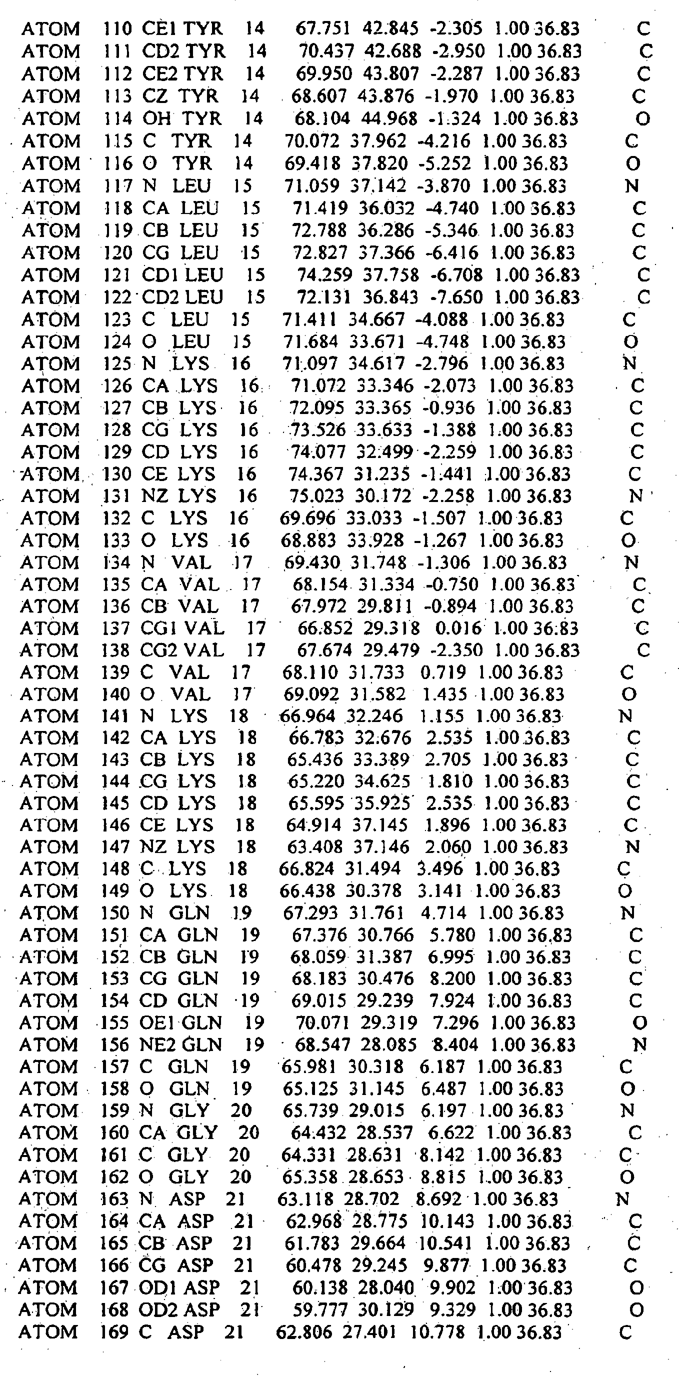

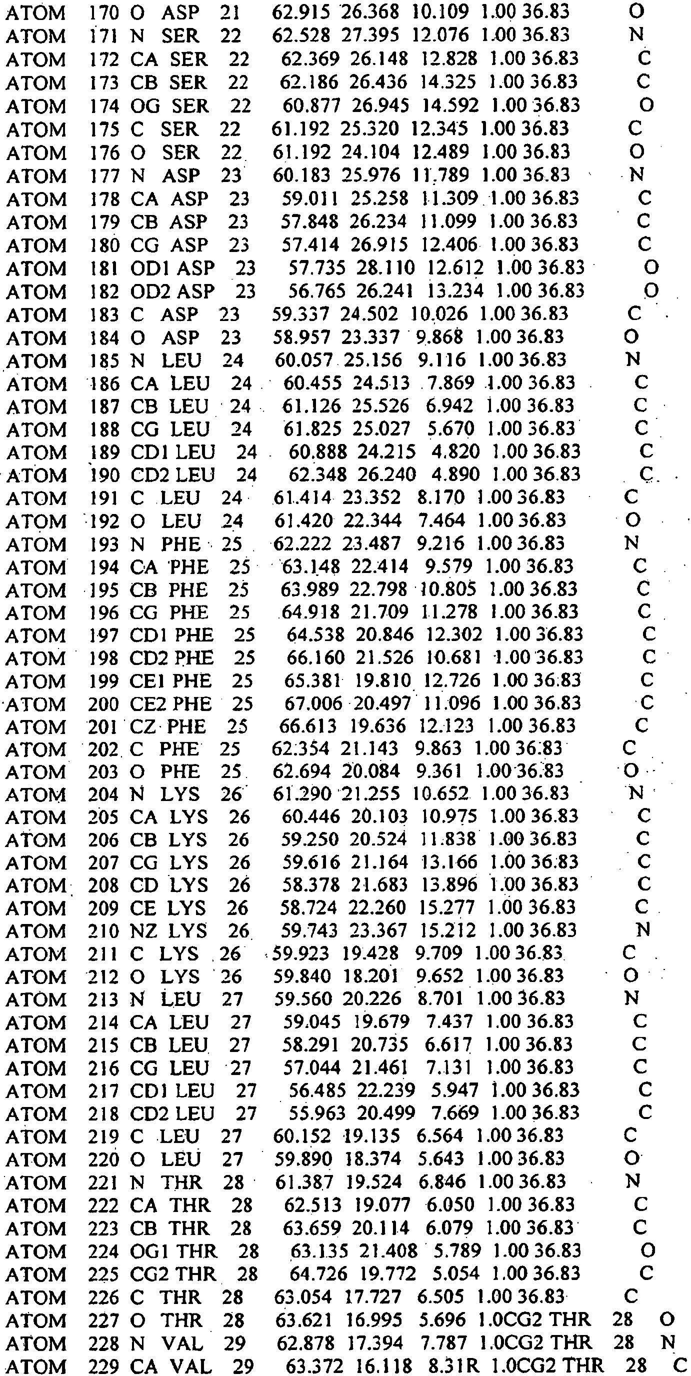

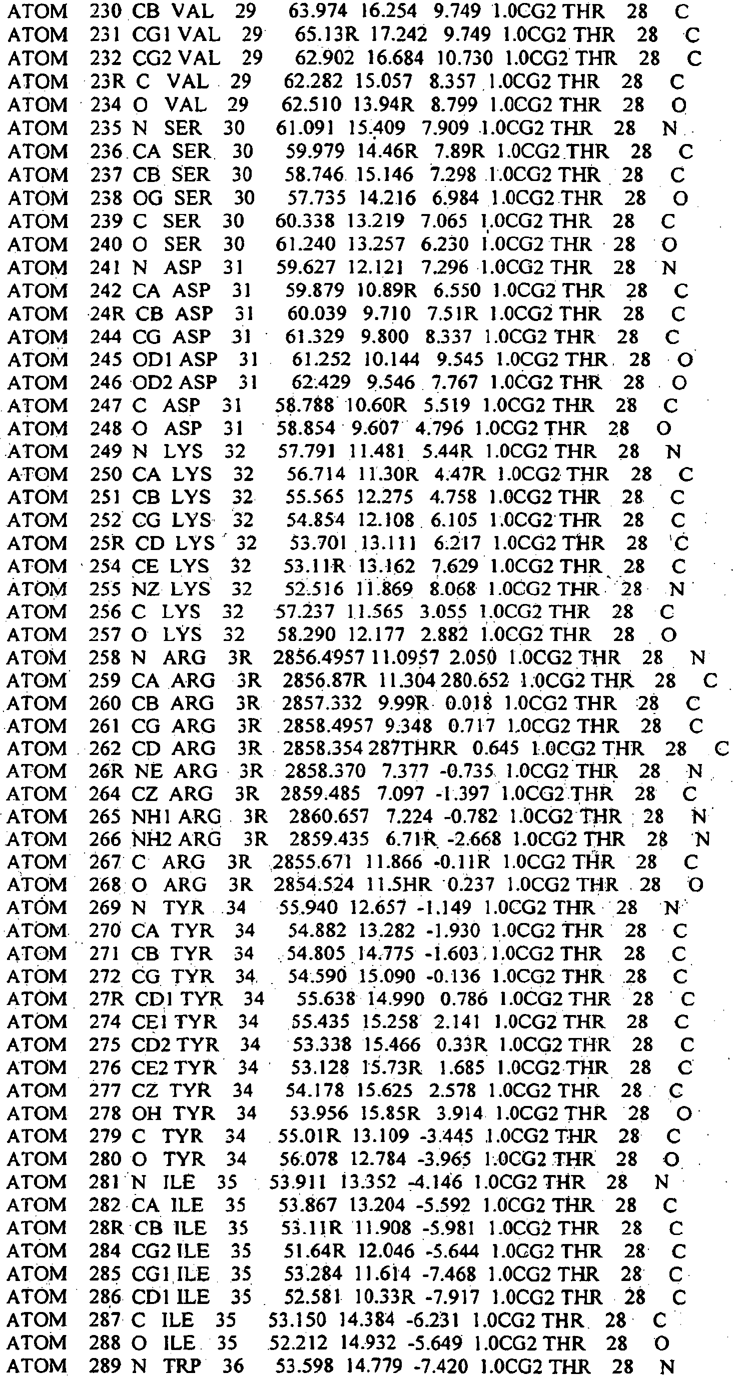

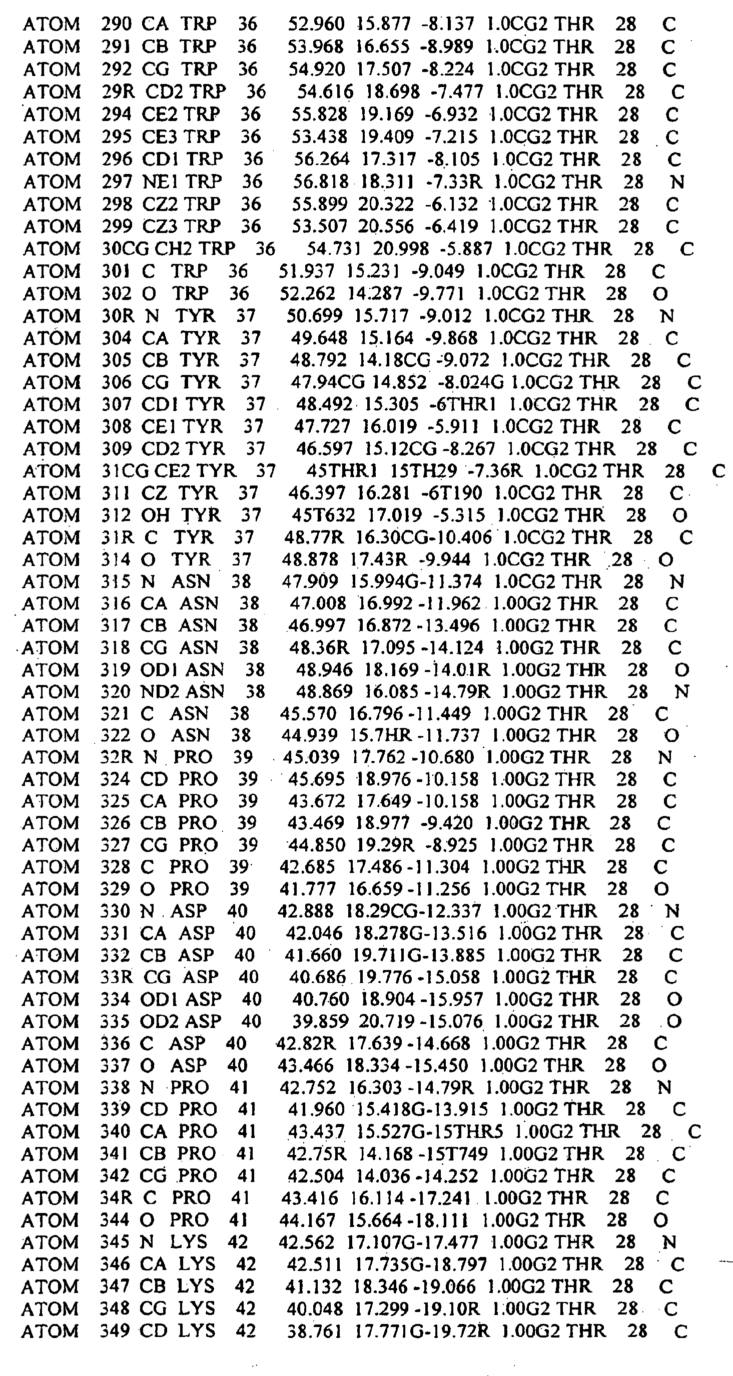

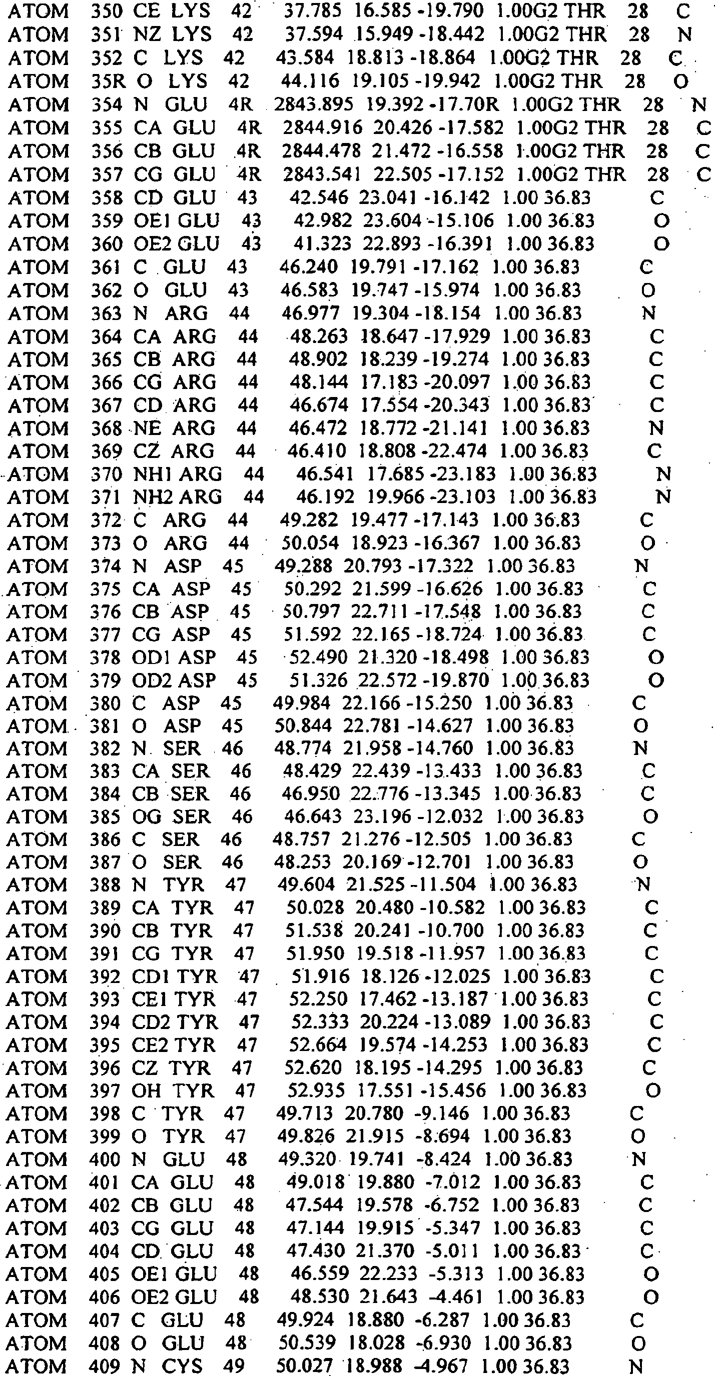

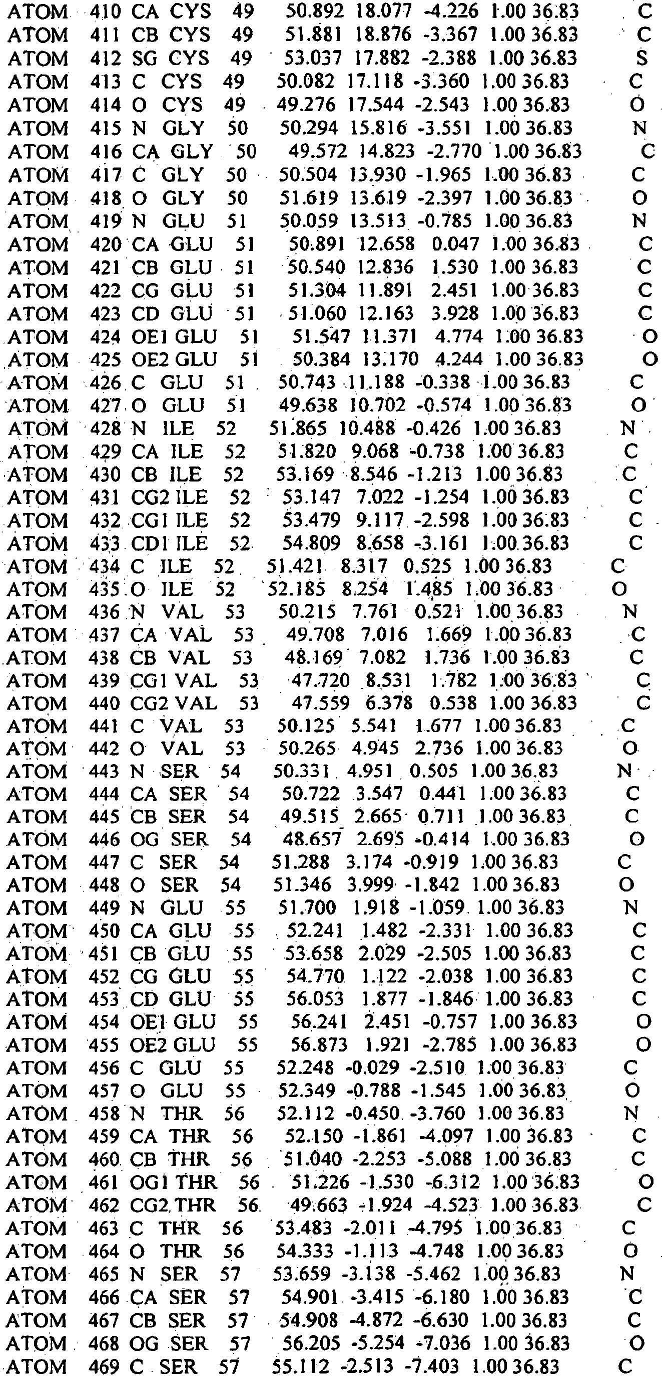

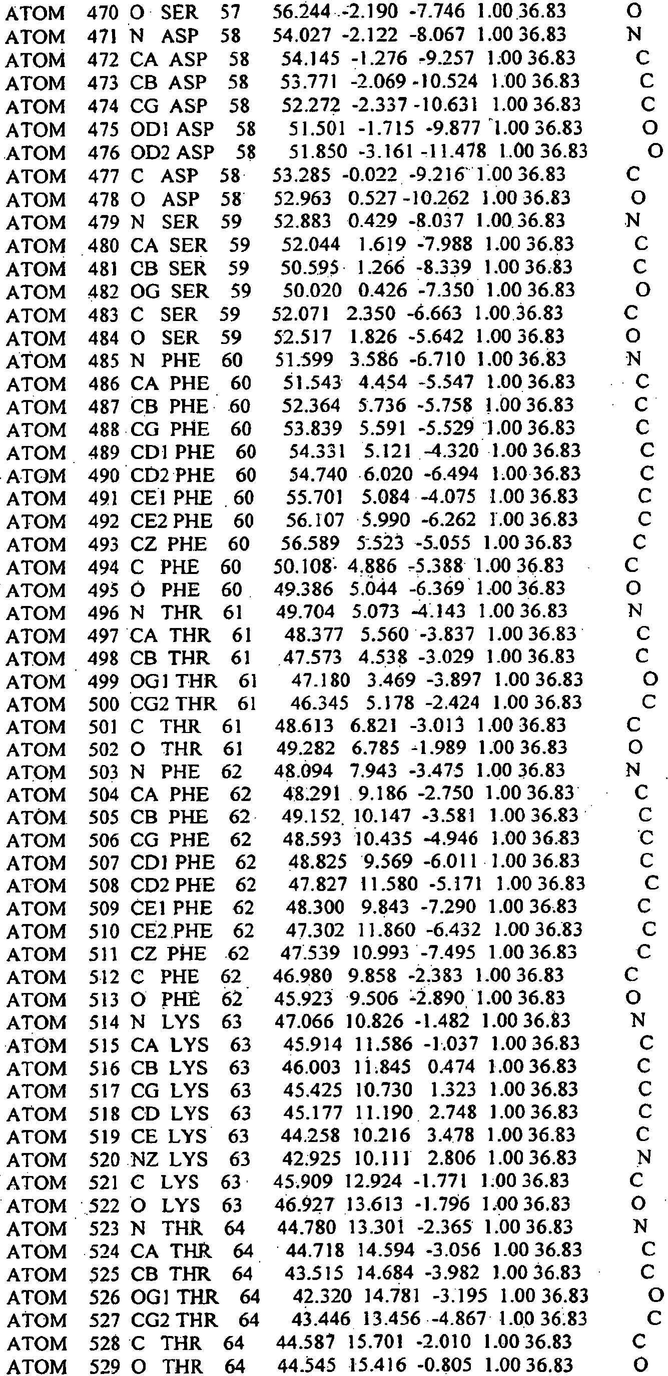

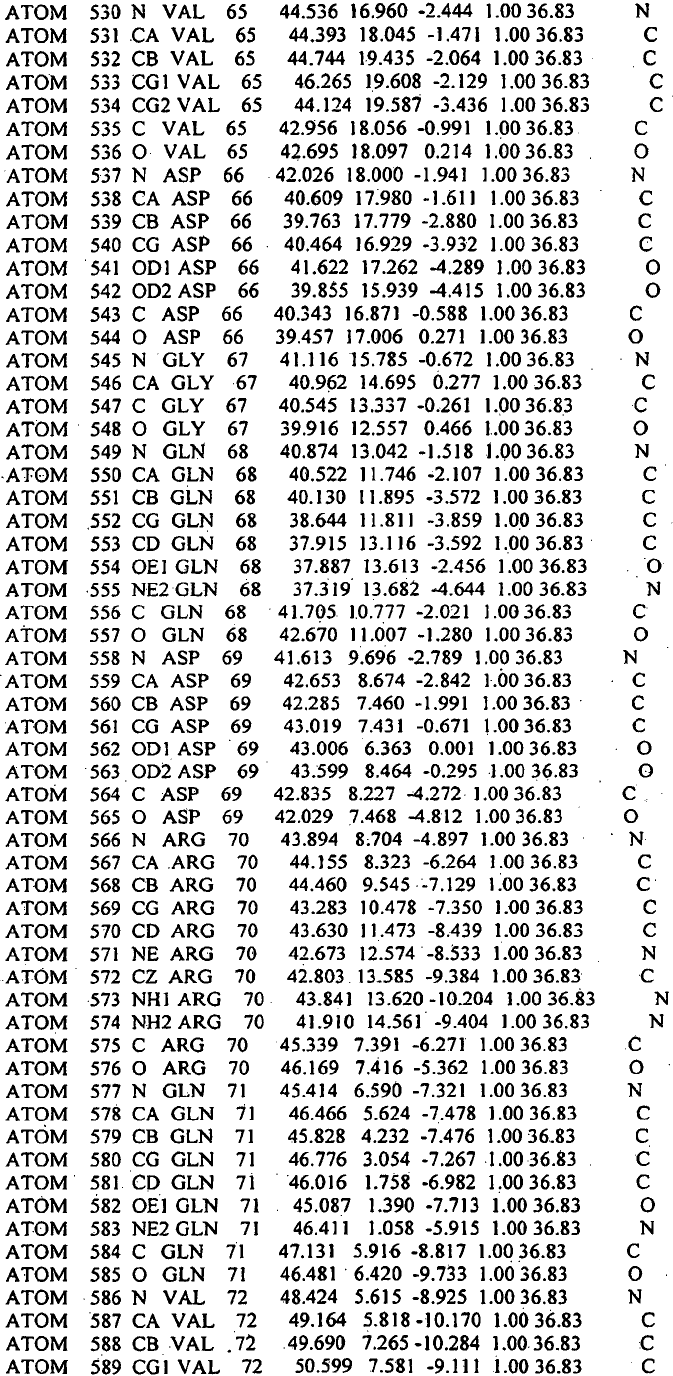

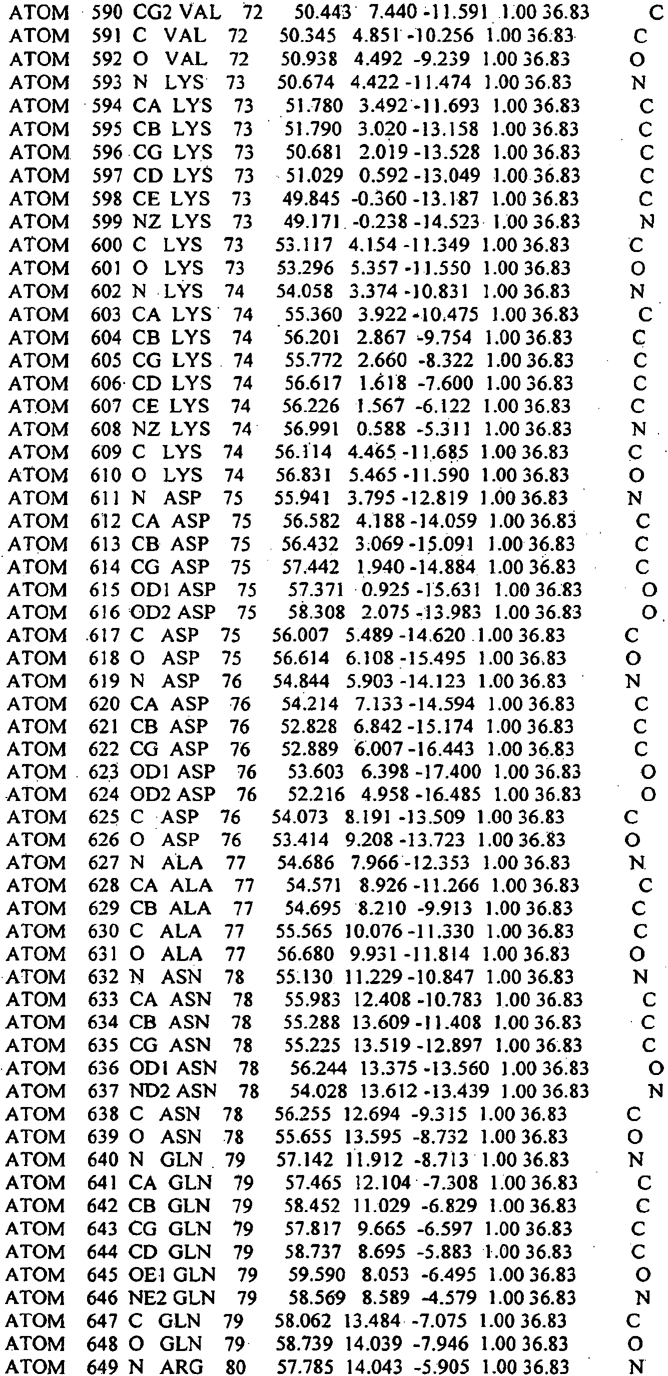

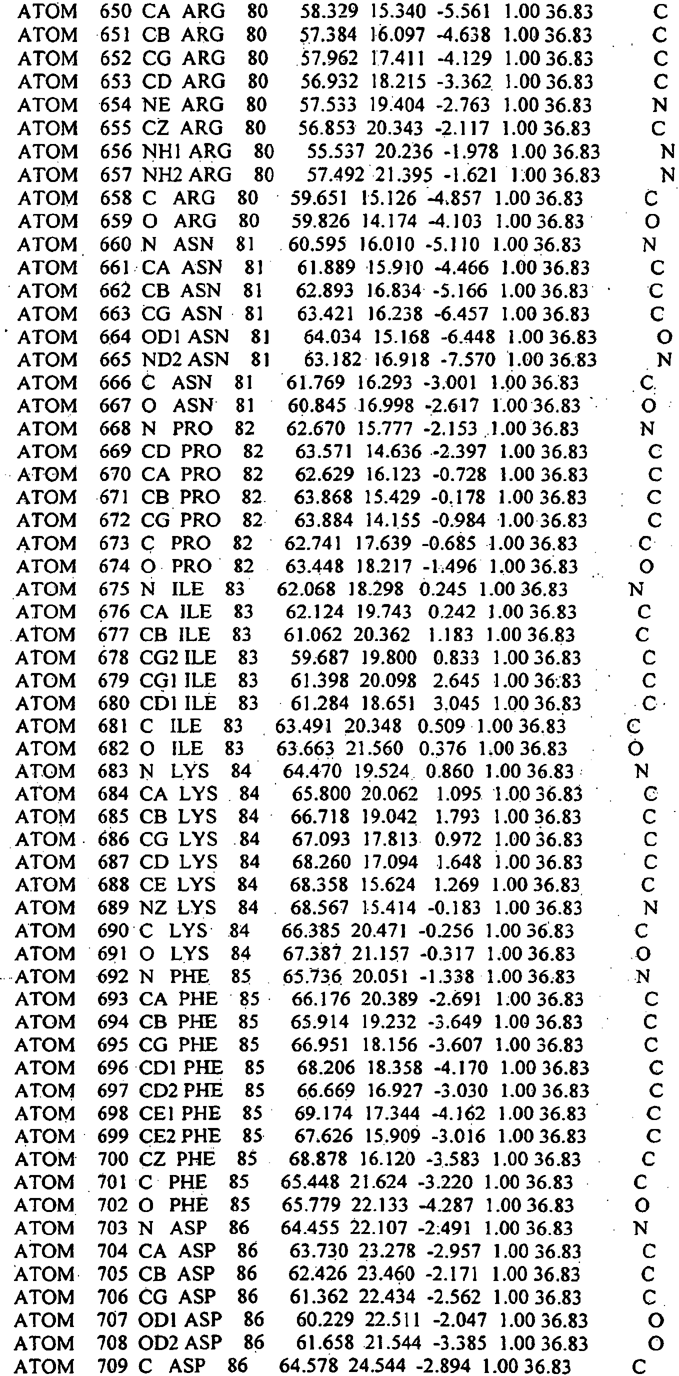

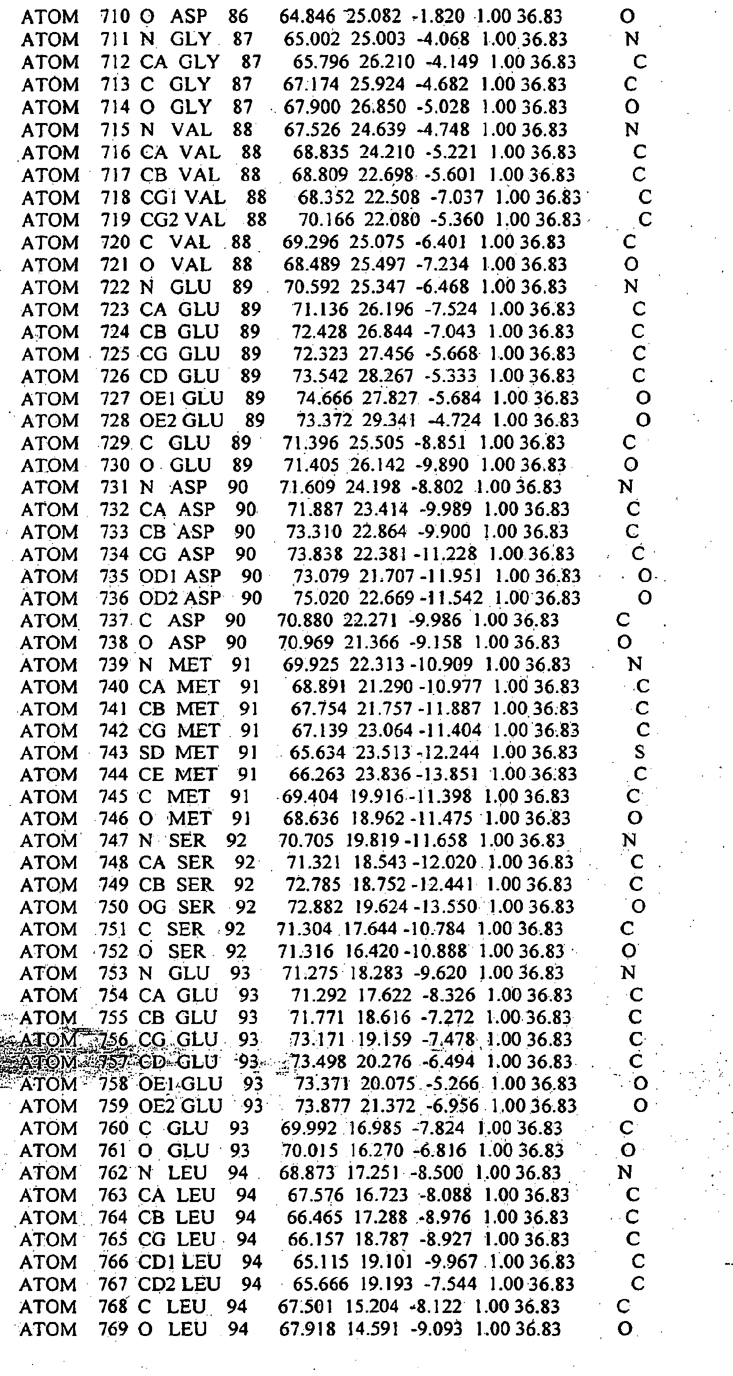

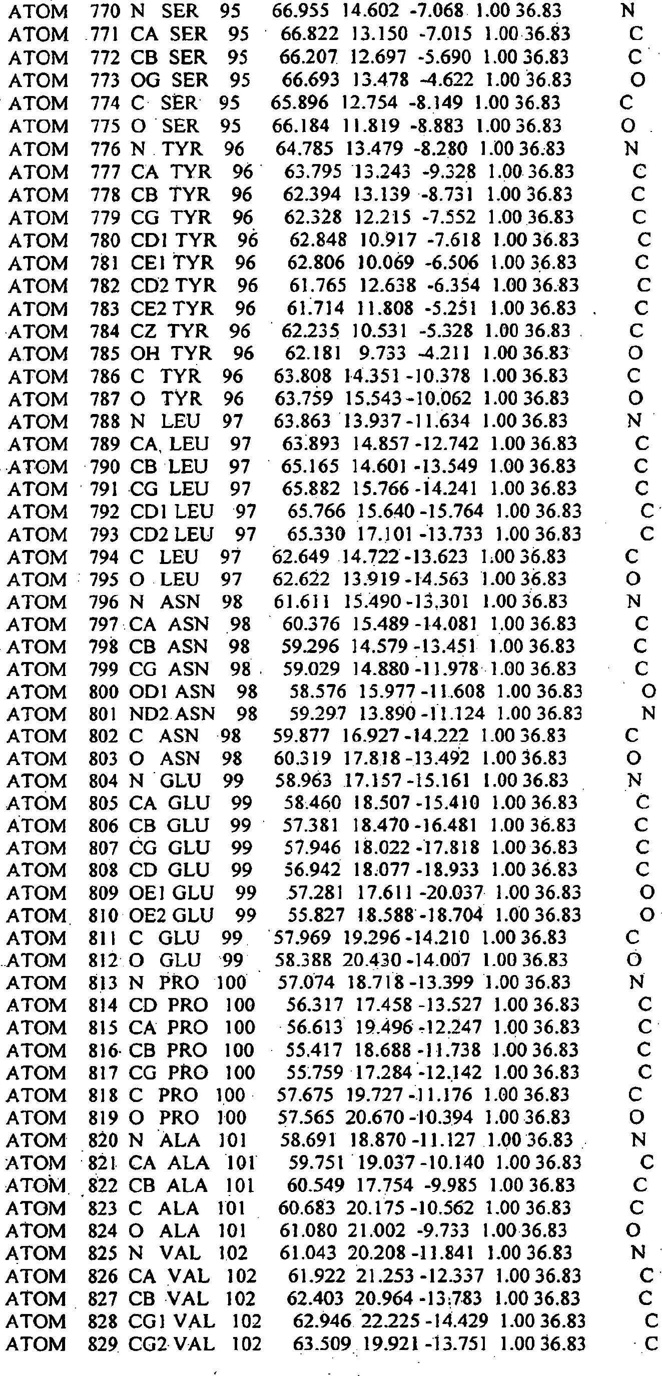

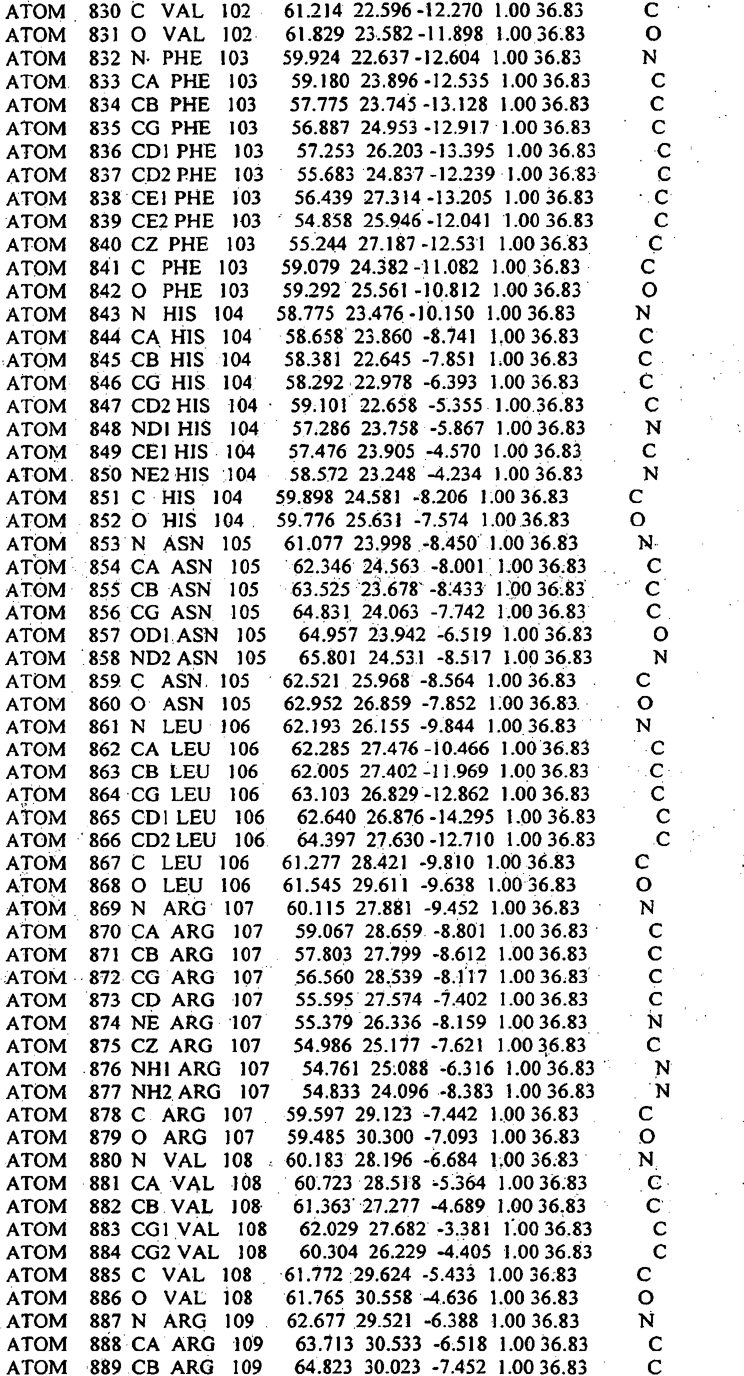

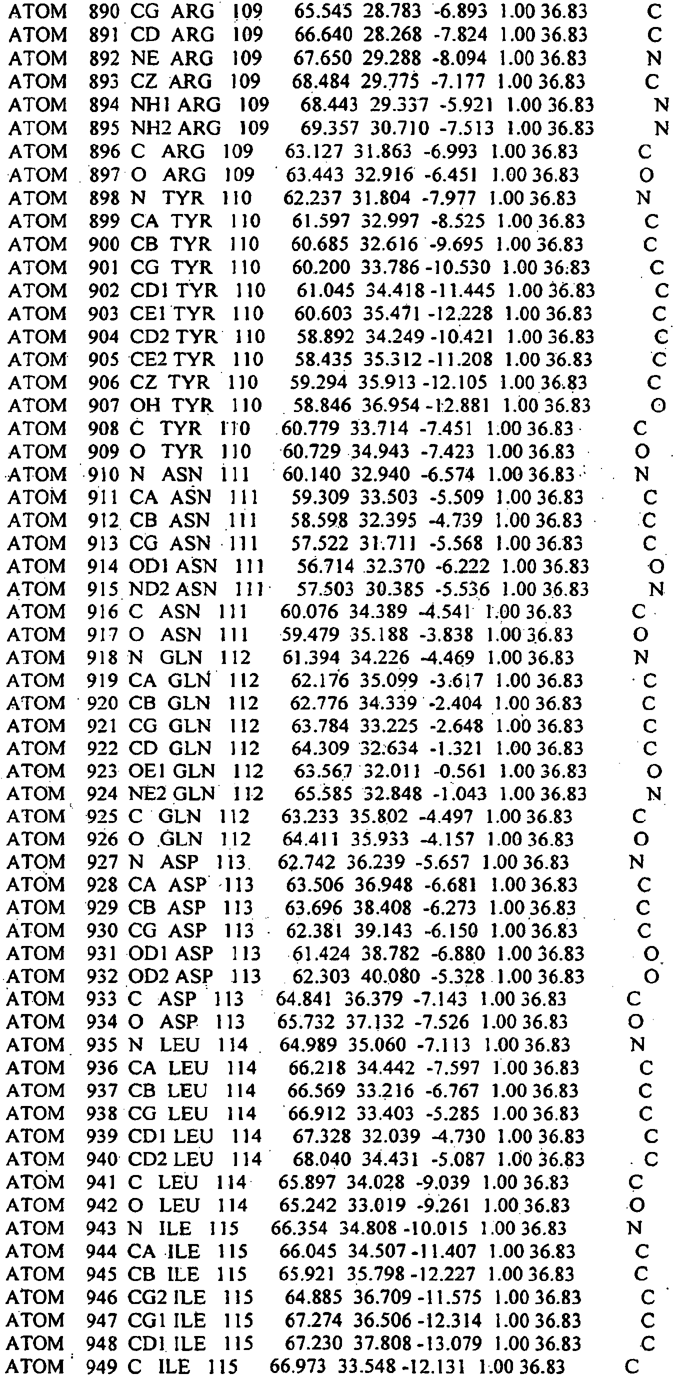

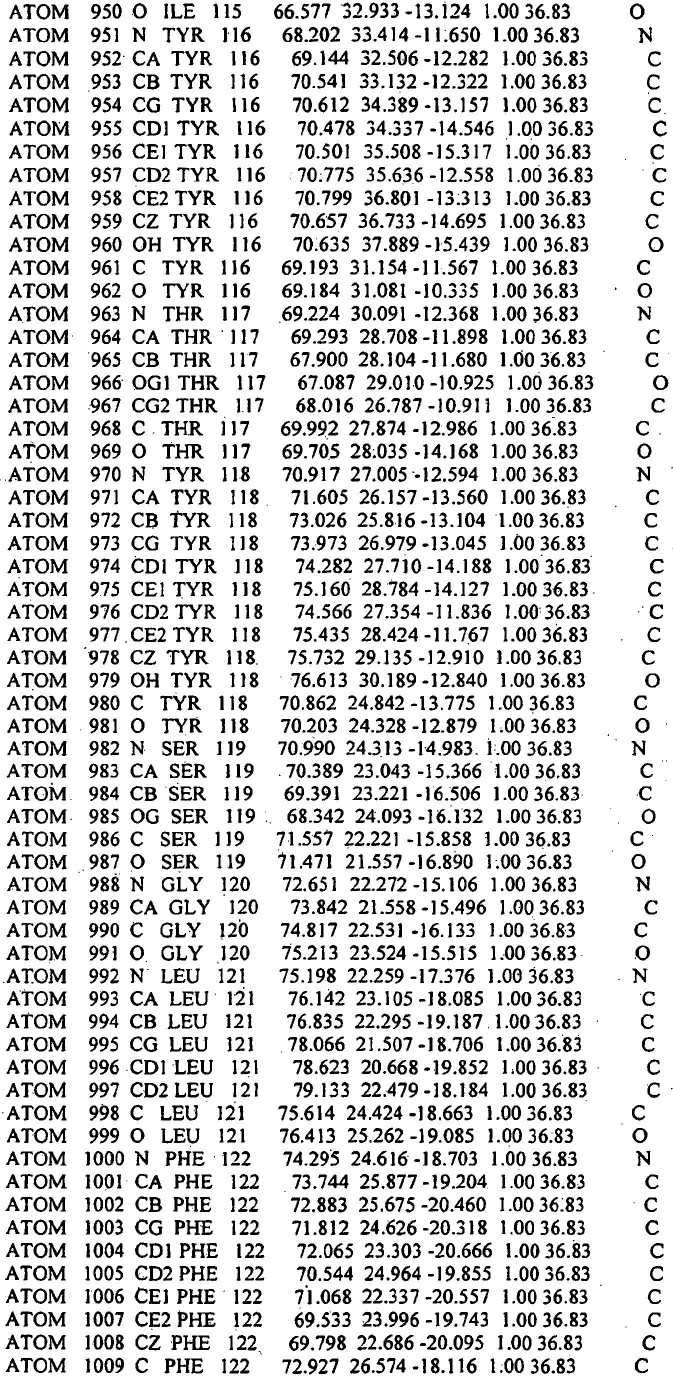

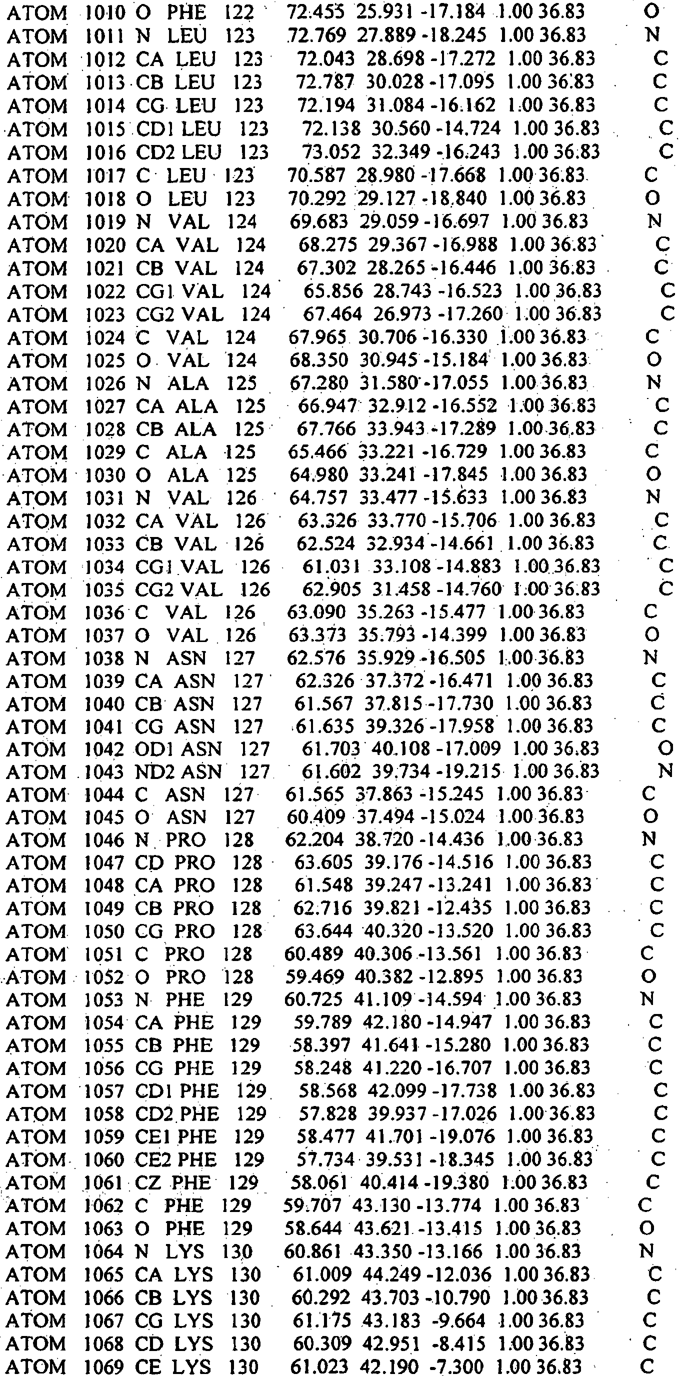

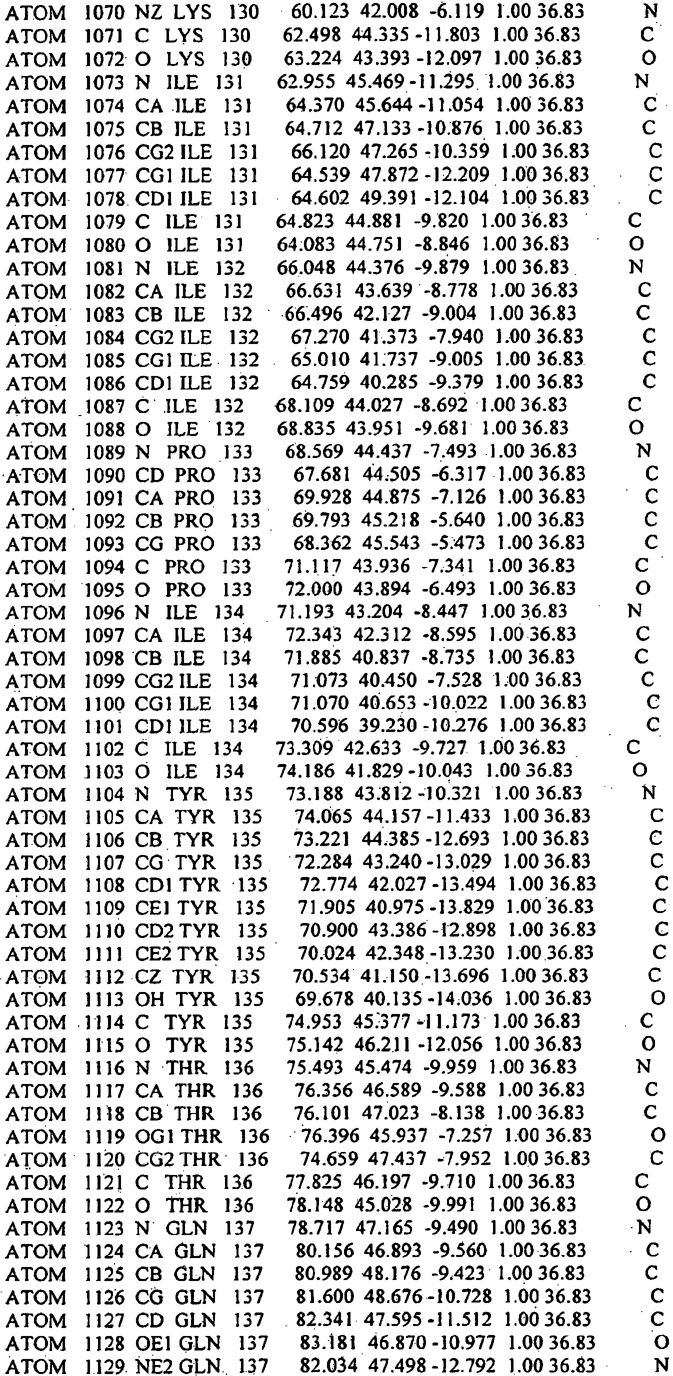

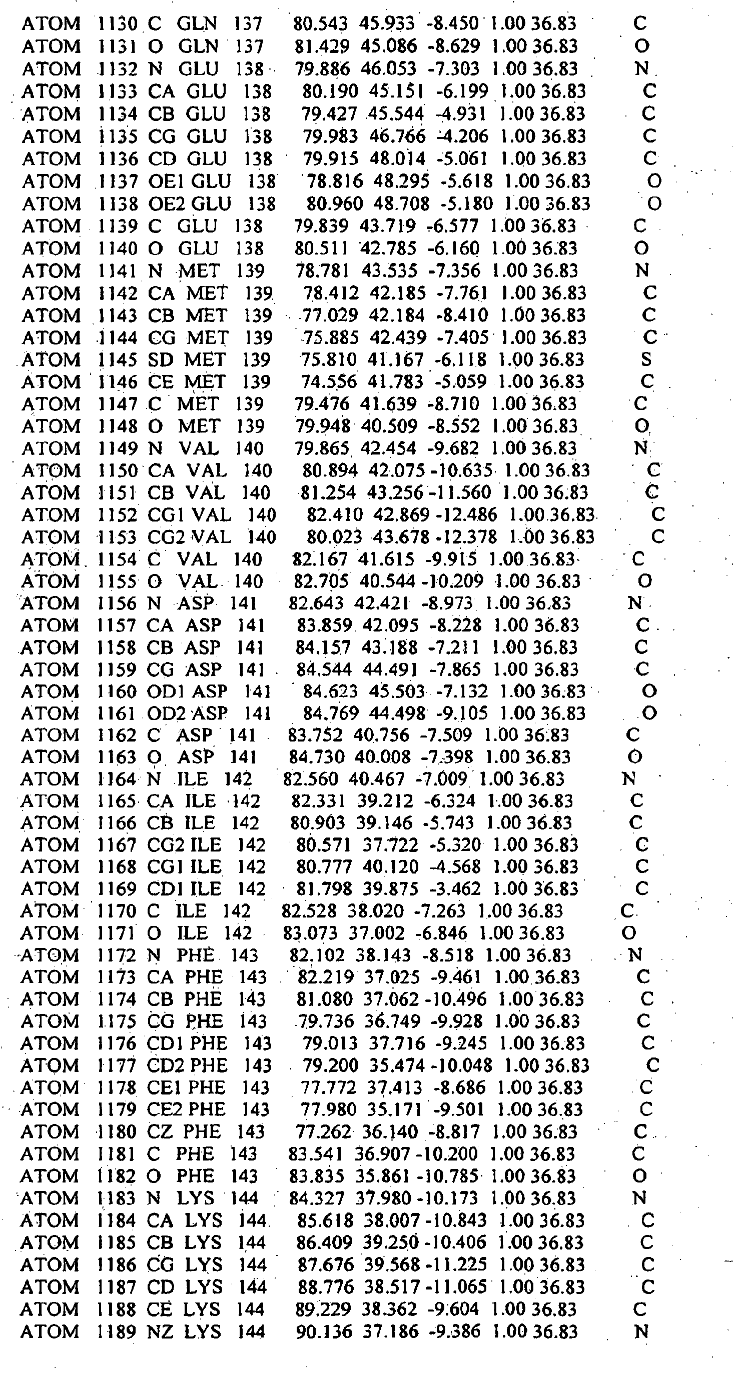

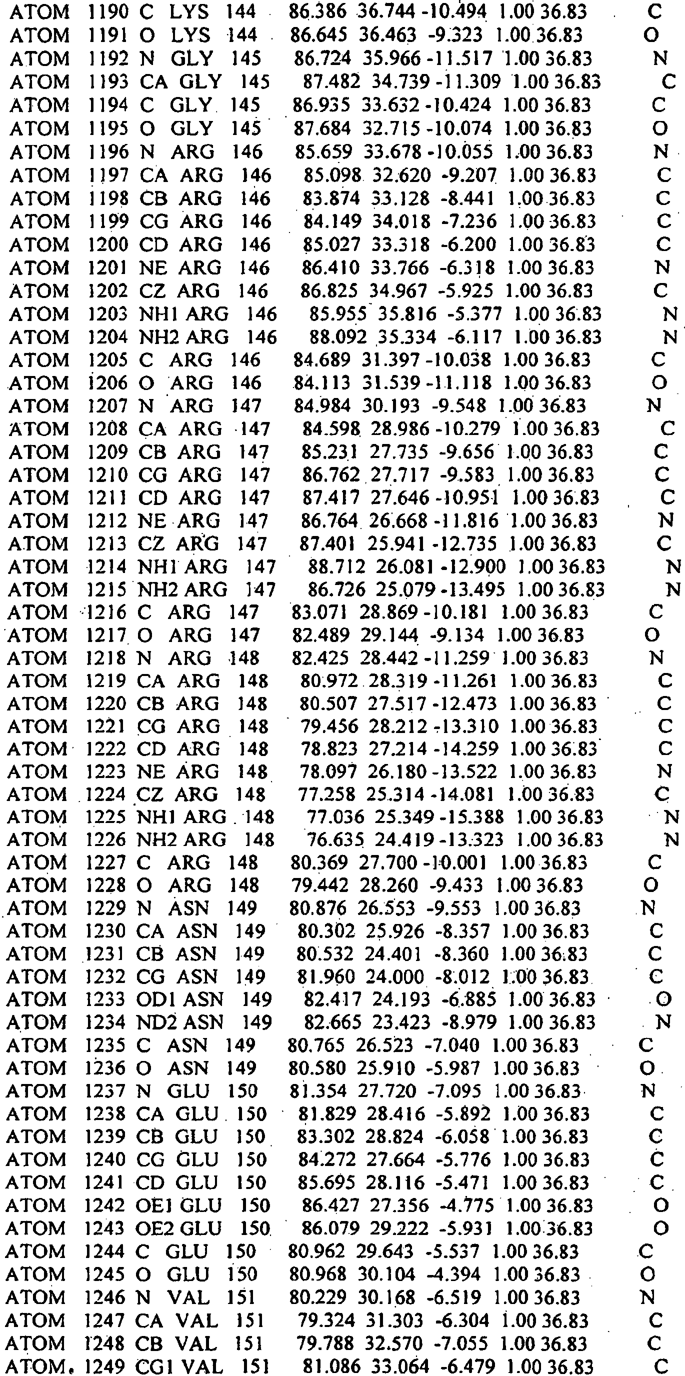

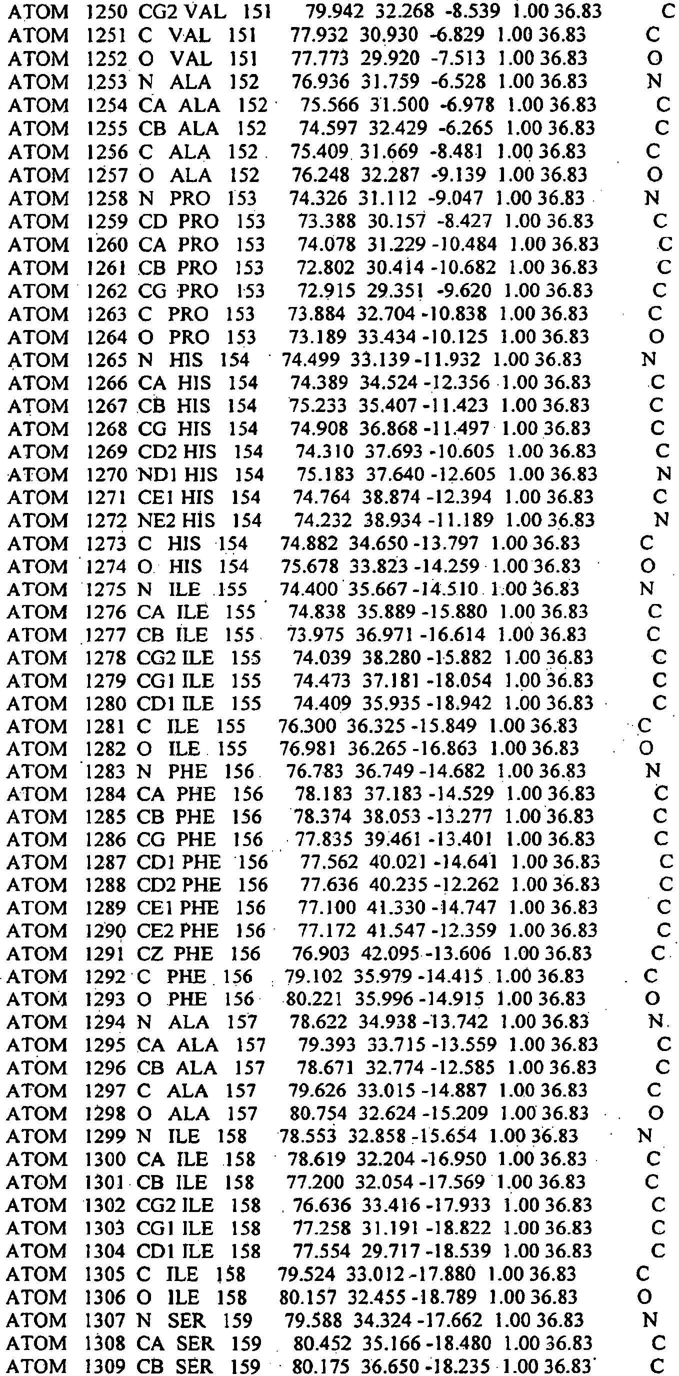

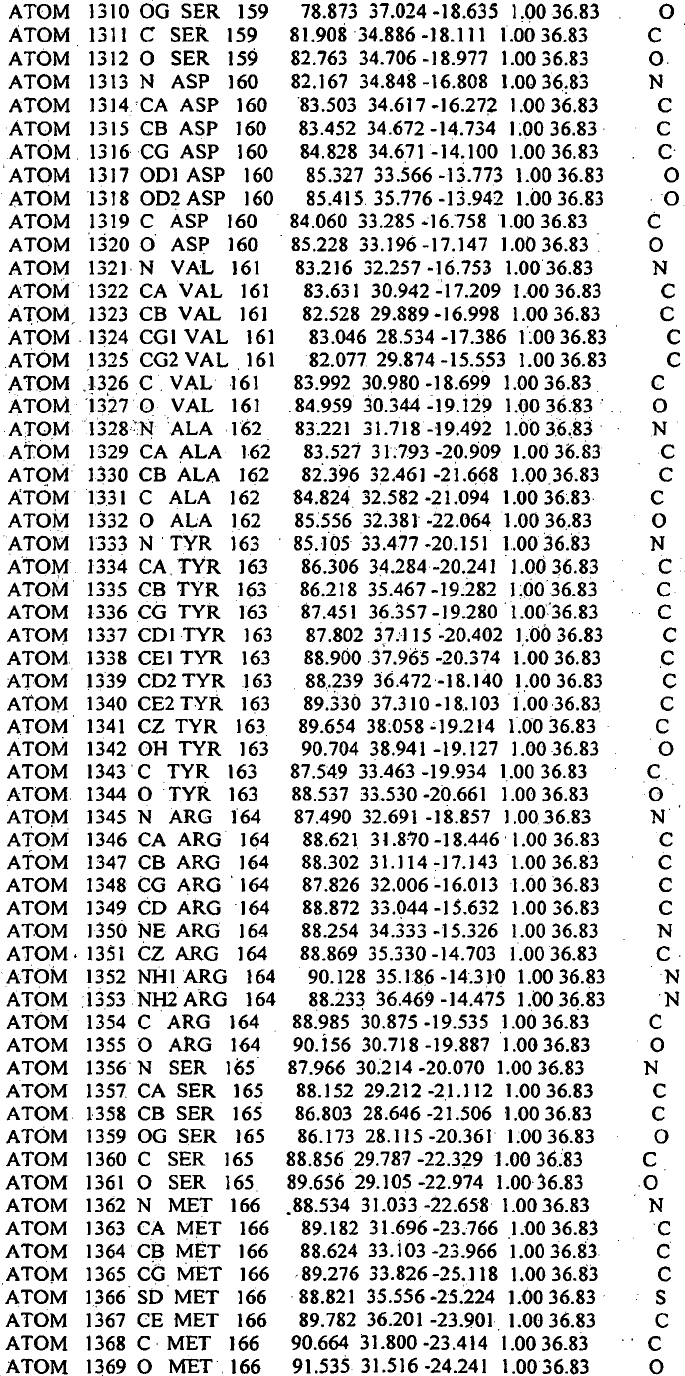

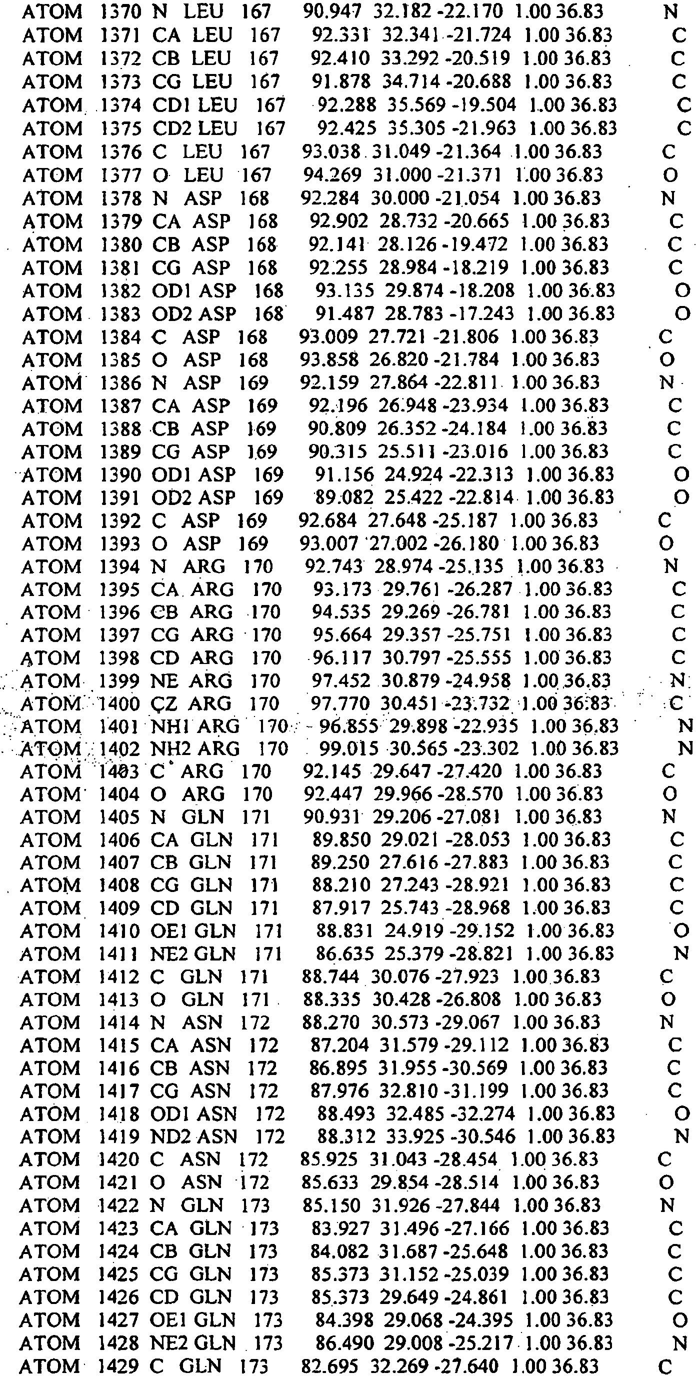

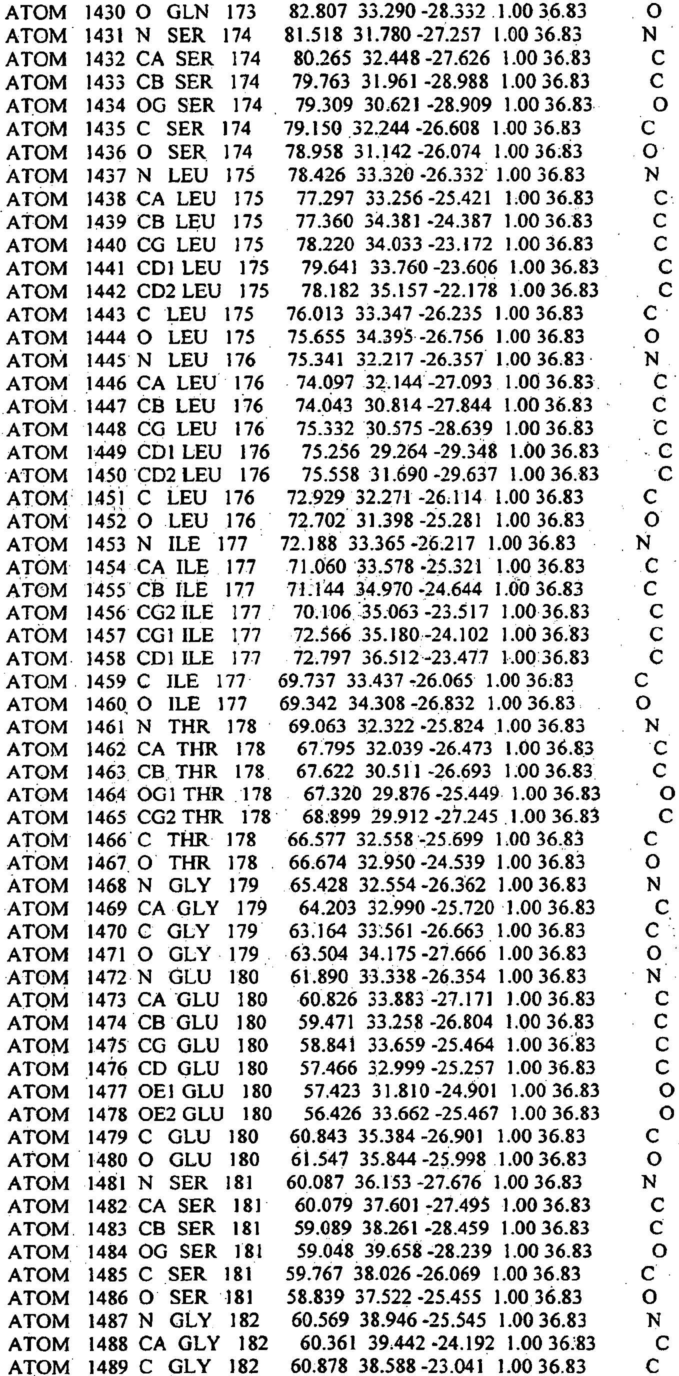

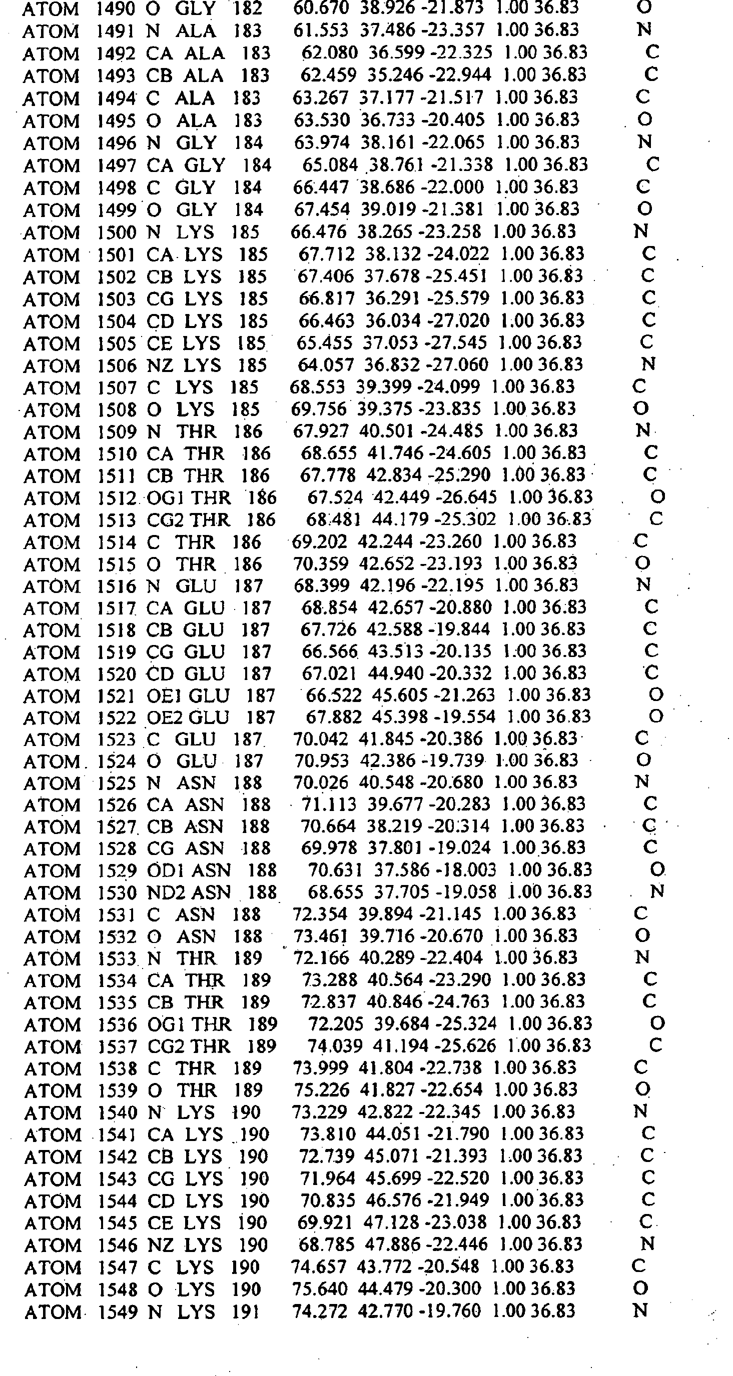

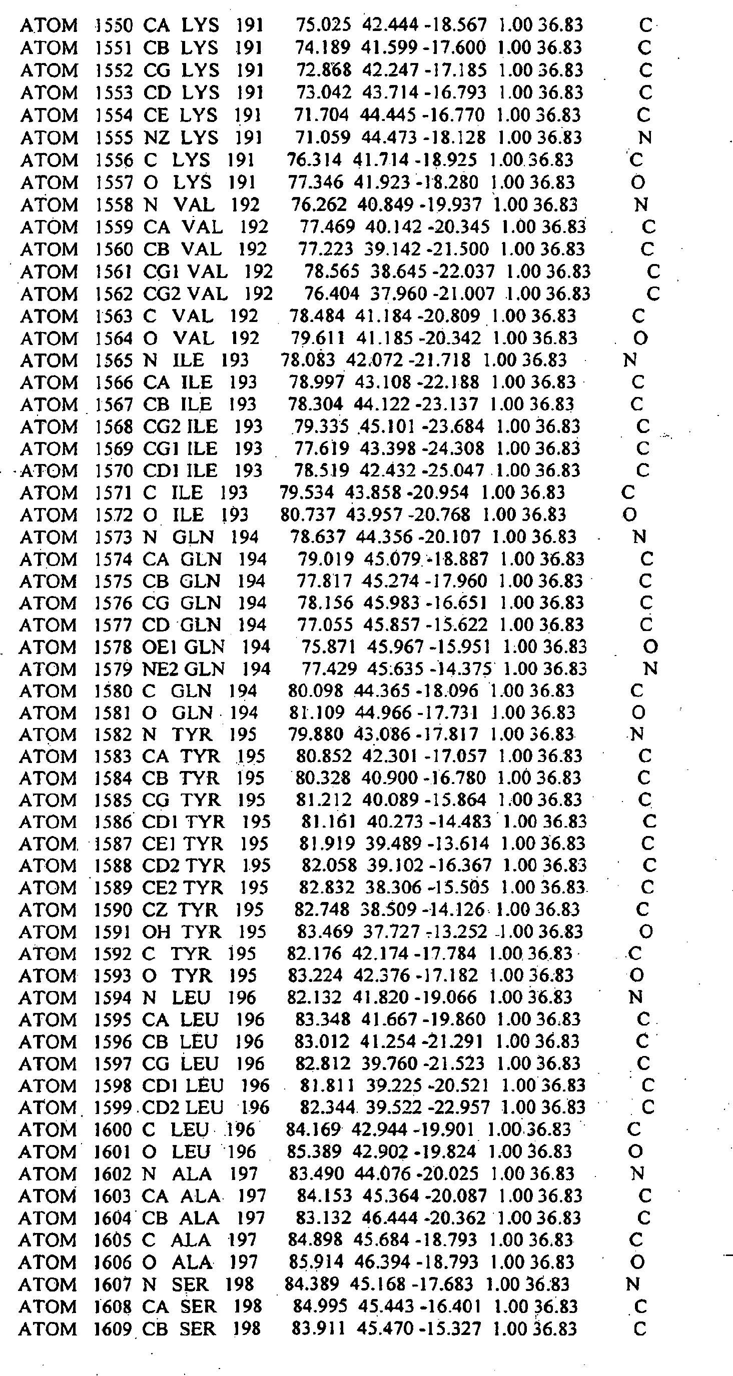

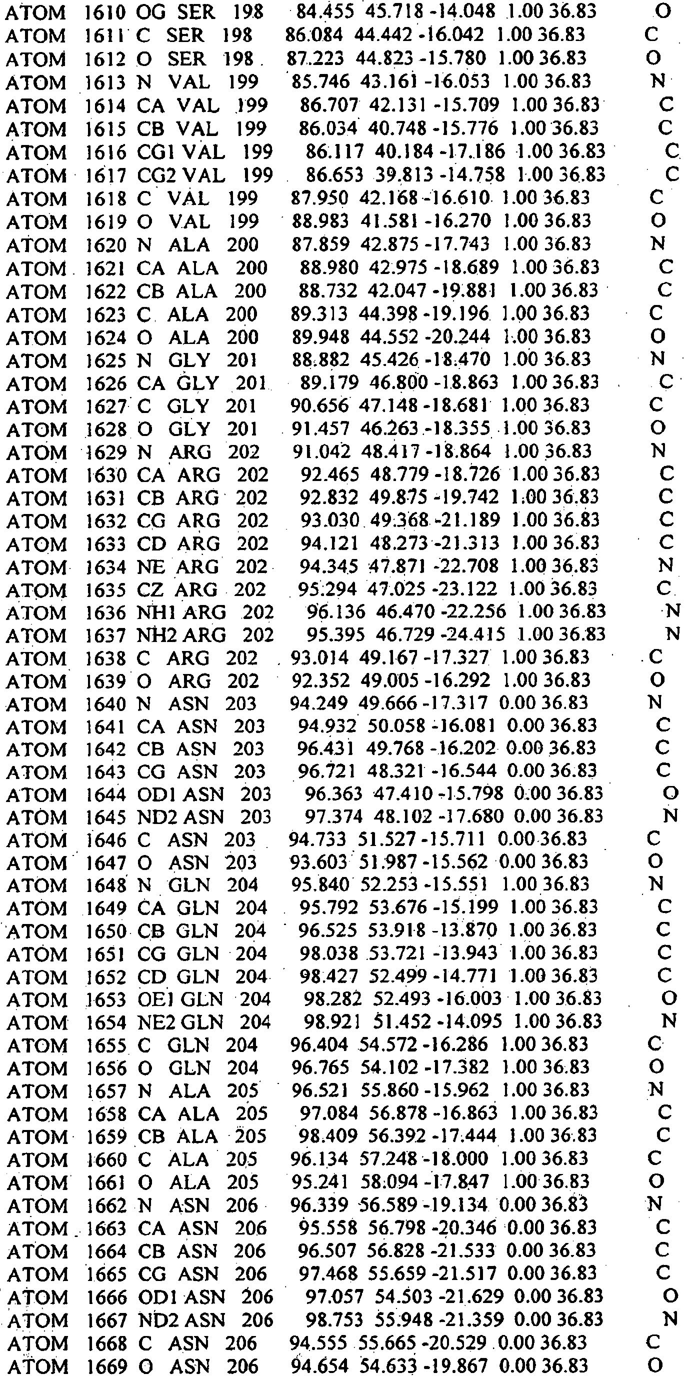

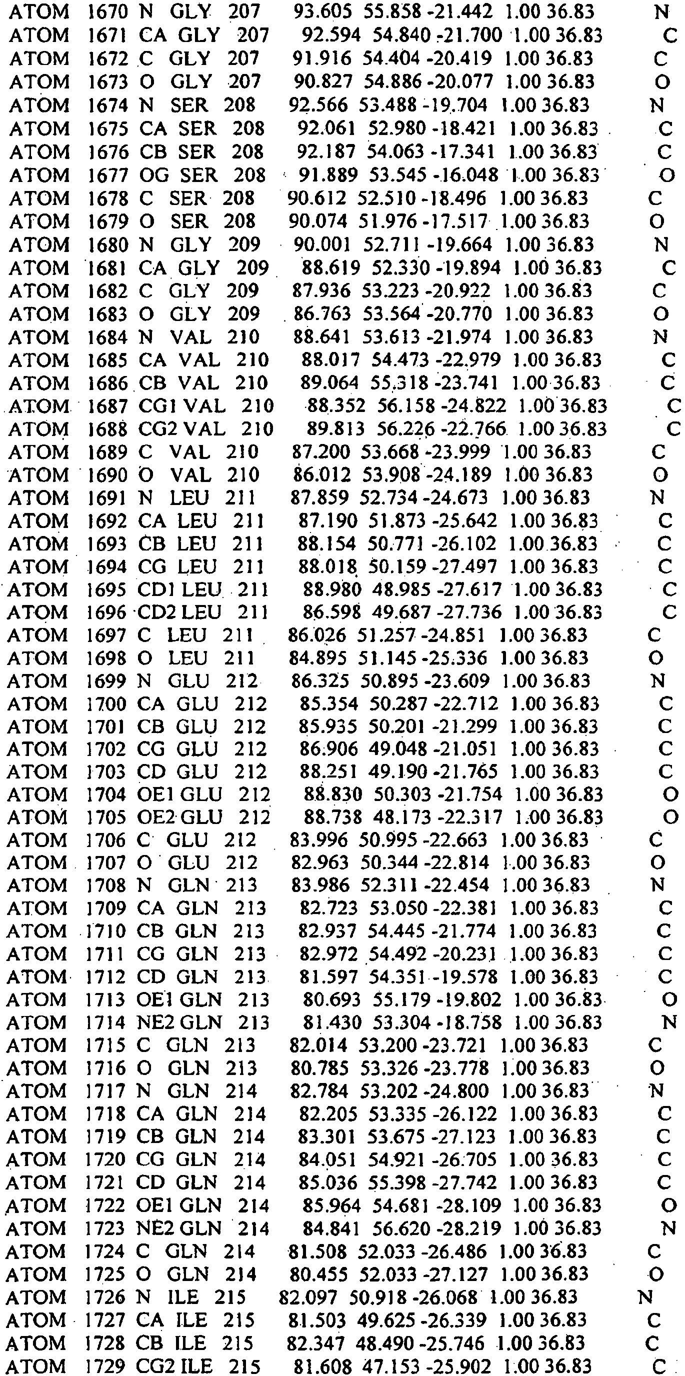

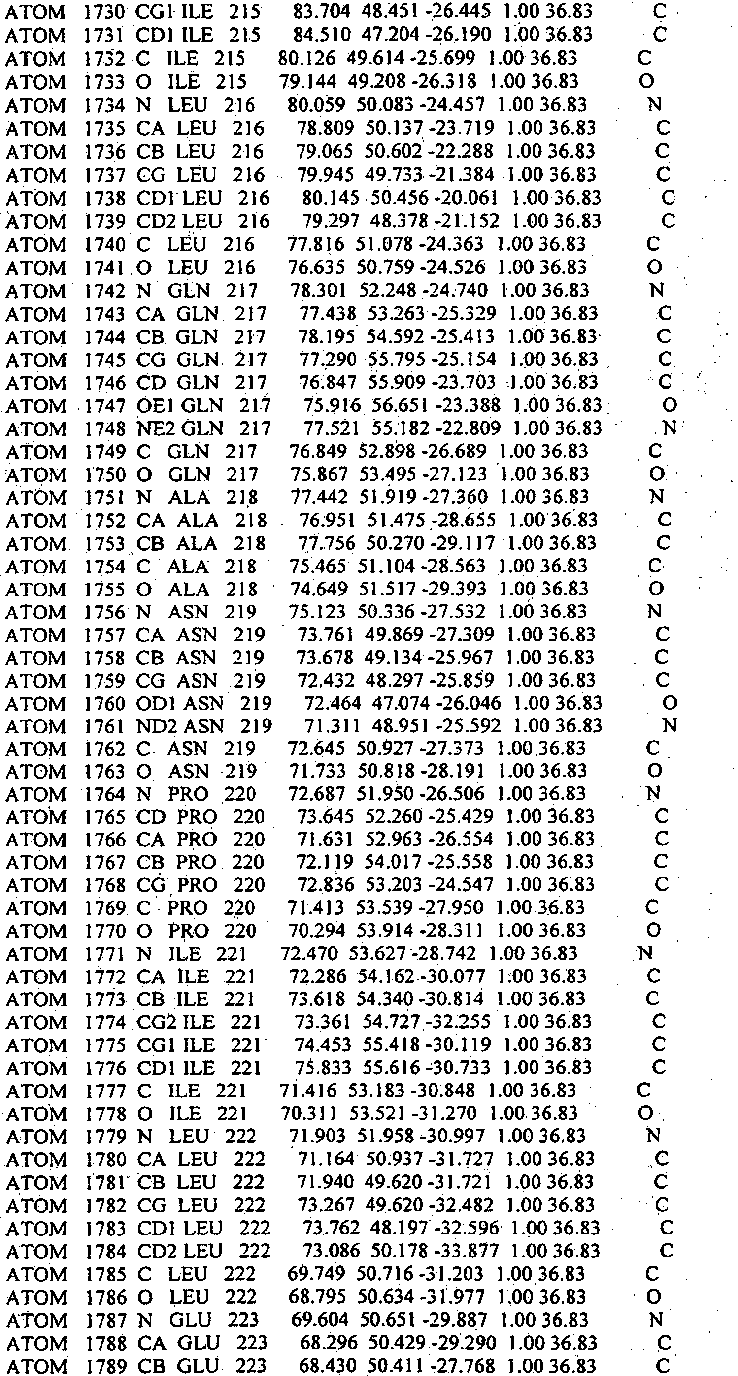

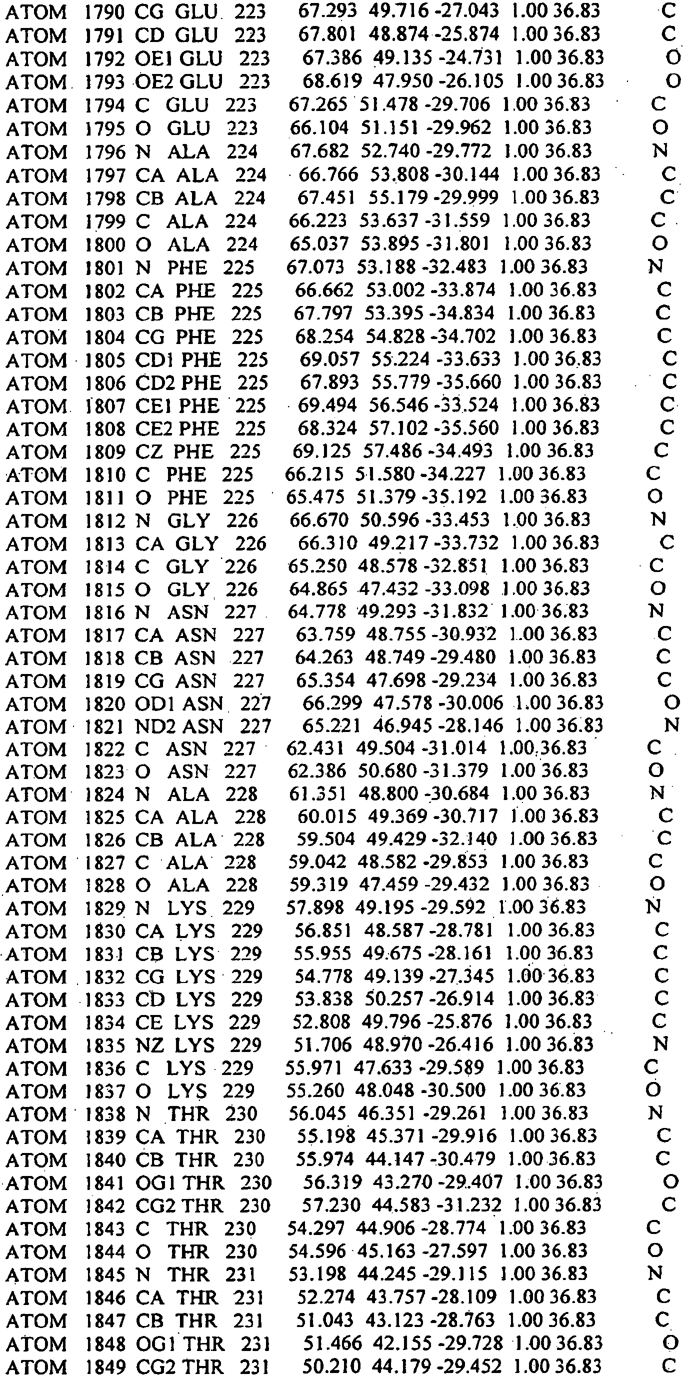

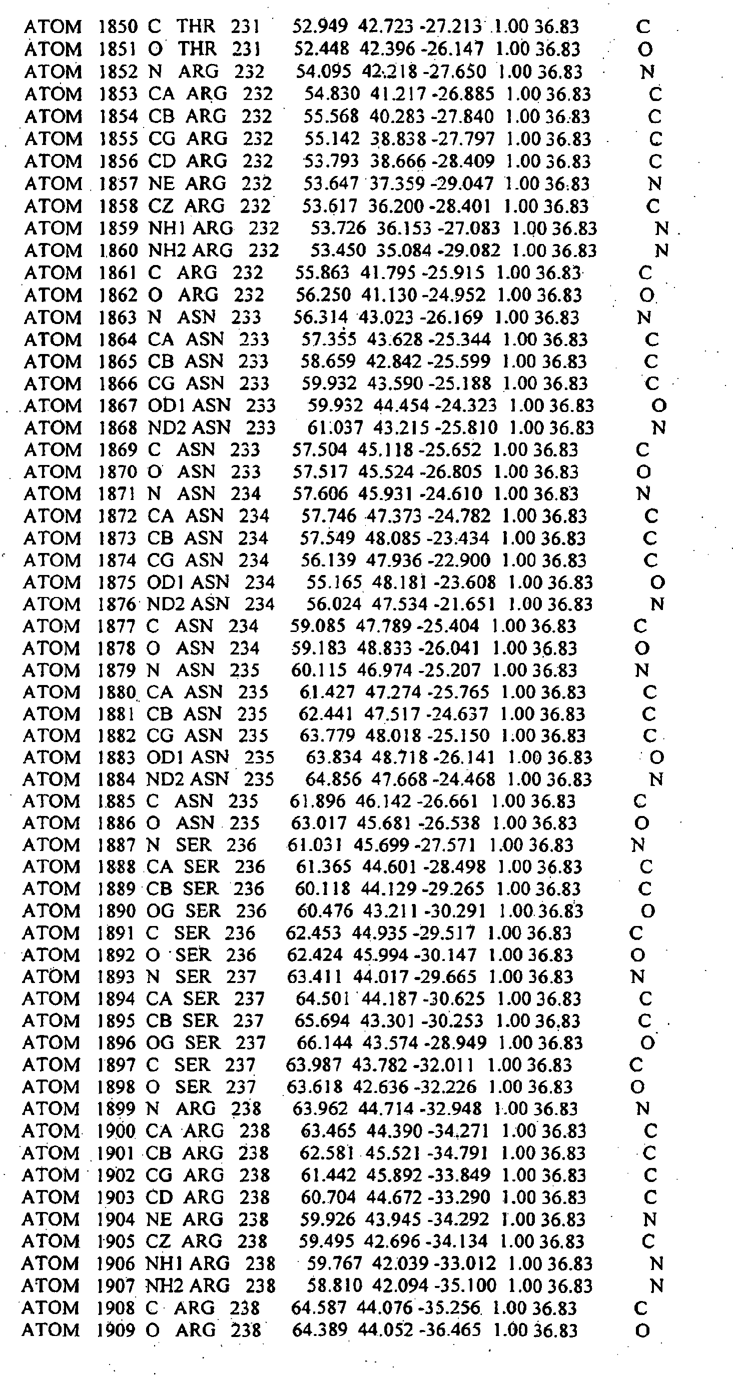

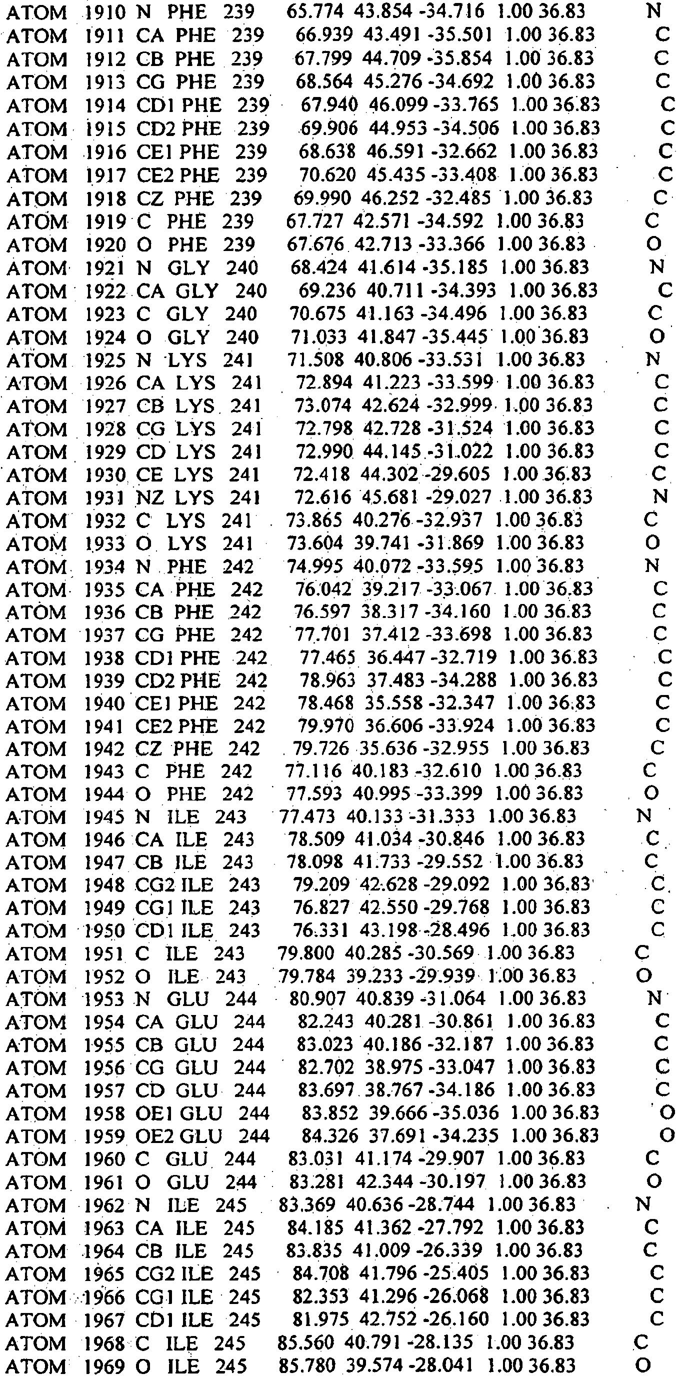

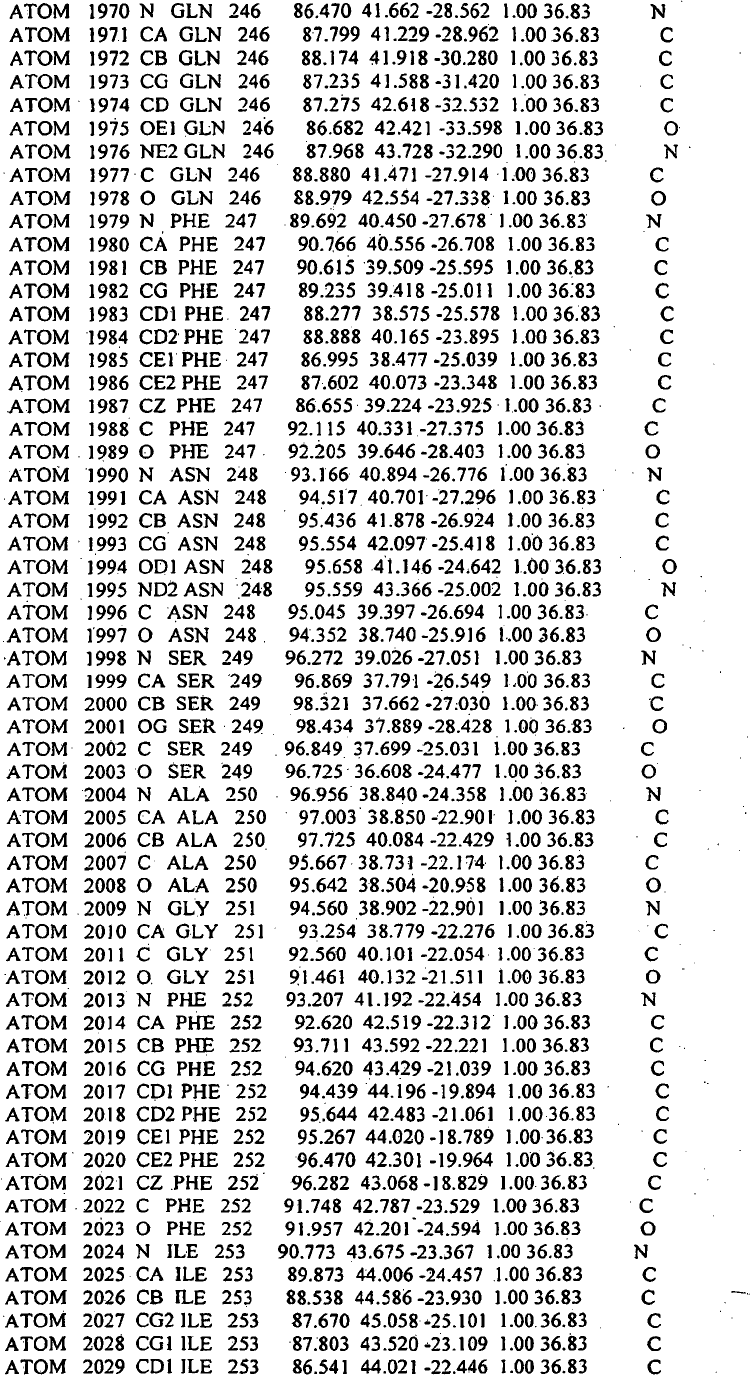

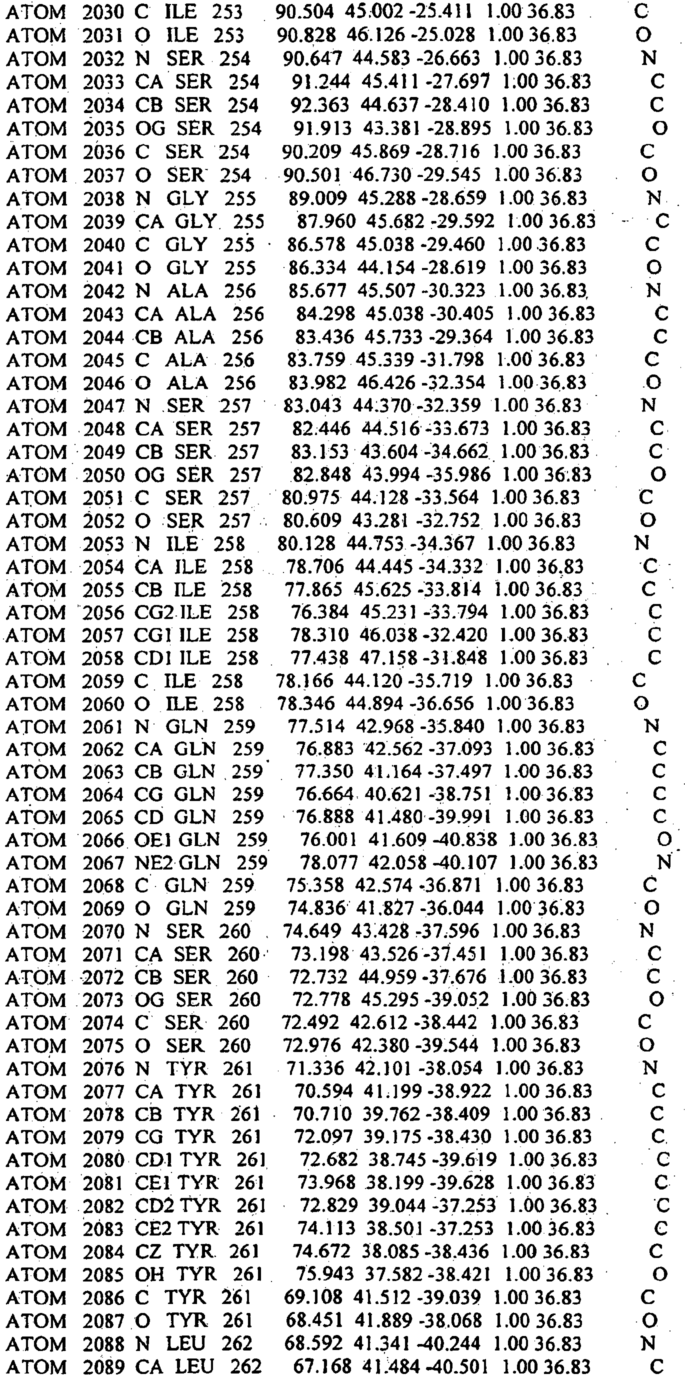

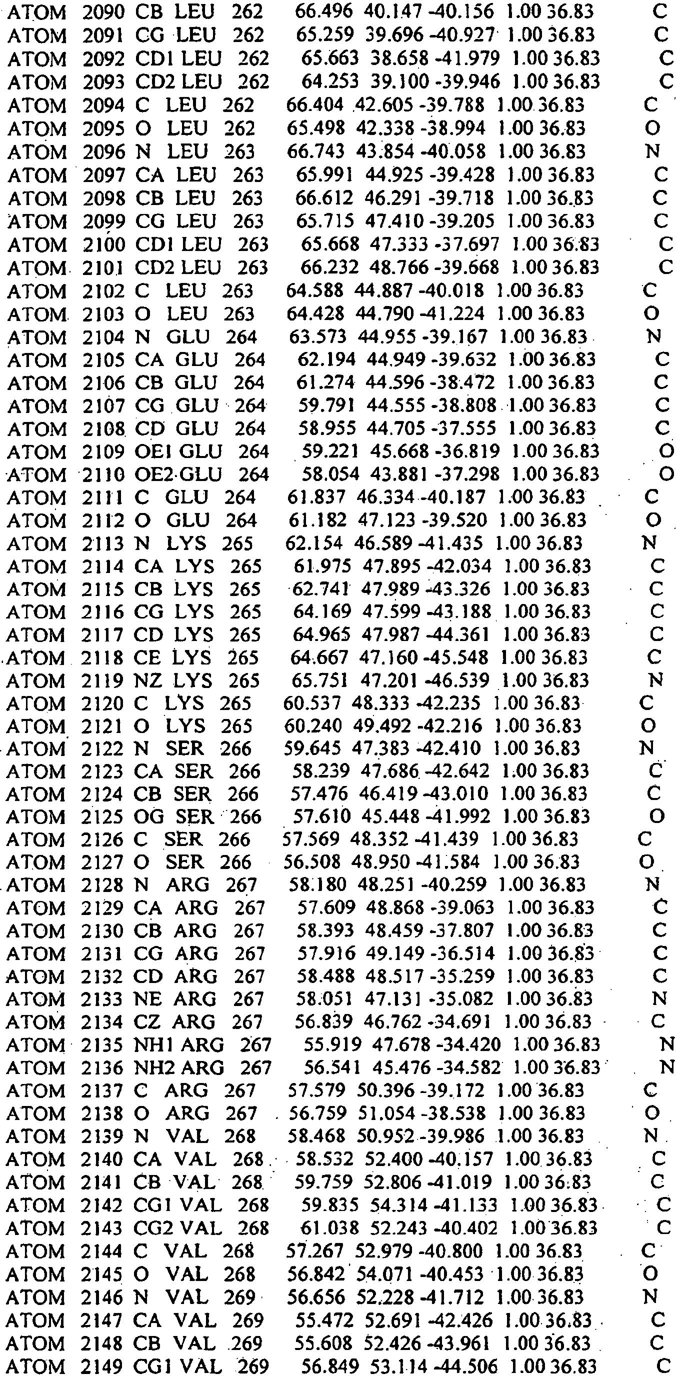

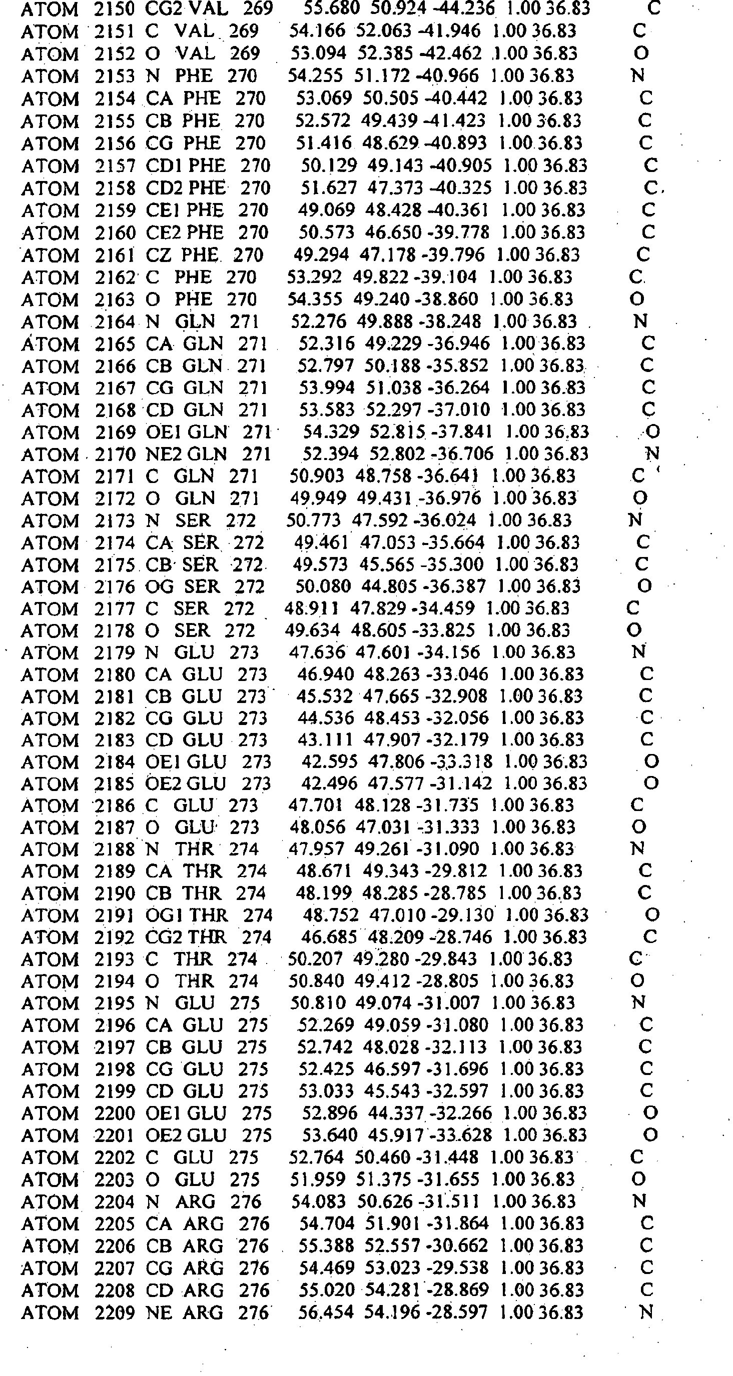

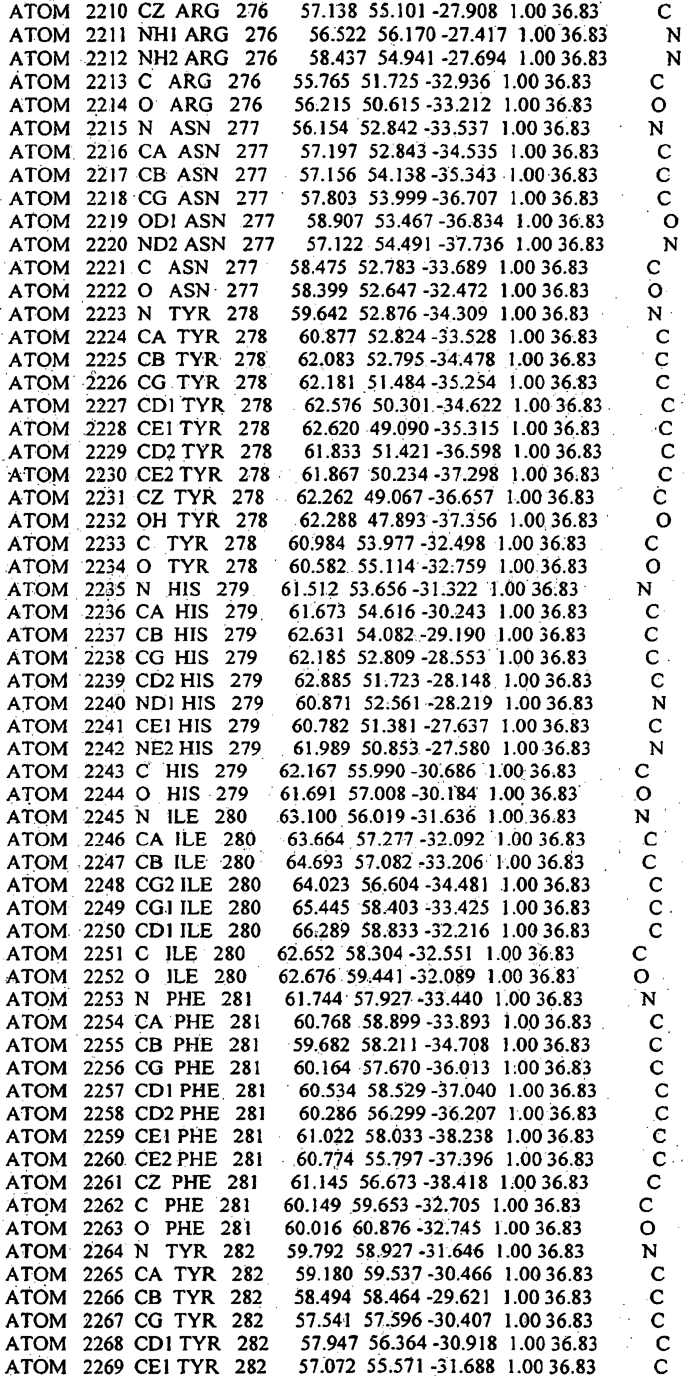

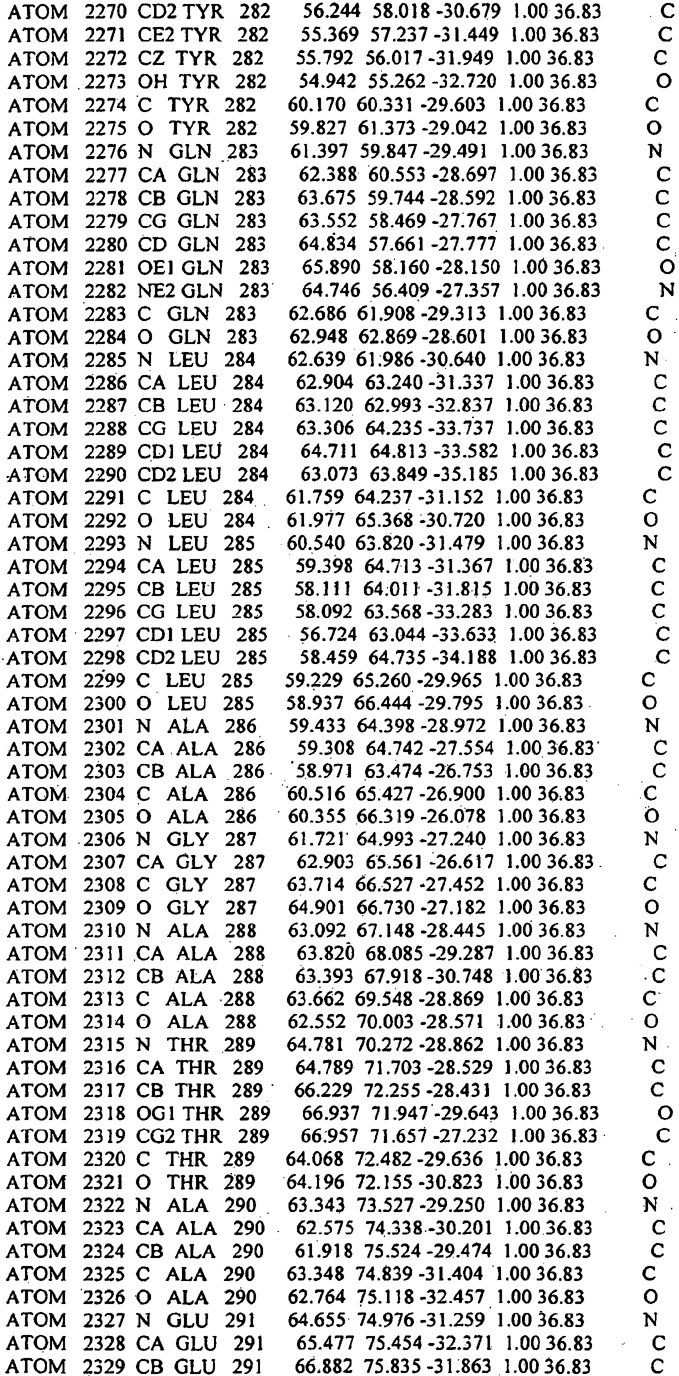

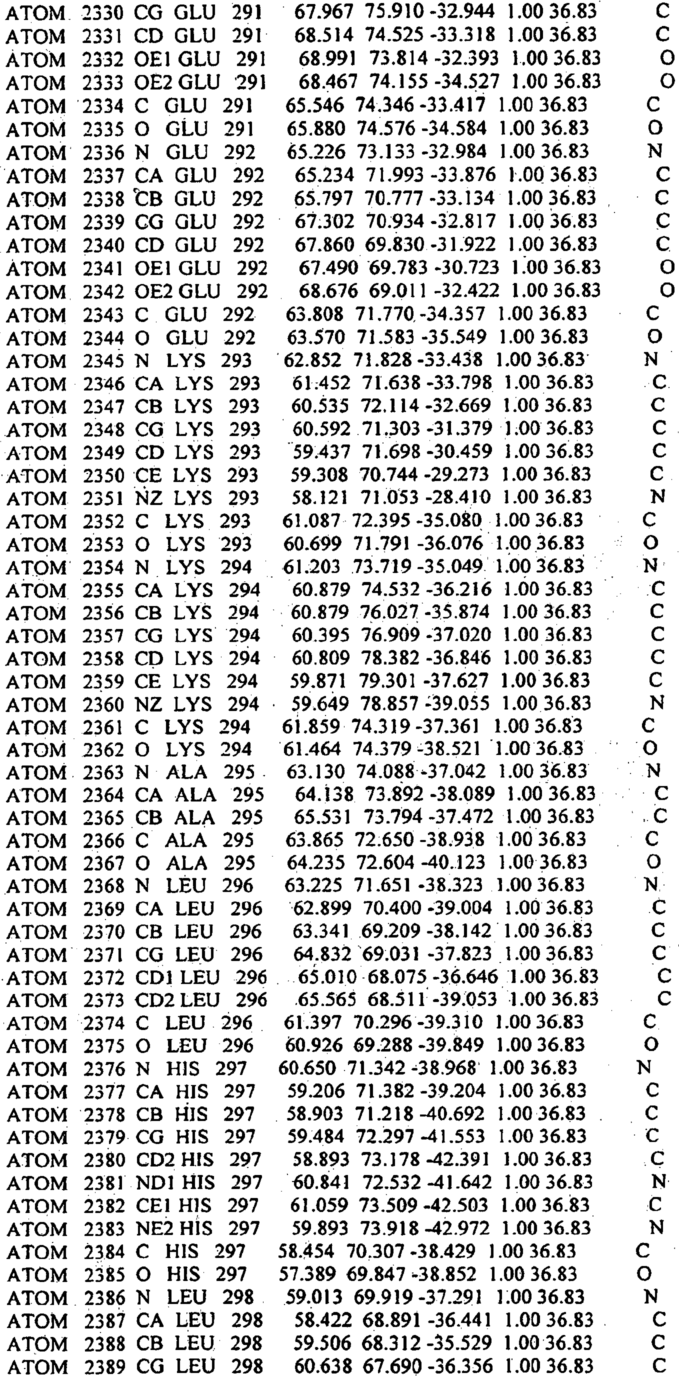

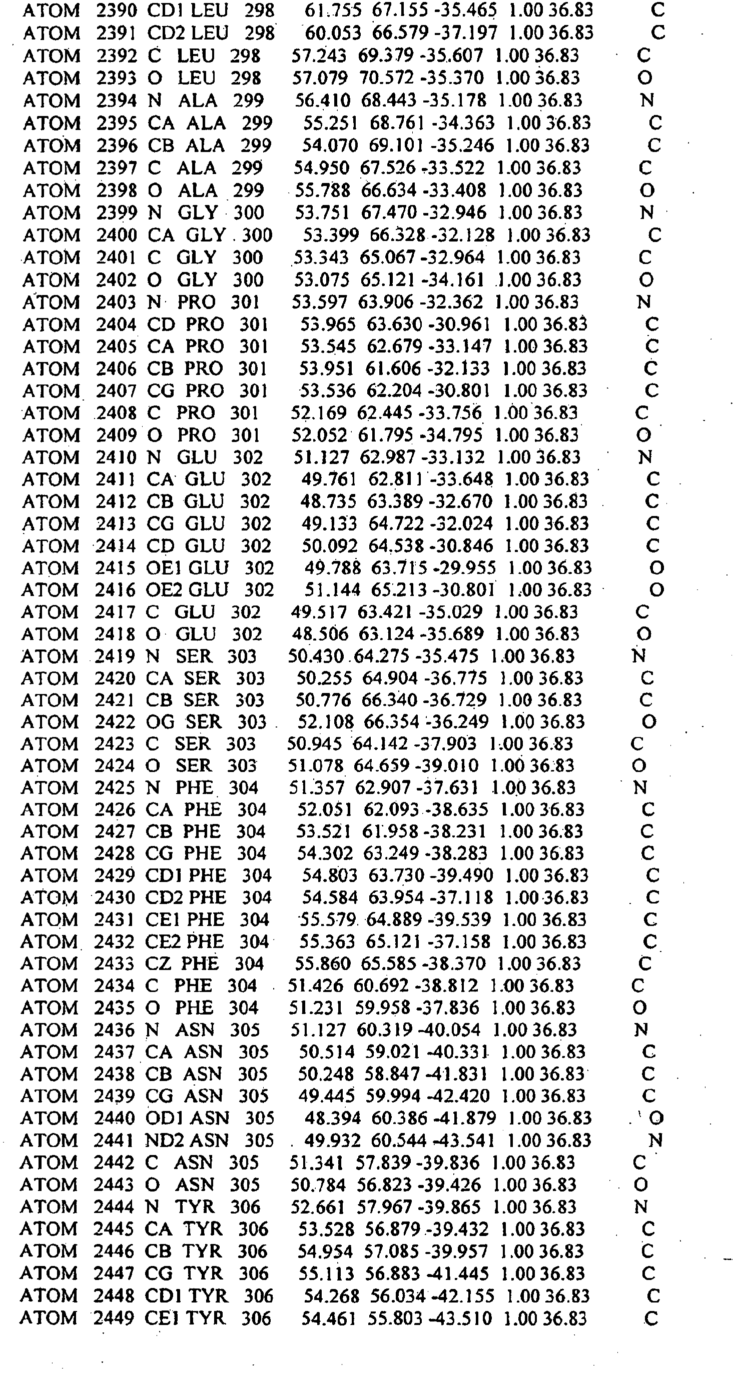

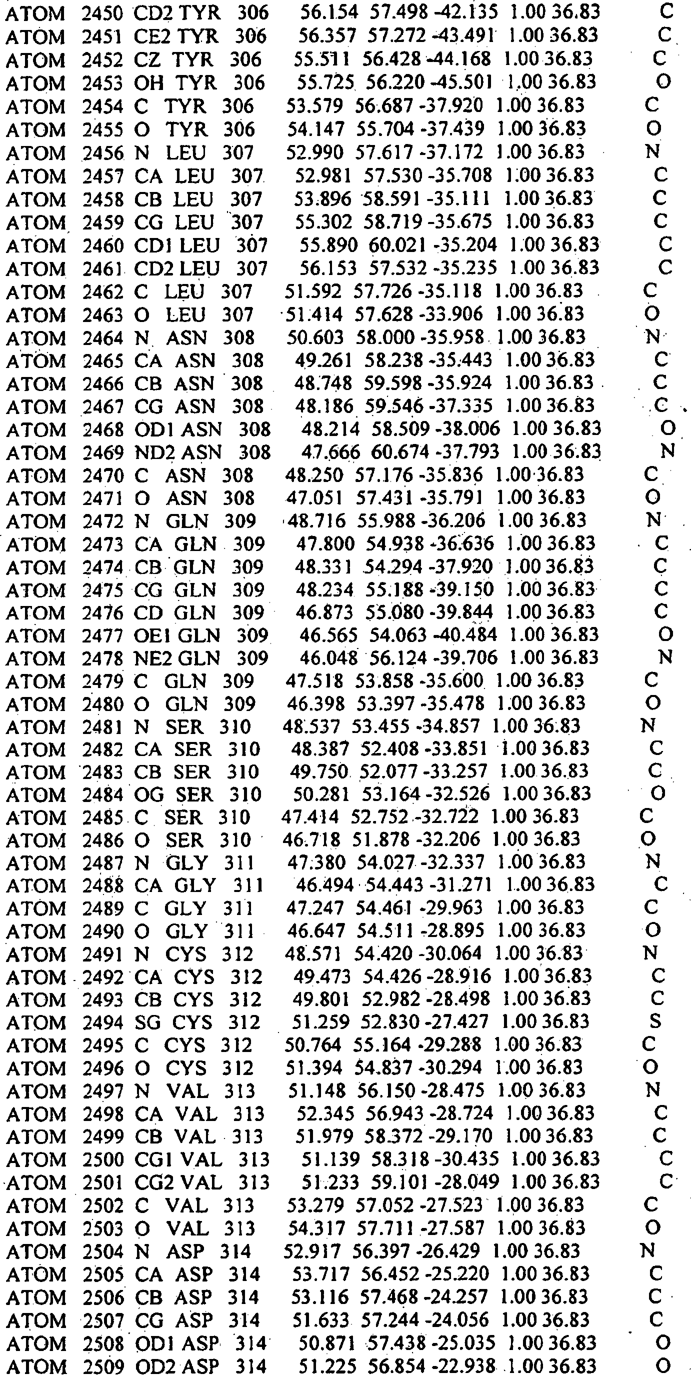

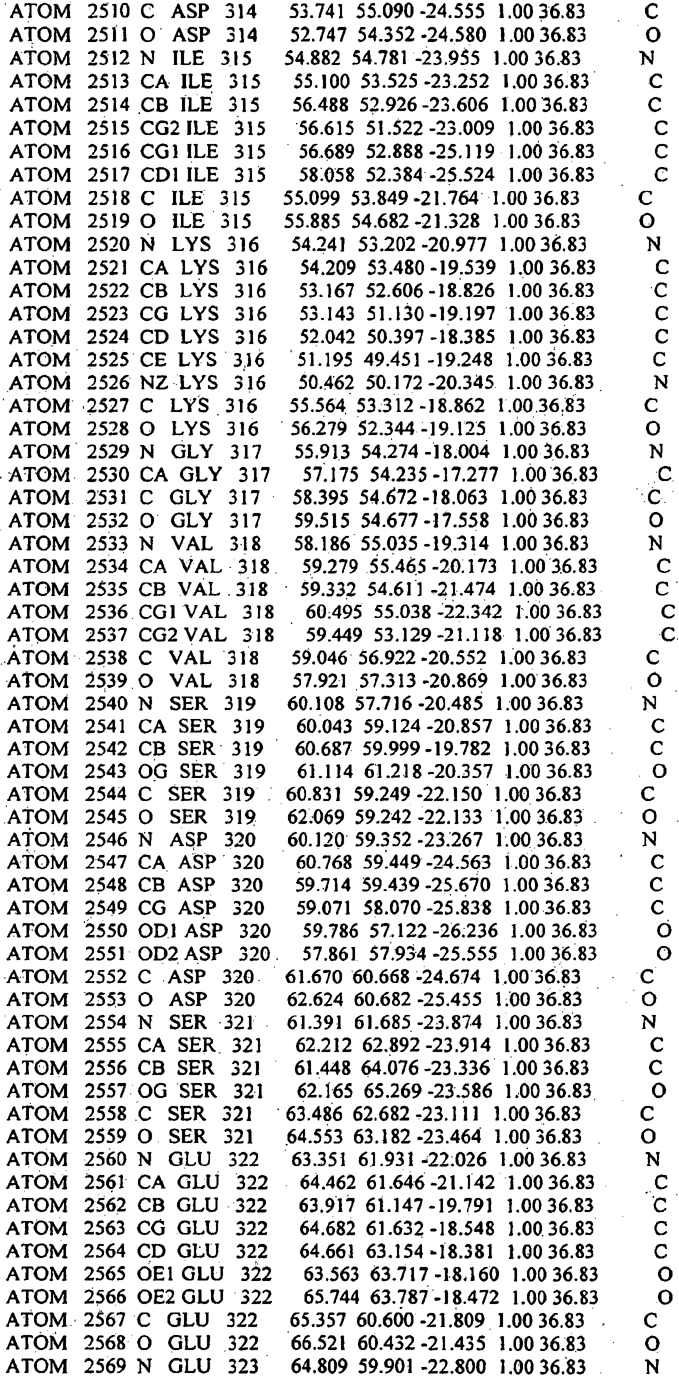

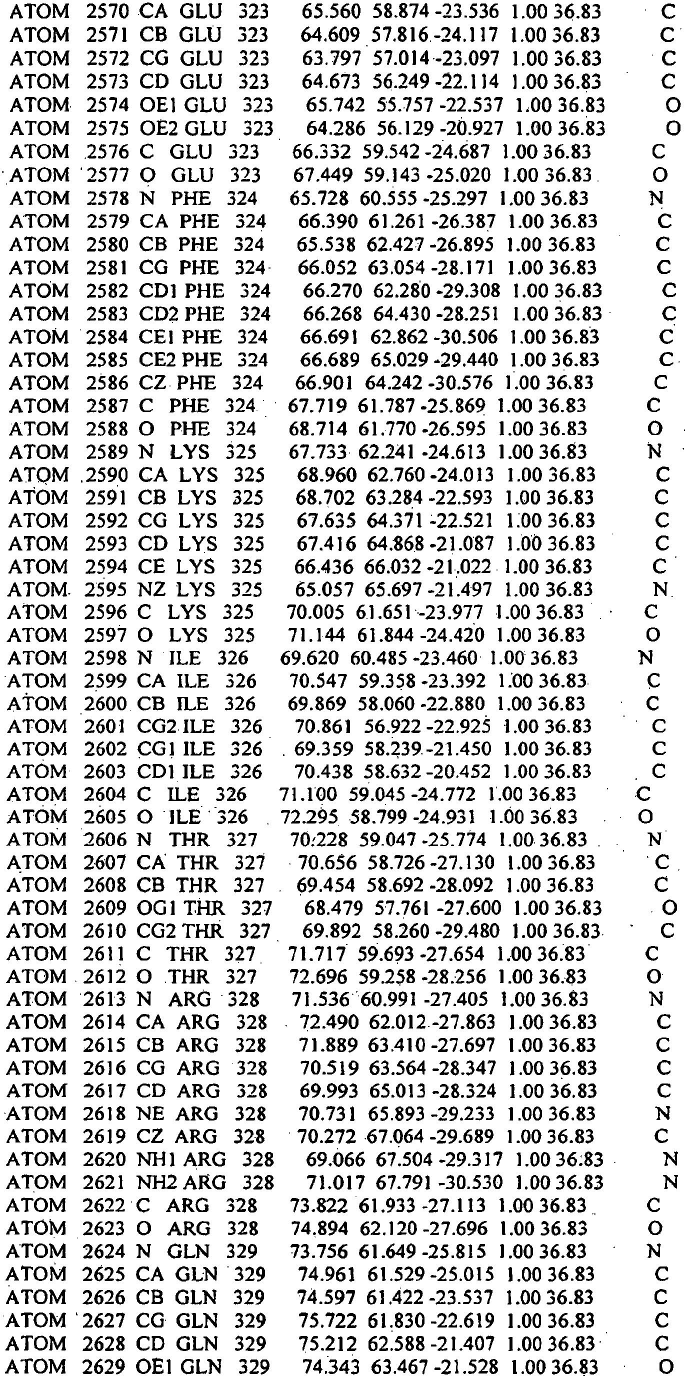

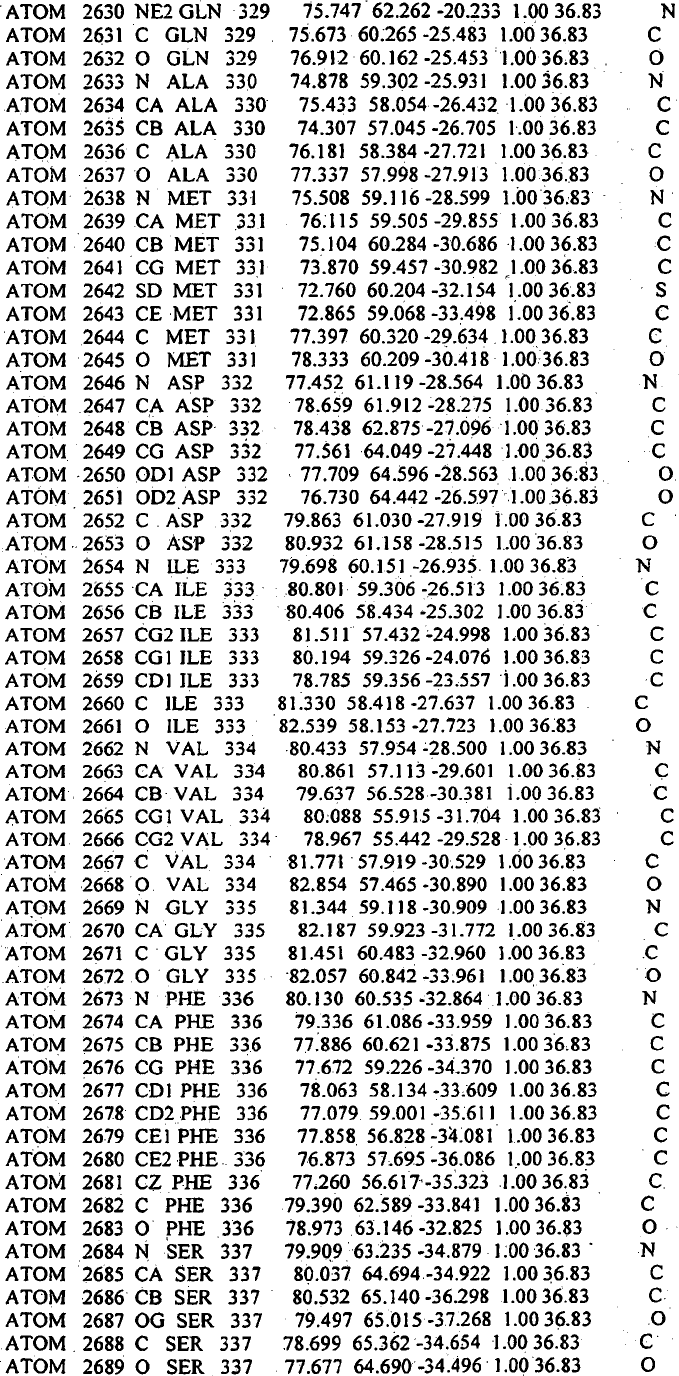

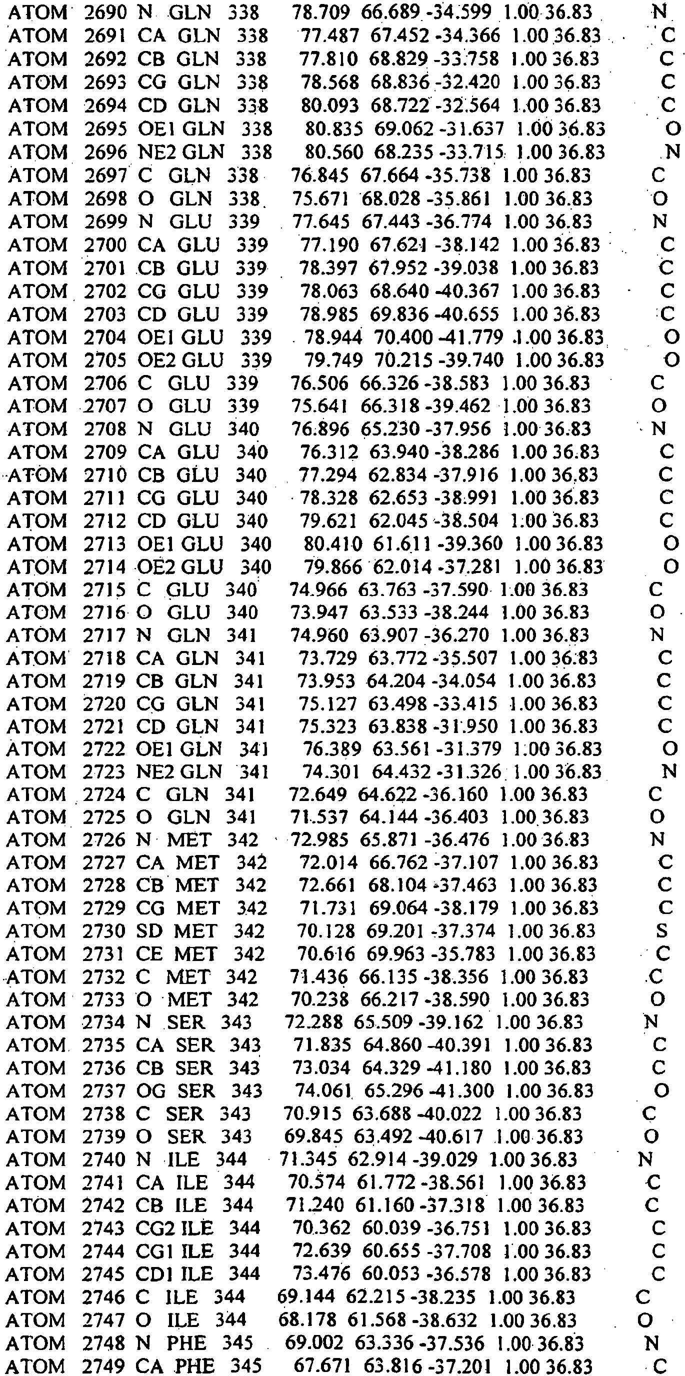

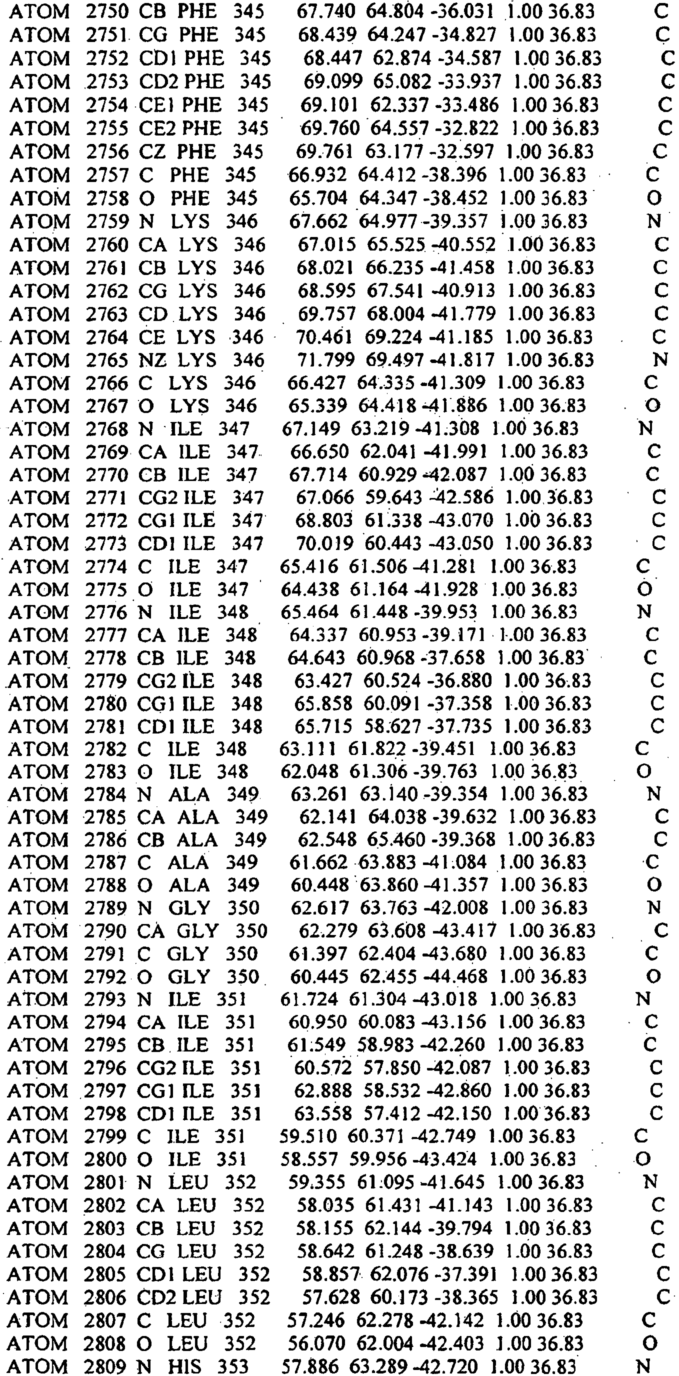

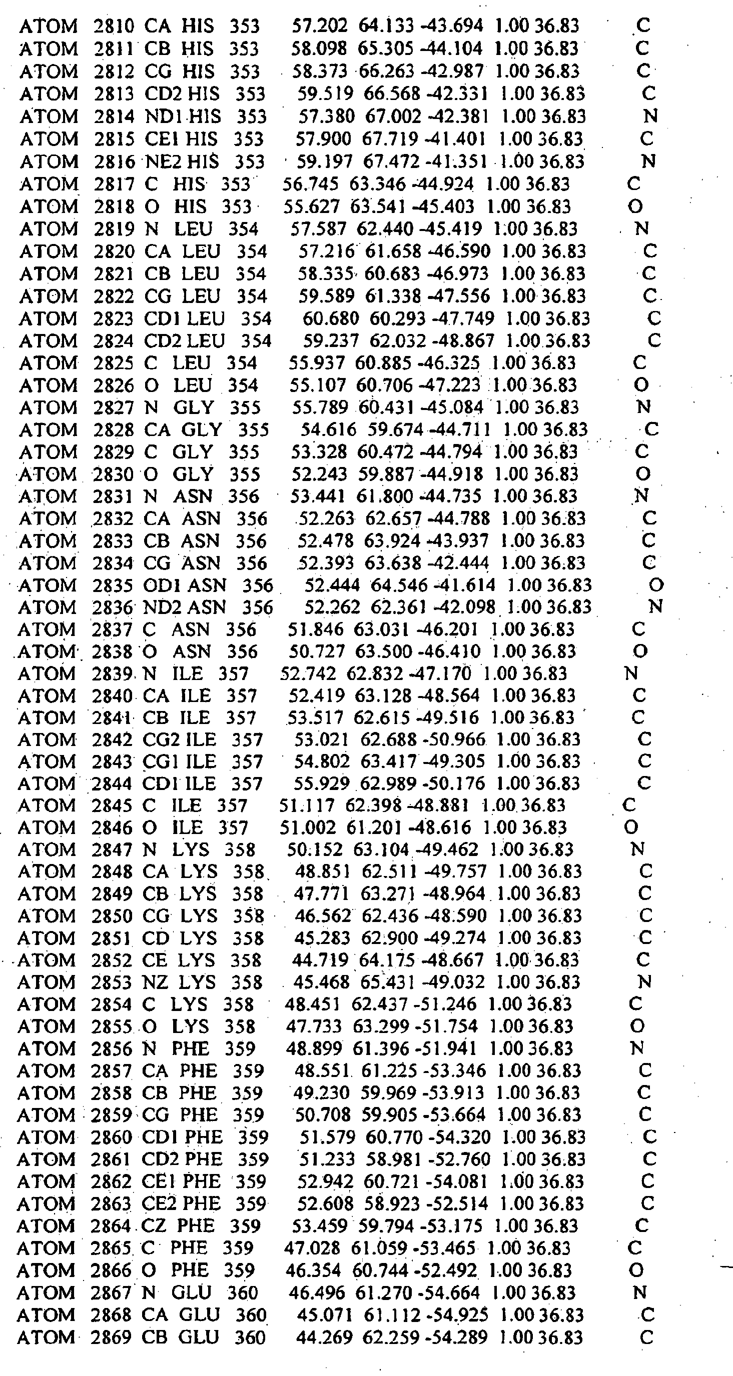

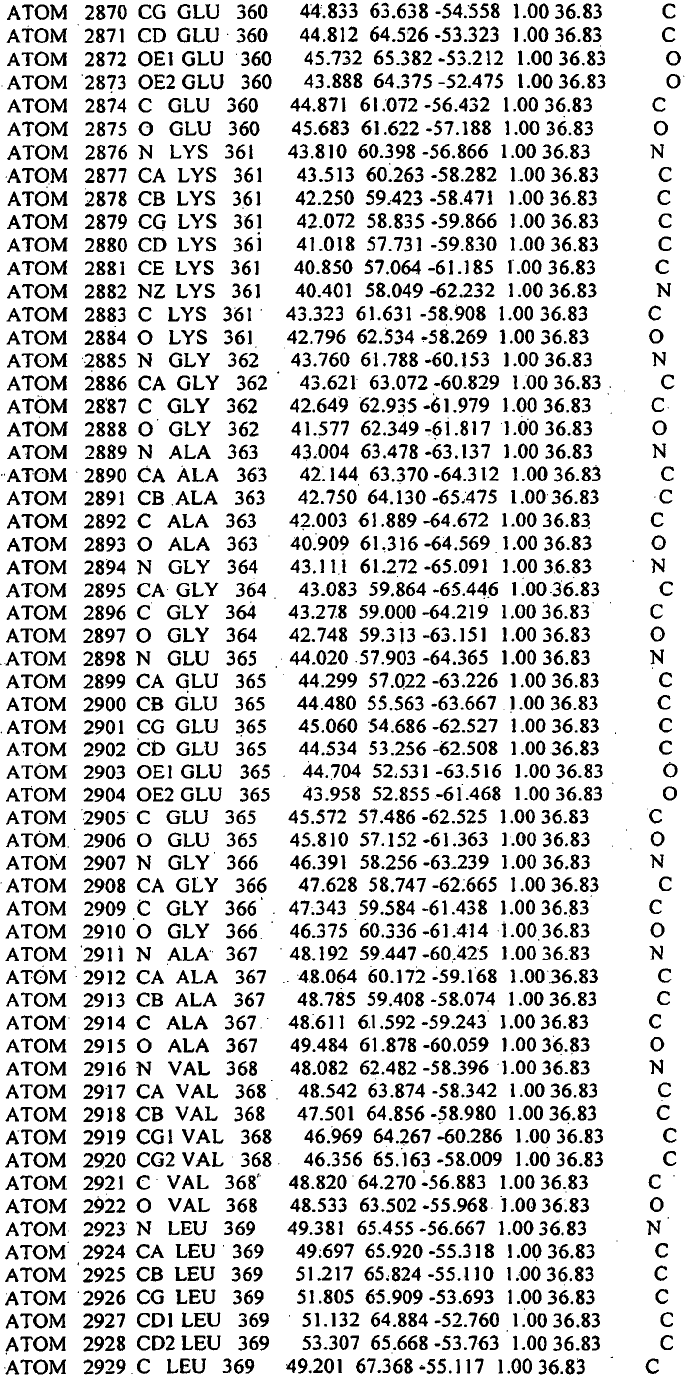

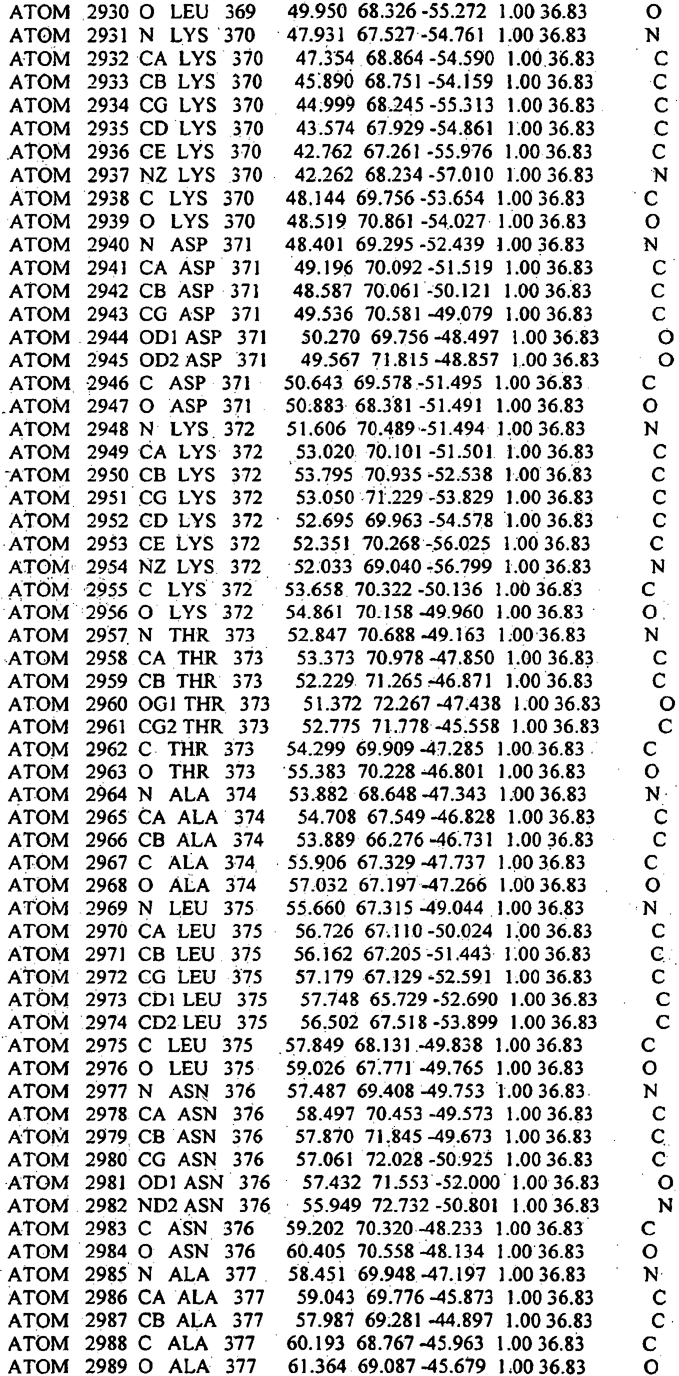

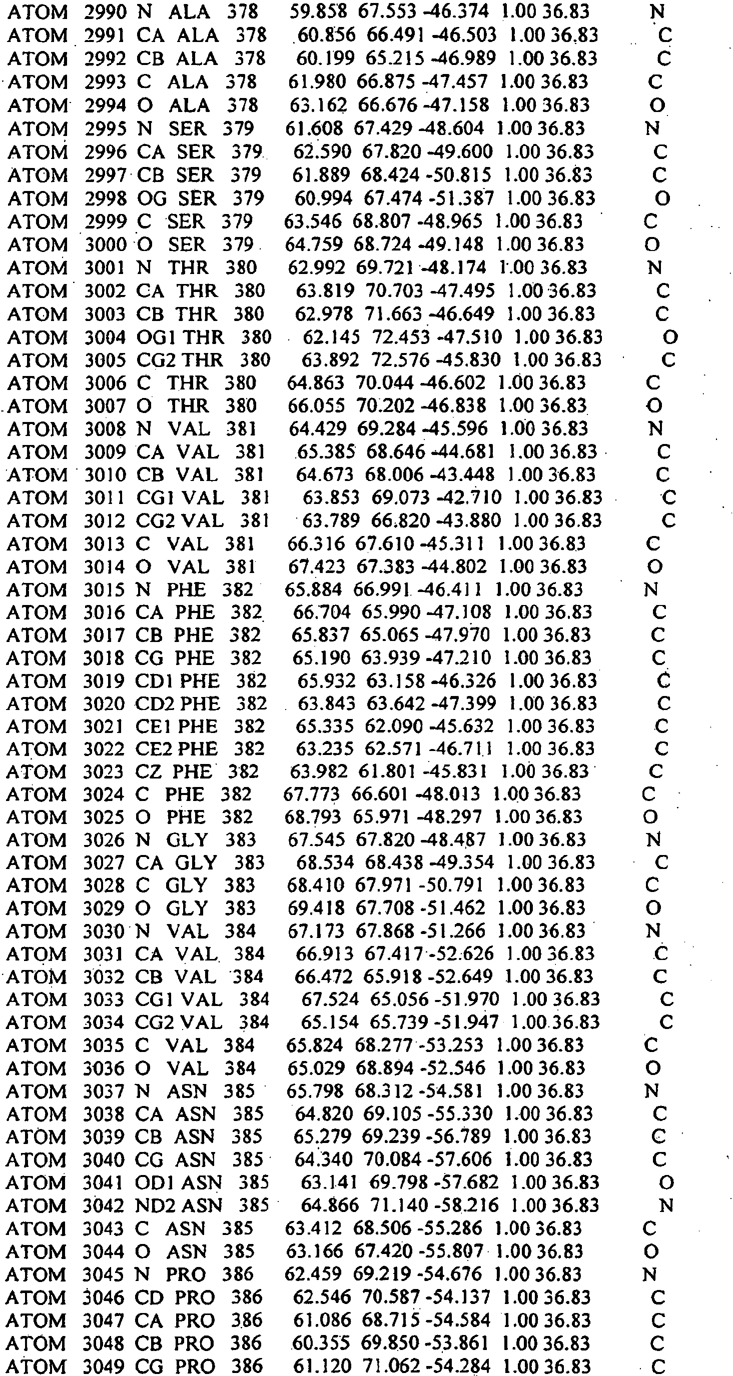

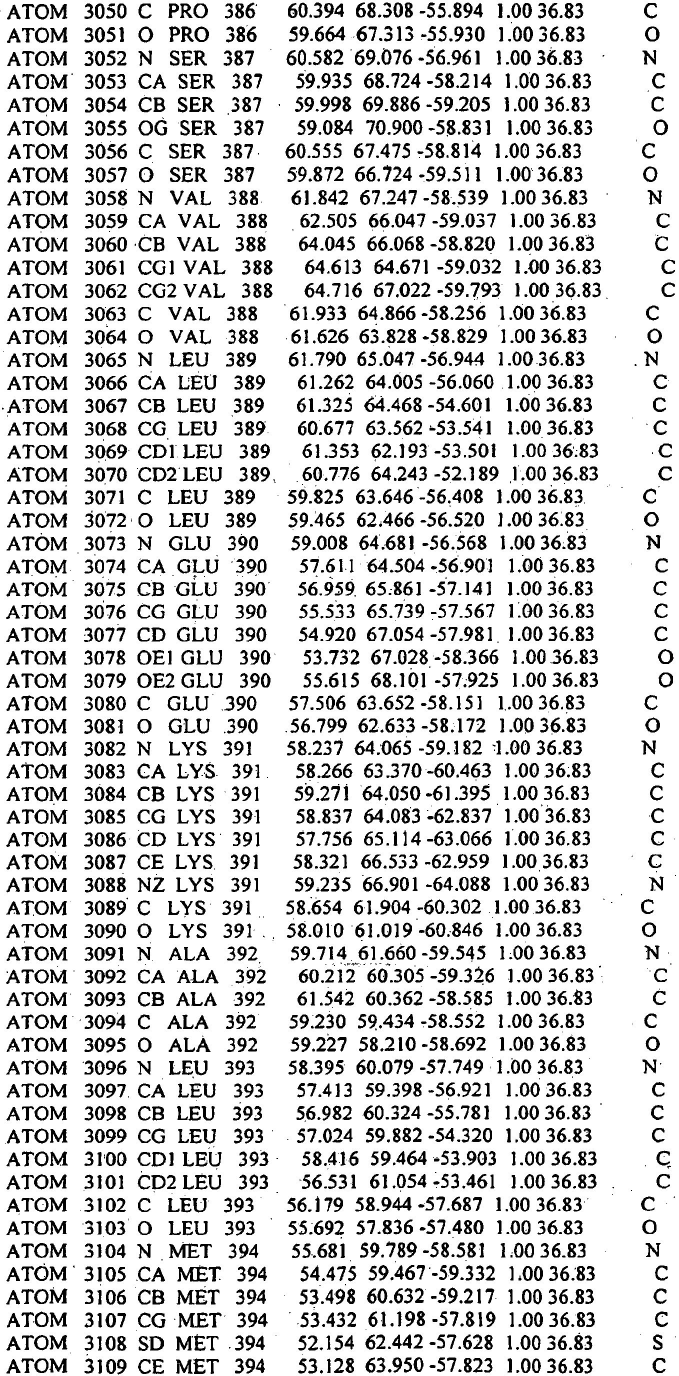

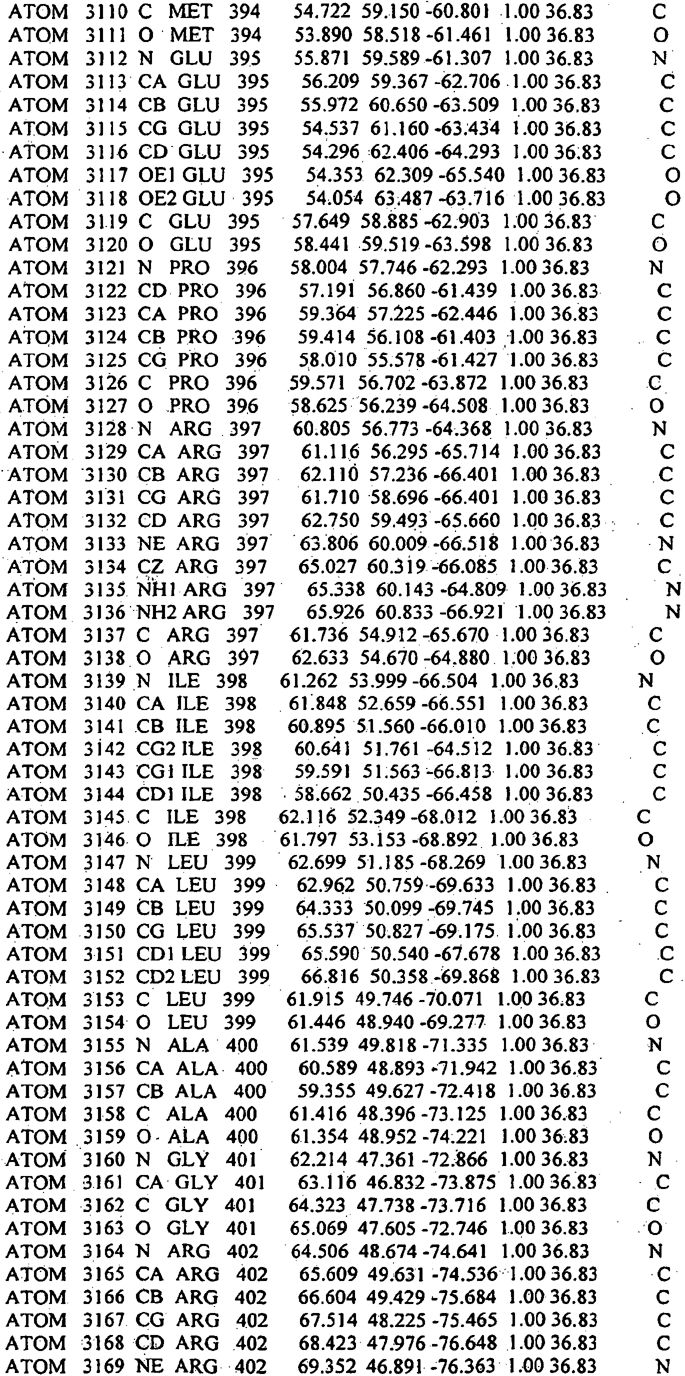

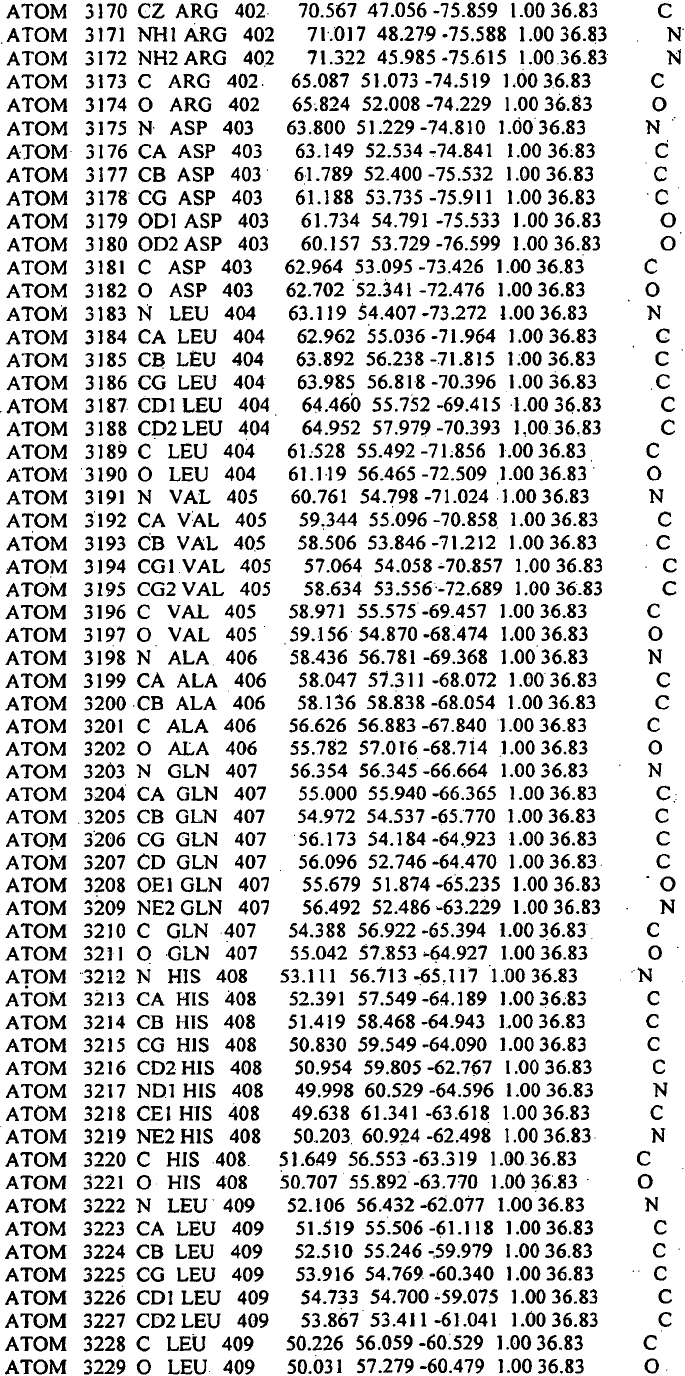

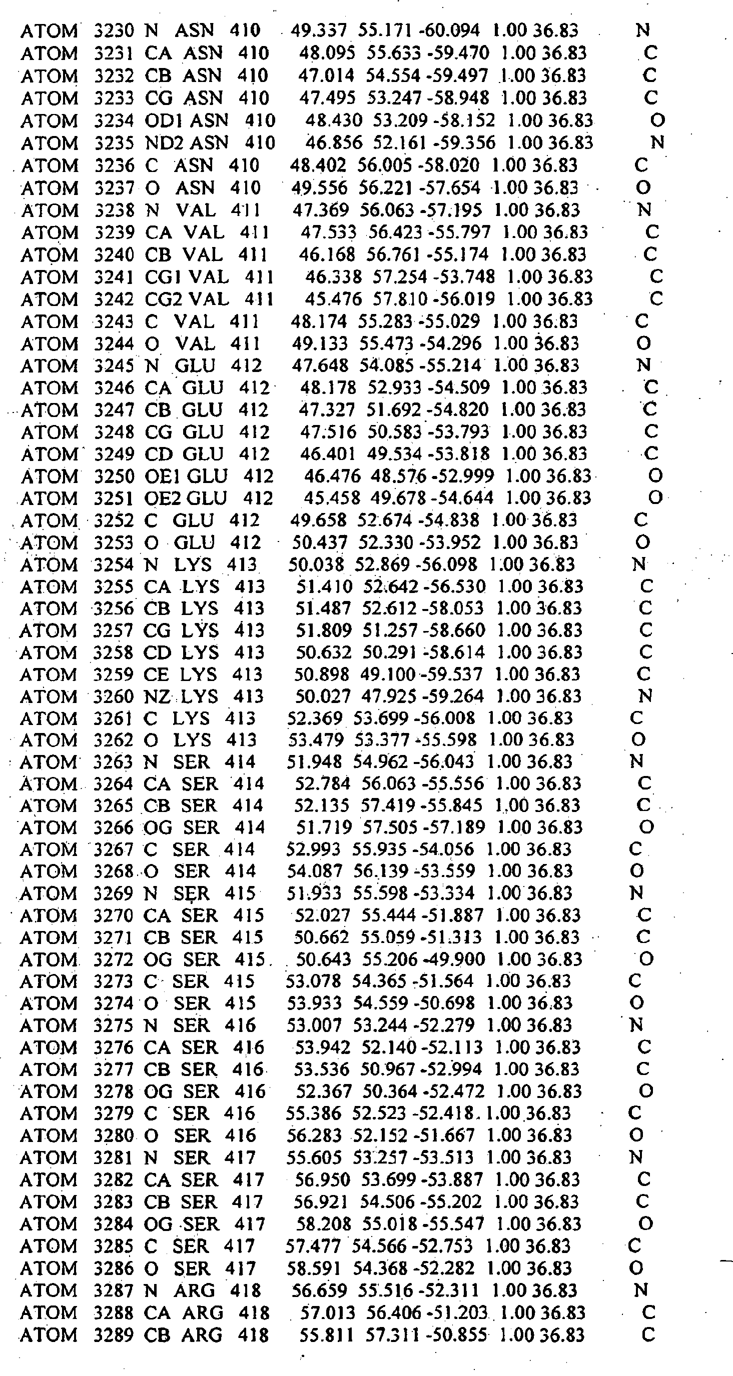

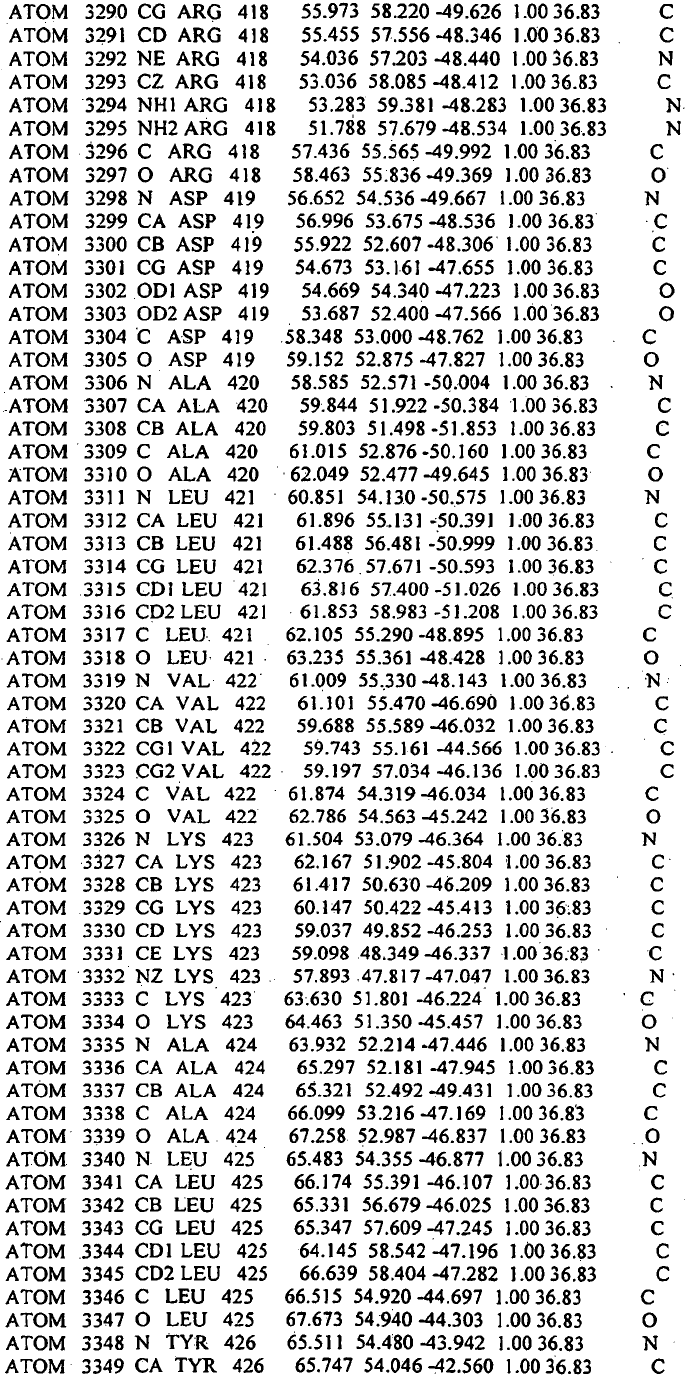

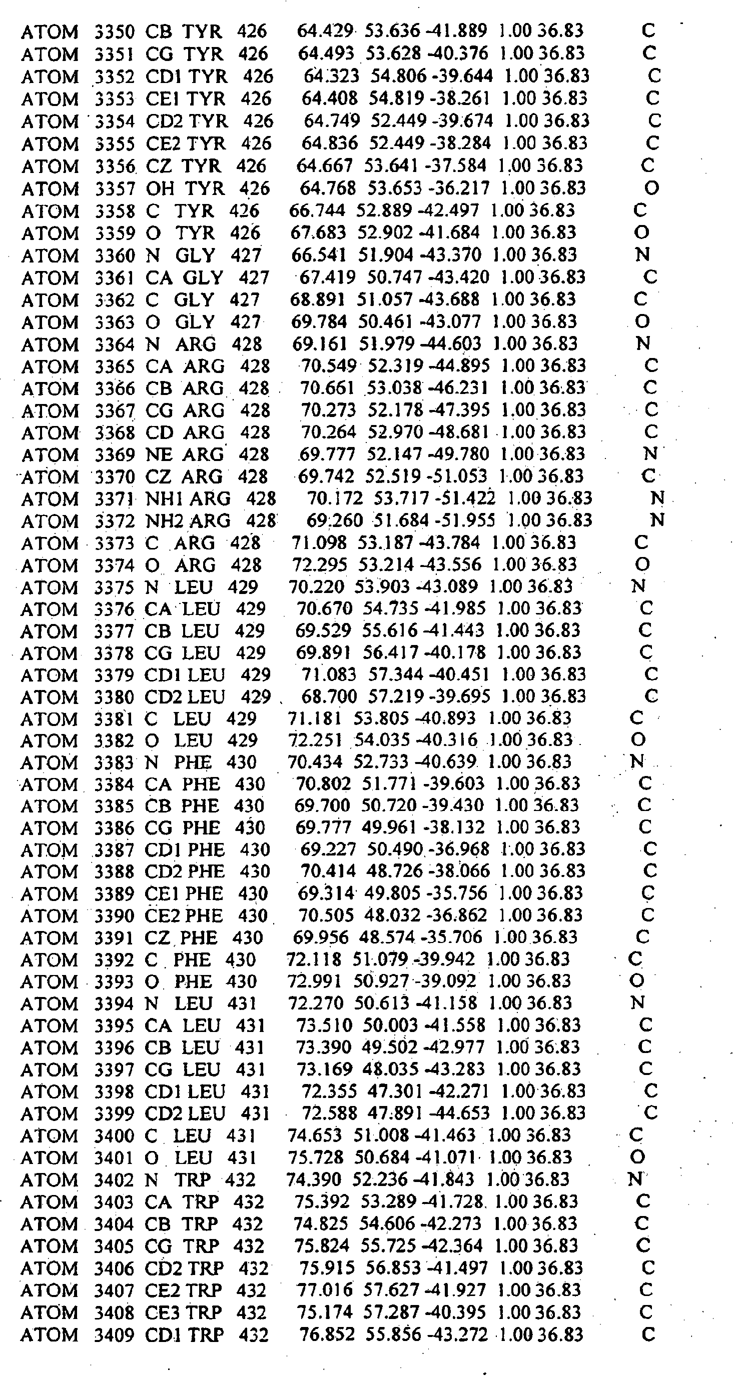

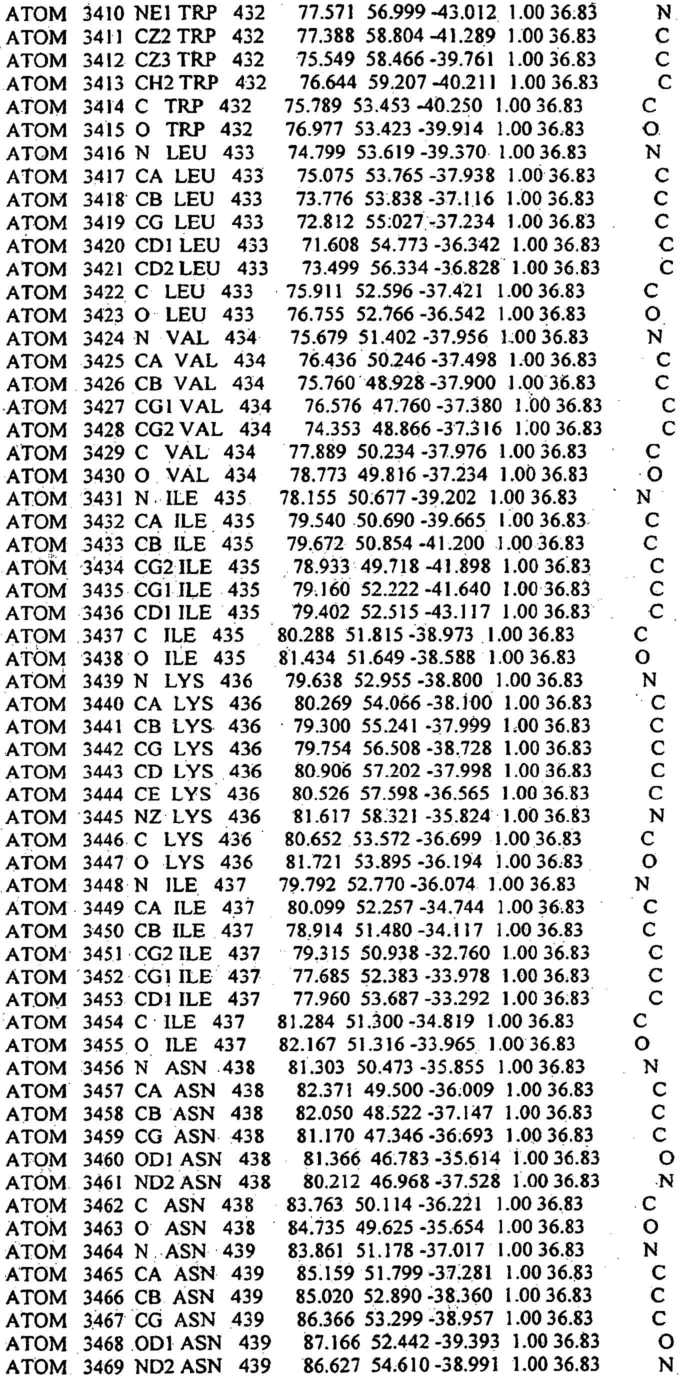

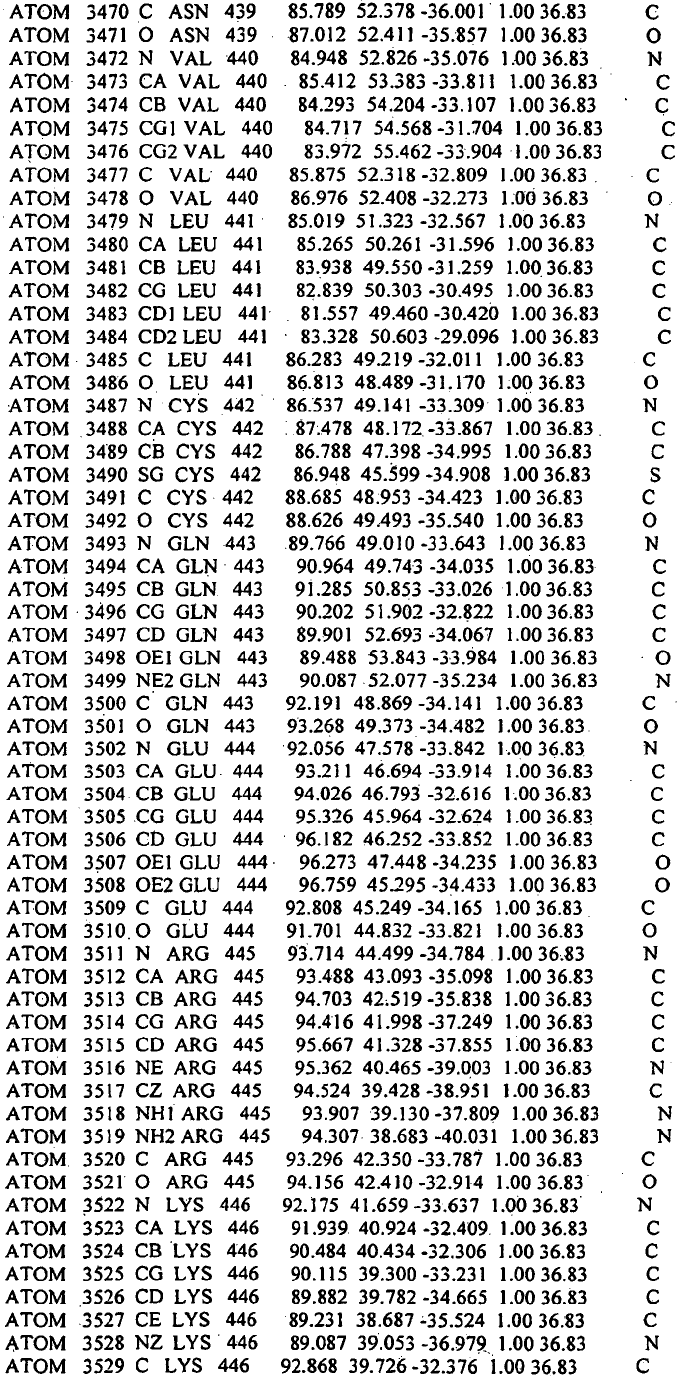

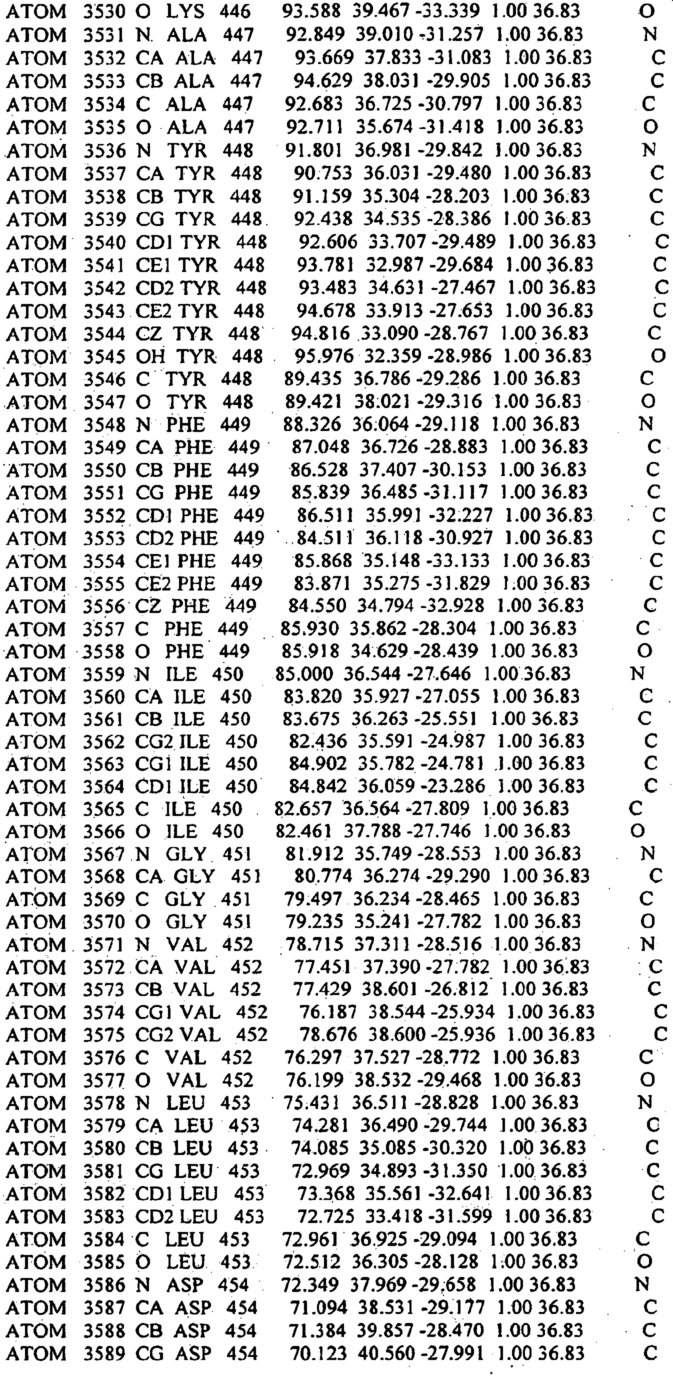

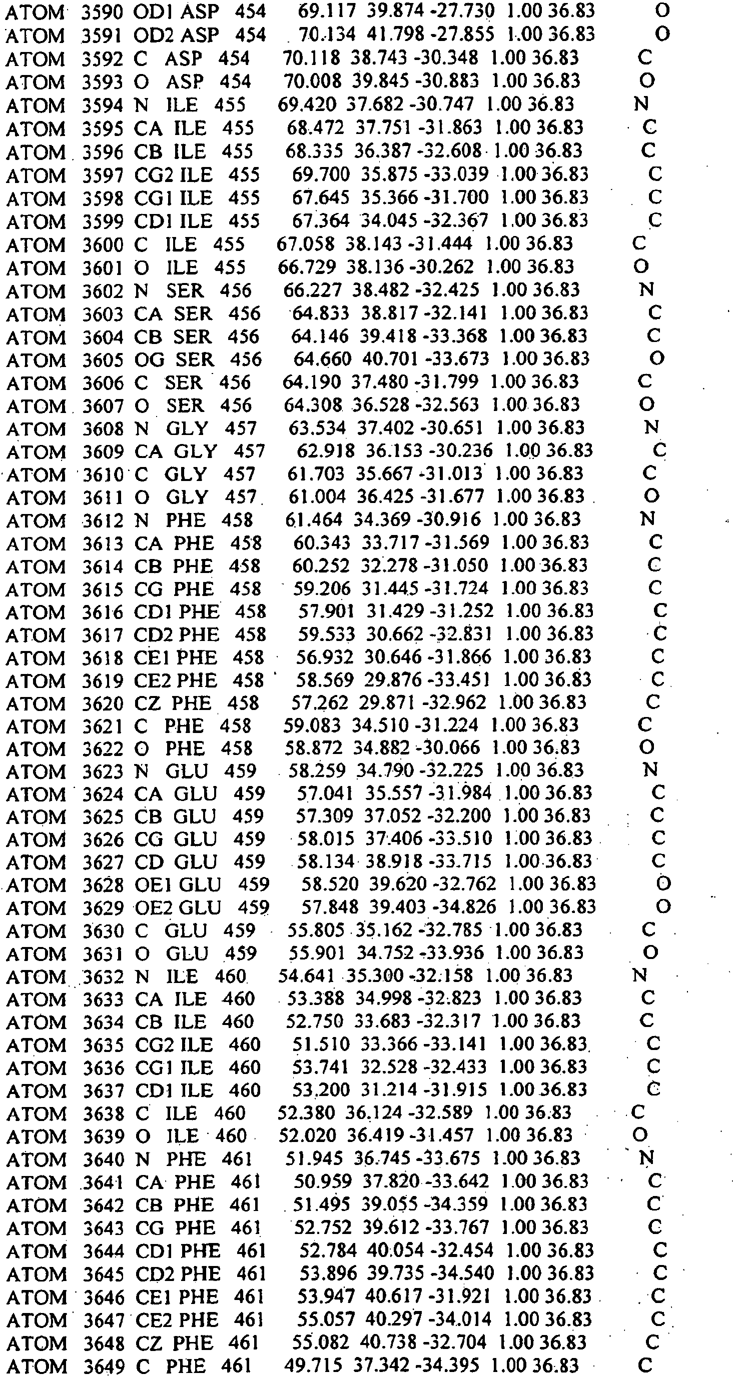

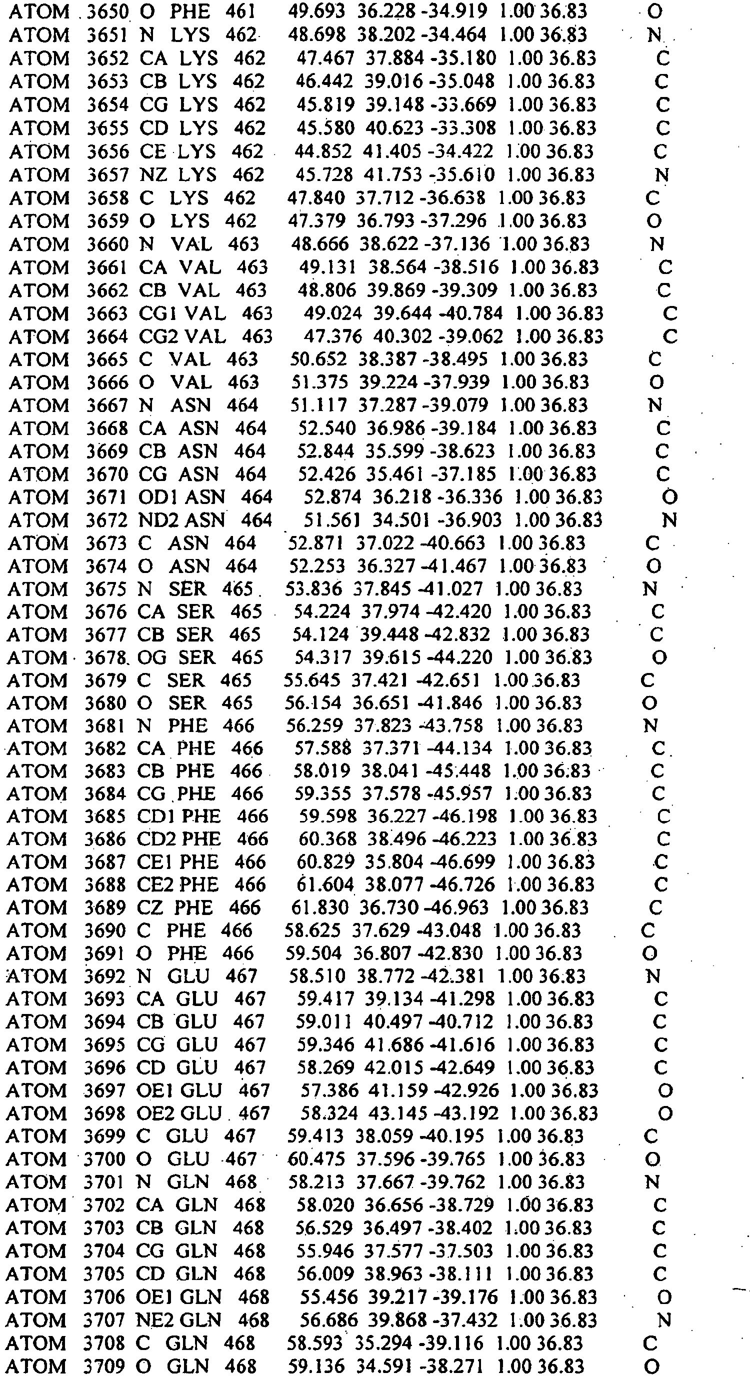

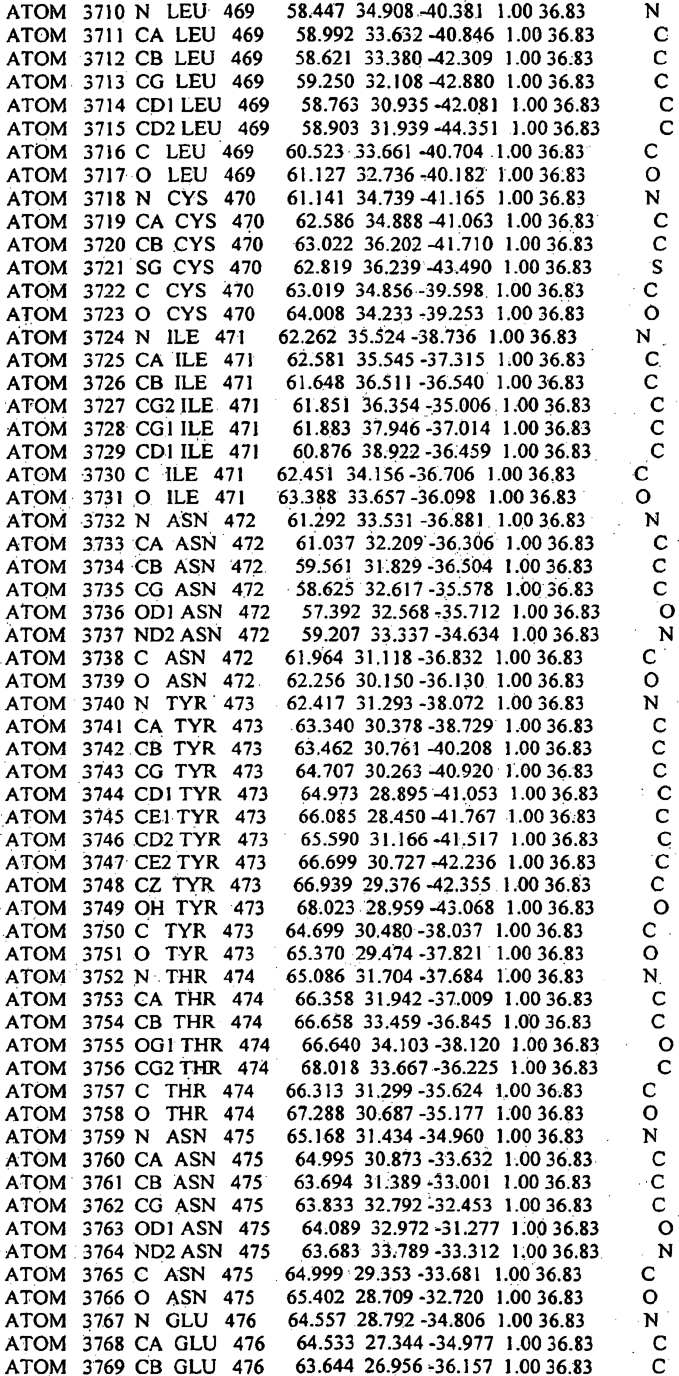

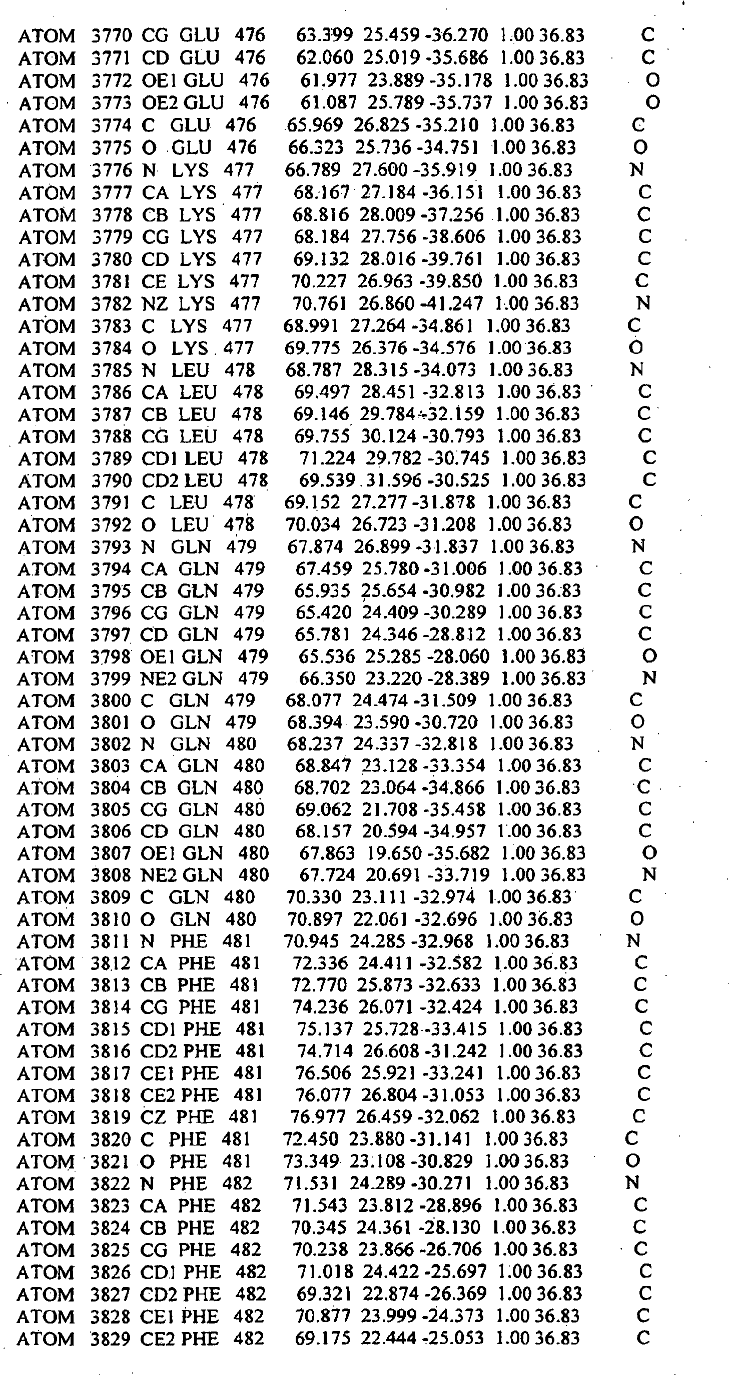

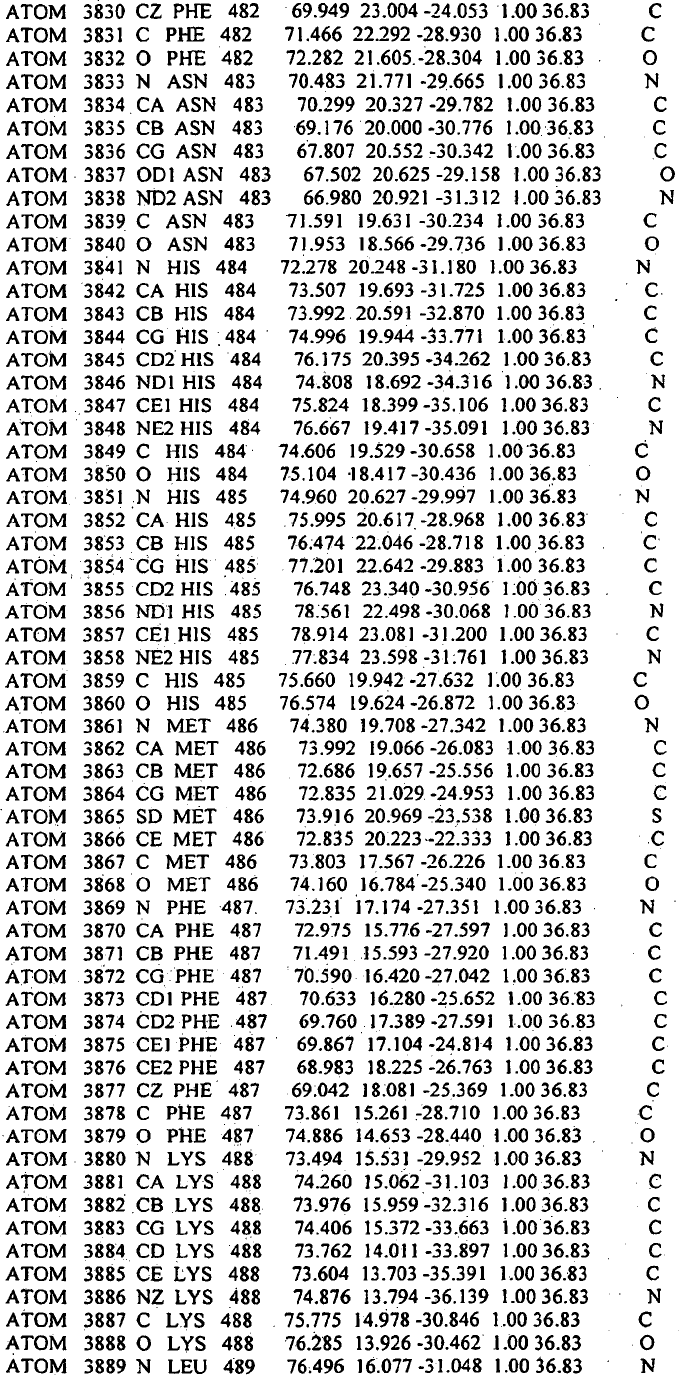

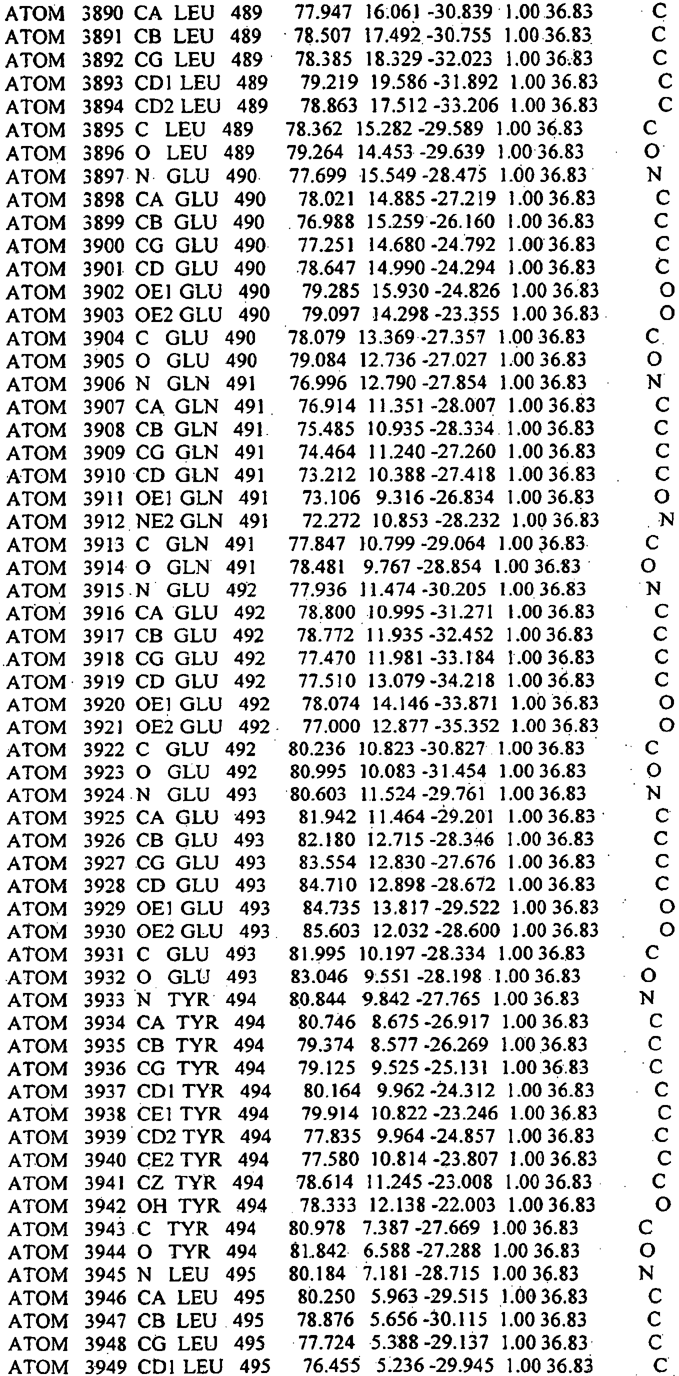

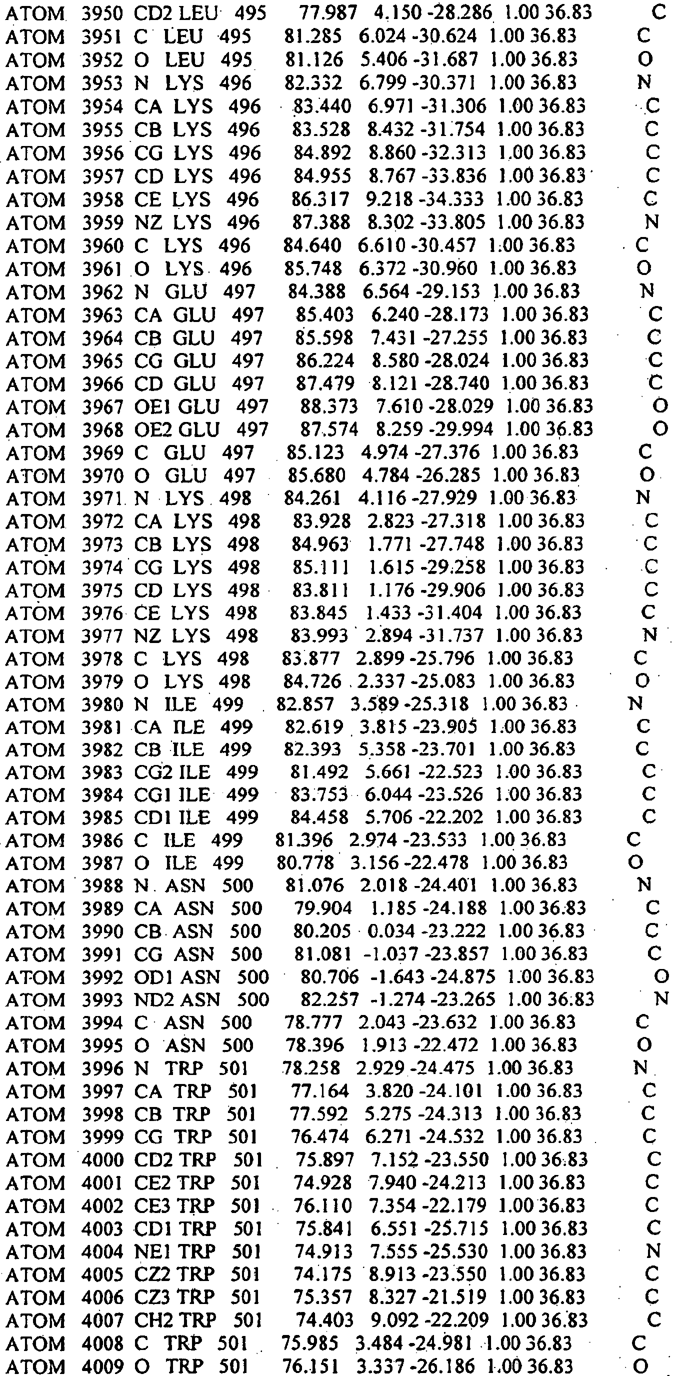

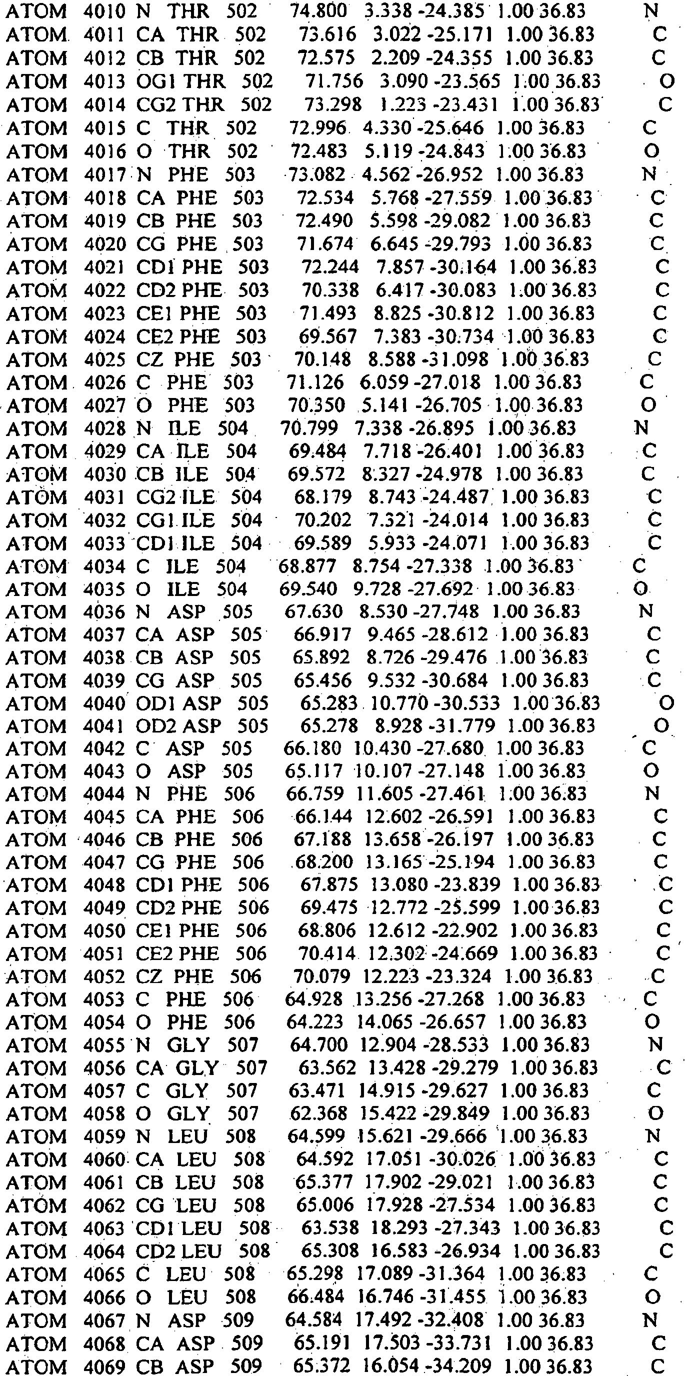

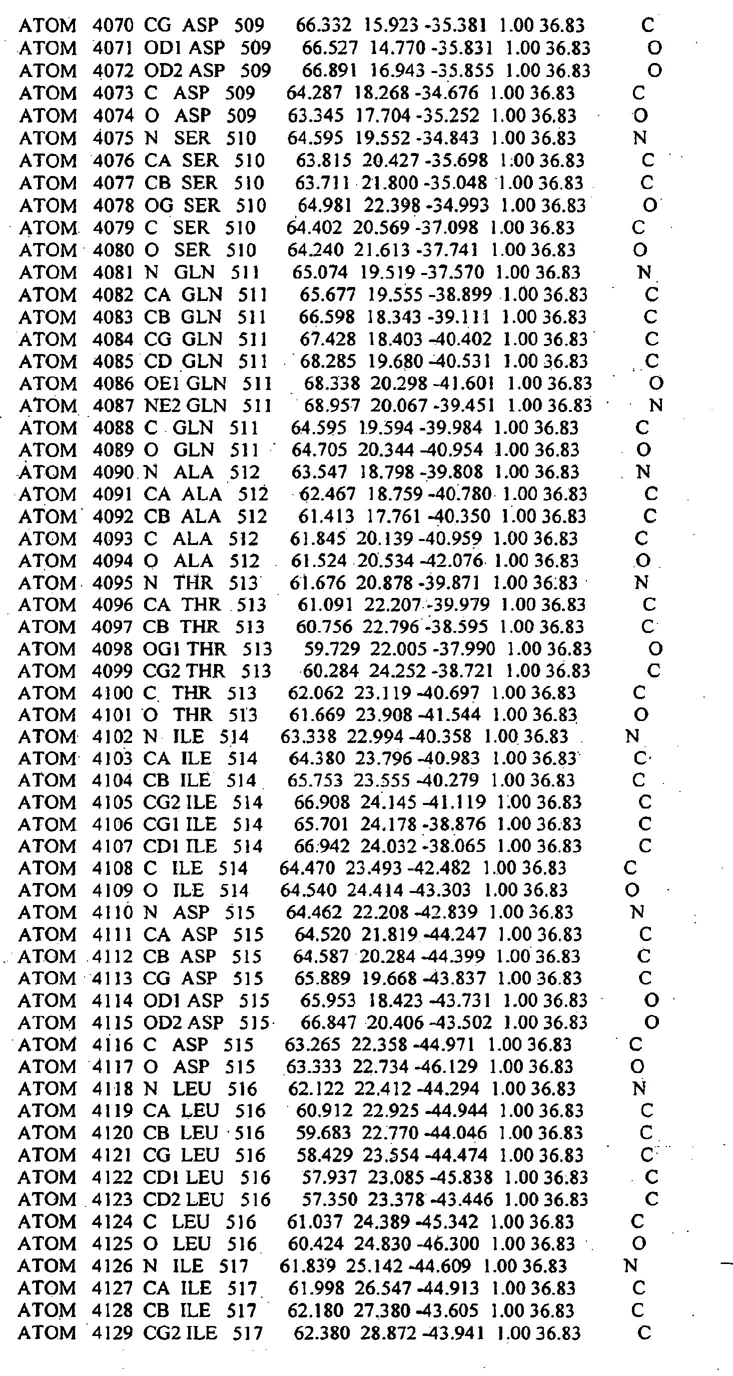

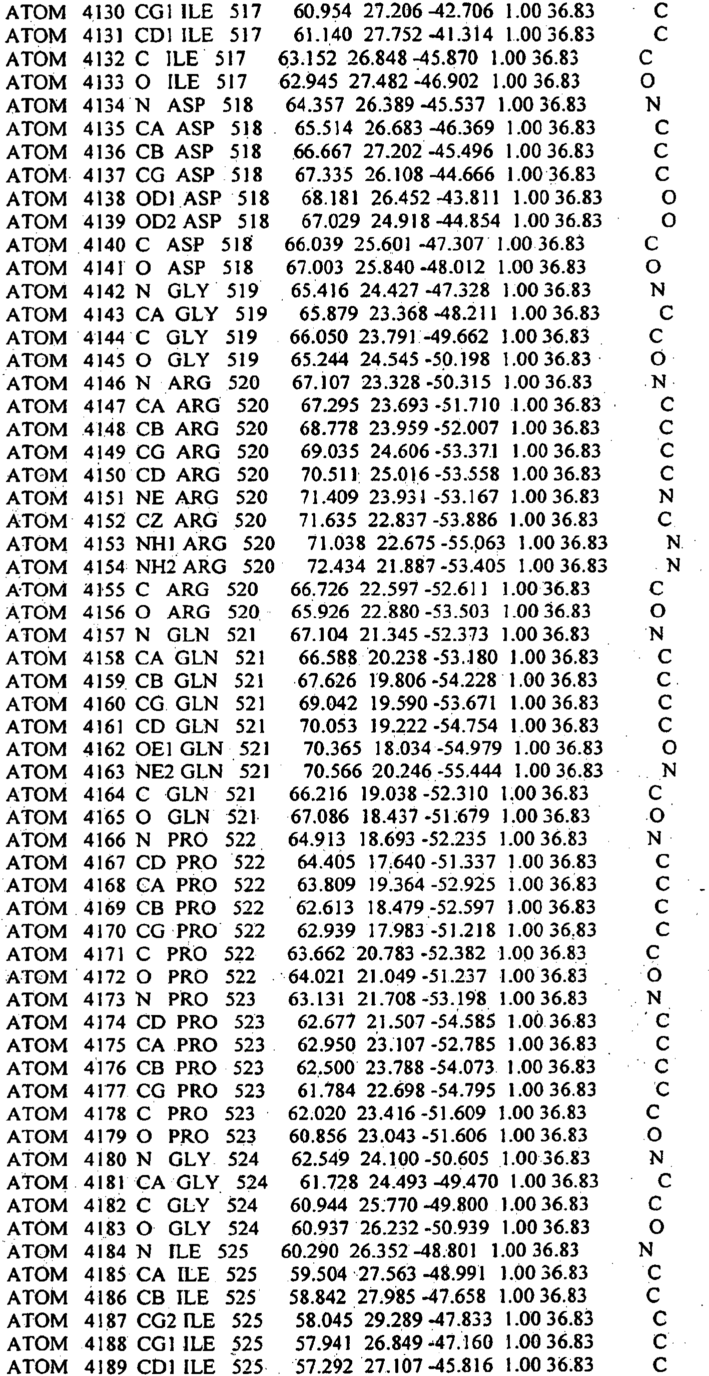

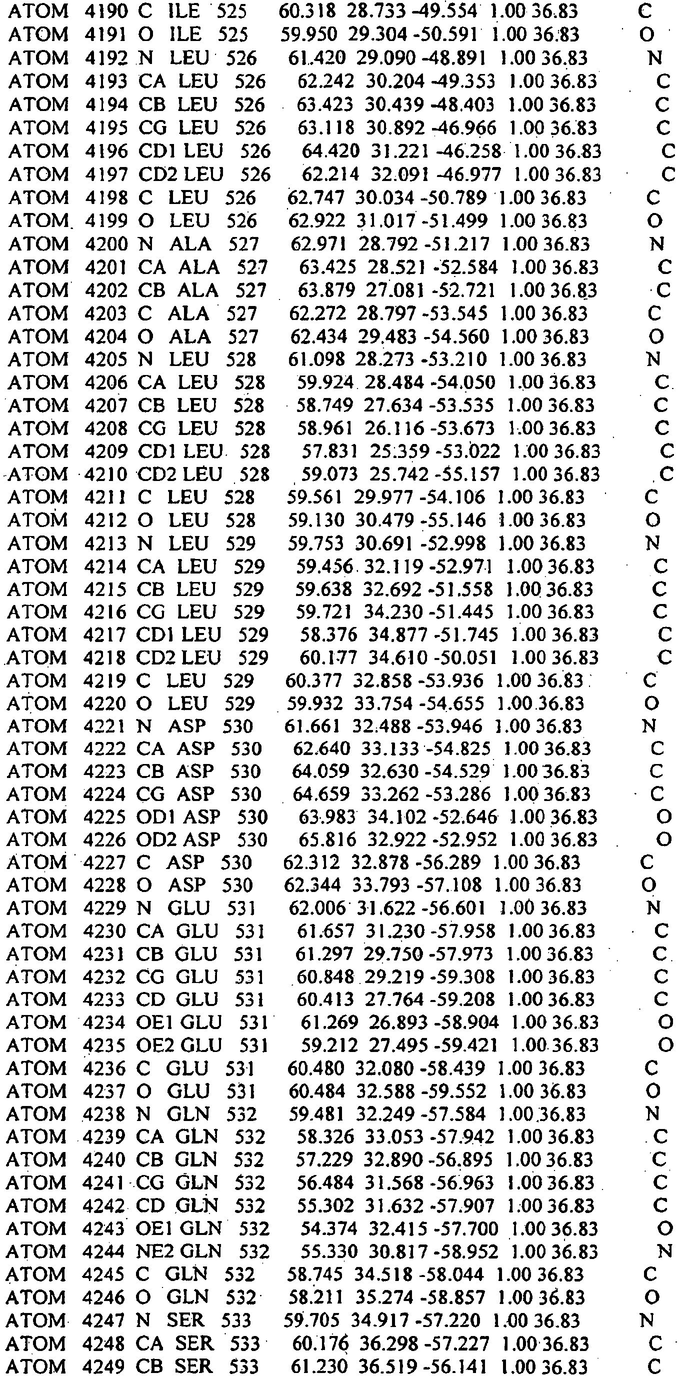

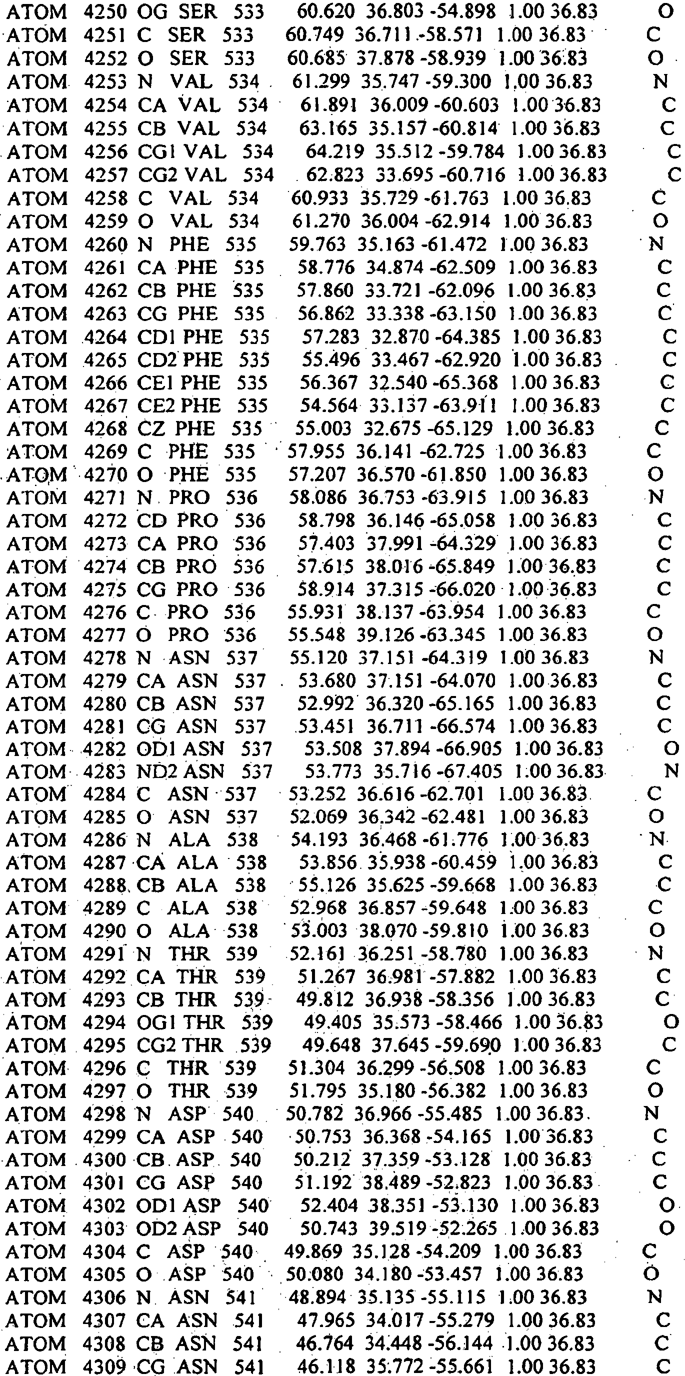

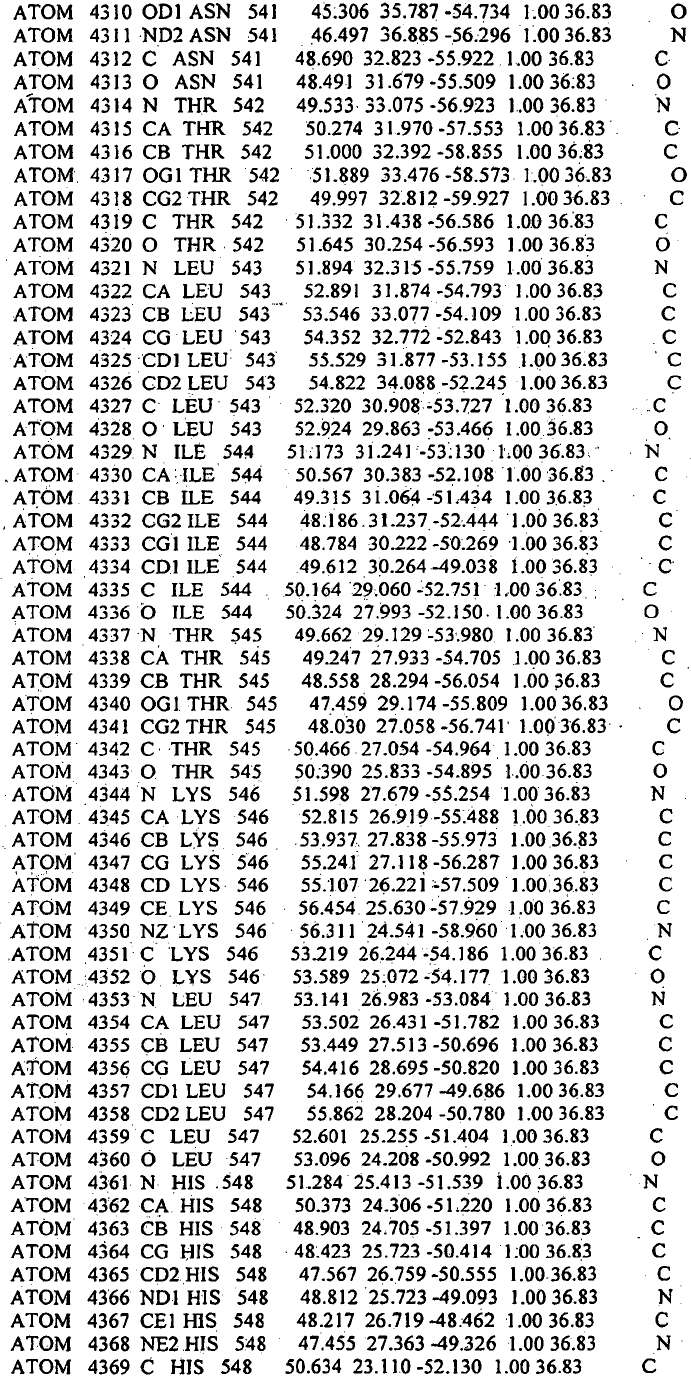

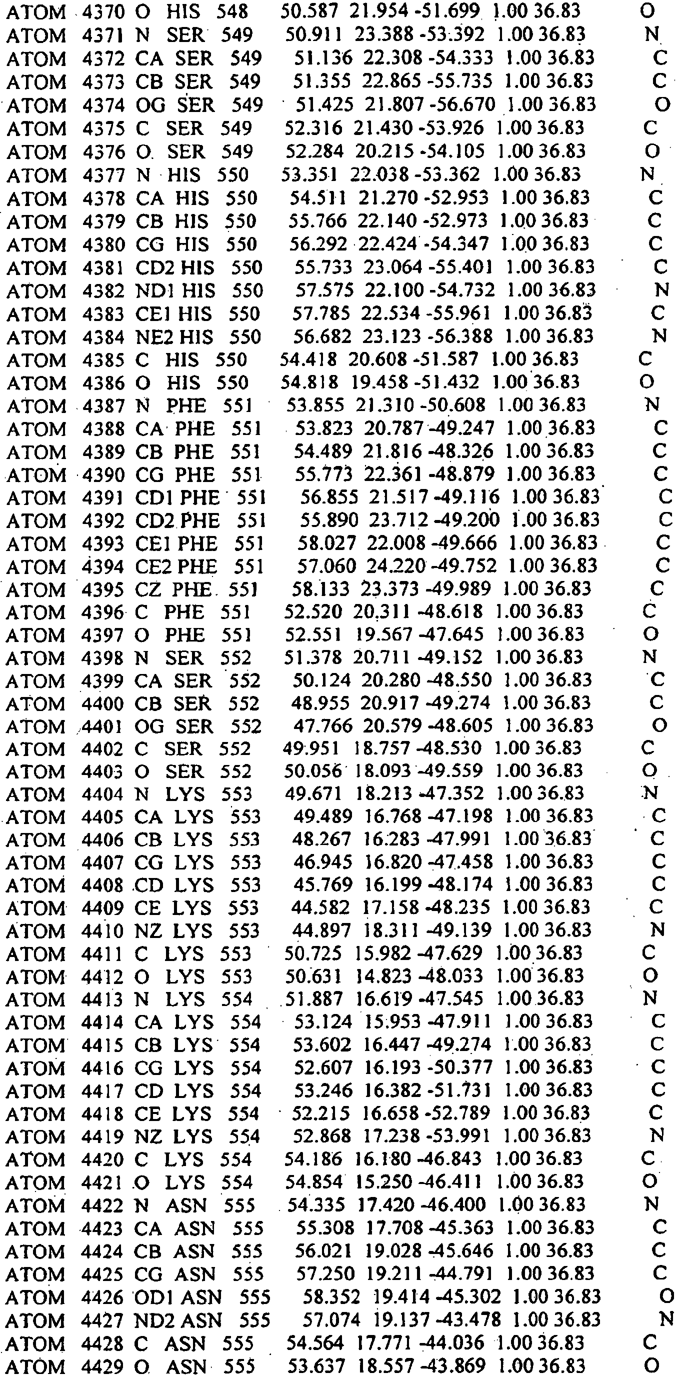

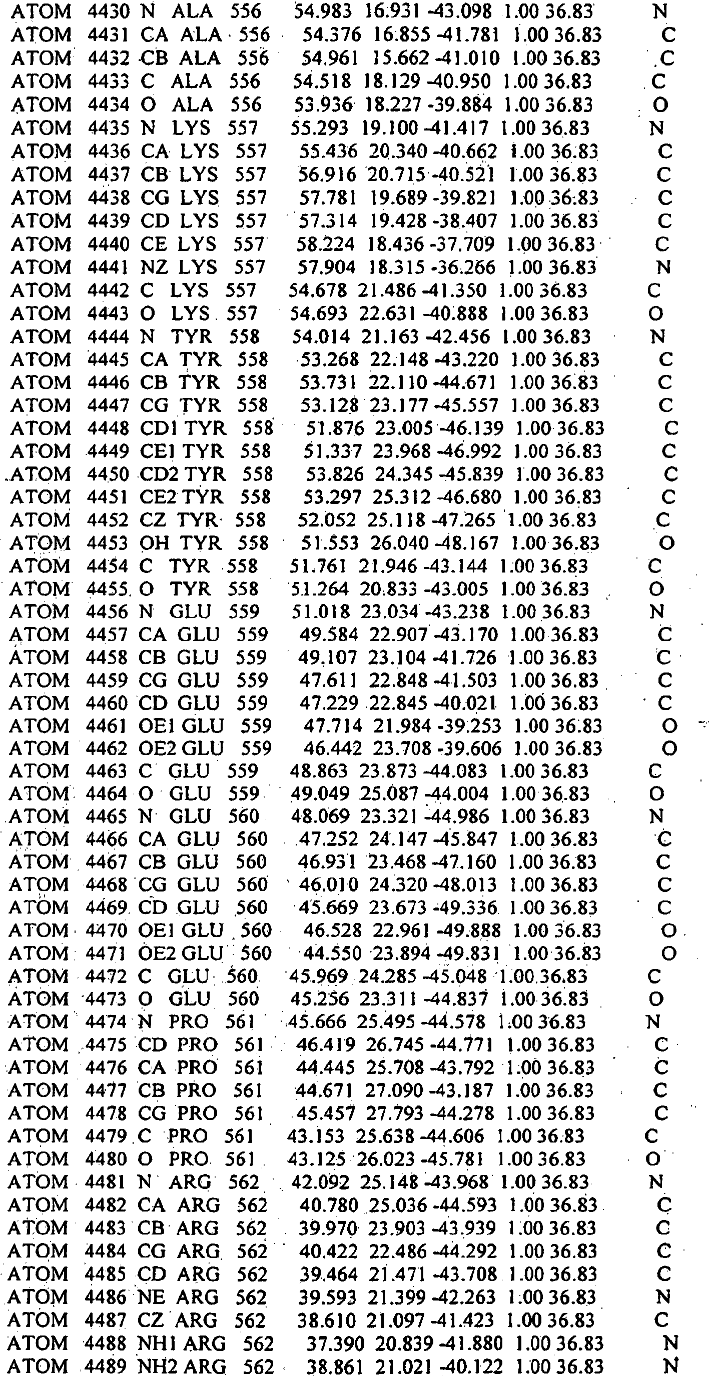

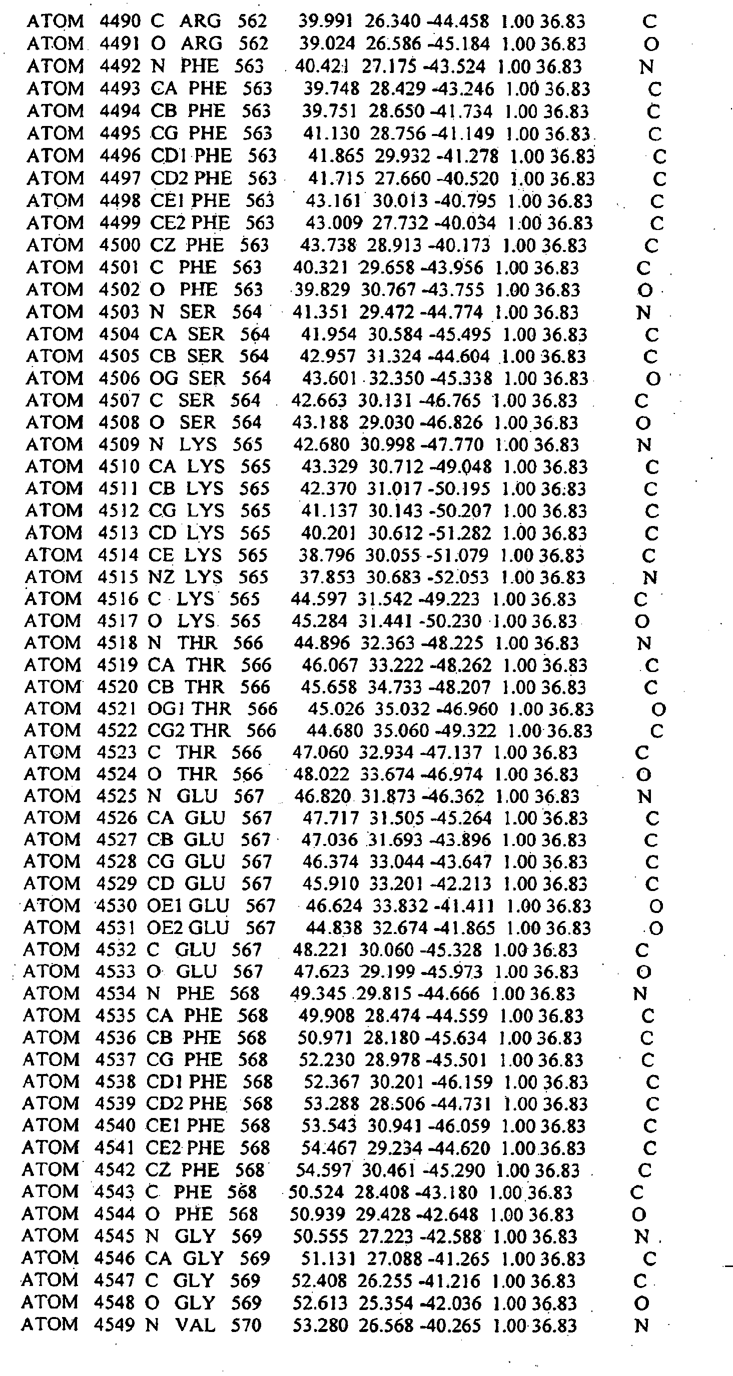

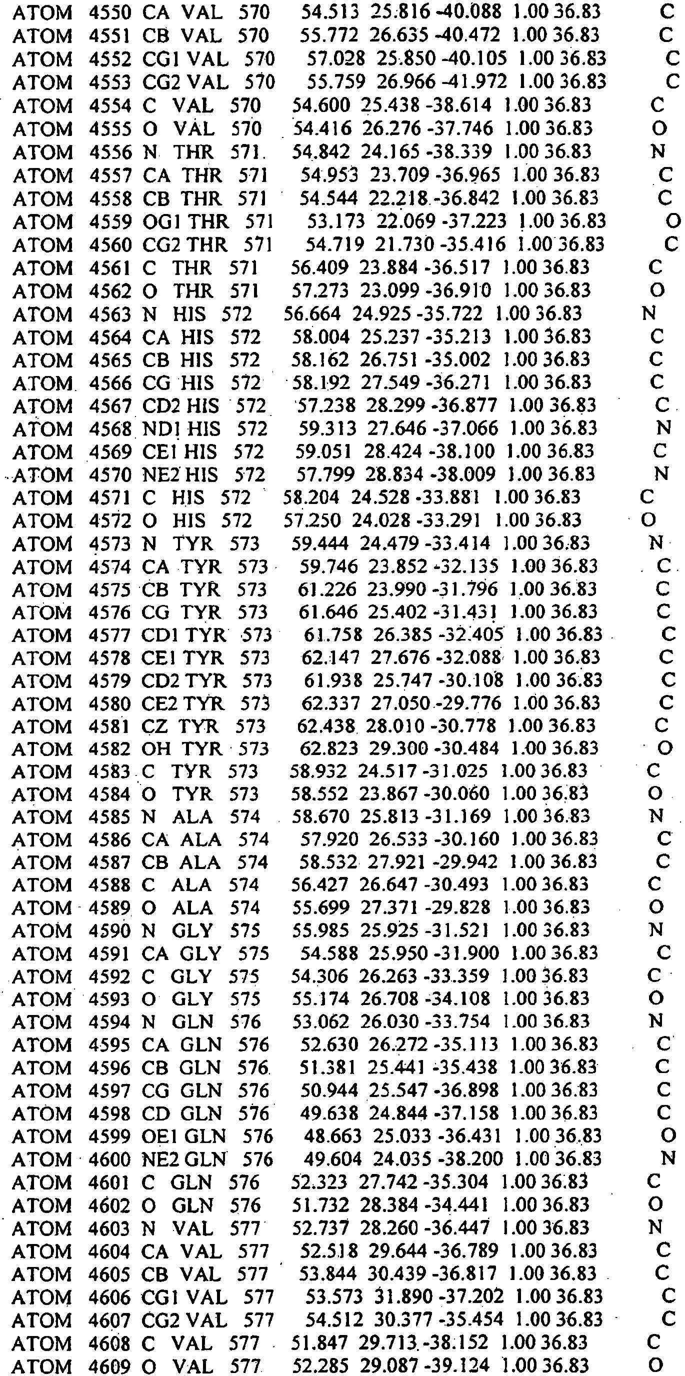

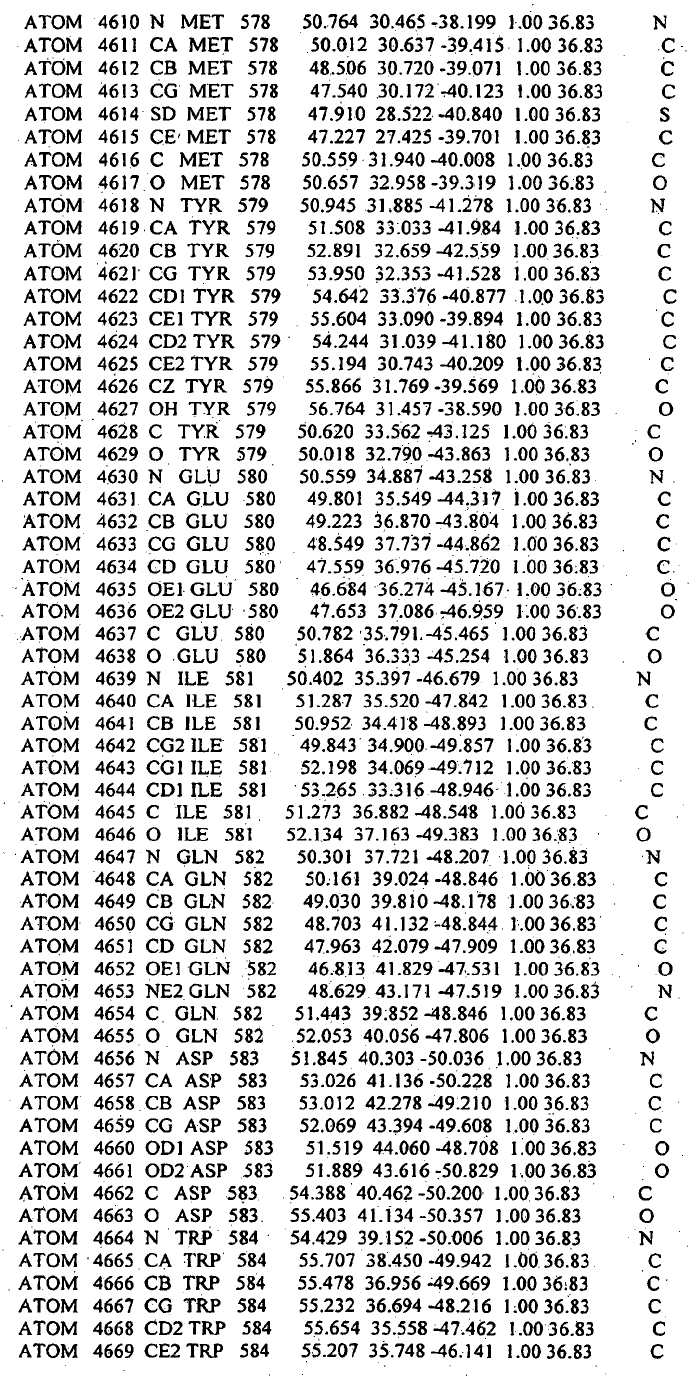

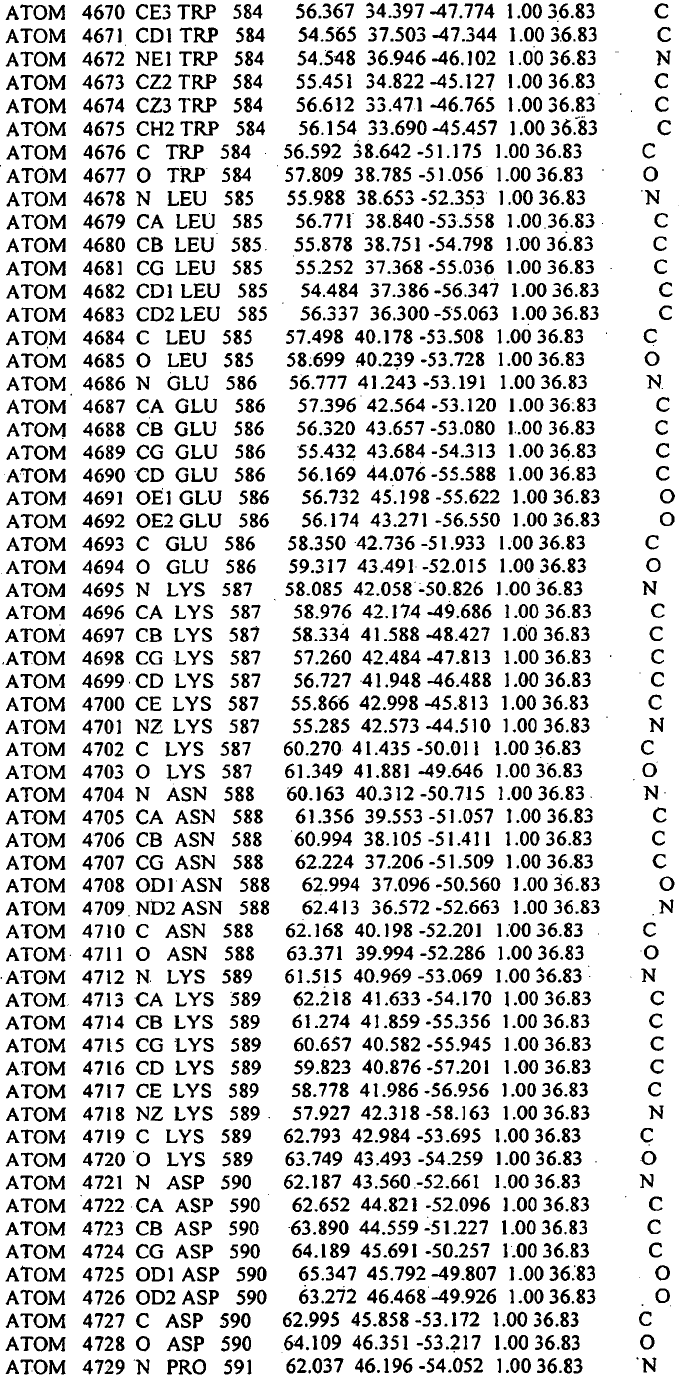

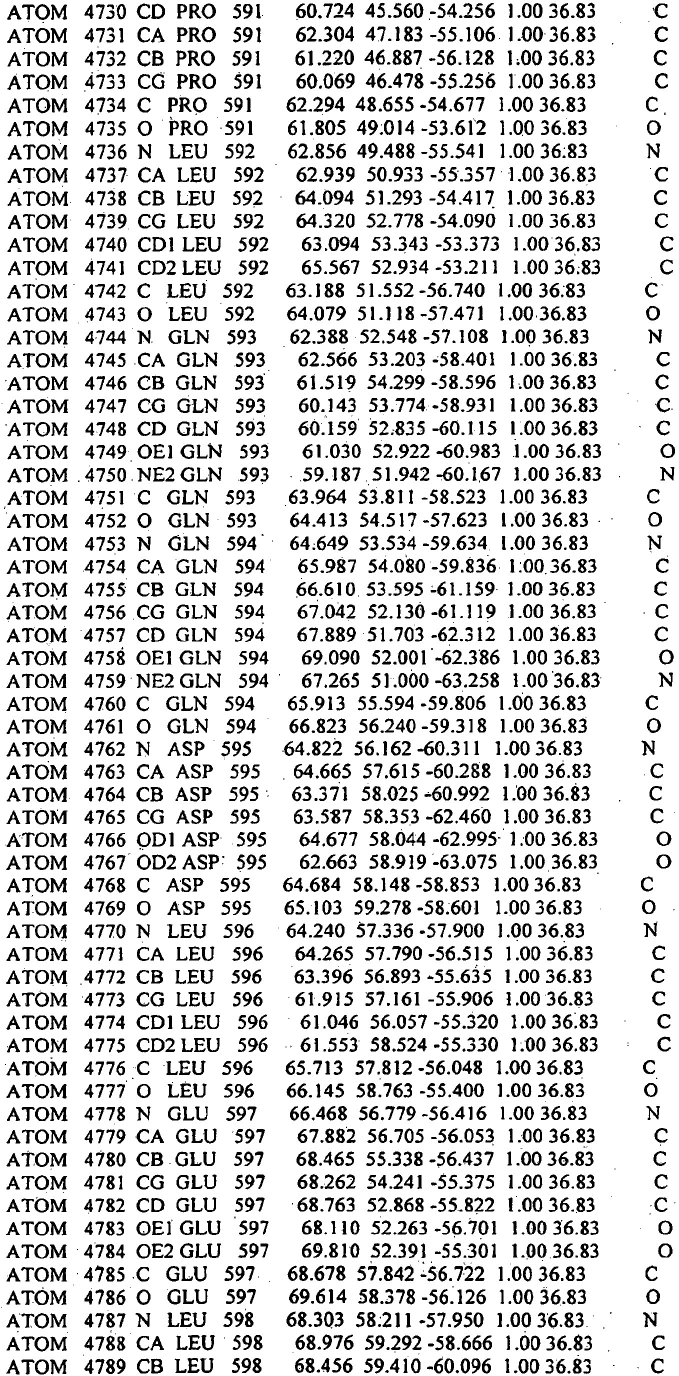

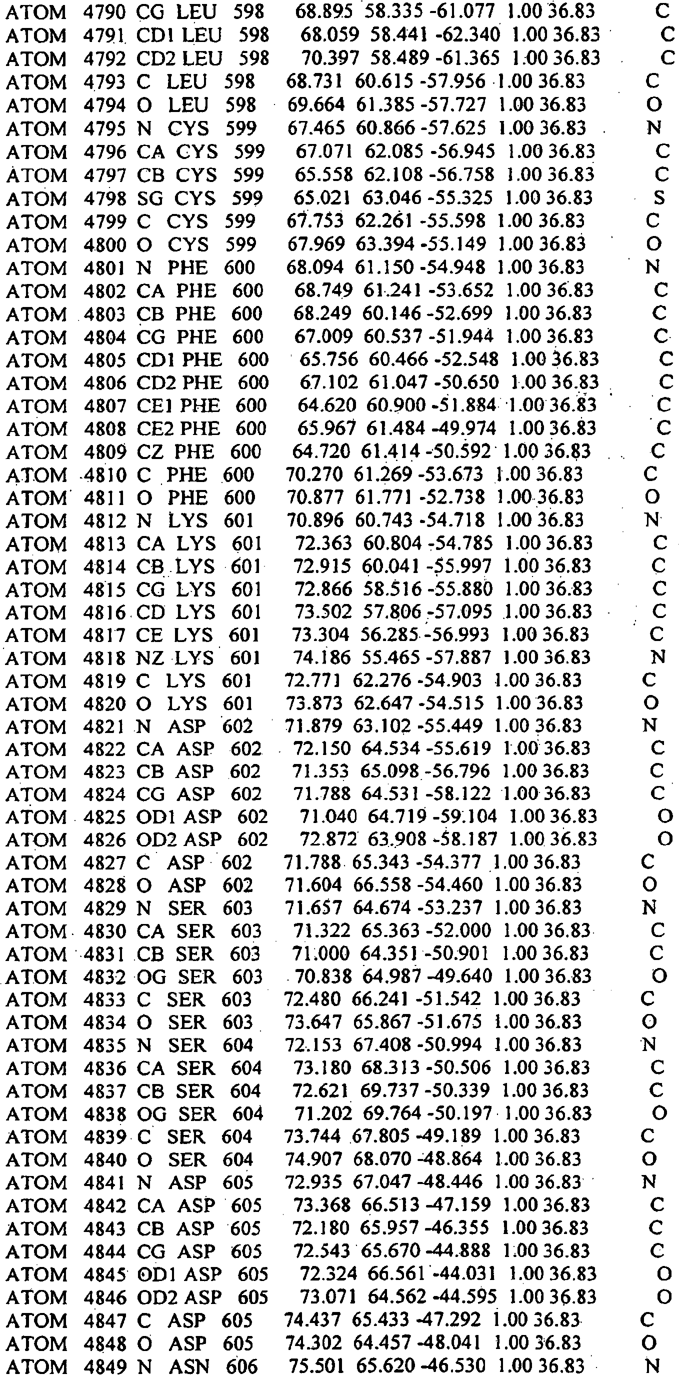

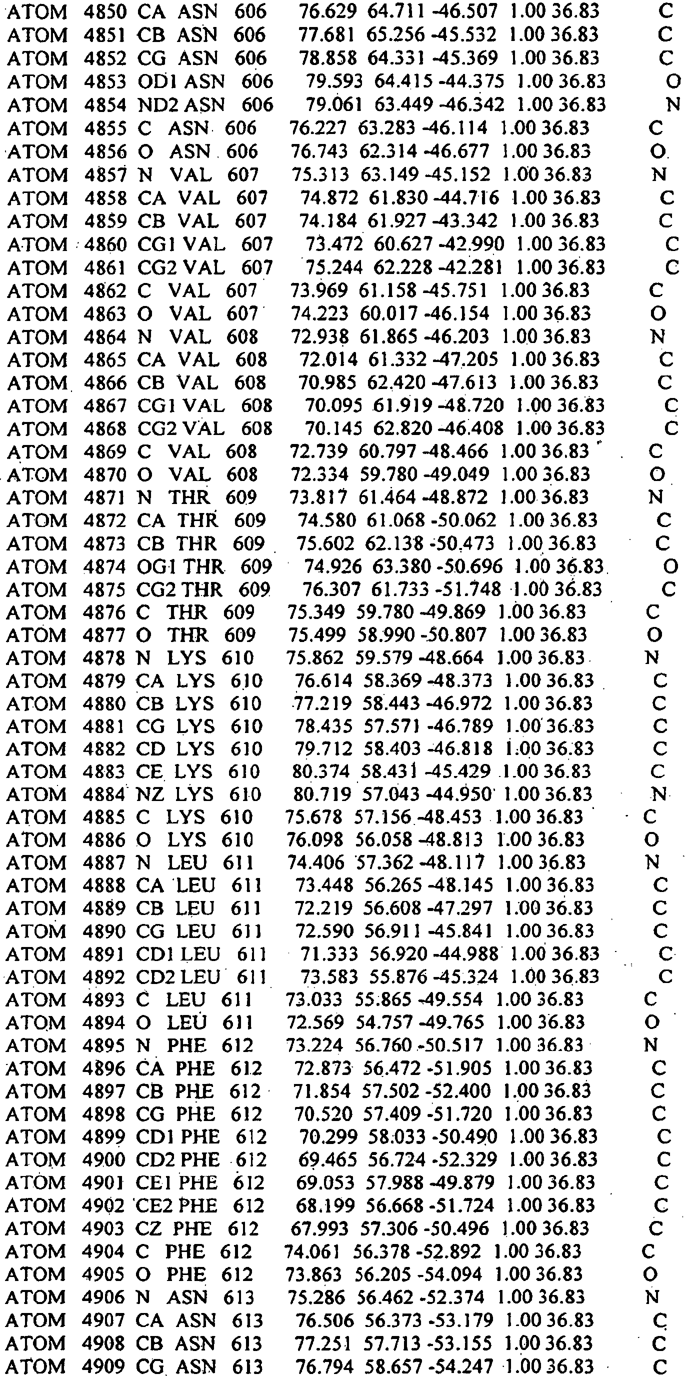

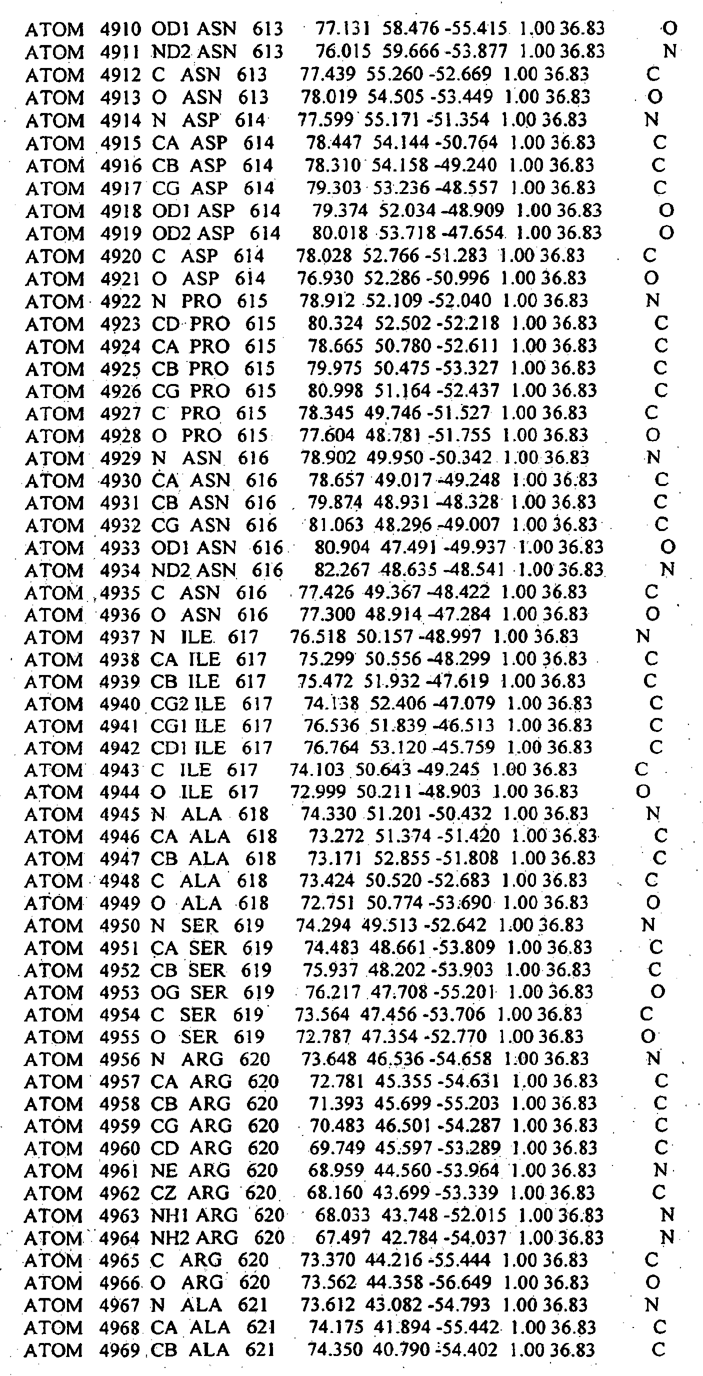

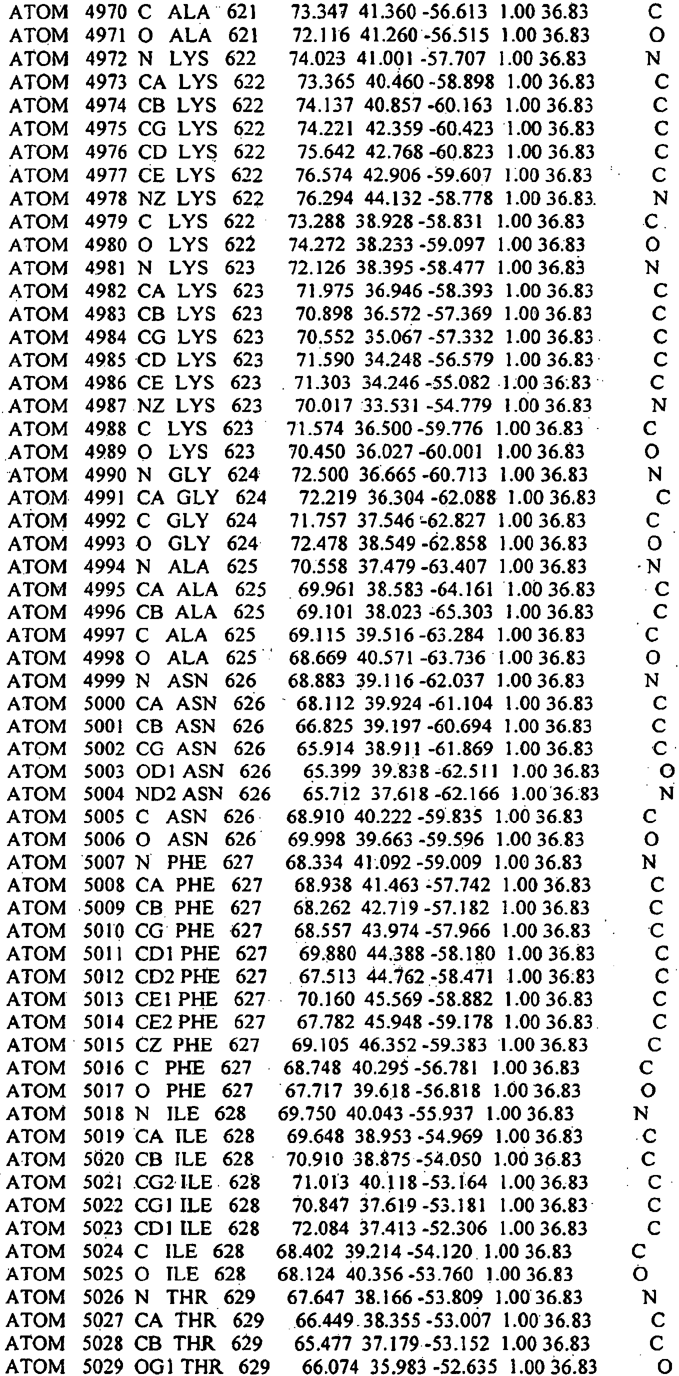

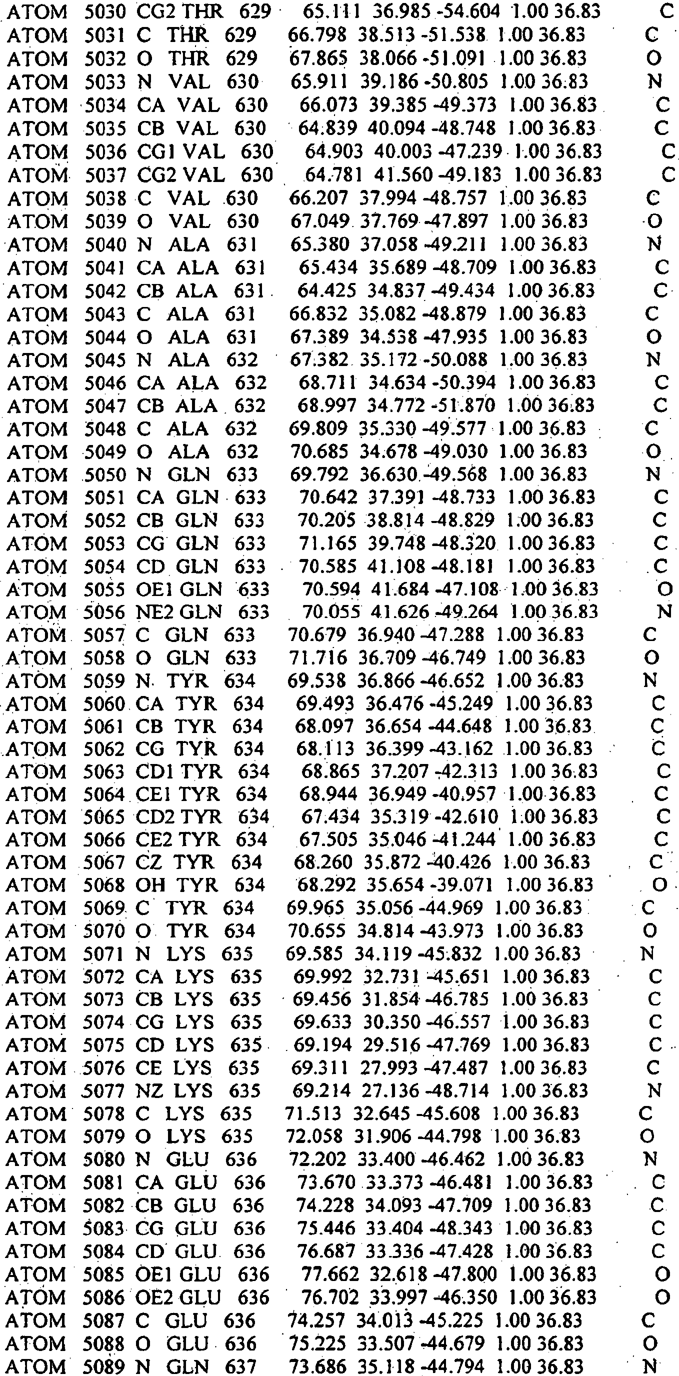

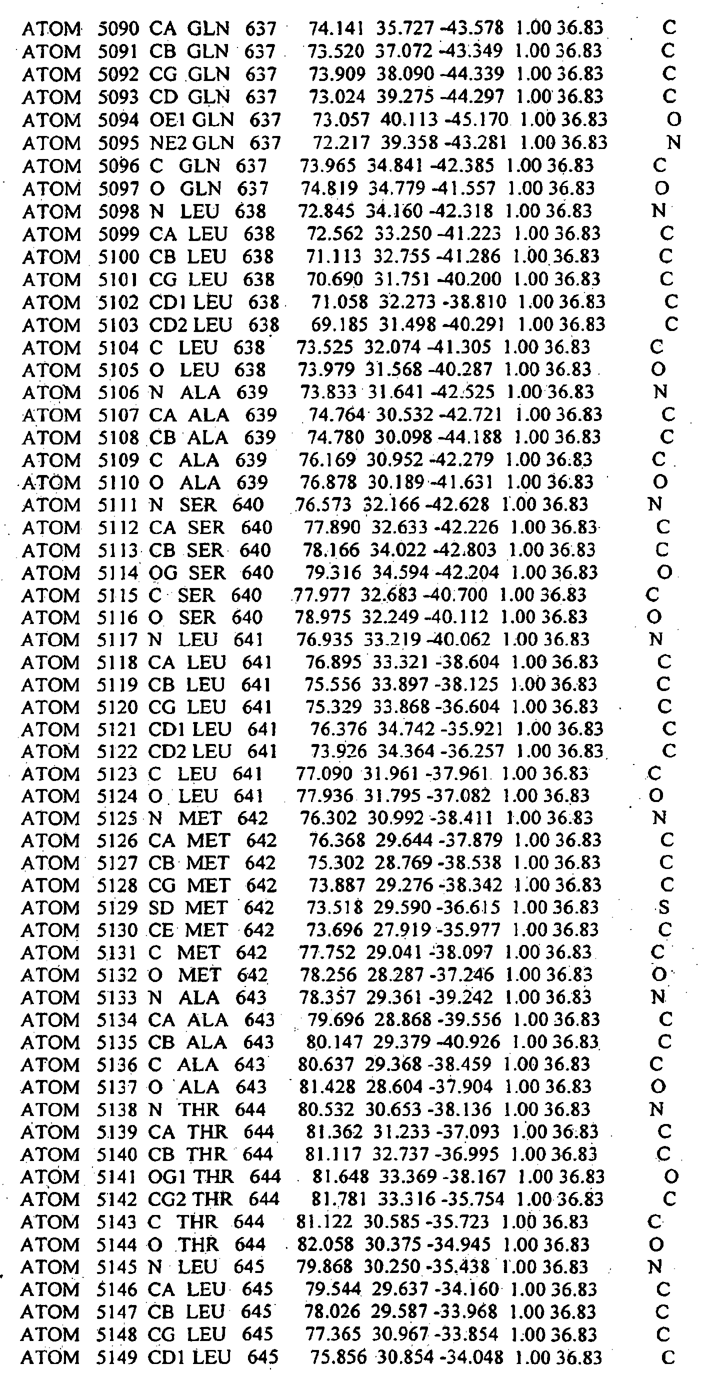

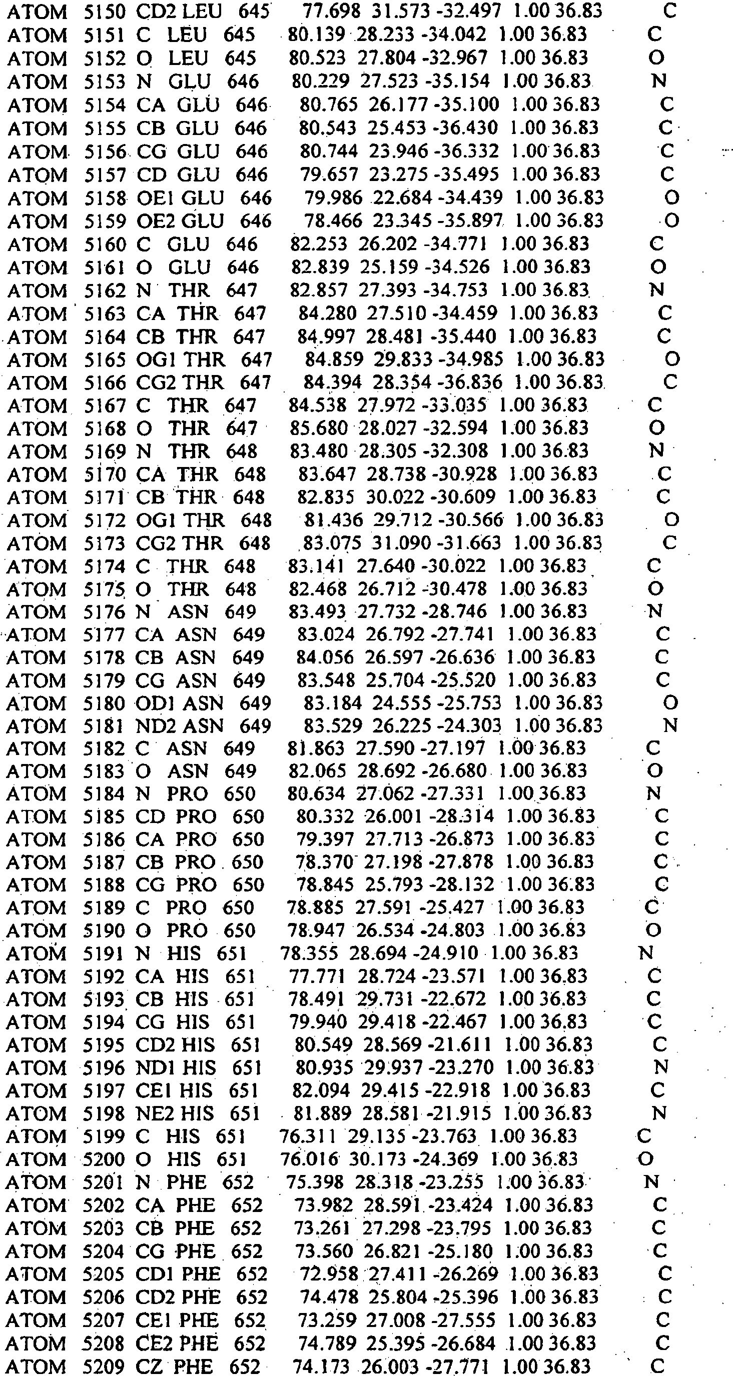

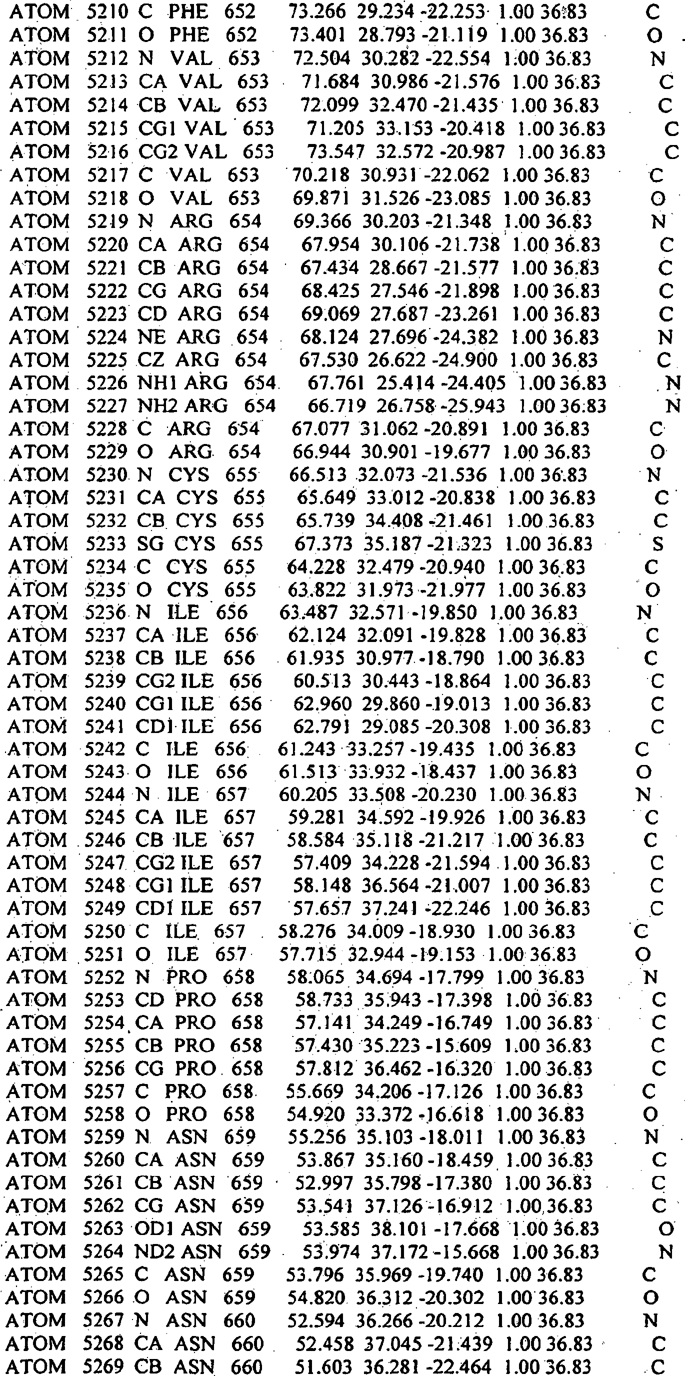

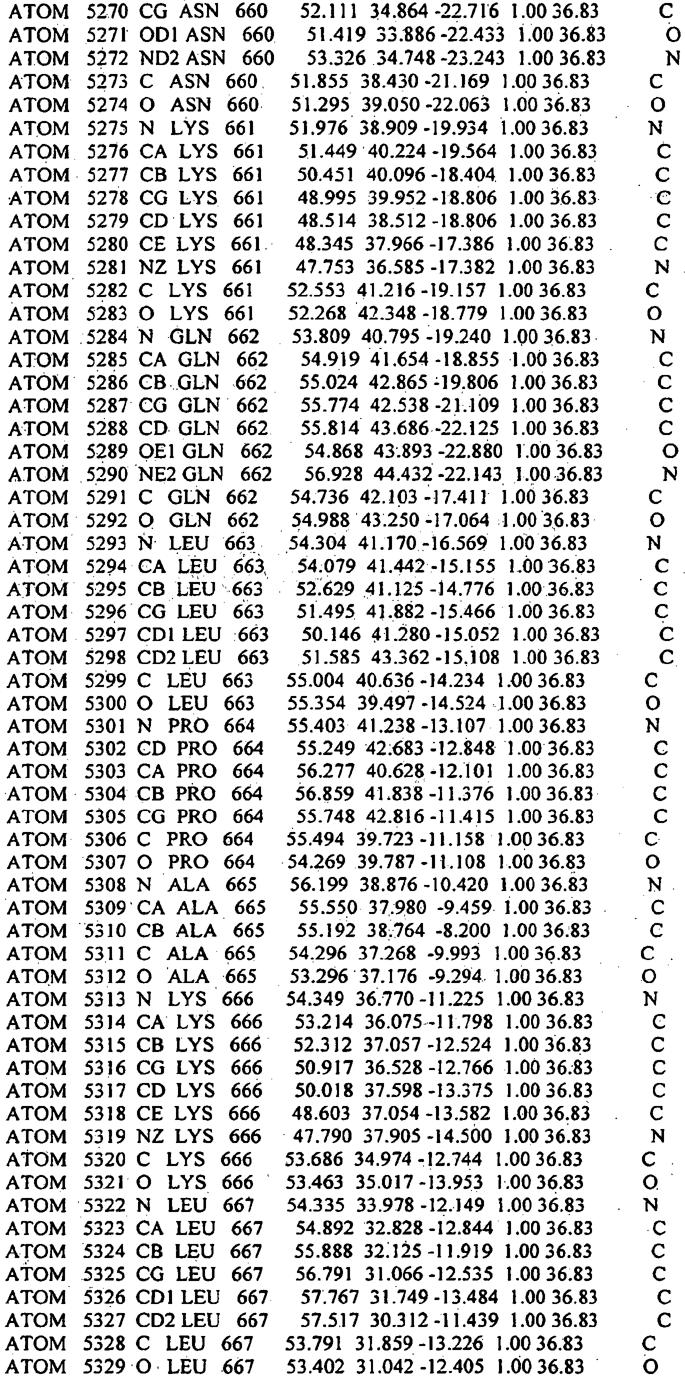

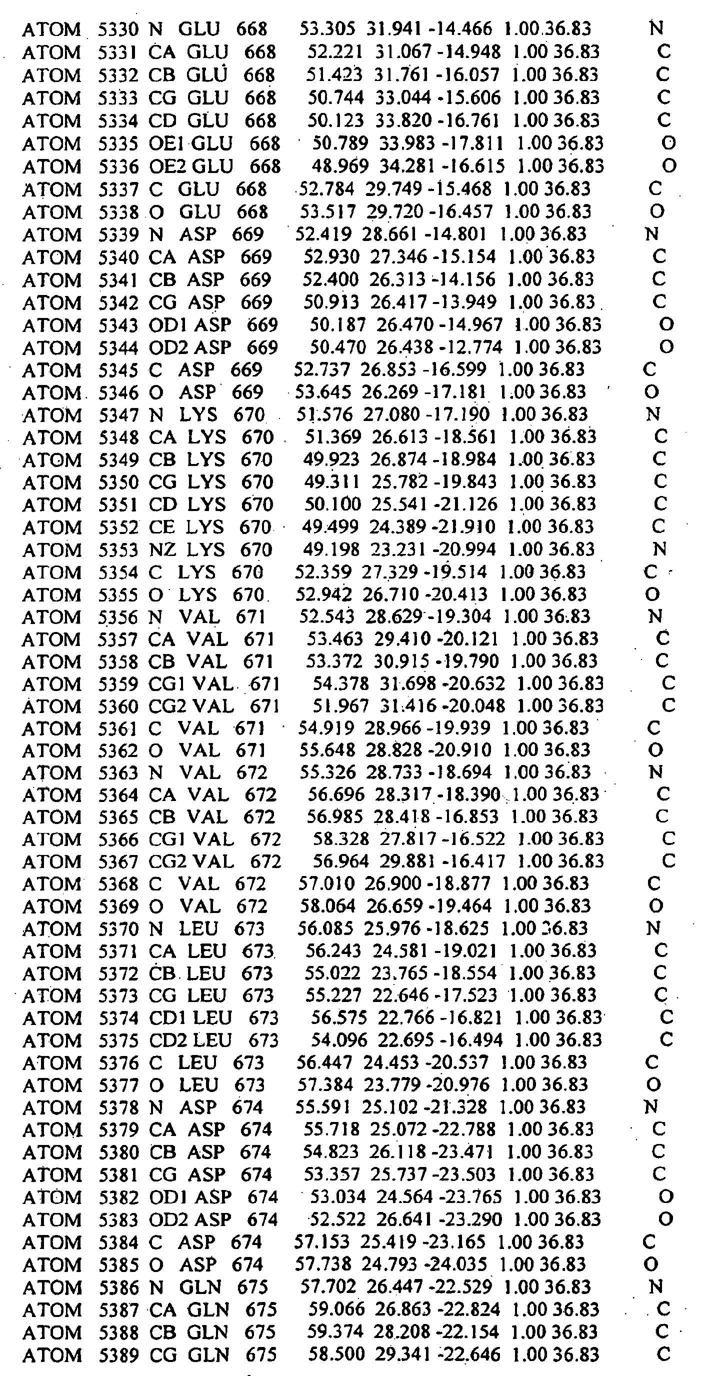

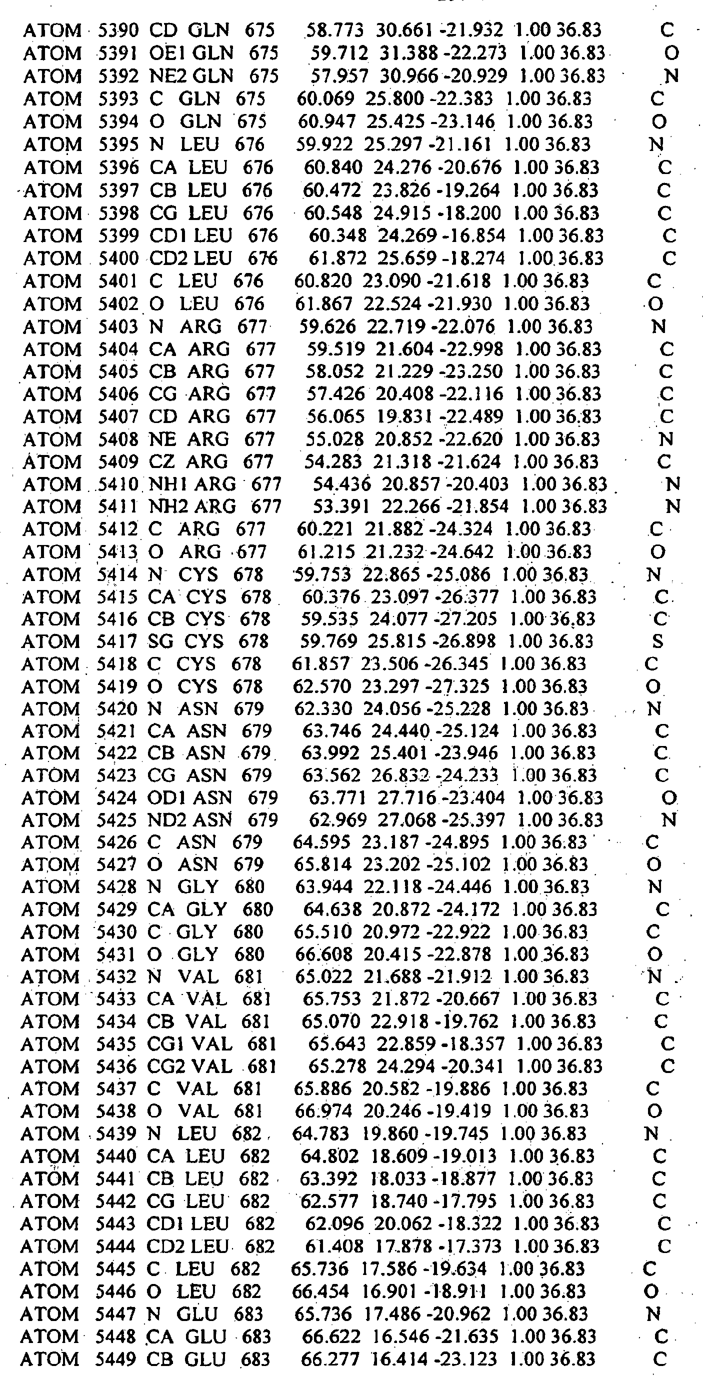

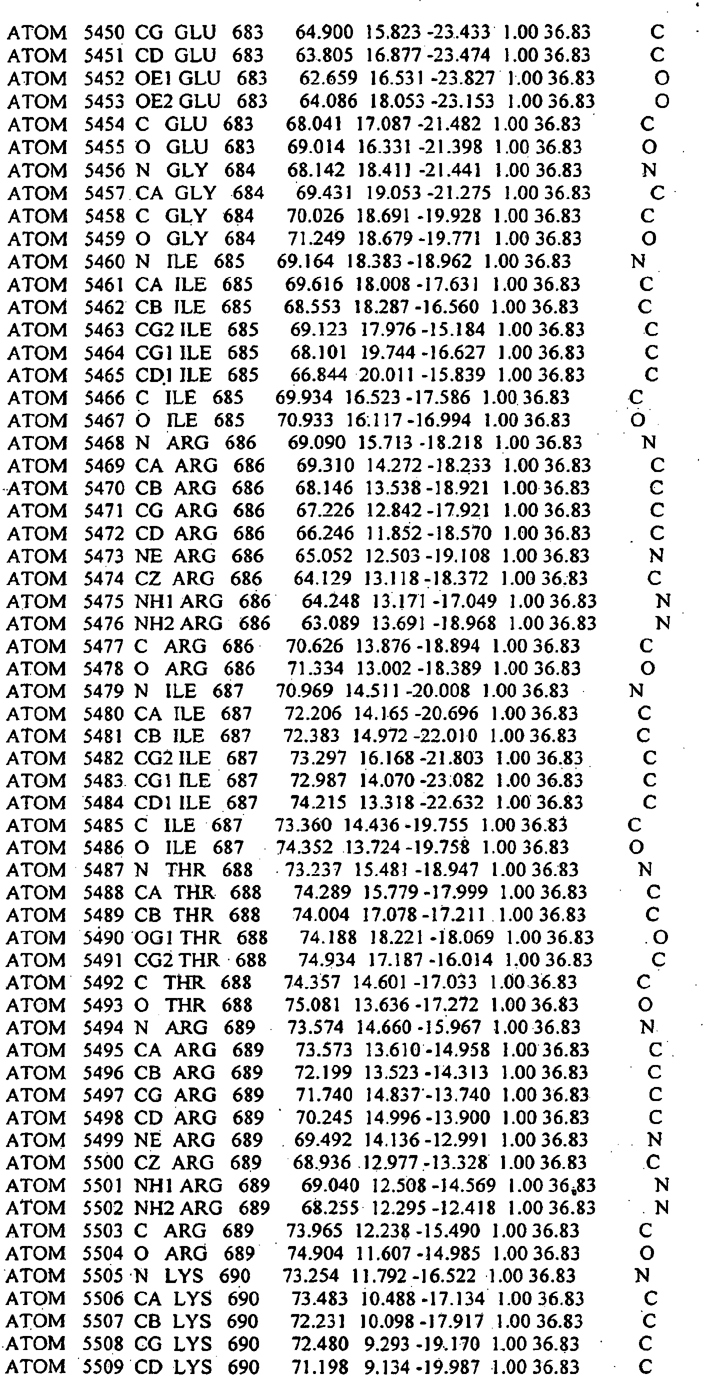

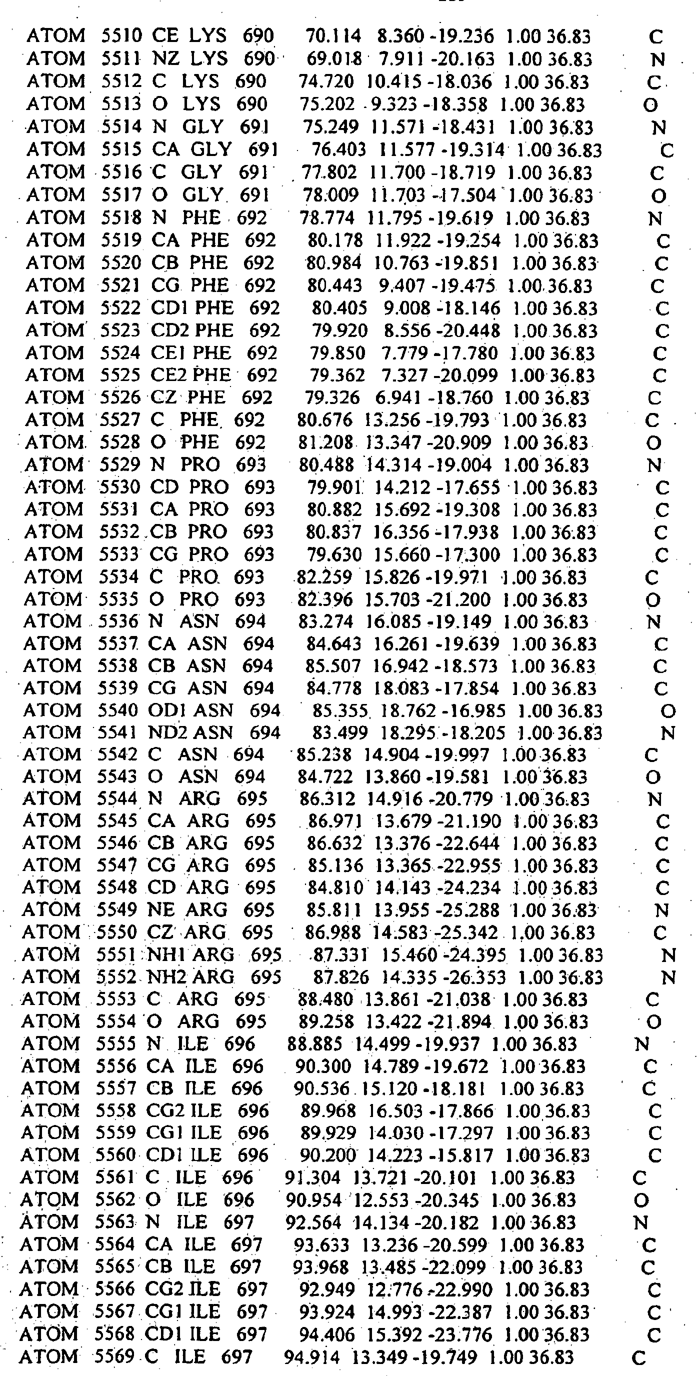

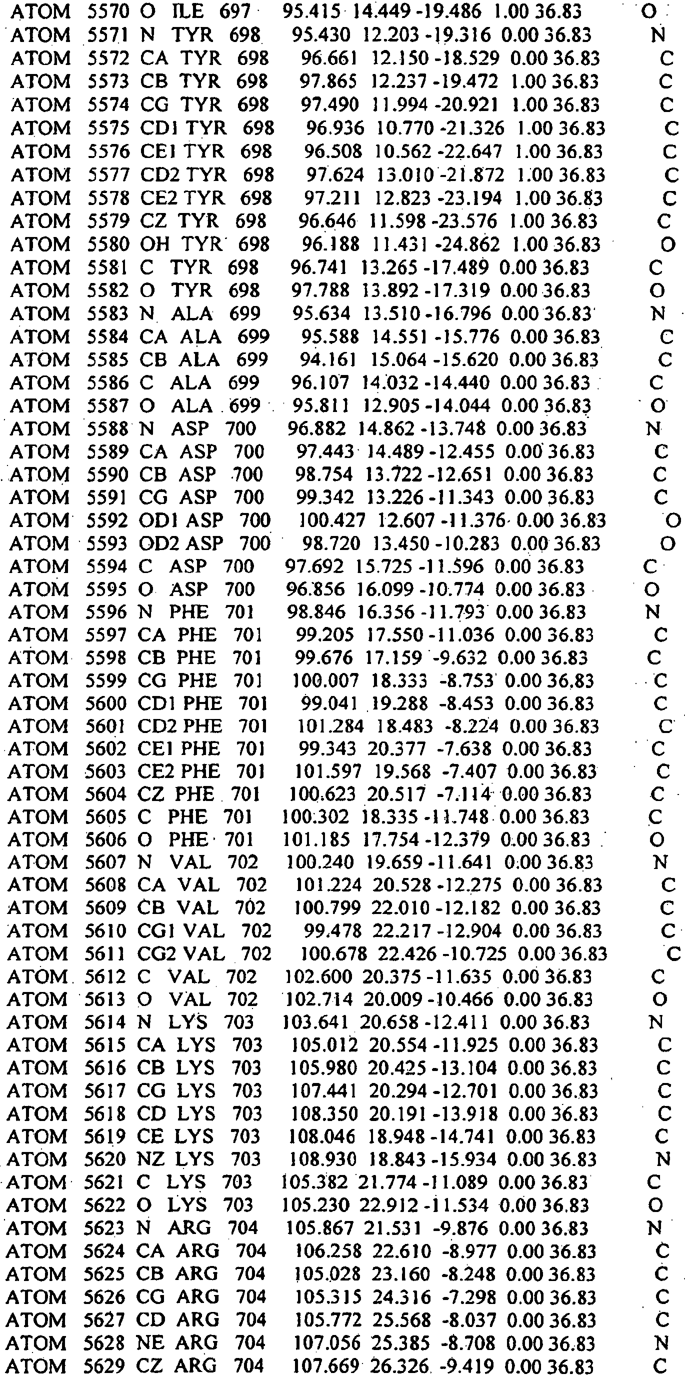

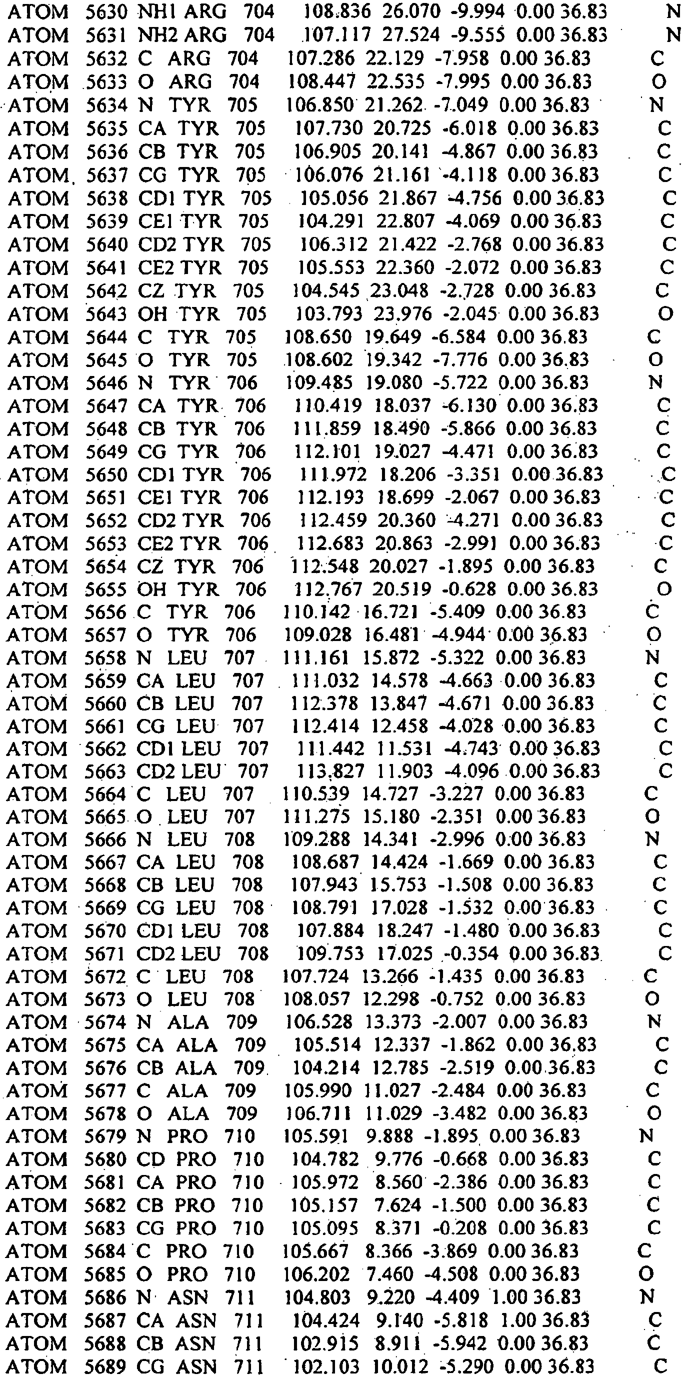

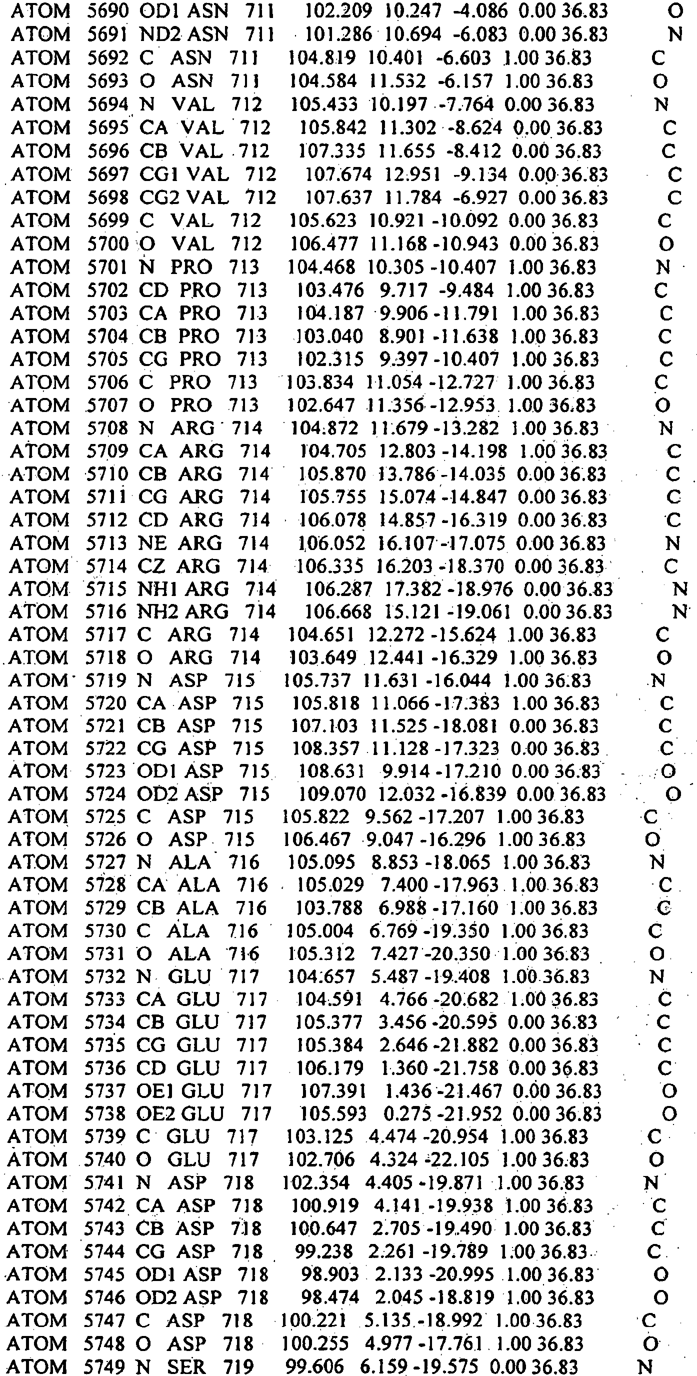

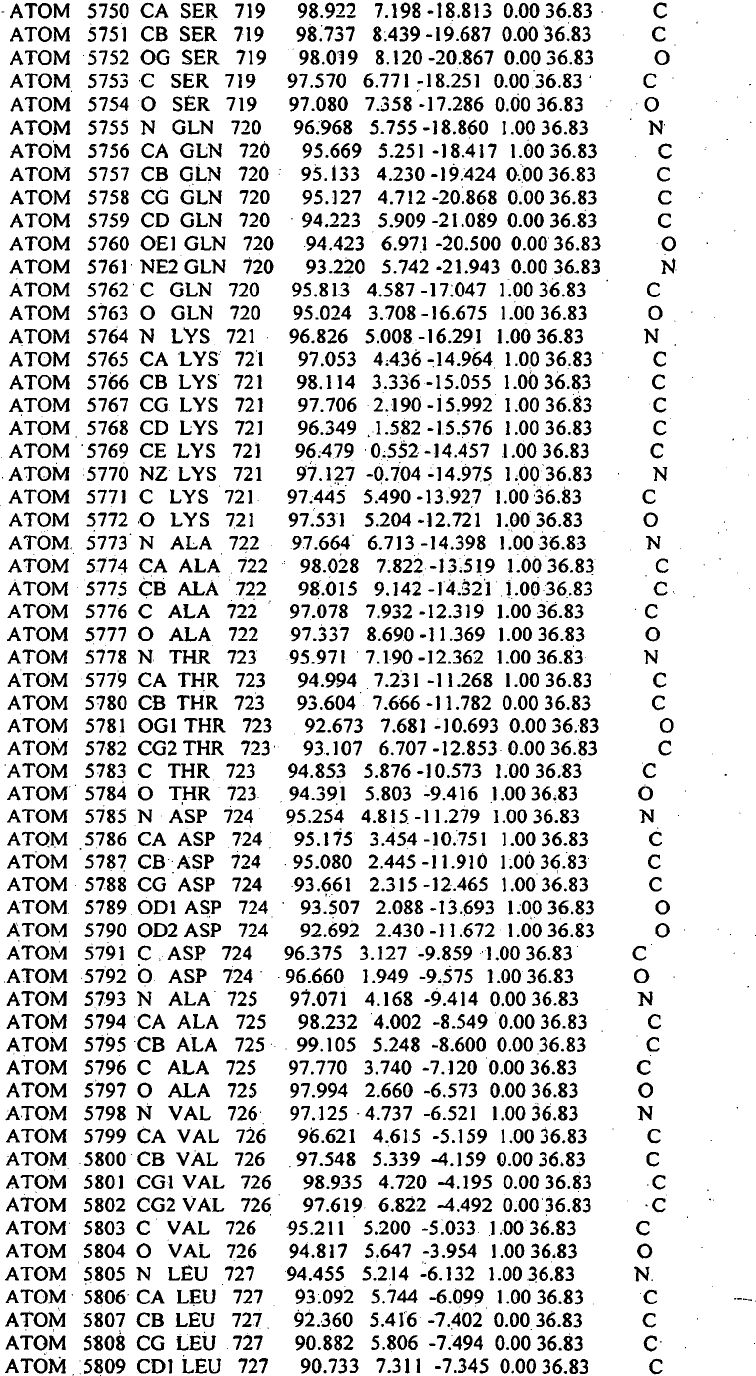

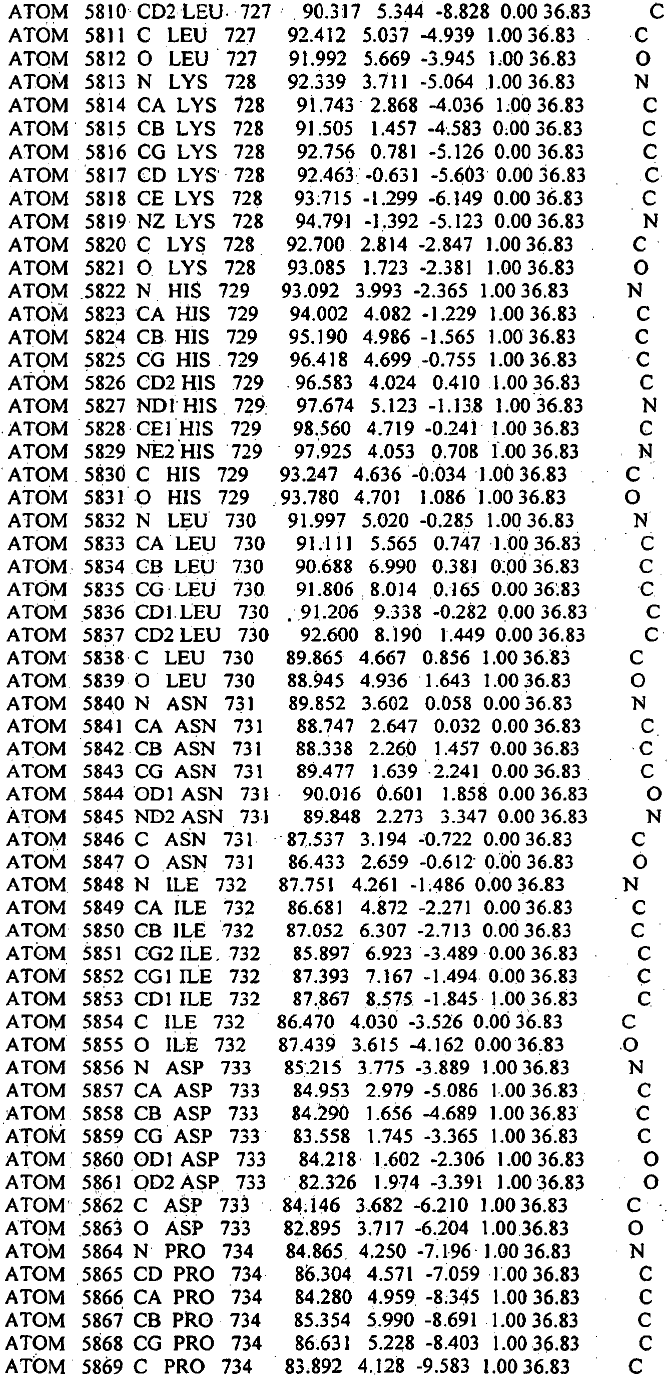

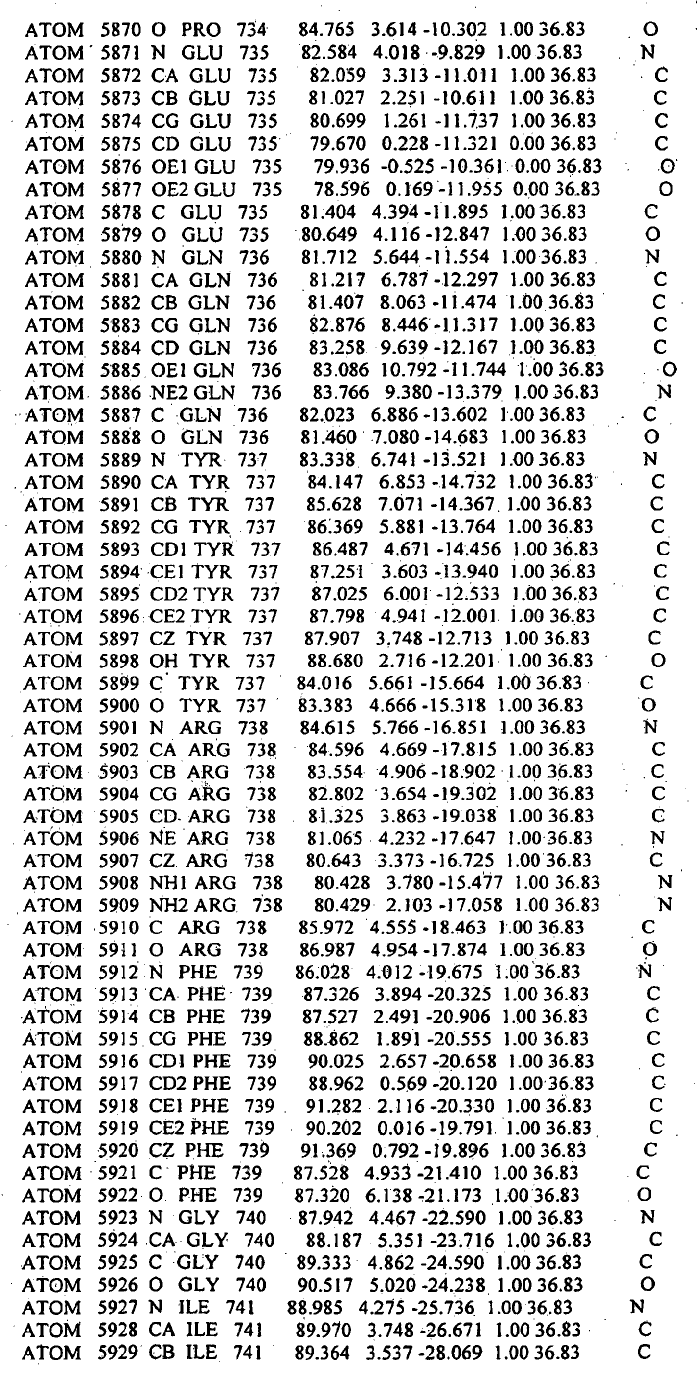

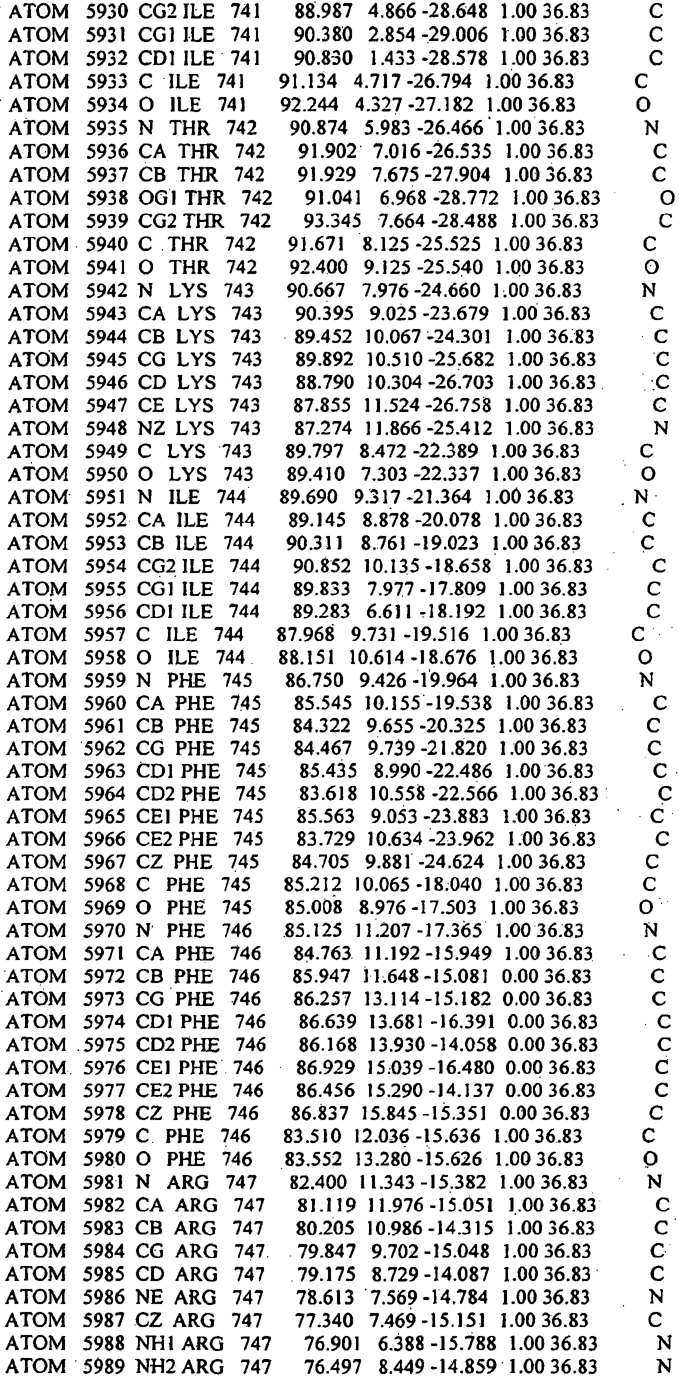

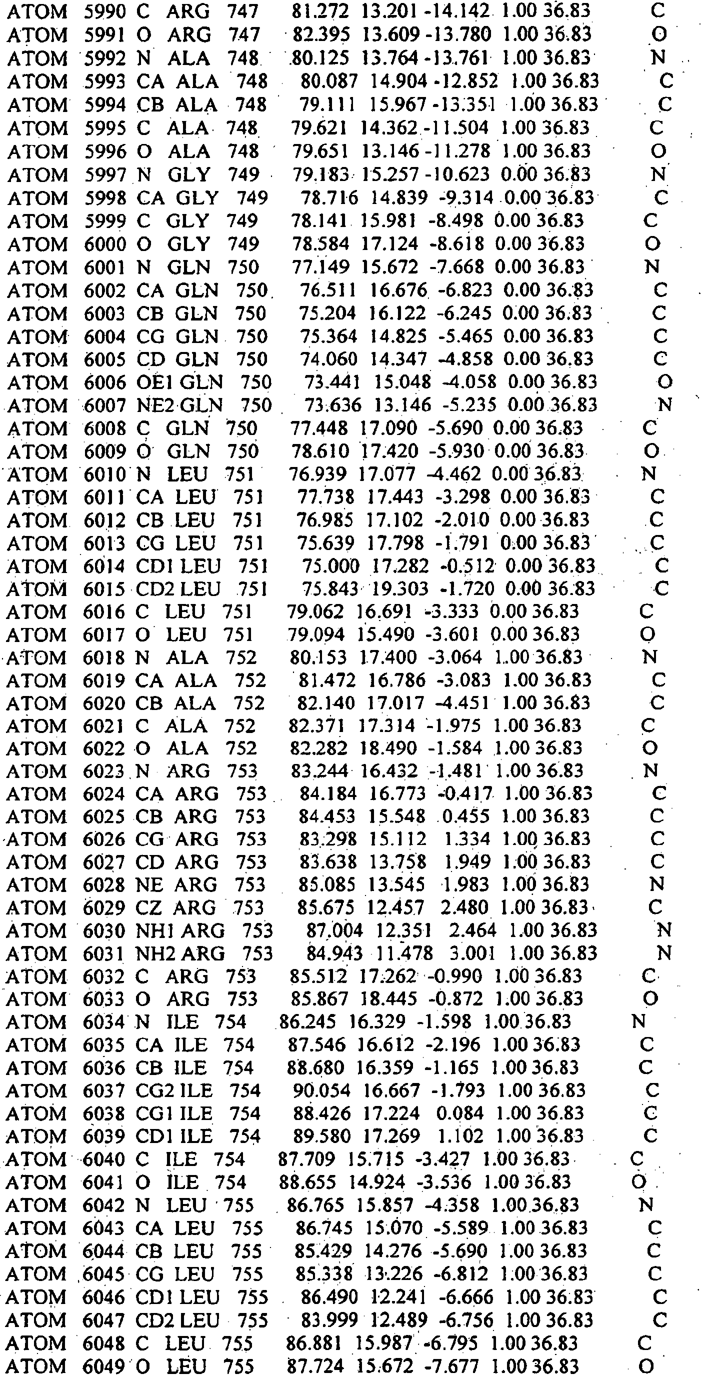

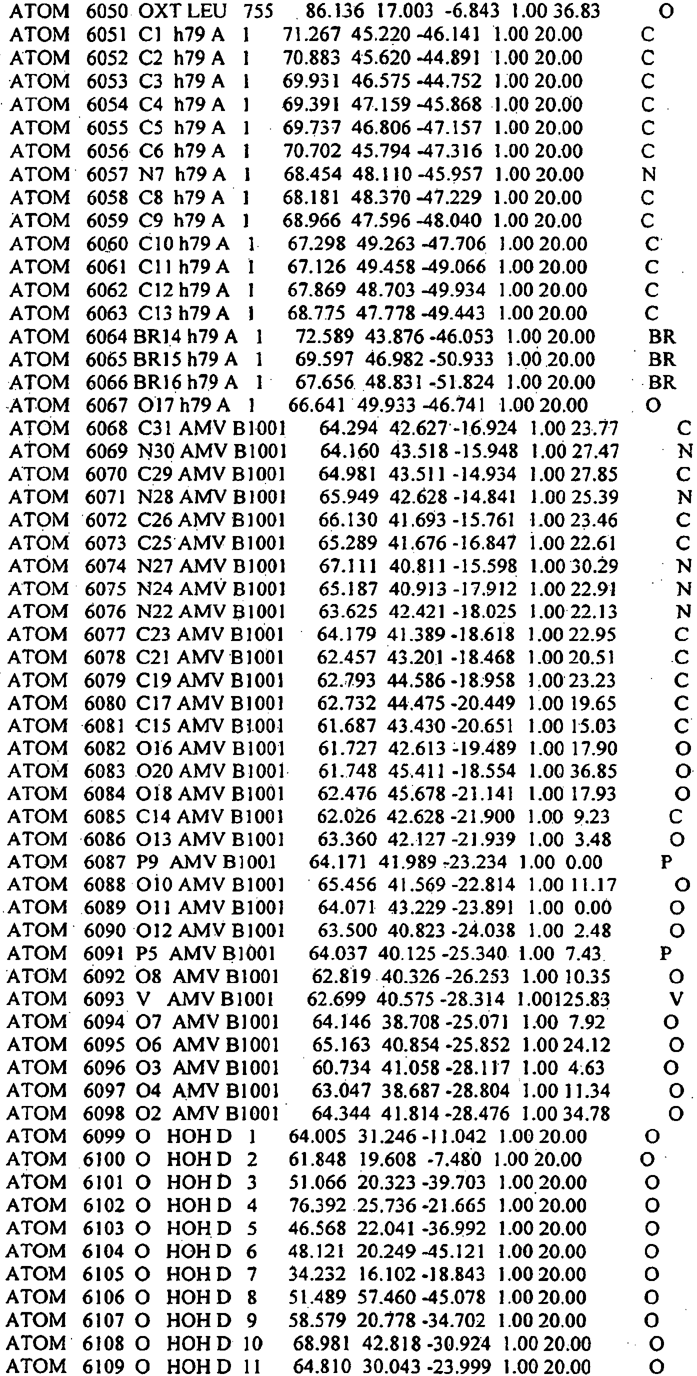

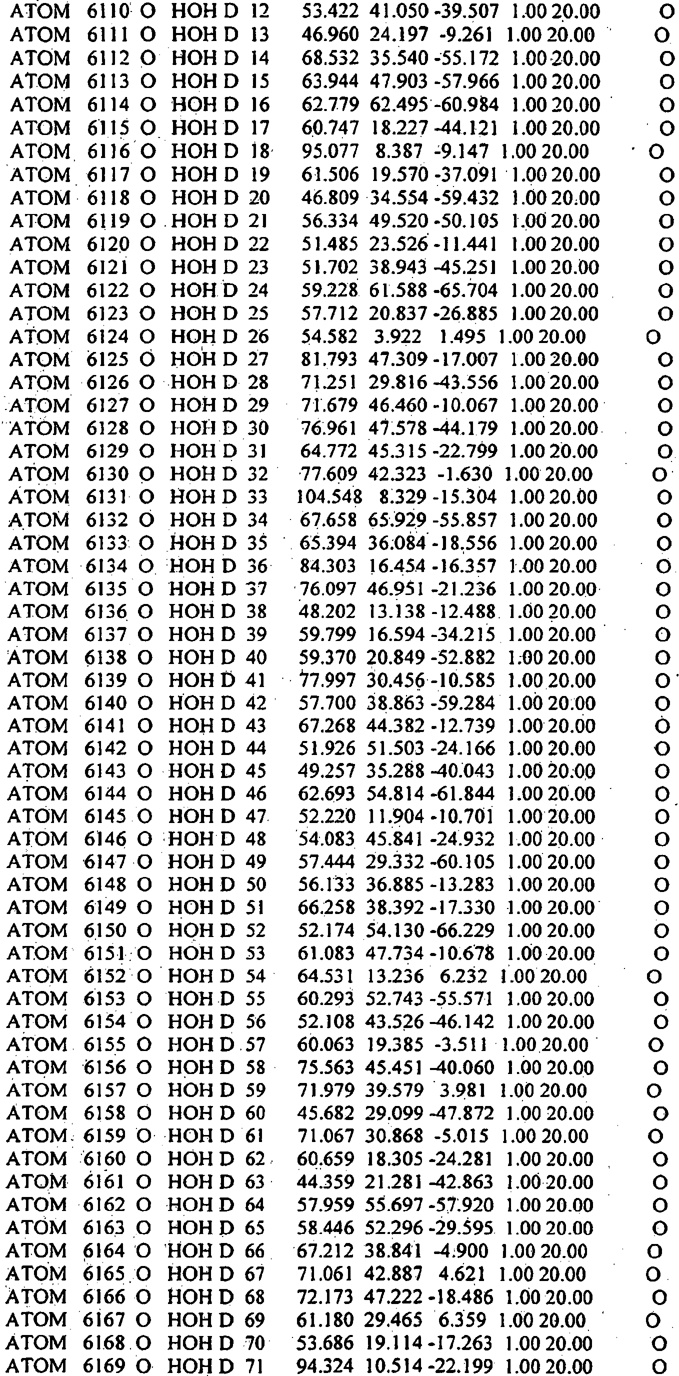

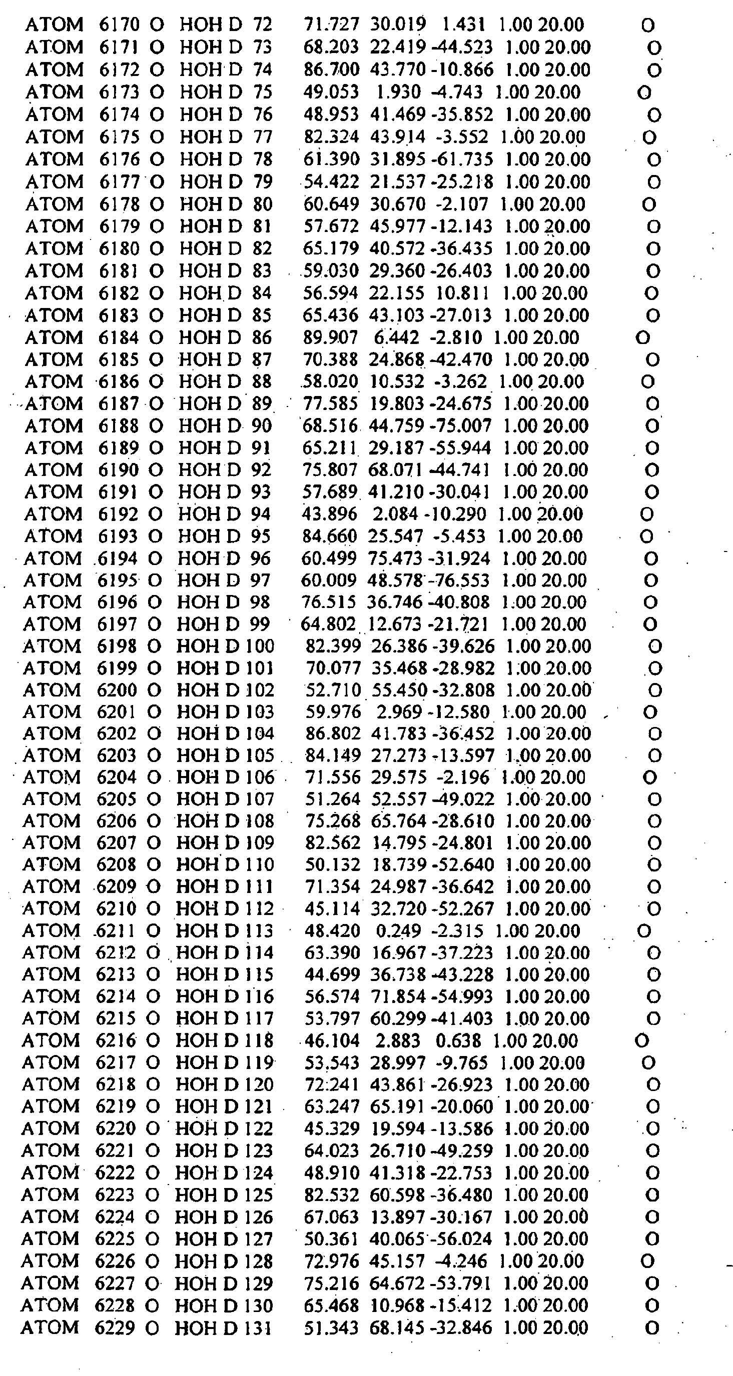

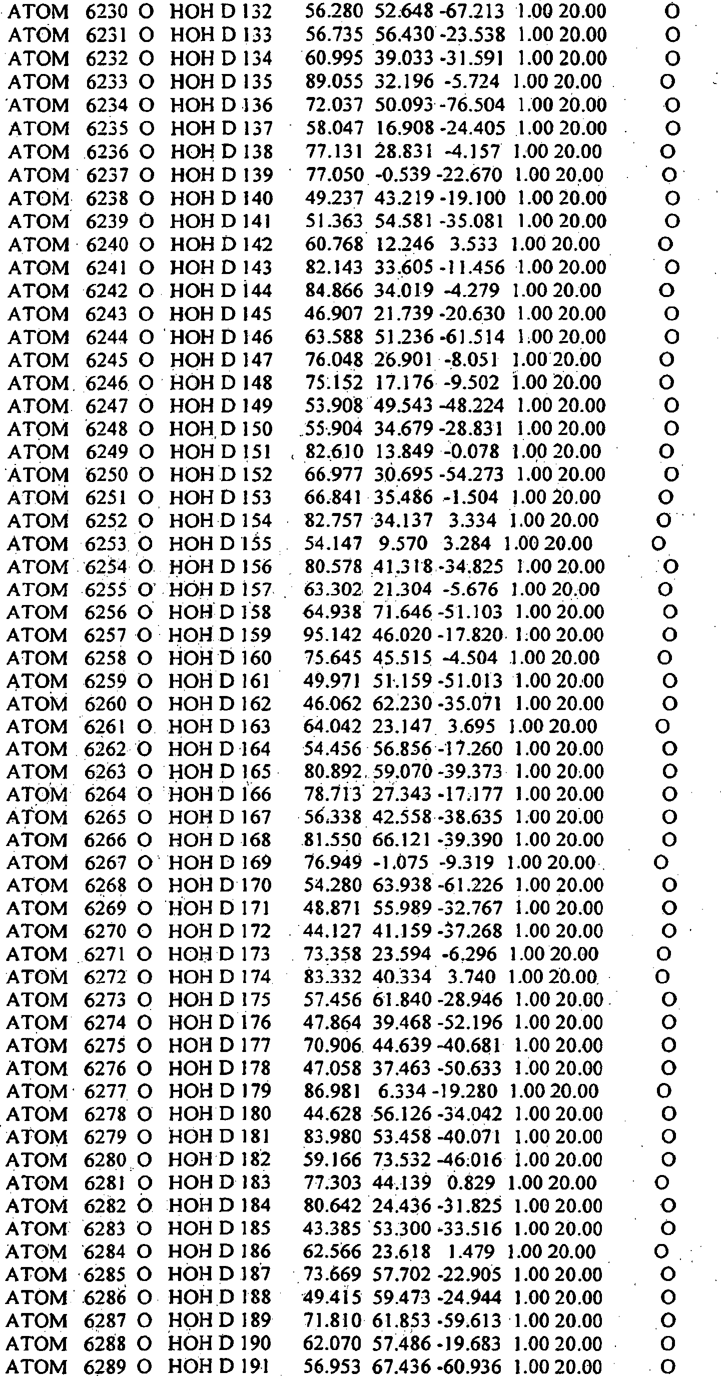

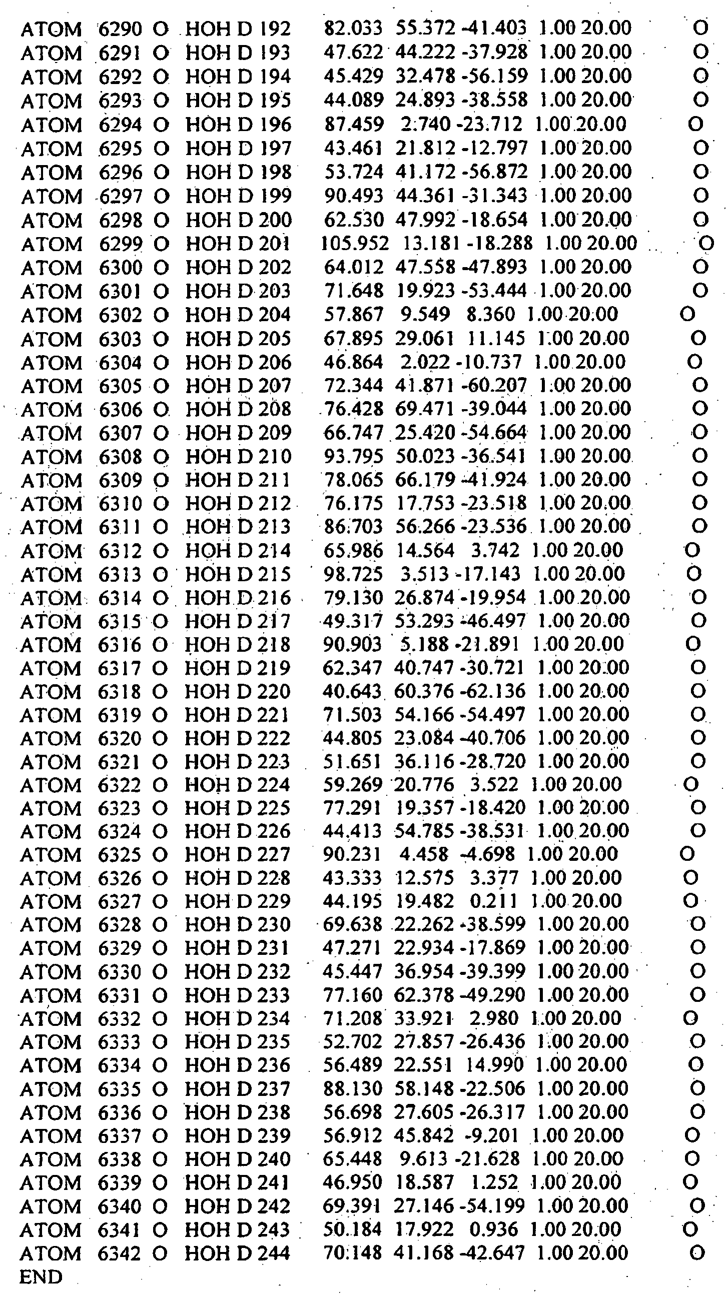

### Further References

Rey M, Valenzuela-Fernandez A, Urzainqui A, Yanez-Mo M, Perez-Martinez M, Penela P, Mayor F, Jr., Sanchez-Madrid F. Myosin IIA is involved in the endocytosis of CXCR4 induced by SDF-1alpha. J Cell Sci 120:1126-1133 (2007)
Minamiya, Y., Nakagawa. T., Saito, H.: loncreased expression of myosin light chain kinase mRNA is related to metastasis in non-small cell lung cancer. Tumour Biol 26: 153-157 (2005)
Lehmann, M.J., Sherer, N.M., Marks, C.B., Pypaert, M., Mothes, W.: Actin-and myosin-driven movement of viruses along filopodia precedes their entry into cells. J Cell Biol 170: 317-325 (2005)
Sun, X., Whittaker, G.R.: Role of the actin cytoskeleton during influenza virus internalization into polarized epithelial cells. Cell Microbiol 9:1672-1682 (2007)
Kawakami,S.; Watanabe,Y.; Beachy,R.N. Tobacco mosaic virus infection spreads cell to cell as intact replication complexes PNAS 101: 6291-6296 (2004)
Soldati, 0., Foth, B.J., Cowman, AF.: Molecular and functional aspects of parasite invasion. Trends in Parasitology 20: 567-574 (2004)
Desnos C, Huet S, Darchen F. "'Should I stay or go?":myosin V function in organelle trafficking. Biol Cell 99:411-23 (2007)
Sasaki H, Nakamura M, Ohno T, Matsuda Y, Yuda Y, Nonomura Y: Myosin-actin interaction plays an important role in human immunodeficiency virus type 1 release from host cells. Proc Natl Acad Sci U S A. 92:2026-30 (1995)
Desnos C, Huet S, Darchen F.: Should I stay or should I go?': myosin V function in organelle trafficking. Biol Cell. 2007 Aug;99(8):411-23.
Kim MT, Kim BJ, Lee JH, Kwon SC, Yeon DS, Yang DK, So I, Kim KW. Involvement of calmodulin and myosin light chain kinase in activation of mTRPC5 expressed in HEK cells. Am J Physiol Cell Physiol. 2006 Apr;290(4):C1031-40. Epub 2005 Nov 23.
Andzelm, M. M., X. Chen, et al. (2007). "Myosin IIA is required for cytolytic granule exocytosis in human NK cells." J Exp Med 204(10): 2285-91.
Bement, W. M., T. Hasson, et al. (1994). "Identification and overlapping expression of multiple unconventional myosin genes in vertebrate cell types." Proc Natl Acad Sci U S A 91(14): 6549-53.
Bement, W. M., J. A. Wirth, et al. (1994). "Cloning and mRNA expression of human unconventional myosin-IC. A homologue of amoeboid myosins-I with a single IQ motif and an SH3 domain." J Mol Biol 243(2): 356-63.
Berg, J. S., B. C. Powell, et al. (2001). "A millennial myosin census." Mol Biol Cell 12(4): 780-94.
Bohil, A. B., B. W. Robertson, et al. (2006). "Myosin-X is a molecular motor that functions in filopodia formation." Proc Natl Acad Sci U S A 103(33): 12411-6.
Crozet, F., A. el Amraoui, et al. (1997). "Cloning of the genes encoding two murine and human cochlear unconventional type I myosins." Genomics 40(2): 332-41.
Delbac, F., A. Sanger, et al. (2001). "Toxoplasma gondii myosins B/C: one gene, two tails, two localizations, and a role in parasite division." J Cell Biol 155(4): 613-23.
Desjardins, P. R., J. M. Burkman, et al. (2002). "Evolutionary implications of three novel members of the human sarcomeric myosin heavy chain gene family." Mol Biol Evol 19(4): 375-93.
Desnos, C., S. Huet, et al. (2007). "Myosin va mediates docking of secretory granules at the plasma membrane." J Neurosci 27(39): 10636-45.
Donaudy, F., R. Snoeckx, et al. (2004). "Nonmuscle myosin heavy-chain gene MYH14 is expressed in cochlea and mutated in patients affected by autosomal dominant hearing impairment (DFNA4)." Am J Hum Genet 74(4): 770-6.
Dose, A. C. and B. Burnside (2000). "Cloning and chromosomal localization of a human class III myosin." Genomics 67(3): 333-42.
Dose, A. C. and B. Burnside (2002). "A class III myosin expressed in the retina is a potential candidate for Bardet-Biedl syndrome." Genomics 79(5): 621-4.
Dunham, I., N. Shimizu, et al. (1999). "The DNA sequence of human chromosome 22." Nature 402(6761): 489-95.
Dunn, T. A., S. Chen, et al. (2006). "A novel role of myosin VI in human prostate cancer." Am J Pathol 169(5): 1843-54.
Edakuni, N., K. Ikuta, et al. (2006). "Restored expression of the MYO18B gene suppresses orthotopic growth and the production of bloody pleural effusion by human malignant pleural mesothelioma cells in SCID mice." Oncol Res 16(5): 235-43.
Eller, M., H. H. Stedman, et al. (1989). "Nucleotide sequence of full length human embryonic myosin heavy chain cDNA." Nucleic Acids Res 17(9): 3591
Engle, L. J. and R. H. Kennett (1994). "Cloning, analysis, and chromosomal localization of myoxin (MYH12), the human homologue to the mouse dilute gene." Genomics 19(3): 407-16.
Foth, B. J., M. C. Goedecke, et al. (2006). "New insights into myosin evolution and classification." Proc Natl Acad Sci U S A 103(10): 3681-6.
Furusawa, T., S. Ikawa, et al. (2000). "Isolation of a novel PDZ-containing myosin from hematopoietic supportive bone marrow stromal cell lines." Biochem Biophys Res Commun 270(1): 67-75.
Hasson, T., J. F. Skowron, et al. (1996). "Mapping of unconventional myosins in mouse and human." Genomics 36(3): 431-9.
Heintzelman, M. B. and J. D. Schwartzman (1997). "A novel class of unconventional myosins from Toxoplasma gondii." J Mol Biol 271(1): 139-46.
Hofmann, W. A., T. Johnson, et al. (2006). "From transcription to transport: emerging roles for nuclear myosin I." Biochem Cell Biol 84(4): 418-26.
Jaijo, T., E. Aller, et al. (2007). "MYO7A mutation screening in Usher syndrome type I patients from diverse origins." J Med Genet 44(3): e71.
Krendel, M., E. K. Osterweil, et al. (2007). "Myosin 1E interacts with synaptojanin-1 and dynamin and is involved in endocytosis." FEBS Lett 581(4): 644-50.
Lapierre, L. A., R. Kumar, et al. (2001). "Myosin vb is associated with plasma membrane recycling systems." Mol Biol Cell 12(6): 1843-57.
Lee, Y. R. and B. Liu (2004). "Cytoskeletal motors in Arabidopsis. Sixty-one kinesins and seventeen myosins." Plant Physiol 136(4): 3877-83.
Leinwand, L. A., L. Saez, et al. (1983). "Isolation and characterization of human myosin heavy chain genes." Proc Natl Acad Sci U S A 80(12): 3716-20.
Liang, Y., A. Wang, et al. (1999). "Characterization of the human and mouse unconventional myosin XV genes responsible for hereditary deafness DFNB3 and shaker 2." Genomics 61(3): 243-58.
Matsuoka, R., M. C. Yoshida, et al. (1993). "Human smooth muscle myosin heavy chain gene mapped to chromosomal region 16q12." Am J Med Genet 46(1): 61-7.
Mori, K., T. Furusawa, et al. (2003). "Genome structure and differential expression of two isoforms of a novel PDZ-containing myosin (MysPDZ) (Myo18A)." J Biochem (Tokyo) 133(4): 405-13.
Pegoraro, E., B. F. Gavassini, et al. (2007). "MYH7 gene mutation in myosin storage myopathy and scapulo-peroneal myopathy." Neuromuscul Disord 17(4): 321-9.
Rodriguez, O. C. and R. E. Cheney (2002). "Human myosin-Vc is a novel class V myosin expressed in epithelial cells." J Cell Sci 115(Pt 5): 991-1004.
Ruppert, C., R. Kroschewski, et al. (1993). "Identification, characterization and cloning of myr 1, a mammalian myosin-I." J Cell Biol 120(6): 1393-403.
Salas-Cortes, L., F. Ye, et al. (2005). "Myosin Ib modulates the morphology and the protein transport within multi-vesicular sorting endosomes." J Cell Sci 118(Pt 20): 4823-32.
Sanchez, E., B. Z. Alizadeh, et al. (2007). "MYO9B gene polymorphisms are associated with autoimmune diseases in Spanish population." Hum Immunol 68(7): 610-5.
Schachat, F. and M. M. Briggs (2002). "Phylogenetic implications of the superfast myosin in extraocular muscles." J Exp Biol 205(Pt 15): 2189-201.
Scherer, S. W., J. Cheung, et al. (2003). "Human chromosome 7: DNA sequence and biology." Science 300(5620): 767-72.
Shimmen, T. (2007). "The sliding theory of cytoplasmic streaming: fifty years of progress." J Plant Res 120(1): 31-43.
Simons, M., M. Wang, et al. (1991). "Human nonmuscle myosin heavy chains are encoded by two genes located on different chromosomes." Circ Res 69(2): 530-9.
Solomon, S. D., A. A. Geisterfer-Lowrance, et al. (1990). "A locus for familial hypertrophic cardiomyopathy is closely linked to the cardiac myosin heavy chain genes, CRI-L436, and CRI-L329 on chromosome 14 at q11-q12. "Am J Hum Genet 47(3): 389-94.
Strausberg, R. L., E. A. Feingold, et al. (2002). "Generation and initial analysis of more than 15,000 full-length human and mouse cDNA sequences." Proc Natl Acad Sci U S A 99(26): 16899-903.
Venter, J. C., M. D. Adams, et al. (2001). "The sequence of the human genome." Science 291(5507): 1304-51.
Volkmann, D., T. Mori, et al. (2003). "Unconventional myosins of the plant-specific class VIII: endocytosis, cytokinesis, plasmodesmata/pit-fields, and cell-to-cell coupling." Cell Biol Int 27(3): 289-91.
Walsh, T., V. Walsh, et al. (2002). "From flies' eyes to our ears: mutations in a human class III myosin cause progressive nonsyndromic hearing loss DFNB30." Proc Natl Acad Sci U S A 99(11): 7518-23.
Winters, L. M., M. M. Briggs, et al. (1998). "The human extraocular muscle myosin heavy chain gene (MYH13) maps to the cluster of fast and developmental myosin genes on chromosome 17." Genomics 54(1): 188-9.
Yang, C. H., J. Szeliga, et al. (2005). "Identification of the surfactant protein A receptor 210 as the unconventional myosin 18A." J Biol Chem 280(41): 34447-57.
Zhang, L., J. D'Costa, et al. (2006). "Identification of a region on the outer surface of the CBFbeta-SMMHC myeloid oncoprotein assembly competence domain critical for multimerization." Oncogene 25(55): 7289-96.

### References cited in Example 1

1. S. Agarwal, S. Cammerer, S. Filali, W. Fröhner, J. Knöll, M. P. Krahl, K. R. Reddy, H.-J. Knölker, Curr. Org. Chem. 2005, 9, 1601.
2. S. Agarwal, H.-J. Knölker, Org. Biomol. Chem. 2004, 2, 3060.
3. a) V. M. Dembitsky, Russ. J. Bioorg. Chem. 2002, 28, 170. b) G. W. Gribble in The Handbook of Environmental Chemistry; Springer-Verlag, Berlin, 2003; Vol. 3, p. 1.
4. a) H.-J. Knölker, K. R. Reddy, *Chem. Rev.* **2002,** *102,* 4303. b) H.-J. Knölker, *Top. Curr. Chem.* **2005,** *244*, 115.

### References cited in Example 2

1. Burkholder, P. R., Pfister, R. M. & Leitz, F. H. Production of a pyrrole antibiotic by a marine bacterium. Appl Microbiol 14, 649-53 (1966).
2. Laatsch, H. et al. Structure-activity relationships of phenyl- and benzoylpyrroles. Chem Pharm Bull (Tokyo) 43, 537-46 (1995).
3. Ohri, R. V. et al. A Re(V)-catalyzed C-N bond-forming route to human lipoxygenase inhibitors. Org Lett 7, 2501-4 (2005).
4. Boldogh, I. R., Ramcharan, S. L., Yang, H. C. & Pon, L. A. A type V myosin (Myo2p) and a Rab-like G-protein (Ypt11p) are required for retention of newly inherited mitochondria in yeast cells during cell division. Mol Biol Cell 15, 3994-4002 (2004).
5. Itoh, T., Toh, E. A. & Matsui, Y. Mmr1p is a mitochondrial factor for Myo2p-dependent inheritance of mitochondria in the budding yeast. Embo J 23, 2520-30 (2004).
6. Itoh, T., Watabe, A., Toh, E. A. & Matsui, Y. Complex formation with Ypt11 p, a rab-type small GTPase, is essential to facilitate the function of Myo2p, a class V myosin, in mitochondrial distribution in Saccharomyces cerevisiae. Mol Cell Biol 22, 7744-57.(2002).
7. Westermann, B. & Neupert, W. Mitochondria-targeted green fluorescent proteins: convenient tools for the study of organelle biogenesis in Saccharomyces cerevisiae. Yeast 16, 1421-7 (2000).
8. Mnaimneh, S. et al. Exploration of essential gene functions via titratable promoter alleles. Cell 118, 31-44 (2004).
9. Kron, S. J. & Spudich, J. A. Fluorescent actin filaments move on myosin fixed to a glass surface. Proc Natl Acad Sci U S A 83, 6272-6 (1986).
10. Durrwang, U. et al. Dictyostelium myosin-IE is a fast molecular motor involved in phagocytosis. J Cell Sci 119, 550-8 (2006).
11. Woodward, S. K., Geeves, M. A. & Manstein, D. J. Kinetic characterization of the catalytic domain of Dictyostelium discoideum myosin. Biochemistry 34, 16056-64 (1995).
12. Manstein, D. J. & Hunt, D. M. Overexpression of myosin motor domains in Dictyostelium: screening of transformants and purification of the affinity tagged protein. J Muscle Res Cell Motil 16, 325-32 (1995).
13. Lehrer, S. S. & Kerwar, G. Intrinsic fluorescence of actin. Biochemistry 11, 1211-7 (1972).
14. Fedorov, R., Witte, G., Urbanke, C., Manstein, D. J, & Curth, U. 3D structure of Thermus aquaticus single-stranded DNA-binding protein gives insight into the functioning of SSB proteins. Nucleic Acids Res 34, 6708-17 (2006).
15. Manstein, D. J. & Hunt, D. M. Overexpression of myosin motor domains in Dictyostelium: screening of transformants and purification of the affinity tagged protein. J. Mus. Res. Cell Motil. 16, 325-332 (1995).
16. Agarwal, S. & Knolker, H. J. A novel pyrrole synthesis. Org Biomol Chem 2, 3060-2 (2004).
17. Brunger, A. T. et al. Crystallography & NMR system: A new software suite for macromolecular structure determination Acta Crystallographica Section D-Biological Crystallography 54, 905-21 (1998).
18. Schmidt, M. W. et al. The General Atomic and Molecular Electronic Structure System. J. Comput. Chem. 14, 1347-1363 (1993).
19. Furch, M., Geeves, M. A. & Manstein, D. J. Modulation of actin affinity and actomyosin adenosine triphosphatase by charge changes in the myosin motor domain. Biochemistry 37, 6317-26 (1998).
20. Uyeda, T. Q., Kron, S. J. & Spudich, J. A. Myosin step size. Estimation from slow sliding movement of actin over low densities of heavy meromyosin. J Mol Biol 214, 699-710 (1990).
21. Peschke, J. D., Hanefeld, U. & Laatsch, H. Biosynthesis of the marine antibiotic pentabromopseudilin. 2. The pyrrole ring. Biosci Biotechnol Biochem 69, 628-30 (2005).2
22. Lehrer, S. S., and Kerwar, G. (1972)Intrinsic fluorescence of actin, Biochemistry 11, 1211-1217.
23. Kron, S. J. & Spudich, J. A. Fluorescent actin filaments move on myosin fixed to a glass surface. Proc. Natl. Acad. Sci. USA 83, 6272-6 (1986).

## Claims

1. A method of designing an inhibitor of a myosin, the method comprising in silico molecular modeling of a compound such that the modeled compound interacts by means of one or more of charge-charge interactions, charge-dipole interactions, dipole-dipole interactions, hydrogen bonds and hydrophobic interactions with at least three amino acid residues of said myosin, said residues being selected from
(a) positions K265, A420, K423, A424, R428, L431, D590, I617, and A618 of SEQ ID NO: 2, wherein said residues comprise K265 and said myosin comprises or consists of
(i) the sequence of SEQ ID NO: 2;
(ii) the sequence encoded by the sequence of SEQ ID NO: 1; or
(iii) a sequence being at least 40% identical to the sequence of SEQ ID NO: 2 or to the sequence encoded by the sequence of SEQ ID NO: 1;
wherein said sequence of (iii) comprises said at least three amino acid residues; or
(b) positions of any one of SEQ ID NOs: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 or 108, respectively, said positions aligning with the positions of SEQ ID NO: 2 as defined in (a), wherein the alignment of said positions occurs over the entire length of the respective ranges of (a) and (b), wherein said residues comprise the residue aligning with K265 of SEQ ID NO: 2 and said myosin comprises or consists of
(i) the sequence of any one of SEQ ID NO: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 or 108, respectively;
(ii) the sequence encoded by the sequence of any one of SEQ ID NO: 3, 5, 7, 9, 11, 13, 15, 17, 19, 21, 23, 25, 27, 29, 31, 33, 35, 37, 39, 41, 43, 45, 47, 9, 51, 53, 55, 57, 59, 61, 63, 65, 67, 69, 71, 73, 75, 77, 79, 81, 83, 85, 87, 89, 91, 93, 95, 97, 99, 101, 103, 105, 107 or 109, respectively; or
(iii) a sequence being at least 40% identical to the sequence of any one of SEQ ID NO: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 or 108, respectively, or to the sequence encoded by the sequence of any one of SEQ ID NO: 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 or 109, respectively;
wherein said sequence of (iii) comprises said at least three amino acid residues;
thereby obtaining said inhibitor of a myosin.

2. The method of claim 1, further comprising synthesizing said inhibitor, thereby producing said inhibitor.

3. The method of claim 1 or 2, wherein said molecular modeling comprises
(i) measuring at least one intermolecular distance; and/or
(ii) calculating at least one free energy of interaction.

4. A method of identifying an inhibitor of a myosin, the method comprising
(a) bringing into contact a myosin and a test compound;
(b) determining whether said test compound interacts by means of one or more of charge-charge interactions, charge-dipole interactions, dipole-dipole interactions, hydrogen bonds and hydrophobic interactions with at least three amino acid residues selected from
(ba) positions K265, A420, K423, A424, R428, L431, D590, I617, and A618 of SEQ ID NO: 2, wherein said residues comprise K265 and said myosin comprises or consists of
(i) the sequence of SEQ ID NO: 2;
(ii) the sequence encoded by the sequence of SEQ ID NO: 1;
or
(iii) a sequence being at least 40% identical to the sequence of SEQ ID NO: 2 or to the sequence encoded by the sequence of SEQ ID NO: 1;
wherein said sequence of (iii) comprises said at least three amino acid residues; or
(bb) positions of any one of SEQ ID NOs: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 or 108, respectively, said positions aligning with the positions of SEQ ID NO: 2 as defined in (a), wherein the alignment of said ranges occurs over the entire length of the respective positions of (a) and (b), wherein said residues comprise the residue aligning with K265 of SEQ ID NO: 2 and said myosin comprises or consists of
(i) the sequence of any one of SEQ ID NO: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 or 108, respectively;
(ii) the sequence encoded by the sequence of any one of SEQ ID NO: 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 or 109, respectively; or
(iii) a sequence being at least 40% identical to the sequence of any one of SEQ ID NO: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78,80, 82, 84, 86, 88, 90, 92, 94, 96, 98, 100, 102, 104, 106 or 108, respectively, or to the sequence encoded by the sequence of any one of SEQ ID NO: 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 or 109, respectively;
wherein said sequence of (iii) comprises said at least three amino acid residues; wherein said determining is effected by X-ray crystallography and/or NMR spectroscopy;
and
(c) identifying those compounds which interact with said at least three amino acid residues,
thereby identifying said inhibitor of a myosin.

5. The method of claim 4, further comprising the step of
(a')
(i) determining whether said test compound binds to said myosin; and/or
(ii) determining whether said test compound decreases the activity and/or modulates the conformation of said myosin; and/or
(iii) determining the cytotoxicity of said test compound;
wherein step (a') is to be effected after step (a) and prior to step (b), and wherein said determining in step (b) is performed with test compounds determined to bind, to decrease, to modulate, and/or to be cytotoxic in step (a').

6. The method of claim 5, wherein said activity is the capability of said myosin
(i) to bind actin;
(ii) to convert ATP into ADP and Pᵢ; and/or
(iii) to generate force and/or movement.

7. The method of any one of the preceding claims, wherein molecular modeling according to any one of claims 1 to 3 starts from a compound selected from compounds of the general formulae (1) to (4) or a salt or solvate thereof, or 4 to 6 the test compound of any one of claims 4 to 6 is selected from compounds of the general formulae (1) to (4) or a salt or solvate thereof 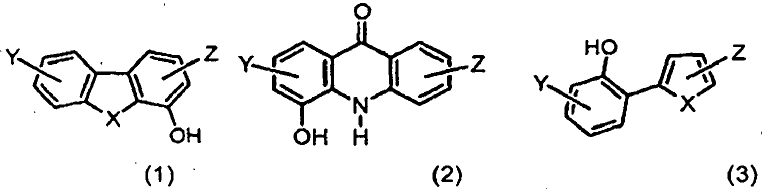 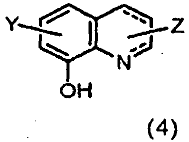 wherein
X is selected from NH, O and S; and
Y and Z designate, as valence permits, one or more substituents, wherein each occurrence of Y and Z is independently selected from F, Cl, Br, I, R and OR;
R being selected from (i) H, (ii) (CO)CH₃, and (iii) linear or branched alkyl, alkenyl or alkinyl with one to four carbon atoms, the moieties (ii) and (iii) being optionally substituted with one or more F, Cl, Br and/or I.

8. The method of claim 7, wherein said general formulae are the general formulae (1) or (2).

## Patentansprüche

1. Verfahren zum Entwerfen eines Inhibitors eines Myosins, wobei das Verfahren in silico molekulares Modellieren einer Verbindung umfasst, so dass die modellierte Verbindung mit mindestens drei Aminosäureresten des Myosins wechselwirkt und zwar mittels einer oder mehrerer Wechselwirkungen ausgewählt aus Ladungs-Ladungs-Wechselwirkungen, Ladungs-Dipol-Wechselwirkungen, Dipol-Dipol-Wechselwirkungen, Wasserstoffbindungen und hydrophoben Wechselwirkungen, wobei die Reste ausgewählt sind aus
(a) Positionen K265, A420, K423, A424, R428, L431, D590, 1617, und A618 der SEQ ID Nr: 2, wobei diese Reste K265 umfassen und das Myosin aus folgender Sequenz besteht oder diese umfasst
(i) der Sequenz der SEQ ID Nr: 2;
(ii) der Sequenz, die von der Sequenz der SEQ ID Nr: 1 kodiert wird; oder
(iii) einer Sequenz, die mindestens zu 40% mit der Sequenz der SEQ ID Nr: 2 oder mit der Sequenz, die von der Sequenz der SEQ ID Nr: 1 kodiert wird, identisch ist;
wobei die Sequenz gemäß (iii) die mindestens drei genannten Aminosäurereste umfasst; oder
(b) Positionen einer der SEQ ID Nrn: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 oder 108, wobei die Positionen sich an den in (a) definierten Positionen der SEQ ID Nr: 2 ausrichten ("align"), wobei die Ausrichtung der Positionen über die gesamte Länge der jeweiligen Bereiche gemäß (a) und (b) auftritt, wobei die Reste jenen Rest umfassen, der sich an K265 der SEQ ID Nr. 2 ausrichtet, und das Myosin aus den folgenden Sequenzen besteht oder diese umfasst
(i) der Sequenz einer der SEQ ID Nrn: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 oder 108;
(ii) der Sequenz, die von der Sequenz einer der SEQ ID Nrn: 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 oder 109, kodiert wird; oder
(iii) einer Sequenz, die mindestens zu 40% mit der Sequenz einer der SEQ ID Nrn: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 oder 108, oder mit der Sequenz, die von der Sequenz einer der SEQ ID Nrn: 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 oder 109 kodiert wird, identisch ist;
wobei die Sequenz gemäß (iii) die mindestens drei genannten Aminosäurereste umfasst;
wodurch ein Inhibitor eines Myosins erhalten wird.

2. Verfahren nach Anspruch 1, ferner umfassend das Synthetisieren des Inhibitors, wodurch der Inhibitor erzeugt wird.

3. Verfahren nach Anspruch 1 oder 2, wobei das molekulare Modellieren
(i) Messen mindestens einer intermolekularen Distanz, und/oder
(ii) Berechnen mindestens einer freien Energie einer Wechselwirkung,
umfasst.

4. Verfahren zum Identifizieren eines Inhibitors eines Myosins, wobei das Verfahren umfasst
(a) Inkontaktbringen eines Myosins mit einer Testverbindung;
(b) Feststellen, ob die Testverbindung mit mindestens drei Aminosäureresten mittels einer oder mehrerer Wechselwirkungen interagiert, wobei die Wechselwirkungen ausgewählt sind aus Ladungs-Ladungs-Wechselwirkungen, Ladungs-Dipol-Wechselwirkungen, Dipol-Dipol-Wechselwirkungen, Wasserstoffbrücken und hydrophoben Wechselwirkungen, und wobei die mindestens drei Aminosäurereste ausgewählt sind aus
(ba) Positionen K265, A420, K423, A424, R428, L431, D590, I617, und A618 der SEQ ID Nr: 2, wobei diese Reste K265 umfassen und das Myosin aus folgender Sequenz besteht oder diese umfasst
(i) der Sequenz der SEQ ID Nr: 2;
(ii) der Sequenz, die von der Sequenz der SEQ ID Nr: 1 kodiert wird; oder
(iii) einer Sequenz, die mindestens zu 40% mit der Sequenz der SEQ ID Nr: 2 oder mit der Sequenz, die von der Sequenz der SEQ ID Nr: 1 kodiert wird, identisch ist;
wobei die Sequenz gemäß (iii) die mindestens drei genannten Aminosäurereste umfasst; oder
(bb) Positionen nach einer der SEQ ID Nrn: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 oder 108, wobei die Positionen sich an den in (a) definierten Positionen der SEQ ID Nr: 2 ausrichten ("align"), wobei die Ausrichtung der Positionen über die gesamte Länge der jeweiligen Bereiche gemäß (a) und (b) auftritt, wobei die Reste jenen Rest umfassen, der sich an K265 der SEQ ID Nr. 2 ausrichtet, und das Myosin aus den folgenden Sequenzen besteht oder diese umfasst
(i) der Sequenz einer der SEQ ID Nrn: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 oder 108;
(ii) der Sequenz, die von der Sequenz einer der SEQ ID Nrn: 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 oder 109, kodiert wird; oder
(iii) einer Sequenz, die mindestens zu 40% mit der Sequenz einer der SEQ ID Nrn: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 oder 108, oder mit der Sequenz, die von der Sequenz einer der SEQ ID Nrn: 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 oder 109 kodiert wird, identisch ist;
wobei die Sequenz gemäß (iii) die mindestens drei genannten Aminosäurereste umfasst; wobei das Bestimmen mittels Röntgenstrukturanalyse und/oder Kernresonanzspektroskopie erfolgt;
und
(c) Identifizieren jener Verbindungen, die mit mindestens drei Aminosäureresten interagieren,
wodurch ein Inhibitor eines Myosins identifiziert wird.

5. Verfahren nach Anspruch 4, ferner umfassend den Schritt
(a')
(i) Feststellen, ob die Testverbindung an das Myosin bindet; und/oder
(ii) Feststellen, ob die Testverbindung die Aktivität des Myosins erniedrigt und/oder seine Konformation moduliert; und/oder
(iii) Feststellen der Zytotoxizität der Testverbindung;
wobei Schritt (a') nach Schritt (a) und vor Schritt (b) auszuführen ist, und wobei das Bestimmen in Schritt (b) mit jenen Testverbindungen durchgeführt wird, für die in Schritt (a') festgestellt wurde, dass sie binden, erniedrigen, modulieren und/oder zytotoxisch sind.

6. Verfahren nach Anspruch 5, wobei die Aktivität die Fähigkeit des Myosins
(i) Aktin zu binden;
(ii) ATP in ADP und Pᵢ umzuwandeln; und/oder
(iii) Kraft und/oder Bewegung zu erzeugen
ist.

7. Verfahren nach einem der vorangegangenen Ansprüche, wobei das molekulare Modellieren nach einem der Ansprüche 1 bis 3 von einer Verbindung ausgeht, die ausgewählt ist aus den Verbindungen mit den allgemeinen Formeln (1) bis (4) oder einem Salz oder Solvat davon, oder die Testverbindung nach einem der Ansprüche 4 bis 6 ausgewählt ist aus Verbindungen der allgemeinen Formeln (1) bis (4) oder einem Salz oder Solvat davon 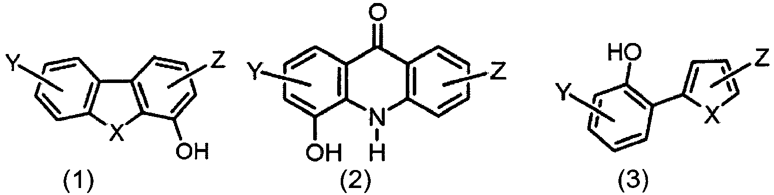 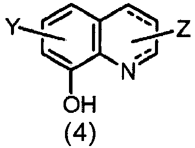 wobei
X ausgewählt ist aus NH, O und S; und
Y und Z, insoweit die Valenz dies erlaubt, einen oder mehrere Substituenten darstellen, wobei jedes Vorkommen von Y und Z unabhängig ausgewählt ist aus F, Cl, Br, I, R und OR;
R ausgewählt ist aus (i) H, (ii) (CO)CH₃, und (iii) linearem oder verzweigtem Alkyl, Alkenyl oder Akinyl mit einem bis vier Kohlenstoffatomen, wobei die Gruppen (ii) und (iii) gegebenenfalls mit einem oder mehreren F, Cl, Br und/oder I substituiert sind.

8. Verfahren nach Anspruch 7, wobei die allgemeinen Formeln die allgemeinen Formeln (1) oder (2) sind.

## Revendications

1. Méthode de conception d'un inhibiteur d'une myosine, la méthode comprenant la modélisation moléculaire *in silico* d'un composé tel que le composé modélisé interagit au moyen d'une ou de plusieurs interactions charge-charge, interactions charge-dipôle, interactions dipôle-dipôle, liaisons hydrogène et interactions hydrophobes avec au moins trois résidus d'acides aminés de ladite myosine, lesdits résidus étant choisis parmi
(a) les positions K265, A420, K423, A424, R428, L431, D590, 1617, et A618 de SEQ ID NO : 2, où lesdits résidus comprennent K265 et ladite myosine comprend ou est constituée de
(i) la séquence de SEQ ID NO : 2 ;
(ii) la séquence codée par la séquence de SEQ ID NO : 1 ; ou
(iii) une séquence au moins identique à 40 % avec la séquence de SEQ ID NO : 2 ou avec la séquence codée par la séquence de SEQ ID NO : 1 ;
dans laquelle ladite séquence de (iii) comprend lesdits au moins trois résidus d'acides aminés ; ou
(b) les positions de l'une quelconque de SEQ ID NO : 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 ou 108, respectivement, lesdites positions s'alignant avec les positions de SEQ ID NO : 2 telles que définies en (a), où l'alignement desdites positions survient sur la longueur entière des plages respectives de (a) et (b), où lesdits résidus comprennent le résidu s'alignant avec K265 de SEQ ID NO : 2 et ladite myosine comprend ou est constituée de
(i) la séquence de l'une quelconque de SEQ ID NO : 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 ou 108, respectivement ;
(ii) la séquence codée par la séquence de l'une quelconque de SEQ I D NO : 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 or 109, respectivement ; ou
(iii) une séquence au moins identique à 40 % avec la séquence de l'une quelconque de SEQ ID NO : 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 ou 108, respectivement, ou avec la séquence codée par la séquence de l'une quelconque de SEQ ID NO : 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 or 109, respectivement ;
dans laquelle ladite séquence de (iii) comprend lesdits au moins trois résidus d'acides aminés ;
obtenant de cette façon ledit inhibiteur d'une myosine.

2. Méthode selon la revendication 1, comprenant en outre la synthèse dudit inhibiteur, produisant de cette façon ledit inhibiteur.

3. Méthode selon la revendication 1 ou 2, dans laquelle ladite modélisation moléculaire comprend
(i) la mesure d'au moins une distance intermoléculaire ; et/ou
(ii) le calcul d'au moins une énergie libre d'interaction.

4. Méthode d'identification d'un inhibiteur d'une myosine, la méthode comprenant
(a) la mise en contact d'une myosine et d'un composé à tester ;
(b) la détermination si ledit composé à tester interagit au moyen d'une ou de plusieurs interactions charge-charge, interactions charge-dipôle, interactions dipôle-dipôle, liaisons hydrogène et interactions hydrophobes avec au moins trois résidus d'acides aminés choisis parmi
(ba) les positions K265, A420, K423, A424, R428, L431, D590, 1617, et A618 de SEQ ID NO : 2, où lesdits résidus comprennent K265 et ladite myosine comprend ou est constituée de
(i) la séquence de SEQ ID NO : 2 ;
(ii) la séquence codée par la séquence de SEQ ID NO : 1 ; ou
(iii) une séquence au moins identique à 40 % avec la séquence de SEQ ID NO : 2 ou avec la séquence codée par la séquence de SEQ ID NO : 1 ;
dans laquelle ladite séquence de (iii) comprend lesdits au moins trois résidus d'acides aminés ; ou
(bb) les positions de l'une quelconque de SEQ ID NO : 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 ou 108, respectivement, lesdites positions s'alignant avec les positions de SEQ ID NO : 2 telles que définies en (a), où l'alignement desdites plages survient sur la longueur entière des positions respectives de (a) et (b), où lesdits résidus comprennent le résidu s'alignant avec K265 de SEQ ID NO : 2 et ladite myosine comprend ou est constituée de
(i) la séquence de l'une quelconque de SEQ ID NO : 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 ou 108, respectivement ;
(ii) la séquence codée par la séquence de l'une quelconque de SEQ ID NO : 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 or 109, respectivement ; ou
(iii) une séquence au moins identique à 40 % avec les séquences de l'une quelconque de SEQ ID NO : 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106 ou 108, respectivement, ou avec la séquence codée par la séquence de l'une quelconque de SEQ ID NO : 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 or 109, respectivement ;
dans laquelle ladite séquence de (iii) comprend lesdits au moins trois résidus d'acides aminés ;
dans laquelle ladite détermination est effectuée par cristallographie aux rayons X et/ou spectroscopie RMN ;
et
(c) l'identification de ces composés qui interagissent avec lesdits au moins trois résidus d'acides aminés,
identifiant de cette façon ledit inhibiteur d'une myosine.

5. Méthode selon la revendication 4, comprenant en outre l'étape de
(a')
(i) détermination si ledit composé à tester se lie à ladite myosine ; et/ou
(ii) détermination si ledit composé à tester diminue l'activité et/ou module la conformation de ladite myosine ; et/ou
(iii) détermination de la cytotoxicité dudit composé à tester ;
dans laquelle l'étape (a') doit être effectuée après l'étape (a) et avant l'étape (b), et dans laquelle ladite détermination dans l'étape (b) est effectuée avec les composés à tester déterminés comme se liant, diminuant, modulant, et/ou étant cytotoxiques dans l'étape (a').

6. Méthode selon la revendication 5, dans laquelle ladite activité est la capacité de ladite myosine
(i) à lier l'actine ;
(ii) à convertir l'ATP en ADP et Pᵢ ; et/ou
(iii) à produire une force et/ou un mouvement.

7. Méthode selon l'une quelconque des revendications précédentes, dans laquelle la modélisation moléculaire selon l'une quelconque des revendications 1 à 3 débute à partir d'un composé choisi parmi des composés des formules générales (1) à (4) ou l'un de leurs sels ou solvates, ou le composé à tester selon l'une quelconque des revendications 4 à 6 est choisi parmi des composés des formules générales (1) à (4) ou l'un de leurs sels ou solvates 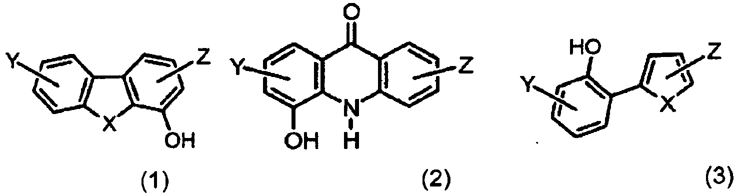 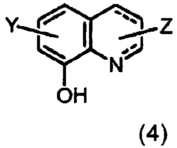 dans lesquelles
X est choisi parmi NH, O et S ; et
Y et Z désignent, lorsque la valence le permet, un ou plusieurs substituants, où chaque occurrence de Y et Z est choisie indépendamment parmi F, CI, Br, I, R et OR ;
R étant choisi parmi (i) H, (ii) (CO)CH₃, et (iii) un groupe linéaire ou ramifié alkyle, alcényle ou alcynyle comportant d'un à quatre atomes de carbone, les radicaux (ii) et (iii) étant éventuellement substitués par un ou plusieurs F, CI, Br et/ou I.

8. Méthode selon la revendication 7, dans laquelle lesdites formules générales sont les formules générales (1) ou (2).
